# EUROPEAN PATENT APPLICATION

(11) **EP 3 572 072 A1**
(43) Date of publication of application: **27.11.2019**
(21) Application number: 19151722.6
(22) Date of filing: 23.01.2012
(51) Int. Cl.: A61K 9/14, A61P 17/10

(54) **NANOPARTICLE COMPOSITIONS**

(30) Priority: 24.01.2011 US 201161435780 P
(62) Divisional of application: 12702700.1
(71) Applicant: Anterios, Inc., Madison, NJ 07940 (US)
(72) Inventor: EDELSON, Jonathan, Nashua NH 03062 (US); KOTYLA, Timothy, Natick MA 01760 (US); THEOBALD, Klaus, Paoli PA 19301 (US)
(74) Representative: Ruiz Antoli, Soledad Cecilia

(57) **Abstract**

The present invention describes novel nanoparticle compositions, and systems and methods utilizing them for treating disorders and/or conditions associated with the epidermal and/or dermal level of the skin. Such disorders include acne, hyperhidrosis, bromhidrosis chromhidrosis, rosacea, hair loss, dermal infection, actinic keratosis, facial wrinkles, muscle contracture, and headache. Methods generally involved administering nanoparticle compositions to the skin.

## Description

### Related Applications

This application claims priority to and benefit of U.S. provisional application serial number 61/435,780 filed January 24, 2011, the entire contents of which are incorporated herein by reference.

### Background

Nanoparticle compositions are useful in a variety of contexts. Nanoparticle compositions have proven to be particularly useful and/or effective in the context of medical applications, including administering therapeutic agents to patients in need thereof. Nanoparticle compositions have proven to be particularly useful and/or effective in the context of topical administration of therapeutic agents (see, *e.g*., PCT patent application number PCT US06/46236, filed December 1, 2006, published as WO 08/045107 on April 17, 2008, and entitled "BOTULINUM NANOEMULSIONS; in PCT patent application number PCT US07/86018, filed November 30, 2007, published as WO 08/070538 on June 12, 2008, and entitled "AMPHIPHILIC ENTITY NANOPARTICLES"; and/or in PCT patent application number PCT US09/48972, filed June 26, 2009, published as WO 09/158687 on December 30, 2009, and entitled "DERMAL DELIVERY"; the contents of all of which are incorporated herein by reference).

Conditions or disorders associated with skin (including the surface of the skin, sweat glands, sebaceous glands, *etc.*) can cause a great deal of unhappiness and psychological debilitation for those who suffer from them, and current treatments are not very successful and often have undesirable side effects. For example, according to studies, acne often leads to reduced self esteem, and sometimes even to depression or suicide (see, *e.g*., Goodman, 2006, Aust. Fam. Physician 35:503, 2006; Purvis et al., 2006, J. Paediatr. Child. Health 42:793; both of which are incorporated herein by reference). Similar challenges are observed with hyperhidrosis (excessive sweating), bromhidrosis (body odor), chromhidrosis (colored sweat), psoriasis, dermal infection (*e.g*., herpes simplex virus infection, human papillomavirus infection, fungal infection, *etc.*), hair loss, actinic keratosis, rosacea, facial wrinkles, neck lines, platysma bands, neuromuscular disorders and conditions involving muscular spasm and/or contracture, and other afflictions of the skin. Nanoparticle compositions have been described which may be useful and/or effective for topical administration for treatment of such disorders, but there is a need for improved nanoparticle compositions for topical administration for more effective treatment of disorders such as those associated with the skin.

### Summary of the Invention

The present invention provides particular compositions as described herein.

In some embodiments, the present invention encompasses the recognition that nanoparticle compositions (e.g., nanoemulsions) are useful as therapeutic agents. Thus, nanoparticle compositions containing a known therapeutic agent and/or independently active biologically active agent, and systems and methods relating thereto, are contemplated by the present invention. Empty nanoparticle compositions (*e.g*., nanoparticle compositions that do not contain any known therapeutic agent and/or independently active biologically active agent), and systems and methods relating thereto, are also contemplated by the present invention.

Nanoparticle compositions, such as those described in PCT patent application number PCT US06/46236, filed December 1, 2006, published as WO 08/045107 on April 17, 2008, and entitled "BOTULINUM NANOEMULSIONS; in PCT patent application number PCT US07/86018, filed November 30, 2007, published as WO 08/070538 on June 12, 2008, and entitled "AMPHIPHILIC ENTITY NANOPARTICLES"; and/or in PCT patent application number PCT US09/48972, filed June 26, 2009, published as WO 09/158687 on December 30, 2009, and entitled "DERMAL DELIVERY" (the contents of all of which are incorporated herein by reference) have been successfully used for topical administration of therapeutic agents. The present inventors have undertaken extensive studies of this class of composition, and, as described in the Examples, have made important and surprising findings that define certain embodiments and/or classes of such compositions as particularly and unexpectedly useful and/or advantageous.

In some embodiments, the present invention provides particular nanoparticle compositions as described herein. In some embodiments, nanoparticle compositions comprise an oil, and aqueous dispersion medium, a surfactant and, optionally, an independently biologically active agent and/or a known therapeutic agent.

In some embodiments, provided nanoparticle compositions comprise 1349 oil. In some embodiments, provided nanoparticle compositions comprise polysorbate 80. In some embodiments, provided nanoparticle compositions comprise propylparaben. In some embodiments, provided nanoparticle compositions comprise methylparaben. In some embodiments, provided nanoparticle compositions comprise isotonic sodium chloride solution. In some embodiments, provided nanoparticle compositions comprise purified water. In some embodiments, provided nanoparticle compositions comprise gelatin. In some embodiments, provided nanoparticle compositions comprise sodium phosphate dibasic. In some embodiments, provided nanoparticle compositions comprise concentrated hydrochloric acid. In some embodiments, provided nanoparticle compositions do not comprise any parabens. In some embodiments, provided nanoparticle compositions do not comprise methylparaben. In some embodiments, provided nanoparticle compositions do not comprise propylparaben.

In some embodiments, nanoparticle compositions comprise a nanoparticle composition, including, but not limited to, a nanoemulsion. In some embodiments, a nanoemulsion comprises 1349 oil, polysorbate 80, propylparaben, isotonic sodium chloride solution, methylparaben, a buffer solution (comprising gelatin, sodium phosphate dibasic, purified water, and hydrochloric acid) and, optionally, a known therapeutic agent and/or independently active biologically active agent. In some embodiments, a nanoemulsion comprises 1349 oil, polysorbate 80, isotonic sodium chloride solution, a buffer solution (comprising gelatin, sodium phosphate dibasic, purified water, and hydrochloric acid) and, optionally, a known therapeutic agent and/or independently active biologically active agent. In some embodiments, the nanoemulsion comprises oil and surfactant at a ratio of 0.67:1. In some embodiments, a nanoemulsion comprises oil and an isotonic sodium chloride solution at a ratio of 1:10. In some embodiments, a nanoemulsion comprises surfactant and an isotonic sodium chloride solution at a ratio of 1:1.67. In some embodiments, a nanoemulsion comprises the components set forth in any of Examples 1-20.

In some embodiments, provided nanoparticle compositions comprise a saline solution. In some embodiments, the saline solution comprises isotonic sodium chloride solution. In some embodiments, the saline solution comprises isotonic sodium chloride solution and/or water. In some embodiments, the saline solution comprises isotonic sodium chloride solution and methylparaben. In some embodiments, the saline solution comprises the components set forth in any of Examples 1-20.

The present invention encompasses the recognition that provided nanoparticle compositions can be formulated for delivery to a subject in need thereof via topical and/or transdermal administration (*e.g*., by lotions, creams, powders, ointments, liniments, gels, drops, *etc.*)*.* In some embodiments, provided nanoparticle compositions are administered to a subject in need thereof via topical and/or transdermal (*e.g*., by lotions, creams, powders, ointments, liniments, gels, drops, *etc.*) administration.

The present invention encompasses the recognition that provided nanoparticle compositions can be particularly useful for topical and/or transdermal administration. The present invention encompasses the recognition that provided nanoparticle compositions can be particularly useful for delivery of agents to the dermal level of the skin. In some embodiments, provided nanoparticle compositions are formulated for topical and/or transdermal delivery to a subject in need thereof. In some embodiments, provided nanoparticle compositions are administered to a subject in need thereof via topical and/or transdermal delivery.

The present invention provides the surprising finding of particular cream and/or lotion formulations with unexpected and/or beneficial properties.

The present invention encompasses the recognition that provided cream and/or lotion formulations can be used for delivery of agents to a subject in need thereof via topical and/or transdermal (*e.g.*, by lotions, creams, powders, ointments, liniments, gels, drops, *etc.*) administration. In some embodiments, provided cream and/or lotion formulations are administered to a subject in need thereof via topical and/or transdermal (*e.g*., by lotions, creams, powders, ointments, liniments, gels, drops, *etc.*) administration. In some embodiments, provided cream and/or lotion formulations are formulated with provided nanoparticle compositions. In some embodiments, provided cream and/or lotion formulations that are formulated with provided nanoparticle compositions are useful and/or effective for topical administration to a subject.

The present invention encompasses the recognition that provided cream and/or lotion formulations can be particularly useful for topical and/or transdermal administration. The present invention encompasses the recognition that provided cream and/or lotion formulations can be particularly useful for delivery of agents to the dermal level of the skin. In some embodiments, provided cream and/or lotion formulations are formulated for topical and/or transdermal delivery to a subject in need thereof. In some embodiments, provided cream and/or lotion formulations are administered to a subject in need thereof via topical and/or transdermal delivery.

In some embodiments, provided cream and/or lotion formulations comprise purified water, methylparaben, mineral oil, isopropyl myristate, white petrolatum, emulsifying wax, and propylparaben. In some embodiments, provided cream and/or lotion formulations comprise purified water, mineral oil, isopropyl myristate, white petrolatum, and emulsifying wax. In some embodiments, provided cream and/or lotion formulations comprise the components set forth in Examples 1-20.

In some embodiments, the present invention provides particular cream and/or lotion formulations as described herein. In some embodiments, provided cream and/or lotion formulations comprise water. In some embodiments, provided cream and/or lotion formulations comprise methylparaben. In some embodiments, provided cream and/or lotion formulations comprise mineral oil. In some embodiments, provided cream and/or lotion formulations comprise isopropyl myristate. In some embodiments, provided cream and/or lotion formulations comprise white petrolatum. In some embodiments, provided cream and/or lotion formulations comprise emulsifying wax. In some embodiments, provided cream and/or lotion formulations comprise propylparaben. In some embodiments, provided cream and/or lotion formulations do not comprise any parabens. In some embodiments, provided cream and/or lotion formulations do not comprise methylparaben. In some embodiments, provided cream and/or lotion formulations do not comprise propylparaben.

The present invention encompasses the recognition that provided cream and/or lotion formulations can be particularly useful for formulating nanoparticle compositions, such as those described herein, for administration to a subject. The present invention encompasses the recognition that provided cream and/or lotion formulations can be particularly useful for formulating nanoemulsions, such as those described herein, for administration to a subject.

The present invention provides that surprising and/or unexpected results can be achieved when cream and/or lotion formulations are formulated with particular provided nanoparticle compositions as described herein.

In some embodiments, provided compositions comprise a mixture of a provided nanoparticle composition and one or more pharmaceutically acceptable excipients. In some embodiments, provided compositions comprise a mixture of a provided nanoparticle composition, a saline solution, and a provided cream and/or lotion formulation, as described herein.

In some embodiments, provided compositions comprise provided nanoparticle compositions. In some embodiments, provided compositions comprise provided cream and/or lotion formulations. In some embodiments, provided compositions comprise both provided nanoparticle compositions and provided cream and/or lotion formulations. In some embodiments, provided compositions comprise provided nanoparticle compositions but do not comprise provided cream and/or lotion formulations. In some embodiments, provided compositions comprise provided cream and/or lotion formulations but do not comprise provided nanoparticle compositions.

In some embodiments, provided compositions comprise a mixture of a provided nanoparticle composition and one or more pharmaceutically acceptable excipients, *e.g*., for topical and/or transdermal (*e.g*., by lotions, creams, powders, ointments, liniments, gels, drops, *etc.*) administration.

The present invention provides methods of treating conditions or disorders using any of the provided compositions (*e.g*., provided nanoparticle composition; cream and/or lotion formulation; combination of provided nanoparticle composition and cream and/or lotion formulation; *etc.*) as described herein.

In some embodiments, such methods involve administration of a provided composition to a patient suffering from and/or susceptible to a disease, condition, or disorder. In some embodiments, such methods involve administration of a provided nanoparticle composition to a patient suffering from and/or susceptible to a disease, condition, or disorder associated with the dermal layer of the skin. In some embodiments, such methods involve administration of an empty nanoparticle composition (*e.g*., a nanoparticle composition not containing a known therapeutic agent and/or independently active biologically active agent) to a patient suffering from and/or susceptible to a disease, condition, or disorder. In some embodiments, such methods involve administration of a nanoparticle composition comprising at least one known therapeutic agent and/or independently active biologically active agent to a patient suffering from and/or susceptible to a disease, condition, or disorder. In some embodiments, such methods involve administration of a nanoparticle composition and/or at least one known therapeutic agent and/or independently active biologically active agent formulated with a provided cream and/or lotion formulation to a patient suffering from and/or susceptible to a disease, condition, or disorder. In some embodiments, such methods involve administration of provided compositions via topical and/or transdermal (*e.g*., by lotions, creams, powders, ointments, liniments, gels, drops, *etc*.) administration.

In some embodiments, the present invention provides methods of treating any conditions or disorders. In some embodiments, the present invention demonstrates that certain compositions as described herein can achieve controlled delivery of active agents efficiently and specifically to biologically relevant target sites (*e.g*., particular tissues, locations within the skin, cells, *etc*.)*.* In some embodiments, the present invention demonstrates controlled delivery and/or achievement of therapeutic effect in a certain biologically relevant target site without significant side effects associated with delivery to other areas.

In some embodiments, the present invention provides methods of treating conditions or disorders associated with epidermal and/or dermal structures (*e.g*., sweat glands, sebaceous glands, hair follicles, *etc.*)*.* In some embodiments, the present invention demonstrates that provided compositions as described herein (*e.g*., provided nanoparticle composition; cream and/or lotion formulation; combination of provided nanoparticle composition and cream and/or lotion formulation; *etc.*) can deliver active agents efficiently and specifically to the dermis, and that provided compositions as described herein can have therapeutic effects upon administration to the skin of a subject. In some embodiments, the present invention demonstrates dermal delivery and/or achievement of therapeutic effect without significant side effects associated with delivery to other areas (*e.g*., to subdermal or extradermal structures and/or to tissues other than dermis). In some embodiments, provided compositions as described herein (*e.g*., provided nanoparticle composition; cream and/or lotion formulation; combination of provided nanoparticle composition and cream and/or lotion formulation; *etc.*) can transdermally deliver active agents, such as therapeutic agents (*e.g*., botulinum toxins, monoclonal antibodies, etc.).

The present invention provides methods of treating conditions or disorders by administering to a patient a provided composition as described herein (*e.g*., provided nanoparticle composition; cream and/or lotion formulation; combination of provided nanoparticle composition and cream and/or lotion formulation; *etc.*)*.* In some embodiments, the present invention provides methods of treating conditions or disorders by administering to a patient a composition containing a provided nanoparticle composition (*e.g*., a nanoemulsion) as described herein. In some embodiments, administration is local administration. In some embodiments, local administration is topical administration.

In general, for nanoparticle compositions comprising a known therapeutic agent and/or independently active biologically active agent, such nanoparticle compositions are arranged and constructed such that an amount of therapeutic agent is delivered to a desired target site (*e.g*., to epidermal and/or dermal structures) that is sufficient to treat the condition or disorder. In some embodiments, provided nanoparticle compositions are arranged and constructed (*e.g*., through selection and/or combination of agents, structure of composition, *etc*.) such that they achieve the desired therapeutic effect upon administration to the skin. In some embodiments, provided nanoparticle compositions are arranged and constructed such that they do not induce unwanted clinical effects inside and/or outside of the desired site of action (*e.g*., surface of skin, dermis, *etc.*)*.* In some embodiments, provided nanoparticle compositions are arranged and constructed such that they have systemic effects.

In some embodiments, provided compositions may be formulated and/or delivered so that systemic delivery is achieved; in some embodiments, provided compositions may be formulated and/or delivered so that local, but not systemic, delivery is achieved.

The present disclosure specifically demonstrates effective and efficient delivery of a therapeutic agent (and, in particular, a large biologic agent, such as botulinum toxin) to the dermis using provided compositions. For example, in some embodiments, the present invention provides methods comprising administration of a composition as described herein without clinically significant side effects. To give but one example, when topical delivery is contemplated, clinically significant side effects include, but are not limited to, unwanted systemic side effects, damage to nervous tissue underlying the dermis (*e.g*., neuronal paralysis), unwanted effects on muscles (*e.g*., muscle paralysis), and/or undesirable blood levels of therapeutic agent, *etc.* The present invention provides the surprising benefits and/or capabilities of particular provided compositions (*e.g*., provided nanoparticle compositions and/or provided cream and/or lotion formulations) as compared with nanoparticle compositions in general.

The present invention further provides technologies for identifying the component or components present in the provided compositions that are responsible for the observed activity of nanoparticle compositions and/or cream and/or lotion formulations. To the extent that such technologies identify component(s) that can achieve the observed results independent of a nanoparticle structure, the present invention also provides use in medicine, and in particular in the treatment of conditions or disorders associated with dermal structures (*e.g*., sweat glands, sebaceous glands, hair follicles, *etc.*), of compositions containing one or more individual components of provided compositions. As used herein, a "provided composition" may contain one or more individual components of nanoparticle compositions and/or cream and/or lotion formulations.

This application refers to various patent and non-patent publications, all of which are incorporated herein by reference.

### Definitions

*Abrasion*: The term "abrasion," as used herein, refers to any means of altering, disrupting, removing, or destroying the top layer of the skin. In some embodiments, abrasion refers to a mechanical means of altering, disrupting, removing, or destroying the top layer of the skin. In some embodiments, abrasion refers to a chemical means of altering, disrupting, removing, or destroying the top layer of skin. To give but a few examples, agents such as exfoliants, fine particles (*e.g*., magnesium or aluminum particles), acids (*e.g*., alpha-hydroxy acids or beta-hydroxy acids), and/or alcohols may cause abrasion. In general, permeation enhancers such as those described, for example, by Donovan (see, *e.g.*, U.S. Patent Publications 2004/009180 and 2005/175636; and PCT Publication WO 04/06954; all of which are incorporated herein by reference), and Graham (see, *e.g.*, U.S. Patent 6,939,852 and U.S. Patent Publication 2006/093624; both of which are incorporated herein by reference), *etc*., are expected to cause abrasion. Of course, those of ordinary skill in the art will appreciate that a particular agent may cause abrasion when present at one concentration, or in association with one or more other agents, but may not cause abrasion under different circumstances. Thus, whether or not a particular material is an "abrasive agent" depends on context. Abrasion can readily be assessed by those of ordinary skill in the art, for example by observation of redness or irritation of the skin and/or histologic examination of skin showing alteration, disruption, removal, or erosion of the stratum corneum.

*Administration*: The term "administration," as used herein refers to the delivery and/or administration of a provided composition to a subject. For example, the present invention contemplates routes of delivering or administering that include, but are not limited to, topical and/or transdermal (*e.g*., by lotions, creams, liniments, ointments, powders, gels, drops, deodorants, antiperspirants, sunscreens, *etc.*)*.*

*Amino acid*: As used herein, term "amino acid," in its broadest sense, refers to any compound and/or substance that can be incorporated into a polypeptide chain. In some embodiments, an amino acid has the general structure H₂N-C(H)(R)-COOH. In some embodiments, an amino acid is a naturally-occurring amino acid. In some embodiments, an amino acid is a synthetic amino acid; in some embodiments, an amino acid is a D-amino acid; in some embodiments, an amino acid is an L-amino acid. "Standard amino acid" refers to any of the twenty standard L-amino acids commonly found in naturally occurring peptides. "Nonstandard amino acid" refers to any amino acid, other than the standard amino acids, regardless of whether it is prepared synthetically or obtained from a natural source. Amino acids, including carboxy- and/or amino-terminal amino acids in peptides, can be modified by methylation, amidation, acetylation, and/or substitution with other chemical groups that can change the peptide's circulating half-life without adversely affecting their activity. Amino acids may participate in a disulfide bond. The term "amino acid" is used interchangeably with "amino acid residue," and may refer to a free amino acid and/or to an amino acid residue of a peptide. It will be apparent from the context in which the term is used whether it refers to a free amino acid or a residue of a peptide.

*Animal*: As used herein, the term "animal" refers to any member of the animal kingdom. In some embodiments, "animal" refers to humans, at any stage of development. In some embodiments, "animal" refers to non-human animals, at any stage of development. In certain embodiments, the non-human animal is a mammal (*e.g*., a rodent, a mouse, a rat, a rabbit, a monkey, a dog, a cat, a sheep, cattle, a primate, and/or a pig). In some embodiments, animals include, but are not limited to, mammals, birds, reptiles, amphibians, fish, and/or worms. In some embodiments, an animal may be a transgenic animal, genetically-engineered animal, and/or a clone.

*Approximately*: As used herein, the terms "approximately" or "about" in reference to a number are generally taken to include numbers that fall within a range of 5%, 10%, 15%, or 20% in either direction (greater than or less than) of the number unless otherwise stated or otherwise evident from the context (except where such number would be less than 0% or exceed 100% of a possible value).

*Biologically active agent*: As used herein, the phrase "biologically active agent" refers to any substance that has activity in a biological system and/or organism. For instance, a substance that, when administered to an organism, has a biological effect on that organism is considered to be biologically active. In some embodiments, where a substance (*e.g*., a polypeptide, nucleic acid, antibody, *etc*.) is biologically active, a portion of that substance that shares at least one biological activity of the whole substance is typically referred to as a "biologically active" portion.

*Botulinum nanoparticle composition*: The term "botulinum nanoparticle composition," as used herein, refers to any nanoparticle composition described herein in which at least one nanoparticle includes botulinum toxin. The botulinum toxin may be present within the nanoparticle, on the nanoparticle surface and/or within a micellar membrane defining the nanoparticle.

*Botulinum toxin*: The term "botulinum toxin," as used herein, refers to any neurotoxin produced by *Clostridium botulinum.* Except as otherwise indicated, the term encompasses fragments or portions (*e.g*., the light chain and/or the heavy chain) of such neurotoxin that retain appropriate activity (*e.g*., muscle relaxant activity). The phrase "botulinum toxin," as used herein, encompasses the botulinum toxin serotypes type A, type Ab, type Af, type B, type Bf, type C1, type C2, type D, type E, type F, and type G; mutants thereof; variants thereof; fragments thereof; characteristic portions thereof; and/or fusions thereof. In some embodiments, botulinum toxin is present as any of the subtypes described in Sakaguchi, 1982, Pharmacol. Ther., 19:165; and/or Smith et al., 2005, Infect. Immun., 73:5450; both of which are incorporated herein by reference. Botulinum toxin, as used herein, also encompasses both a botulinum toxin complex (*i.e*., for example, the 300, 600, and 900 kD complexes) as well as the purified (*i.e*., for example, isolated) botulinum toxin (*i.e.*, for example, about 150 kD). "Purified botulinum toxin" is defined as a botulinum toxin that is isolated, or substantially isolated, from other proteins, including proteins that form a botulinum toxin complex. A purified toxin may be greater than 80% pure, greater than 85% pure, greater than 90% pure, greater than 95% pure, greater than 98% pure, and/or greater than 99% pure. Those of ordinary skill in the art will appreciate that the present invention is not limited to any particular source of botulinum toxin. For example, botulinum toxin for use in accordance with the present invention may be isolated from *Clostridium botulinum*, may be chemically synthesized, may be produced recombinantly (*i.e*., in a host cell or organism other than *Clostridium botulinum*), *etc.*

*Cosmetic formulation*: The term "cosmetic formulation" is used herein to refer to a topically applied composition that contains one or more agents having cosmetic properties. To give but a few examples, a cosmetic formulation may be a skin softener, nutrition lotion type emulsion, cleansing lotion, cleansing cream, skin milk, emollient lotion, massage cream, emollient cream, make-up base, lipstick, facial pack or facial gel, cleaner formulation such as shampoos, rinses, body cleanser, hair-tonics, or soaps, and/or a dermatological composition such as a lotion, ointment, gel, cream, patch, deodorant, antiperspirant, and/or spray.

*Cream*: The term "cream" refers to a spreadable composition, typically formulated for application to the skin. Creams typically contain an oil and/or fatty acid based-matrix. Creams formulated according to the present invention may contain nanoparticles and may be capable of substantially complete penetration (*e.g*., of such nanoparticles) through the skin upon topical administration. Such a cream could also act as a carrier for incorporated materials (*e.g*., for example, for one or more known therapeutic agents and/or independently active biologically active agents).

*Dispersion medium*: The term "dispersion medium" as used herein, refers to a liquid medium in which particles (*e.g*., empty nanoparticles and/or nanoparticles containing one or more known therapeutic agents and/or independently active biologically active agents) are dispersed. In general, a dispersion is formed when at least two immiscible materials are combined. An "oil-in-water" dispersion is one in which oily particles are dispersed within an aqueous dispersion medium. A "water-in-oil" dispersion is one in which aqueous particles are dispersed within an oily dispersion medium. Those of ordinary skill in the art will appreciate that a dispersion can be formed from any two immiscible media and is not limited strictly to combinations of aqueous and oily media. The term "dispersion medium" therefore applies broadly to *any* dispersion medium notwithstanding that it is common to refer to "aqueous" and "oily" categories.

*Encapsulated*: The term "encapsulated" (also "encapsulate" or "encapsulating") is used herein to mean that the encapsulated entity is completely surrounded by another material. To give but one example, a biologically active agent (*e.g*., botulinum toxin) may be encapsulated within a nanoparticle in an emulsion. Such encapsulation may be achieved, for example, during formation of a nanoparticle composition (*e.g*., a nanoemulsion), for example during microfluidization. To give but another example, known therapeutic agents and/or independently active biologically active agents are not encapsulated within empty nanoparticles in an emulsion.

*Empty nanoparticle composition*: The term "empty nanoparticle composition," as used herein, refers to a nanoparticle composition which does not include a known therapeutic agent and/or an independently active biologically active agent.

*Homology*: As used herein, the term "homology" refers to the overall relatedness between polymeric molecules, *e.g*., between polynucleotide molecules (*e.g*., DNA molecules and/or RNA molecules) and/or between polypeptide molecules. In some embodiments, polymeric molecules are considered to be "homologous" to one another if their sequences are at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or at least 99% identical. In some embodiments, polymeric molecules are considered to be "homologous" to one another if their sequences are at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or at least 99% similar.

*Identity*: As used herein, the term "identity" refers to the overall relatedness between polymeric molecules, *e.g*., between polynucleotide molecules (*e.g*., DNA molecules and/or RNA molecules) and/or between polypeptide molecules. Calculation of the percent identity of two nucleic acid sequences, for example, can be performed by aligning the two sequences for optimal comparison purposes (*e.g.*, gaps can be introduced in one or both of a first and a second nucleic acid sequences for optimal alignment and non-identical sequences can be disregarded for comparison purposes). In certain embodiments, the length of a sequence aligned for comparison purposes is at least 30%, at least 40%, at least 50%, at least 60%, at least 70%, at least 80%, at least 90%, at least 95% or 100% of the length of the reference sequence. The nucleotides at corresponding nucleotide positions are then compared. When a position in the first sequence is occupied by the same nucleotide as the corresponding position in the second sequence, then the molecules are identical at that position. The percent identity between the two sequences is a function of the number of identical positions shared by the sequences, taking into account the number of gaps, and the length of each gap, which needs to be introduced for optimal alignment of the two sequences. The comparison of sequences and determination of percent identity between two sequences can be accomplished using a mathematical algorithm. For example, the percent identity between two nucleotide sequences can be determined using the algorithm of Meyers and Miller (CABIOS, 1989, 4: 11-17), which has been incorporated into the ALIGN program (version 2.0) using a PAM120 weight residue table, a gap length penalty of 12 and a gap penalty of 4. The percent identity between two nucleotide sequences can, alternatively, be determined using the GAP program in the GCG software package using an NWSgapdna.CMP matrix.

*In conjunction with*: As used herein, the phrase "delivered in conjunction with" and/or "administered in conjunction with" refers to the co-delivery and/or co-administration of two or more substances or agents. In some embodiments, according to the present invention, the phrase is used herein in reference to delivery of a biologically active agent with nanoparticles and/or provided nanoparticle compositions. A substance or agent is delivered in conjunction with nanoparticles when the substance or agent is combined with nanoparticles and/or nanoparticle compositions; is encapsulated or completely surrounded by nanoparticles; is embedded within a nanoparticle micellar membrane; and/or is associated with the outer surface of a nanoparticle micellar membrane. A substance or agent to be delivered in conjunction with nanoparticles and/or nanoparticle compositions may or may not be covalently linked to the nanoparticles and/or nanoparticle compositions. A substance or agent to be delivered in conjunction with nanoparticles and/or nanoparticle compositions may or may not be attached to the nanoparticles and/or nanoparticle compositions by adsorption forces. In some embodiments, according to the present invention, the phrase is used herein in reference to simultaneous administration of a composition comprising empty nanoparticles with another composition comprising a known therapeutic agent and/or independently active biologically active agent. In such embodiments, a known therapeutic agent and/or independently active biologically active agent is not part of the empty nanoparticle composition, but instead, is administered separately to the subject (*e.g*., either as a separate composition, or having been admixed and/or formulated together with the empty nanoparticle composition. In some embodiments, a known therapeutic and/or independently active biologically active agent is not incorporated nanoparticles of a nanoparticle composition; in some embodiments, a known therapeutic and/or independently active biologically active agent is not encapsulated within nanoparticles of a nanoparticle composition; in some embodiments, a known therapeutic and/or independently active biologically active agent is not otherwise in association with nanoparticles of the nanoparticle composition).

*Independently active biologically active agent*: The term "independently active biologically active agent" refers to an agent that shows biological activity whether or not the agent is present in a nanoparticle composition as described herein. In some embodiments, one or more particular biological activities of the agent is/are improved in a nanoparticle composition; in some embodiments, one or more biological activities of the agent is/are not improved in a nanoparticle composition.

*Isolated*: As used herein, the term "isolated" refers to a substance and/or entity that has been (1) separated from at least some of the components with which it was associated when initially produced (whether in nature and/or in an experimental setting), and/or (2) produced, prepared, and/or manufactured by the hand of man. Isolated substances and/or entities may be separated from at least about 10%, about 20%, about 30%, about 40%, about 50%, about 60%, about 70%, about 80%, about 90%, or more of the other components with which they were initially associated. In some embodiments, isolated substances and/or entities are more than 90%, 91%, 92%, 93%, 94%, 95%, 96%, 97%, 98%, or 99% pure.

*Known therapeutic agent*: As used herein, the term "known therapeutic agent" describes a biologically active agent known, prior to its incorporation in a nanoparticle composition, to have a particular biological effect *e.g*., on a dermal structure (*e.g.*, for example, on sweat glands, sebaceous glands, hair follicles, *etc*)*.* In some embodiments, a known therapeutic agent describes a biologically active agent known prior to filing of the present application to have a particular biological effect, *e.g.*, on a dermal structure (*e.g.*, for example, on sweat glands, sebaceous glands, hair follicles, *etc*)*.* Exemplary known therapeutic agents known to have a particular biological effect on sweat glands include aluminum chloride, aluminum chlorohydrate, aluminum chlorohydrex compounds, aluminum dichlorohydrate, aluminum dichlorohydrex compounds, aluminum sesquichlorohydrate, aluminum sesquichlorohydrex compounds, aluminum zirconium tetrachlorohydrex gly, aluminum zirconium trichlorohydrex gly, ammonium alum, aluminum sulfate compounds, aluminum zirconium compounds, botulinum toxin, oral medication (*e.g*., diphenhydramine hydrochloride, hydroxyzine, glycopyrrolate, *etc*.), anticholinergic drugs (*e.g*., oxybutynin, glycopyrrolate, propantheline bromide, benztropine, *etc.*), beta-blockers, antidepressants, anxiolytics, talc, baby powder, and/or combinations thereof. Exemplary known therapeutic agents known to have a particular biological effect on sebaceous glands include botulinum toxin, cleansers or soaps, a topical bactericidal (*e.g*., benzoyl peroxide, triclosan, and/or chlorhexidine gluconate), topical antibiotics (*e.g*., externally-applied erythromycin, clindamycin, tetracycline, *etc.*), oral antibiotics (*e.g*., erythromycin, tetracycline, oxytetracycline, doxycycline, minocycline, lymecycline, trimethoprim, *etc.*), hormonal treatments (*e.g*., estrogen/progestogen oral contraceptives, low dose spironolactone, cortisone, *etc.*), a keratolytic (*i.e.*, a substance that dissolves keratin plugging pores), benzoyl peroxide, a topical retinoid (*e.g*., tretinoin [RETIN-A®], adapalene [DIFFERIN®], and tazarotene [TAZORAC®], retinol, isotretinoin, *etc.*), oral retinoids (*e.g*., isotretinoin [ACCUTANE®, AMNESTEEM™, SOTRET™, CLARA VIS™]), retinoic acids, a natural product with anti-acne activity (*e.g*., aloe vera, aruna, haldi [*i.e*., turmeric], papaya, *etc.*), azelaic acid (brand names AZELEX™, FINACEA®, FINEVIN®, SKINOREN, *etc.*), antiinflammatory agents (*e.g*., naproxen, ibuprofen, rofecoxib, *etc.*), nicotinamide (*i.e.*, vitamin B3), tea tree oil (melaleuca oil), aminolevulinic acid, azithromycin, methylaminolevuninate, nadifloxacine, PRK124, talarozole, zileuton, rofecoxib, zinc, an agent described in Krowchuk (2000, Pediatric Dermatology, 47:841-857; incorporated herein by reference) and/or in Johnson et al. (2000, American Family Physician, 62:1823-1830 and 1835-1836; incorporated herein by reference), and/or combinations thereof. Exemplary known therapeutic agents known to have a particular biological effect on hair follicles include minoxidil (ROGAINE®/REGAINE®), finasteride (PROPECIA®), dutasteride (AVODART®), an antiandrogen (*e.g*., ketoconazole, fluconazole, spironolactone, *etc.*), saw palmetto, caffeine, copper peptides, nitroxide spin labels TEMPO and TEMPOL, unsaturated fatty acids (*e.g*., gamma linolenic acid), hedgehog agonists, azelaic acid and zinc in combination, Chinese knotweed, pumpkin seed, tretinoin, zinc, stinging nettle, Tempol alcohol-based gel *(e.g.,* MTS-01, *etc*.), Aldara, alefacept, AS101, bimatoprost, capsaicin, efalizumab, FK506, GP11046, GP11511, hydroxychloroquine, latanoprost, MK0906, roxithromycin, Targretin Gel 1%, tetrapeptide aldehyde proteasome inhibitor (*e.g.*, NEOSH101, *etc.*), and/or combinations thereof. Exemplary known therapeutic agents known to have a particular biological effect on the treatment and/or prevention of psoriasis include, but are not limited to, botulinum toxin; coal tar; dithranol (anthralin); a corticosteroid such as desoximetasone (TOPICORT®); a vitamin D3 analog (*e.g.*, calcipotriol); a retinoid; argan oil; topical administration of psoralen with exposure to ultraviolet A light (PUVA); milk thistle; methotrexate; cyclosporine; the antimetabolite tioguanine; hydroxyurea; sulfasalazine; mycophenolate mofetil; azathioprine; tacrolimus; and/or antibody-based therapeutics (*e.g*., alefacept [AMEVIEVE®], etanercept [EMBREL®], infliximab [REMICADE efalizumab [RAPTTVA®], *etc*).

*Microfluidized*: As used herein, the term "microfluidized" means exposed to high shear forces. In some embodiments, such exposure to high shear forces is accomplished by exposure to high pressure; in some embodiments such high pressure is within the range of about 15,000 psi to about 26,000 psi. In some embodiments, such exposure to high shear forces is accomplished by cavitation. In some embodiments, such exposure to high shear forces is accomplished by passing a sample through an instrument such as, for example, a Microfluidizer® (Microfluidics Corporation/MFIC Corporation) or other like device that may be useful in creating a uniform nanoparticle composition. In some embodiments, a sample is microfluidized through exposure to high shear forces for a period of time less than about 10 minutes. In some embodiments, the period of time is less than about 9, about 8, about 7, about 6, about 5, about 4, about 3, about 2, or about 1 minute(s). In some embodiments, the period of time is within the range of about 1 - about 2 minutes. In some embodiments, the period of time is about 30 seconds. In some embodiments, a sample is "microfluidized" through a single exposure to high shear forces; such embodiments are referred to as "single pass" microfluidization.

*Nanoemulsion*: An emulsion is traditionally defined in the art "as a system ... consisting of a liquid dispersed with or without an emulsifier in an immiscible liquid usually in droplets of larger than colloidal size" Medline Plus Online Medical Dictionary, Merriam Webster (2005). The term "nanoemulsion," as used herein, refers to an emulsion in which at least some of the droplets (or particles) have diameters in the nanometer size range. As will be understood by those of ordinary skill in the art, a nanoemulsion is characterized by droplets or particles one thousand fold smaller than microemulsion droplets or particles.

*Nanoparticle*: As used herein, the term "nanoparticle" refers to any particle having a diameter of less than 1000 nanometers (nm). In some embodiments, a nanoparticle has a diameter of less than 300 nm, as defined by the National Science Foundation. In some embodiments, a nanoparticle has a diameter of less than 100 nm as defined by the National Institutes of Health. In some embodiments, nanoparticles are micelles in that they comprise an enclosed compartment, separated from the bulk solution by a micellar membrane. A "micellar membrane" comprises amphiphilic entities which have aggregated to surround and enclose a space or compartment (*e.g*., to define a lumen).

*Nanoparticle composition:* As used herein, the term "nanoparticle composition" refers to any substance that contains at least one nanoparticle. In some embodiments, a nanoparticle composition is a uniform collection of nanoparticles. In some embodiments, nanoparticle compositions are dispersions or emulsions. In general, a dispersion or emulsion is formed when at least two immiscible materials are combined. An "oil-in-water" dispersion is one in which oily particles (or hydrophobic or non-polar) are dispersed within an aqueous dispersion medium. A "water-in-oil" dispersion is one in which aqueous (or hydrophilic or polar) particles are dispersed within an oily dispersion medium. Those of ordinary skill in the art will appreciate that a dispersion can be formed from any two immiscible media and is not limited strictly to combinations of aqueous and oily media. The term "dispersion medium" therefore applies broadly to *any* dispersion medium notwithstanding that it is common to refer to "aqueous" and "oily" categories. In some embodiments, nanoparticle compositions are nanoemulsions. In some embodiments, nanoparticle compositions comprise micelles. In some embodiments, a nanoparticle composition comprises particles such as those described in U.S. patent number 7,763,663, issued on July 27, 2010, and entitled "POLYSACCHARIDE-CONTAINING BLOCK COPOLYMER PARTICLES AND USES THEREOF" (incorporated herein by reference). In some embodiments, a nanoparticle composition comprises a nanoemulsion as described in PCT patent application number PCT/US06/026918, filed July 11, 2006, published as WO 08/010788 on January 24, 2008, and entitled "COMPOSITIONS AND METHODS FOR MAKING AND USING NANOEMULSIONS" (incorporated herein by reference). In some embodiments, a nanoparticle composition comprises a nanoemulsion as described in PCT patent application number PCT US06/46236, filed December 1, 2006, published as WO 08/045107 on April 17, 2008, and entitled "BOTULINUM NANOEMULSIONS" (incorporated herein by reference). In some embodiments, a nanoparticle composition comprises amphiphilic entity nanoparticles as described in PCT patent application number PCT/US07/86018, filed November 30, 2007, published as WO 08/070538 on June 12, 2008, and entitled "AMPHIPHILIC ENTITY NANOPARTICLES" (incorporated herein by reference). In some embodiments, a nanoparticle composition comprises particles as described in PCT application serial number PCT/US08/65329, filed May 30, 2008, published as PCT publication WO 08/151022 on December 11, 2008, and entitled "NUCLEIC ACID NANOPARTICLES AND USES THEREFOR" (incorporated herein by reference). In some embodiments, a nanoparticle composition comprises particles as described in PCT patent application number PCT/ US07/86040, filed November 30, 2007, published as PCT publication WO 08/140594 on November 20, 2008, and entitled "PEPTIDE NANOPARTICLES AND USES THEREFOR" (incorporated herein by reference). In some-embodiments, a nanoparticle composition comprises particles as described in PCT patent application number PCT US09/48972, filed June 26, 2009, published as WO 09/158687 on December 30, 2009, and entitled "DERMAL DELIVERY" (incorporated herein by reference). In some embodiments, nanoparticle compositions are stable. In some embodiments, nanoparticle compositions include one or more biologically active agents to be delivered in conjunction with the nanoparticles. In some embodiments, nanoparticle compositions are empty nanoparticle compositions (e.g., they do not contain any known therapeutic agents and/or independently active biologically active agents).

*Not contaminated with*: The phrase "not contaminated with," when used herein to refer to a nanoparticle composition, is synonymous with "substantially free of' and describes a nanoparticle composition containing no more than about 50% of the recited material. For example, if a nanoparticle composition is said to be "substantially free of' particles whose diameter is outside of a stated range, then no more than about 50% of the particles in that composition have diameters outside of the range. In some embodiments, no more than 25% of the particles are outside of the range. In some embodiments, no more than 20%, 19%, 18%, 17%, 16%, 15%, 14%, 13%, 12%, 10%, 9%, 8%, 7%, 6%, 5%, 4%, 3%, 2%, 1%, 0.5% or less of the particles have diameters outside of the stated range.

*Nucleic acid*: As used herein, the term "nucleic acid," in its broadest sense, refers to any compound and/or substance that is or can be incorporated into an oligonucleotide chain. In some embodiments, a nucleic acid is a compound and/or substance that is or can be incorporated into an oligonucleotide chain via a phosphodiester linkage. In some embodiments, "nucleic acid" refers to individual nucleic acid residues (*e.g*., nucleotides and/or nucleosides). In some embodiments, "nucleic acid" refers to an oligonucleotide chain comprising individual nucleic acid residues. As used herein, the terms "oligonucleotide" and "polynucleotide" can be used interchangeably. In some embodiments, "nucleic acid" encompasses RNA as well as single and/or double-stranded DNA and/or cDNA. Furthermore, the terms "nucleic acid," "DNA," "RNA," and/or similar terms include nucleic acid analogs, *e.g*., analogs having other than a phosphodiester backbone. For example, the so-called "peptide nucleic acids," which are known in the art and have peptide bonds instead of phosphodiester bonds in the backbone, are considered within the scope of the present invention. The term "nucleotide sequence encoding an amino acid sequence" includes all nucleotide sequences that are degenerate versions of each other and/or encode the same amino acid sequence. Nucleotide sequences that encode proteins and/or RNA may include introns. Nucleic acids can be purified from natural sources, produced using recombinant expression systems and optionally purified, chemically synthesized, *etc.* Where appropriate, *e.g*., in the case of chemically synthesized molecules, nucleic acids can comprise nucleoside analogs such as analogs having chemically modified bases or sugars, backbone modifications, *etc.* A nucleic acid sequence is presented in the 5' to 3' direction unless otherwise indicated. The term "nucleic acid segment" is used herein to refer to a nucleic acid sequence that is a portion of a longer nucleic acid sequence. In many embodiments, a nucleic acid segment comprises at least 3, 4, 5, 6, 7, 8, 9, 10, or more residues. In some embodiments, a nucleic acid is or comprises natural nucleosides (*e.g*., adenosine, thymidine, guanosine, cytidine, uridine, deoxyadenosine, deoxythymidine, deoxyguanosine, and deoxycytidine); nucleoside analogs (*e.g*., 2-aminoadenosine, 2-thiothymidine, inosine, pyrrolo-pyrimidine, 3-methyl adenosine, 5-methylcytidine, C-5 propynyl-cytidine, C-5 propynyl-uridine, 2-aminoadenosine, C5-bromouridine, C5-fluorouridine, C5-iodouridine, C5-propynyl-uridine, C5-propynyl-cytidine, C5-methylcytidine, 2-aminoadenosine, 7-deazaadenosine, 7-deazaguanosine, 8-oxoadenosine, 8-oxoguanosine, O(6)-methylguanine, and 2-thiocytidine); chemically modified bases; biologically modified bases (*e.g*., methylated bases); intercalated bases; modified sugars (*e.g*., 2'-fluororibose, ribose, 2'-deoxyribose, arabinose, and hexose); and/or modified phosphate groups (*e.g*., phosphorothioates and 5'-*N-*phosphoramidite linkages). In some embodiments, the present invention is specifically directed to "unmodified nucleic acids," meaning nucleic acids (*e.g*., polynucleotides and residues, including nucleotides and/or nucleosides) that have not been chemically modified.

*Patient*: As used herein, the term "patient" or "subject" refers to any organism to which a provided composition may be administered, *e.g*., for experimental, diagnostic, prophylactic, cosmetic, and/or therapeutic purposes. Typical patients include animals (*e.g*., mammals such as mice, rats, rabbits, non-human primates, and humans). In some embodiments, a patient is a human.

*Pharmaceutically acceptable*: The term "pharmaceutically acceptable" as used herein, refers to agents that, within the scope of sound medical judgment, are suitable for use in contact with the tissues of human beings and animals without excessive toxicity, irritation, allergic response, or other problem or complication, commensurate with a reasonable benefit/risk ratio.

*Premix*: As used herein, the term "premix" refers to any combination of components that is subsequently used to generate a nanoparticle composition according to the present invention. For example, a premix is any collection of ingredients that, when subjected to high shear forces, generates nanoparticles according to the present invention. In some embodiments, a premix contains two or more immiscible solvents. In some embodiments, a premix contains components that self-assemble into nanoparticles. In some embodiments, a premix contains components that self-assemble into micelles. In some embodiments, a premix contains one or more amphiphilic entities as described in co-pending PCT application serial number PCT/US07/86018, filed November 30, 2007, published as WO 08/070538 on June 12, 2008, and entitled "AMPHIPHILIC ENTITY NANOPARTICLES." In some embodiments, a premix contains one or more known therapeutic agents and/or independently active biologically active agents. In some embodiments, a premix does not contain any known therapeutic agents and/or independently active biologically active agents. In some embodiments, a premix is agitated, mixed, and/or stirred; in some embodiments, a premix is agitated, mixed, and/or stirred prior to being subjected to high shear force. In some embodiments, a premix comprises at least one solubilized component (*i.e*., at least one component that is in solution); in some such embodiments, the premix is subjected to high shear force after such solubilization is achieved.

*Pure*: As used herein, a substance and/or entity is "pure" if it is substantially free of other components. For example, a preparation that contains more than about 90% of a particular substance and/or entity is typically considered to be a pure preparation. In some embodiments, a substance and/or entity is at least 91%, at least 92%, at least 93%, at least 94%, at least 95%, at least 96%, at least 97%, at least 98%, or at least 99% pure.

*Refractory*: The term "refractory" as used herein, refers to any subject that does not respond with an expected clinical efficacy following the administration of provided compositions as normally observed by practicing medical personnel.

*Self-administration*: The term "self-administration," as used herein, refers to the situation where a subject has the ability to administer a composition to him or herself without requiring medical supervision. In some embodiments, self-administration may be performed outside of a clinical setting. To give but one example, in some embodiments, a facial cosmetic cream may be administered by a subject in one's own home.

*Shear force*: As used herein, the term "shear force" refers to a force that is parallel or tangential to the face of a material, as opposed to a force that is perpendicular to the face of a material. In some embodiments, a composition is exposed to high shear forces in order to produce a uniform nanoparticle composition. Any method known in the art can be used to generate high shear forces. In some embodiments, cavitation is used to generate high shear forces. In some embodiments, high pressure homogenization is used to generate high shear forces. Alternatively or additionally, high shear force may be administered by exposure to high pressure, for example about 15,000 psi. In some embodiments, such high pressure is within the range of about 18,000 psi to about 26,000 psi; in some embodiments, it is within the range of about 20,000 psi to about 25,000 psi. In some embodiments, and to give but one example, a Microfluidizer® Processor (Microfluidics Corporation/MFIC Corporation) or other like device is used to generate high shear force. Microfluidizer® Processors provide high pressure and a resultant high shear rate by accelerating a composition through microchannels (typically having dimensions on the order of 75 microns) at a high velocity (typically in the range of 50 m/s - 300 m/s) for size reduction to the nanoscale range. As the fluid exits the microchannels it forms jets which collide with jets from opposing microchannels. In the channels the fluid experiences high shear (up to 10⁷ 1/s) which is orders of magnitude higher than that of conventional technologies. Jet collisions result in mixing at submicron levels. Therefore, in such devices, high shear and/or impact can achieve particle size reduction and mixing of multiphase. In some embodiments, a sample is exposed to high shear forces for a period of time less than about 10 minutes. In some embodiments, the period of time is less than about 9 minutes, about 8 minutes, about 7 minutes, about 6 minutes, about 5 minutes, about 4 minutes, about 3 minutes, about 2 minutes, or about 1 minute. In some embodiments, the period of time is within the range of about 1 minute to about 2 minutes; in some embodiments, the period of time is less than about 1 minute; in some embodiments, the period of time is about 30 seconds. In some embodiments, a sample is "microfluidized" through a single exposure to high shear forces; such embodiments are referred to herein as "single pass" microfluidization.

*Similarity*: As used herein, the term "similarity" refers to the overall relatedness between polymeric molecules, *e.g*., between polynucleotide molecules (*e.g.*, DNA molecules and/or RNA molecules) and/or between polypeptide molecules. Calculation of percent similarity of polymeric molecules to one another can be performed in the same manner as a calculation of percent identity, except that calculation of percent similarity takes into account conservative substitutions as is understood in the art.

*Small Molecule*: In general, a "small molecule" is a molecule that is less than about 5 kilodaltons (kD) in size. In some embodiments, the small molecule is less than about 4 kD, 3 kD, about 2 kD, or about 1 kD. In some embodiments, the small molecule is less than about 800 daltons (D), about 600 D, about 500 D, about 400 D, about 300 D, about 200 D, or about 100 D. In some embodiments, a small molecule is less than about 2000 g/mol, less than about 1500 g/mol, less than about 1000 g/mol, less than about 800 g/mol, or less than about 500 g/mol. In some embodiments, small molecules are non-polymeric. In some embodiments, in accordance with the present invention, small molecules are not proteins, polypeptides, oligopeptides, peptides, polynucleotides, oligonucleotides, polysaccharides, glycoproteins, proteoglycans, *etc.*

*Stable*: The term "stable," when applied to provided compositions herein, means that the compositions maintain one or more aspects of their physical structure (*e.g*., size range and/or distribution of particles) over a period of time. In some embodiments, a stable nanoparticle composition is one for which the average particle size, the maximum particle size, the range of particle sizes, and/or the distribution of particle sizes (*i.e*., the percentage of particles above a designated size and/or outside a designated range of sizes) is maintained for a period of time. In some embodiments, the period of time is at least about one hour; in some embodiments the period of time is about 5 hours, about 10 hours, about one (1) day, about one (1) week, about two (2) weeks, about one (1) month, about two (2) months, about three (3) months, about four (4) months, about five (5) months, about six (6) months, about eight (8) months, about ten (10) months, about twelve (12) months, about twenty-four (24) months, about thirty-six (36) months, or longer. In some embodiments, the period of time is within the range of about one (1) day to about twenty-four (24) months, about two (2) weeks to about twelve (12) months, about two (2) months to about five (5) months, *etc.* For example, if a population of nanoemulsion particles is subjected to prolonged storage, temperature changes, and/or pH changes and a majority of the nanoparticles in the composition maintain a diameter within a stated range (for example, between approximately 10 nm and approximately 120 nm), the nanoparticle composition is stable. For some such populations, a majority is more than about 50%, about 60%, about 70%, about 80%, about 90%, about 95%, about 96%, about 97%, about 98%, about 99%, about 99.5%, about 99.6%, about 99.7%, about 99.8%, about 99.9% or more. In some embodiments of the invention, where a provided composition comprises a known therapeutic agent and/or an independently active biologically active agent, the provided composition is considered stable if the concentration of therapeutic agent and/or an independently active biologically active agent (*e.g*., botulinum toxin) is maintained in the composition over the designated period of time under a designated set of conditions. In some embodiments of the invention, where a provided composition comprises at least one therapeutic agent and/or an independently active biologically active agent, the provided composition is considered stable if the concentration of therapeutic agent and/or an independently active biologically active agent (*e.g*., botulinum toxin) is maintained in the composition over the designated period of time under a designated set of conditions. In some embodiments of the invention, where a provided composition comprises a therapeutic agent and/or an independently active biologically active agent, the provided composition is considered stable if the bioavailability of the active agent (*e.g*., botulinum toxin) is maintained over the designated period of time under a designated set of conditions. As will be appreciated by those of skill in the art, bioavailability of a therapeutic agent and/or an independently active biologically active agent in a nanoemulsion, in some embodiments, may reflect the amount or concentration of the agent in active form (*e.g.*, not degraded or otherwise inactivated); in some embodiments, bioavailability may be independent of amount or concentration of the active agent. That is, in some embodiments, bioavilability may be maintained although amount or concentration increases (*e.g*., up to X% of study level) or decreases (e*.g*., down to Y% of starting level).

*Substantially*: As used herein, the term "substantially" refers to the qualitative condition of exhibiting total or near-total extent or degree of a characteristic or property of interest. One of ordinary skill in the biological arts will understand that biological and chemical phenomena rarely, if ever, go to completion and/or proceed to completeness or achieve or avoid an absolute result. The term "substantially" is therefore used herein to capture the potential lack of completeness inherent in many biological and chemical phenomena.

*Substantially free of*: A nanoparticle composition is said to be "substantially free of' particles whose diameter is outside of a stated range when no more than about 50% of the particles in that composition have diameters outside of the range. In some embodiments, no more than 25% of the particles are outside of the range. In some embodiments, no more than 20%, 19%, 18%, 17%, 16%, 15%, 14%, 13%, 12%, 10%, 9%, 8%, 7%, 6%, 5%, 4%, 3%, 2%, 1%, 0.5% or less of the particles have diameters outside of the stated range.

*Suffering from*: An individual who is "suffering from" a disease, disorder, or condition (*e.g*., any disease, disorder, or condition, including, but not limited to, any disease, disorder, or condition described herein) has been diagnosed with or exhibits symptoms of the disease, disorder, or condition. In some embodiments, exemplary diseases, disorders, or conditions include, but are not limited to, a condition associated with sweat glands or sebaceous glands, such as acne; hyperhidrosis; unwanted sweating; bromhidrosis; body odor; chromhidrosis; hair loss; psoriasis; actinic keratosis; dermal infection; eczematous dermatitis (*e.g*., atopic dermatitis, *etc*.); excess sebum-producing disorder; burns; Raynaud's phenomenon; lupus erthythematosus; hyperpigmentation disorder; hypopigmentation disorder; skin cancer; *etc.*

*Susceptible to*: An individual who is "susceptible to" a disease, disorder, or condition (e.g., any disease, disorder, or condition, including, but not limited to, any disease, disorder, or condition described herein) is at risk for developing the disease, disorder, or condition. In some embodiments, an individual who is susceptible to a disease, disorder, or condition does not display any symptoms of the disease, disorder, or condition. In some embodiments, an individual who is susceptible to a disease, disorder, or condition has not been diagnosed with the disease, disorder, and/or condition. In some embodiments, an individual who is susceptible to a disease, disorder, or condition is an individual who has been exposed to conditions associated with development of the disease, disorder, or condition (*e.g*., the individual has been exposed to an infectious agent; the individual has been exposed to an environmental hazard thought to cause the disease, disorder, and/or condition; *etc.*). In some embodiments, a risk of developing a disease, disorder, and/or condition is a population-based risk (*e.g*., an individual carries a gene and/or allele associated with the disease, disorder, and/or condition).

*Symptoms are reduced*: According to the present invention, "symptoms are reduced" when one or more symptoms of a particular disease, disorder or condition is reduced in magnitude (*e.g.*, intensity, severity, *etc.*) or frequency. For purposes of clarity, a delay in the onset of a particular symptom is considered one form of reducing the frequency of that symptom. To give but a few examples, where the condition in question is acne, symptoms of that condition are reduced when the (*e.g*., diameter, volume, *etc.*) and/or severity (*e.g.*, redness, inflammatory response, *etc.*) of one or more blemishes in the selected area is reduced, and/or when the number of total blemishes is reduced (*e.g.*, on a subject's face, back, *etc.*)*.* Where the condition in question is hyperhidrosis and/or unwanted sweating, symptoms are reduced when the subject produces less sweat. It is not intended that the present invention be limited only to cases where the symptoms are eliminated. The present invention specifically contemplates treatment such that one or more symptoms is/are reduced (and the condition of the subject is thereby "improved"), albeit not completely eliminated.

*Therapeutically effective amount*: As used herein, the term "therapeutically effective amount" means an amount that is sufficient, when administered to a population suffering from or susceptible to a disease, disorder, and/or condition, to treat the disease, disorder, and/or condition. In some embodiments, a therapeutically effective amount is one that reduces the incidence and/or severity of, and/or delays onset of, one or more symptoms of the disease, disorder, and/or condition. Those of ordinary skill in the art will appreciate that the term "therapeutically effective amount" does not in fact require successful treatment be achieved in a particular individual. Rather, a therapeutically effective amount may be that amount that provides a particular desired pharmacological response in a significant number of subjects when administered to patients in need of such treatment. It is specifically understood that particular subjects may, in fact, be "refractory" to a "therapeutically effective amount." To give but one example, a refractory subject may have a low bioavailability such that clinical efficacy is not obtainable. In some embodiments, reference to a therapeutically effective amount may be a reference to an amount as measured in one or more specific tissues. Those of ordinary skill in the art will appreciate that, in some embodiments, a therapeutically effective agent may be formulated and/or administered in a single dose. In some embodiments, a therapeutically effective agent may be formulated and/or administered in a plurality of doses, for example, as part of a dosing regimen.

*Therapeutic agent*: As used herein, the phrase "therapeutic agent" refers to any agent that has a therapeutic effect and/or elicits a desired biological and/or pharmacological effect, when administered to a subject. Exemplary therapeutic agents include but are not limited to botulinum toxins and monoclonal antibodies and fragments thereof.

*Toxic solvent*: As used herein, the term "toxic solvent" refers to any substance that may alter, disrupt, remove, or destroy an animal's tissue. As would be understood by one of ordinary skill in the art, an animal's tissue can include living cells, dead cells, extracellular matrix, cellular junctions, biological molecules, *etc.* To give but a few examples, toxic solvents include dimethyl sulfoxide, dimethyl acetimide, dimethyl formamide, chloroform, tetramethyl formamide, acetone, acetates, and alkanes.

*Treatment*: As used herein, the term "treatment" (also "treat" or "treating") refers to any administration of a substance (*e.g*., provided compositions) that partially or completely alleviates, ameliorates, relives, inhibits, delays onset of, reduces severity of, and/or reduces incidence of one or more symptoms or features of a particular disease, disorder, and/or condition. Such treatment may be of a subject who does not exhibit signs of the relevant disease, disorder and/or condition and/or of a subject who exhibits only early signs of the disease, disorder, and/or condition. Alternatively or additionally, such treatment may be of a subject who exhibits one or more established signs of the relevant disease, disorder and/or condition. In some embodiments, treatment may be of a subject who has been diagnosed as suffering from the relevant disease, disorder, and/or condition. In some embodiments, treatment may be of a subject known to have one or more susceptibility factors that are statistically correlated with increased risk of development of the relevant disease, disorder, and/or condition.

*Uniform*: The term "uniform," when used herein in reference to a nanoparticle composition, refers to a nanoparticle composition in which the individual nanoparticles have a specified range of particle diameter sizes. For example, in some embodiments, a uniform nanoparticle composition is one in which the difference between the minimum diameter and maximum diameter does not exceed about 600 nm, about 550 nm, about 500 nm, about 450 nm, about 400 nm, about 350 nm, about 300 nm, about 250 nm, about 200 nm, about 150 nm, about 100 nm, about 90 nm, about 80 nm, about 70 nm, about 60 nm, about 50 nm, or fewer nm. In some embodiments, particles (*e.g*., empty particles and/or particles containing one or more known therapeutic agents and/or independently active biologically active agents) within uniform provided nanoparticle compositions have diameters that are smaller than about 600 nm, about 550 nm, about 500 nm, about 450 nm, about 400 nm, about 350 nm, about 300 nm, about 250 nm, about 200 nm, about 150 nm, about 130 nm, about 120 nm, about 115 nm, about 110 nm, about 100 nm, about 90 nm, about 80 nm, or less. In some embodiments, particles (*e.g*., empty particles and/or particles containing one or more known therapeutic agents and/or independently active biologically active agents) within uniform provided nanoparticle compositions have diameters within the range of about 10 nm and about 600 nm. In some embodiments, particles within uniform provided nanoparticle compositions have diameters within the range of about 10 nm and about 300 nm, about 10 nm and about 200 nm, about 10 nm and about 150 nm, about 10 nm and about 130 nm, about 10 nm and about 120 nm, about 10 nm and about 115 nm, about 10 nm and about 110 nm, about 10 nm and about 100 nm, or about 10 nm and about 90 nm. In some embodiments, particles within provided nanoparticle compositions have an average particle size that is under about 300 nm, about 250 nm, about 200 nm, about 150 nm, about 130 nm, about 120 nm, about 115 nm, about 110 nm, about 100 nm, or about 90 nm. In some embodiments, the average particle size is within the range of about 10 nm and about 300 nm, about 50 nm and about 250 nm, about 60 nm and about 200 nm, about 65 nm and about 150 nm, about 70 nm and about 130 nm. In some embodiments, the average particle size is between about 80 nm and about 110 nm. In some embodiments, the average particle size is about 90 nm to about 100 nm. In some embodiments, a majority of the particles within uniform provided nanoparticle compositions have diameters below a specified size or within a specified range. In some embodiments, the majority is more than 50%, 60%, 70%, 75%, 80%, 85%, 90%, 95%, 96%, 97%, 98%, 99%, 99.5%, 99.6%, 99.7%, 99.8%, 99.9% or more of the particles in the composition. In some embodiments, a uniform nanoparticle composition is achieved by microfluidization of a sample. In some embodiments, a uniform nanoparticle composition is achieved by single-pass microfluidization of a sample. In some embodiments, a uniform nanoparticle composition is prepared by exposure to high shear force, *e.g*., by microfluidization.

*Unwanted side effects*: As used herein, the term "unwanted side effects" refers to one or more effects and/or symptoms associated with administration of a substance to a patient that are not the desired and/or intended effects and/or are unpleasant to the patient. Exemplary unwanted side effects include pain; bruising; ecchymosis; hematoma; botulism poisoning; unwanted systemic effects; undesirable blood levels of the administered substance; damage to underlying nervous tissue (*e.g*., neuronal paralysis); unwanted effects on muscles (*e.g*., muscle paralysis); flu-like symptoms; morbidity; mortality; alteration in body weight; alteration in enzyme levels; pathological changes detected at the microscopic, macroscopic, and/or physiological levels; infection; hemorrhage; inflammation; scarring; loss of function; changes in local blood flow; fever; malaise; teratogenesis; pulmonary hypertension; stroke; heart disease; heart attack; neuropathy; nausea; vomiting; dizziness; diarrhea; headache; dermatitis; dry mouth; addiction; miscarriage; abortion; uterine hemorrhage; birth defects; bleeding; cardiovascular disease; deafness; kidney damage and/or failure; liver damage and/or failure; dementia; depression; diabetes; erectile dysfunction; glaucoma; hair loss; anaemia; insomnia; lactic acidosis; melasma; thrombosis; priapism; rhabdomyolysis; seizures; drowsiness; increase in appetite; decrease in appetite; increase in libido; decrease in libido; tardive dyskinesia; non-axillary sweating; injection site pain and hemorrhage; pharyngitis; neck pain; back pain; pruritus; anxiety; follicular obstruction; and/or combinations thereof. In some embodiments, topical administration of a provided composition (*e.g*., provided nanoparticle composition, such as an empty nanoparticle composition and/or nanoparticle composition containing one or more known therapeutic agents and/or independently active biologically active agents; cream and/or lotion formulation; combination of provided nanoparticle composition and cream and/or lotion formulation; *etc.*) reduces unwanted side effects by about 50%, about 60%, about 70%, about 80%, about 90%, about 95%, about 98%, about 99%, or about 100% relative to non-topical administration (*e.g*., injection, oral administration, *etc*.) of the same substance.

### Description of Certain Embodiments

The present invention provides particular compositions as described herein.

In some embodiments, provided compositions comprise a provided nanoparticle composition. In some embodiments, provided compositions comprise a provided cream and/or lotion formulation. In some embodiments, provided compositions comprise a provided nanoparticle composition and a provided cream and/or lotion formulation. In some embodiments, provided compositions comprise both a provided nanoparticle composition but do not comprise a provided cream and/or lotion formulation. In some embodiments, provided compositions comprise a provided cream and/or lotion formulation but do not comprise a provided nanoparticle composition.

The present invention provides particular nanoparticle compositions as described herein. In some embodiments, provided nanoparticle compositions comprise an oil, and aqueous dispersion medium, a surfactant and, optionally, an independently biologically active agent and/or a known therapeutic agent. In some embodiments, the present invention provides nanoparticle compositions containing individual specific ingredients described herein. In some embodiments, the present invention provides nanoparticle compositions containing specific combinations of ingredients described herein (*e.g.*, the recipe described in any of Examples 1-20). In some embodiments, the present invention provides nanoparticle compositions with particular structural and/or functional attributes. In some embodiments, the present invention demonstrates one or more unexpected and/or surprising advantages and/or unusual characteristics of provided nanoparticle compositions.

In some embodiments, nanoparticle compositions described herein can be used in over the counter (OTC) products, including, but not limited to, deodorants and antiperspirants. In some embodiments, nanoparticle compositions described herein can be used in prescription formulations.

In some embodiments, the present invention provides methods involving use of the particular nanoparticle compositions described herein for the treatment of disorders or conditions. In some embodiments, such methods involve administration of provided nanoparticle compositions via topical and/or transdermal administration, as described herein.

In some embodiments, the present invention provides particular cream and/or lotion formulations as described herein. In some embodiments, provided cream and/or lotion formulations can be useful for topical and/or transdermal administration of any agent to be delivered to a subject. In some embodiments, provided cream and/or lotion formulations can be useful for administration of a nanoparticle composition to a subject. In some embodiments, provided cream and/or lotion formulations can be useful for topical and/or transdermal administration of a nanoparticle composition to a subject. In some embodiments, provided cream and/or lotion formulations can be useful for administration of a provided nanoparticle composition to a subject. In some embodiments, provided cream and/or lotion formulations can be useful for topical and/or transdermal administration of a provided nanoparticle composition to a subject. In some embodiments, provided cream and/or lotion formulations do not contain a nanoparticle composition. In some embodiments, provided cream and/or lotion formulations contain an active agent, such as a therapeutic agent.

### Provided Nanoparticle Compositions

The present invention provides particular nanoparticle compositions that are particularly effective and/or useful in medical contexts, *e.g*., for therapeutic purposes. The present invention provides particular nanoparticle compositions that are particularly effective and/or useful for topical administration of agents to a subject in need thereof.

In some embodiments, provided nanoparticle compositions comprise an aqueous dispersion medium that comprises an isotonic sodium chloride solution. In some embodiments, provided nanoparticle compositions comprise an aqueous dispersion medium that consists essentially of an isotonic sodium chloride solution. In some embodiments, provided nanoparticle compositions comprise an aqueous dispersion medium that consists of an isotonic sodium chloride solution. In some embodiments, provided nanoparticle compositions comprise an aqueous dispersion medium that comprises gelatin. In some embodiments, provided nanoparticle compositions comprise an aqueous dispersion medium that comprises sodium phosphate. In some embodiments, provided nanoparticle compositions comprise an aqueous dispersion medium that comprises purified water. In some embodiments, nanoparticle compositions comprise an aqueous dispersion medium that comprises hydrochloric acid. In some embodiments, provided nanoparticle compositions comprise an aqueous dispersion medium that comprises gelatin, sodium phosphate, purified water, and hydrochloric acid. In some embodiments, provided nanoparticle compositions comprise an aqueous dispersion medium that consists essentially of gelatin, sodium phosphate, purified water, and hydrochloric acid. In some embodiments, provided nanoparticle compositions comprise an aqueous dispersion medium that consists of gelatin, sodium phosphate, purified water, and hydrochloric acid.

Those of ordinary skill in the art will also be well aware of suitable oily media that can be used as dispersion media or as media to be dispersed in accordance with the present invention. In some embodiments, oils may comprise one or more fatty acid groups or salts thereof. In some embodiments, a fatty acid group may comprise digestible, substituted or unsubstituted hydrocarbons. In some embodiments, a fatty acid group may be a C₆-C₅₀ fatty acid or salt thereof. In some embodiments, a fatty acid group may be a C₆-C₂₀ fatty acid or salt thereof. In some embodiments, a fatty acid group may be a C₆-C₁₆ fatty acid or salt thereof. In some embodiments, a fatty acid group may be a C₆-C₁₂ fatty acid or salt thereof. In some embodiments, a fatty acid group may be a C₆ fatty acid or salt thereof. In some embodiments, a fatty acid group may be a C₈ fatty acid or salt thereof. In some embodiments, a fatty acid group may be a C₁₀ fatty acid or salt thereof. In some embodiments, a fatty acid group may be a C₁₂ fatty acid or salt thereof. In some embodiments, a fatty acid group may be unsaturated. In some embodiments, a fatty acid group may be monounsaturated. In some embodiments, a fatty acid group may be polyunsaturated. In some embodiments, a double bond of an unsaturated fatty acid group may be in the *cis* conformation. In some embodiments, a double bond of an unsaturated fatty acid may be in the *trans* conformation. In some embodiments, a fatty acid group may be one or more of butyric, caproic, caprylic, capric, lauric, myristic, palmitic, stearic, arachidic, behenic, lignoceric acid, and/or combinations thereof. In some embodiments, a fatty acid group may be one or more of palmitoleic, oleic, vaccenic, linoleic, alpha-linolenic, gamma-linoleic, arachidonic, gadoleic, arachidonic, eicosapentaenoic, docosahexaenoic, erucic acid, and/or combinations thereof.

In some embodiments, a provided nanoparticle composition comprises an oily dispersion medium that comprises, consists essentially of, or consists of a medium chain triglyceride (e.g., fatty acids containing 6-12 carbons atoms, such as caprylic acid, octanoic acid, capric acid, decanoic acid, lauric acid, *etc*., which, in some embodiments, may be obtained from coconut oil or palm kernel oil). Exemplary medium chain triglycerides include monounsaturated, and/or polyunsaturated soybean oil, coconut oil, canola oil, safflower oil, olive oil, corn oil, cottonseed oil, linseed oil, safflower oil, palm oil, peanut oil, flaxseed oil, sunflower oil, rice bran oil, sesame oil, rapeseed oil, cocoa butter, almond oil, cashew oil, hazelnut oil, mongongo nut oil, acai oil, borage seed oil, evening primrose oil, carob pod oil, amaranth oil, apple seed oil, artichoke oil, avocado oil, babassu oil, ben oil, borneo tallow nut oil, cocoa butter, cocklebur oil, cohune oil, dika oil, grape seed oil, hemp oil, kapok seed oil, kenaf seed oil, lallemantia oil, marula oil, meadowfoam seed oil, mustard oil, papaya seed oil, perilla seed oil, pequi oil, poppyseed oil, prune kernel oil, quinoa oil, tea seed oil, thistle oil, tigernut oil, tomato seed oil, wheat germ oil, Labrafac™ Lipophile WL 1349 oil, a silicone oil, a mineral oil, a lauroyl macrogol-6 glyceride, a lauroyl polyoxyl-6 glyceride, an oleoyl macrogol-6 glyceride, an oleoyl polyoxyl-6 glyceride, a linoleoyl macrogol-6 glyceride, a linoleoyl polyoxyl-6 glyceride, propylene glycol monocaprylate, propylene glycol monolaurate, propylene glycol monolaurate, polglyceryl-3 dioleate, propylene glycol dicaprylocaprate, diethylene glycol monethyl ether, a caprylocaproyl macrogol-8 glyceride, a caprylocaproyl polyoxyl-8 glyceride, and/or combinations thereof.

In some embodiments, an oil is or comprises saturated, monounsaturated, and/or polyunsaturated short-chain fatty acids, medium-chain fatty acids, long-chain fatty acids, very-long-chain fatty acids, and/or combinations thereof. In some embodiments, exemplary very-long-chain fatty acids include, but are not limited to, myristoleic acid, palmitoleic acid, sapienic acid, oleic acid, linoleic acid, alpha linolenic acid, arachidonic acid, eicosapentaenoic acid, erucic acid,, docoshexaenoic acid, lauric acid, myristic acid, palmitic acid, stearic acid, arachidic acid, behenic acid, lignoceric acid, cerotic acid, and/or combinations thereof.

In some embodiments, an oil is selected from the group consisting of short-chain triglycerides, medium-chain triglycerides, long-chain triglycerides, and/or combinations thereof. In some embodiments, a short-chain triglyceride, a medium-chain triglyceride, and/or a long-chain triglyceride selected from the group consisting of saturated, monounsaturated, and/or polyunsaturated soybean oil, coconut oil, canola oil, safflower oil, olive oil, corn oil, cottonseed oil, linseed oil, safflower oil, palm oil, peanut oil, flaxseed oil, sunflower oil, rice bran oil, sesame oil, rapeseed oil, cocoa butter, almond oil, cashew oil, hazelnut oil, macadamia oil, mongongo nut oil, pecan oil, pine nut oil, pistachio oil, sachainchi oil, walnut oil, bottle gourd oil, buffalo gourd oil, butternut squash seed oil, pumpkin seed oil, watermelon seed oil, acai oil, blackcurrant seed oil, borage seed oil, evening primrose oil, carob pod oil, amaranth oil, apricot oil, apricot kernel oil, apple seed oil, argan oil, artichoke oil, avocado oil, babassu oil, ben oil, borneo tallow nut oil, cape chestnut oil, cassia oil, cocoa butter, cocklebur oil, cohune oil, coriander seed oil, dika oil, grape seed oil, hemp oil, kapok seed oil, kenaf seed oil, lallemantia oil, marula oil, meadowfoam seed oil, mustard oil, nutmeg butter, okra seed oil, papaya seed oil, perilla seed oil, pequi oil, poppyseed oil, prune kernel oil, quinoa oil, ramtil oil, royle oil, tea seed oil, thistle oil, tigernut oil, tomato seed oil, wheat germ oil, radish oil, salicornia oil, tung oil, algae oil, copaiba oil, honge oil, jatropha oil, petroleum nut oil, WL 1349 oil, a silicone oil, a mineral oil, a lauroyl macrogol-6 glyceride, a lauroyl polyoxyl-6 glyceride, an oleoyl macrogol-6 glyceride, an oleoyl polyoxyl-6 glyceride, a linoleoyl macrogol-6 glyceride, a linoleoyl polyoxyl-6 glyceride, propylene glycol monocaprylate, propylene glycol monolaurate, propylene glycol monolaurate, polglyceryl-3 dioleate, propylene glycol dicaprylocaprate, diethylene glycol monethyl ether, a caprylocaproyl macrogol-8 glyceride, a caprylocaproyl polyoxyl-8 glyceride, and/or combinations thereof.

In some embodiments, an oil agent is or comprises saturated, monounsaturated, and/or polyunsaturated soybean oil, coconut oil, canola oil, safflower oil, olive oil, corn oil, cottonseed oil, linseed oil, safflower oil, palm oil, peanut oil, flaxseed oil, sunflower oil, rice bran oil, sesame oil, rapeseed oil, cocoa butter, almond oil, cashew oil, hazelnut oil, macadamia oil, mongongo nut oil, pecan oil, pine nut oil, pistachio oil, sachainchi oil, walnut oil, bottle gourd oil, buffalo gourd oil, butternut squash seed oil, pumpkin seed oil, watermelon seed oil, acai oil, blackcurrant seed oil, borage seed oil, evening primrose oil, carob pod oil, amaranth oil, apricot oil, apricot kernel oil, apple seed oil, argan oil, artichoke oil, avocado oil, babassu oil, ben oil, borneo tallow nut oil, cape chestnut oil, cassia oil, cocoa butter, cocklebur oil, cohune oil, coriander seed oil, dika oil, grape seed oil, hemp oil, kapok seed oil, kenaf seed oil, lallemantia oil, marula oil, meadowfoam seed oil, mustard oil, nutmeg butter, okra seed oil, papaya seed oil, perilla seed oil, pequi oil, poppyseed oil, prune kernel oil, quinoa oil, ramtil oil, royle oil, tea seed oil, thistle oil, tigernut oil, tomato seed oil, wheat germ oil, radish oil, salicornia oil, tung oil, algae oil, copaiba oil, honge oil, jatropha oil, petroleum nut oil, WL 1349 oil, a silicone oil, a mineral oil, a lauroyl macrogol-6 glyceride, a lauroyl polyoxyl-6 glyceride, an oleoyl macrogol-6 glyceride, an oleoyl polyoxyl-6 glyceride, a linoleoyl macrogol-6 glyceride, a linoleoyl polyoxyl-6 glyceride, propylene glycol monocaprylate, propylene glycol monolaurate, propylene glycol monolaurate, polglyceryl-3 dioleate, propylene glycol dicaprylocaprate, diethylene glycol monethyl ether, a caprylocaproyl macrogol-8 glyceride, a caprylocaproyl polyoxyl-8 glyceride, bergamot, cade, camomile, caraway, carnauba, castor, cinnamon, cod liver, coffee, emu, eucalyptus, fish, geraniol, hyssop, jojoba, kukui nut, lavandin, lavender, lemon, litsea cubeba, mallow, mango seed, mink, orange, orange roughy, palm kernel, peach kernel, rosemary, sandalwood, sasquana, savoury, sea buckthorn, shea butter, tea tree, tsubaki, vetiver, butyl stearate, caprylic triglyceride, capric triglyceride, cyclomethicone, diethyl sebacate, dimethicone 360, isopropyl myristate, octyldodecanol, oleyl alcohol, and/or combinations thereof.

In some embodiments, provided nanoparticle compositions comprise an oil agent that comprises 1349 oil. In some embodiments, provided nanoparticle compositions comprise an oil agent that consists essentially of 1349 oil. In some embodiments, provided nanoparticle compositions comprise an oil agent that consists of 1349 oil. In some embodiments, provided nanoparticle compositions comprise an oil agent that comprises soybean oil. In some embodiments, provided nanoparticle compositions comprise an oil agent that consists essentially of soybean oil. In some embodiments, provided nanoparticle compositions comprise an oil agent that consists of soybean oil.

In addition to the two immiscible media, provided nanoparticle compositions may include, for example, one or more surfactants or emulsifying agents. In some embodiments, a surfactant is or comprises an amphiphilic entity in that it contains a hydrophilic moiety and a hydrophobic moiety, typically at opposing ends of the entity. In some embodiments, an amphiphilic entity is said to have a hydrophilic head and a hydrophobic tail. In some embodiments, an amphiphilic entity has a charged (anionic, cationic, or zwitterionic) head group; in some embodiments, an amphiphilic entity has an uncharged head group.

Suitable such surfactants or emulsifying agents include, but are not limited to, pemulen; phosphoglycerides; phosphatidylcholines; dipalmitoyl phosphatidylcholine (DPPC); dioleylphosphatidyl ethanolamine (DOPE); dioleyloxypropyltriethylammonium (DOTMA); dioleoylphosphatidylcholine; cholesterol; cholesterol ester; diacylglycerol; diacylglycerolsuccinate; diphosphatidyl glycerol (DPPG); hexanedecanol; fatty alcohols such as polyethylene glycol (PEG); polyoxyethylene-9-lauryl ether; a surface active fatty acid, such as palmitic acid or oleic acid; fatty acids; fatty acid monoglycerides; fatty acid diglycerides; fatty acid amides; sorbitan trioleate (SPAN®85) glycocholate; sorbitan monolaurate (SPAN®20); polyoxyethylene monostearate; surfactin; a poloxomer; a sorbitan fatty acid ester such as sorbitan trioleate; lecithin; lysolecithin; phosphatidylserine; phosphatidylinositol; sphingomyelin; phosphatidylethanolamine (cephalin); cardiolipin; phosphatidic acid; cerebrosides; dicetylphosphate; dipalmitoylphosphatidylglycerol; stearylamine; dodecylamine; hexadecyl-amine; acetyl palmitate; glycerol ricinoleate; hexadecyl stearate; isopropyl myristate; tyloxapol; poly(ethylene glycol)5000-phosphatidylethanolamine; poly(ethylene glycol)400-monostearate; phospholipids; synthetic and/or natural detergents having high surfactant properties; deoxycholates; cyclodextrins; chaotropic salts; ion pairing agents; sodium dodecyl sulfate; pemulen; an amphiphilic entity having a head group based on a polyoxyethylene glycol sorbitan alkyl ester (*e.g*., as in a polysorbate (TWEEN®), a super-refined polysorbate (TWEEN®), and/or combination thereof; including, but not limited to, polysorbate 20 (TWEEN®20); polysorbate 60 (TWEEN®60); polysorbate 65 (TWEEN®65); polysorbate 80 (TWEEN®80); polysorbate 85 (TWEEN®85); super-refined polysorbate 20 (SR TWEEN®20); super-refined polysorbate 60 (SR TWEEN®60); super-refined polysorbate 65 (SR TWEEN®65); super-refined polysorbate 80 (SR TWEEN®80); super-refined polysorbate 85 (SR TWEEN®85); and/or combinations thereof); an amphiphilic entity having a sulfate-based head group (*e.g*., as in ammonium lauryl sulfate, sodium lauryl sulfate, sodium laureth sulfate, sodium myreth sulfate, *etc.*); an amphiphilic entity having a sulfonate-based head group (*e.g*., as in dioctyl sodium sulfosuccinate, perfluorooctanesulfonate [PFOS], perfluorobutanesulfonate, alkyl benzene sulfonates, CHAPS (3-[(3-Cholamidopropyl)dimethylammonio]-1-propanesulfonate, cocamidopropyl hydroxysultaine, *etc.*); an amphiphilic entity having a phosphate-based head group (*e.g.*, as in alkyl aryl ether phosphate, alkyl ether phosphate, lecithin, *etc.*); an amphiphilic entity having a carboxylate-based head group (*e.g.*, as in fatty acids, sodium stearate, sodium lauroyl sarcosinate, carboxylate fluorosurfactants, perfluorononanoate, perfluorooctanoate [PFOA or PFO], amino acids, imino acids, cocamidopropyl betaine, *etc.*); an amphiphilic entity having an amine-based head group (*e.g.*, a primary, secondary, or tertiary amine, as in octenideine dihydrochloride); an amphiphilic entity having a head group comprising a quaternary ammonium ion (*e.g*., as in cetyl trimethylammonium bromide [CTAB] a.k.a. hexadecyl trimethyl ammonium bromide, cetyl trimethylammonium chloride [CTAC], cetylpyridinium chloride [CPC], polyethoxylated tallow amine [POEA], benzalkonium chloride [BAC], Benzethonium chloride [BZT], 5-Bromo-5-nitro-1,3-dioxane, Dimethyldioctadecylammonium chloride, Dioctadecyldimethylammonium bromide [DODAB]); an amphiphilic entity having a head group based on a fatty alcohol (*e.g*., as in cetyl alcohol, stearyl alcohol, cetostearyl alcohol, oleyl alcohol, *etc*.); an amphiphilic entity having a head group based on a polyoxyethylene glycol alkyl ether (*e.g.*, as in octaethylene glycol monododecyl ether, pentaethylene glycol monododecyl ether); an amphiphilic entity having a head group based on polyoxypropylene glycol alkyl ether; an amphiphilic entity having a head group based on a glucoside alkyl ether (*e.g.*, as in decyl glucoside, lauryl glucoside, octyl glucoside, *etc.*); an amphiphilic entity having a head group based on a polyoxyethylene glycol octylphenol ether (*e.g*., as in Triton X-100); an amphiphilic entity having a head group based on a polyoxyethylene glycol alkylphenol ether (*e.g.*, as in nonosynol-9); an amphiphilic entity having a head group based on a glycerol alkyl ester (*e.g*., as in glyceryl laurate); an amphiphilic entity having a head group based on a sorbitan alkyl ester (*e.g*., spans); an amphiphilic entity that is or comprises cocamide MEA, cocamide DEA< dodecyl dimethylamine oxide; a block copolymer of polyethylene glycol and polypropylene glycol (*i.e*., a poloxamer); an amphiphilic entity having a tail group based on or containing a hydrocarbon chain; an amphiphilic entity having a tail group based on or containing an alkyl ether chain; an amphiphilic entity having a tail group based on or containing a polyethylene; an amphiphilic entity having a tail group based on or containing polypropylene oxide; an amphiphilic entity having a tail group based on or containing a fluorocarbon chain; an amphiphilic entity having a tail group based on or containing a siloxane chain; and/or combinations thereof.

In some embodiments, provided nanoparticle compositions comprise a surfactant that comprises a polysorbate (TWEEN®) substance. In some embodiments, provided nanoparticle compositions comprise a surfactant that comprises a super-refined polysorbate (SR TWEEN®) substance. In some embodiments, provided nanoparticle compositions comprise a surfactant that comprises a polysorbate selected from the group consisting of polysorbate 20 (TWEEN®20); polysorbate 60 (TWEEN®60); polysorbate 65 (TWEEN®65); polysorbate 80 (TWEEN®80); polysorbate 85 (TWEEN®85); super-refined polysorbate 20 (SR TWEEN®20); super-refined polysorbate 60 (SR TWEEN®60); super-refined polysorbate 65 (SR TWEEN®65); super-refined polysorbate 80 (SR TWEEN®80); super-refined polysorbate 85 (SR TWEEN®85); and combinations thereof. In some embodiments, provided nanoparticle compositions comprise a surfactant that comprises polysorbate 80 (TWEEN®80). In some embodiments, provided nanoparticle compositions comprise a surfactant that comprises super-refined polysorbate 80 (SR TWEEN®80). In some embodiments, provided nanoparticle compositions comprise a surfactant that consists essentially of a polysorbate (TWEEN®) substance. In some embodiments, provided nanoparticle compositions comprise a surfactant that consists essentially of a super-refined polysorbate (SR TWEEN®) substance. In some embodiments, provided nanoparticle compositions comprise a surfactant that consists essentially of a polysorbate selected from the group consisting of polysorbate 20 (TWEEN®20); polysorbate 60 (TWEEN®60); polysorbate 65 (TWEEN®65); polysorbate 80 (TWEEN®80); polysorbate 85 (TWEEN®85); super-refined polysorbate 20 (SR TWEEN®20); super-refined polysorbate 60 (SR TWEEN®60); super-refined polysorbate 65 (SR TWEEN®65); super-refined polysorbate 80 (SR TWEEN®80); super-refined polysorbate 85 (SR TWEEN®85); and combinations thereof. In some embodiments, provided nanoparticle compositions comprise a surfactant that consists essentially of polysorbate 80 (TWEEN®80). In some embodiments, provided nanoparticle compositions comprise a surfactant that consists essentially of super-refined polysorbate 80 (SR TWEEN®80). In some embodiments, provided nanoparticle compositions comprise a surfactant that consists of a polysorbate (TWEEN®) substance. In some embodiments, provided nanoparticle compositions comprise a surfactant that consists of a super-refined polysorbate (SR TWEEN®) substance. In some embodiments, provided nanoparticle compositions comprise a surfactant that consists of a polysorbate selected from the group consisting of polysorbate 20 (TWEEN®20); polysorbate 60 (TWEEN®60); polysorbate 65 (TWEEN®65); polysorbate 80 (TWEEN®80); polysorbate 85 (TWEEN®85); super-refined polysorbate 20 (SR TWEEN®20); super-refined polysorbate 60 (SR TWEEN®60); super-refined polysorbate 65 (SR TWEEN®65); super-refined polysorbate 80 (SR TWEEN®80); super-refined polysorbate 85 (SR TWEEN®85); and combinations thereof. In some embodiments, provided nanoparticle compositions comprise a surfactant that consists of polysorbate 80 (TWEEN®80). In some embodiments, provided nanoparticle compositions comprise a surfactant that consists of super-refined polysorbate 80 (SR TWEEN®80). In some embodiments, provided nanoparticle compositions comprise a surfactant that consists essentially of pemulen. In some embodiments, provided nanoparticle compositions comprise a surfactant that consists of pemulen.

In some embodiments, a surfactant is a mixture of different surfactants. Surfactants may be extracted and purified from a natural source or may be prepared synthetically in a laboratory. In some embodiments, surfactants are commercially available.

In some embodiments, provided nanoparticle compositions comprise a gelatin agent selected from the group consisting of a hydrolyzed collagen protein, including, but not limited to, gelatin agents selected from the group consisting of Gelatine, Gelfoam, Puragel, Galfoam, a substance corresponding to CAS number 9000-70-8, other forms of gelatin, and/or combinations thereof.

In light of the teachings provided herein, those of ordinary skill in the art will readily be able to identify alternative or additional gelatin agents. In general, as is known in the art, a gelatin is a protein substance that is produced by partial, typically irreversible hydrolysis of collagen extracted from the boiled bones, connective tissues, organs and some intestines of animals such as domesticated cattle, pigs, and horses.

One of ordinary skill in the art would readily appreciate that gelatin itself may not be the only agent with desirable attributes, such as those described herein, and could readily test a variety of agents, particularly peptide agents, to identify additional agents having similar attributes and/or functions. Exemplary peptide agents that could be tested for attributes and/or functions similar to those exhibited by gelatin include, but are not limited to, proteins derived from blood and/or plasma, including, but not limited to, albumin, fibrin, thrombin, prothrombin, and/or combinations thereof.

In some embodiments, provided nanoparticle compositions may include, for example, one or more excipients. In some embodiments, provided nanoparticle compositions comprise an excipient that comprises methylparaben. In some embodiments, provided nanoparticle compositions comprise an excipient that consists essentially of methylparaben. In some embodiments, provided nanoparticle compositions comprise an excipient that consists of methylparaben. In some embodiments, provided nanoparticle compositions comprise an excipient that comprises propylparaben. In some embodiments, provided nanoparticle compositions comprise an excipient that consists essentially of propylparaben. In some embodiments, provided nanoparticle compositions comprise an excipient that consists of propylparaben. In some embodiments, provided nanoparticle compositions comprise an excipient that comprises propylparaben and methylparaben. In some embodiments, provided nanoparticle compositions comprise an excipient that consists essentially of propylparaben and methylparaben. In some embodiments, provided nanoparticle compositions comprise an excipient that consists of propylparaben and methylparaben. In some embodiments, a premix is substantially or completely free of parabens.

In some embodiments, the present invention provides improved nanoparticle compositions in that the provided nanoparticle compositions comprise one or more of 1349 oil, polysorbate 80, parabens (*e.g*., propylparaben, methylparaben, other parabens, and/or combinations thereof), isotonic sodium chloride solution, purified water, gelatin, sodium phosphate dibasic, and concentrated hydrochloric acid. In some embodiments, the present invention provides improved nanoparticle compositions in that the provided nanoparticle compositions comprise all of 1349 oil, polysorbate 80, parabens (*e.g*., propylparaben, methylparaben, other parabens, and/or combinations thereof), isotonic sodium chloride solution, purified water, gelatin, sodium phosphate dibasic, and concentrated hydrochloric acid. In some embodiments, the present invention provides improved nanoparticle compositions in that the provided nanoparticle compositions comprise all of 1349 oil, polysorbate 80, parabens (*e.g*., propylparaben, methylparaben, other parabens, and/or combinations thereof), and gelatin. In some embodiments, the present invention provides improved nanoparticle compositions in that the provided nanoparticle compositions comprise all of 1349 oil, polysorbate 80, and gelatin. In some embodiments, the present invention provides improved nanoparticle compositions in that the provided nanoparticle compositions comprise all of 1349 oil, polysorbate 80, and parabens (*e.g*., propylparaben, methylparaben, other parabens, and/or combinations thereof). In some embodiments, the present invention provides improved nanoparticle compositions in that the provided nanoparticle compositions comprise all of 1349 oil and polysorbate 80.

In some embodiments, the present invention provides improved nanoparticle compositions in that the provided nanoparticle compositions consist essentially of one or more of 1349 oil, polysorbate 80, parabens (*e.g*., propylparaben, methylparaben, other parabens, and/or combinations thereof), isotonic sodium chloride solution, purified water, gelatin, sodium phosphate dibasic, and concentrated hydrochloric acid. In some embodiments, the present invention provides improved nanoparticle compositions in that the provided nanoparticle compositions consist essentially of all of 1349 oil, polysorbate 80, parabens (*e.g*., propylparaben, methylparaben, other parabens, and/or combinations thereof), and gelatin. In some embodiments, the present invention provides improved nanoparticle compositions in that the provided nanoparticle compositions consist essentially of all of 1349 oil, polysorbate 80, and gelatin. In some embodiments, the present invention provides improved nanoparticle compositions in that the provided nanoparticle compositions consist essentially of all of 1349 oil, polysorbate 80, and parabens (*e.g*., propylparaben, methylparaben, other parabens, and/or combinations thereof). In some embodiments, the present invention provides improved nanoparticle compositions in that the provided nanoparticle compositions consist essentially of all of 1349 oil and polysorbate 80.

In some embodiments, the present invention provides improved nanoparticle compositions in that the provided nanoparticle compositions consist of one or more of 1349 oil, polysorbate 80, isotonic sodium chloride solution, purified water, gelatin, sodium phosphate dibasic, and concentrated hydrochloric acid. In some embodiments, the present invention provides improved nanoparticle compositions in that the provided nanoparticle compositions consist of all of 1349 oil, polysorbate 80, parabens (*e.g*., propylparaben, methylparaben, other parabens, and/or combinations thereof), and gelatin. In some embodiments, the present invention provides improved nanoparticle compositions in that the provided nanoparticle compositions consist of all of 1349 oil, polysorbate 80, and gelatin. In some embodiments, the present invention provides improved nanoparticle compositions in that the provided nanoparticle compositions consist of all of 1349 oil, polysorbate 80, and parabens (*e.g*., propylparaben, methylparaben, other parabens, and/or combinations thereof). In some embodiments, the present invention provides improved nanoparticle compositions in that the provided nanoparticle compositions consist of all of 1349 oil and polysorbate 80.

In some embodiments, the present invention provides improved nanoparticle compositions in that the provided nanoparticle compositions comprise one or more of a medium chain triglyceride, a polysorbate, a paraben (*e.g*., propylparaben, methylparaben, other paraben, and/or combinations thereof), and gelatin. In some embodiments, the present invention provides improved nanoparticle compositions in that the provided nanoparticle compositions comprise all of a medium chain triglyceride, a polysorbate, a paraben (*e.g*., propylparaben, methylparaben, other paraben, and/or combinations thereof), and gelatin. In some embodiments, the present invention provides improved nanoparticle compositions in that the provided nanoparticle compositions comprise all of a medium chain triglyceride, a polysorbate, and gelatin. In some embodiments, the present invention provides improved nanoparticle compositions in that the provided nanoparticle compositions comprise all of a medium chain triglyceride, a polysorbate, and a paraben (*e.g*., propylparaben, methylparaben, other paraben, and/or combinations thereof). In some embodiments, the present invention provides improved nanoparticle compositions in that the provided nanoparticle compositions comprise all of a medium chain triglyceride and a polysorbate.

In some embodiments, the present invention provides improved nanoparticle compositions in that provided nanoparticle compositions do not contain any parabens.

In some embodiments, the present invention provides improved nanoparticle compositions in that provided nanoparticle compositions do not contain any gelatin.

In some embodiments, the present invention provides improved nanoparticle compositions in that the provided nanoparticle compositions comprise one or more of a medium chain triglyceride, a polysorbate, and gelatin. In some embodiments, the present invention provides improved nanoparticle compositions in that the provided nanoparticle compositions comprise all of a medium chain triglyceride, a polysorbate, and gelatin.

In some embodiments, the present invention provides improved nanoparticle compositions in that the provided nanoparticle compositions comprise the components set forth in any of Examples 1-20.

In some embodiments, the present invention provides improved nanoparticle compositions in that the provided nanoparticle compositions comprise oil and surfactant at a ratio of 0.67:1. In some embodiments, the present invention provides improved nanoparticle compositions in that the provided nanoparticle compositions comprise oil and an isotonic sodium chloride solution at a ratio of 1:10. In some embodiments, the present invention provides improved nanoparticle compositions in that the provided nanoparticle compositions comprise surfactant and an isotonic sodium chloride solution at a ratio of 1:1.67. In some embodiments, the present invention provides improved nanoparticle compositions in that the provided nanoparticle compositions comprise the ratios of components set forth in any of Examples 1-20.

In some embodiments, the premix comprises oil and surfactant at a ratio ranging between 0.5 - 10. In some embodiments, the ratio of oil to surfactant is approximately 0.5:1, approximately 1:1, approximately 2:1, approximately 3:1, approximately 4:1, approximately 5:1, approximately 6:1, approximately 7:1, approximately 8:1, approximately 9:1 or approximately 10:1. In some embodiments, the ratio of surfactant to oil is approximately 0.5:1, approximately 1:1, approximately 2:1, approximately 3:1, approximately 4:1, approximately 5:1, approximately 6:1, approximately 7:1, approximately 8:1, approximately 9:1 or approximately 10:1.

In some embodiments, oil and surfactant are utilized at a ratio ranging between 0.1 and 2. In some embodiments, the ratio of oil to surfactant is approximately 0.1:1. In some embodiments, the ratio of oil to surfactant is approximately 0.15:1. In some embodiments, the ratio of oil to surfactant is approximately 0.2:1. In some embodiments, the ratio of oil to surfactant is approximately 0.25:1. In some embodiments, the ratio of oil to surfactant is approximately 0.3:1. In some embodiments, the ratio of oil to surfactant is approximately 0.35:1. In some embodiments, the ratio of oil to surfactant is approximately 0.4:1. In some embodiments, the ratio of oil to surfactant is approximately 0.45:1. In some embodiments, the ratio of oil to surfactant is approximately 0.5:1. In some embodiments, the ratio of oil to surfactant is approximately 0.55:1. In some embodiments, the ratio of oil to surfactant is approximately 0.6:1. In some embodiments, the ratio of oil to surfactant is approximately 0.65:1. In some embodiments, the ratio of oil to surfactant is approximately 0.7:1. In some embodiments, the ratio of oil to surfactant is approximately 0.75:1. In some embodiments, the ratio of oil to surfactant is approximately 0.8:1. In some embodiments, the ratio of oil to surfactant is approximately 0.85:1. In some embodiments, the ratio of oil to surfactant is approximately 0.9:1. In some embodiments, the ratio of oil to surfactant is approximately 0.95:1. In some embodiments, the ratio of oil to surfactant is approximately 1:1. In some embodiments, the ratio of oil to surfactant is approximately 1:1.05. In some embodiments, the ratio of oil to surfactant is approximately 1:1.1. In some embodiments, the ratio of oil to surfactant is approximately 1:1.15. In some embodiments, the ratio of oil to surfactant is approximately 1:1.2. In some embodiments, the ratio of oil to surfactant is approximately 1:1.25. In some embodiments, the ratio of oil to surfactant is approximately 1:1.3. In some embodiments, the ratio of oil to surfactant is approximately 1:1.35. In some embodiments, the ratio of oil to surfactant is approximately 1:1.4. In some embodiments, the ratio of oil to surfactant is approximately 1:1.45. In some embodiments, the ratio of oil to surfactant is approximately 1:1.5. In some embodiments, the ratio of oil to surfactant is approximately 1:1.55. In some embodiments, the ratio of oil to surfactant is approximately 1:1.6. In some embodiments, the ratio of oil to surfactant is approximately 1:1.65. In some embodiments, the ratio of oil to surfactant is approximately 1:1.7. In some embodiments, the ratio of oil to surfactant is approximately 1:1.75. In some embodiments, the ratio of oil to surfactant is approximately 1:1.8. In some embodiments, the ratio of oil to surfactant is approximately 1:1.85. In some embodiments, the ratio of oil to surfactant is approximately 1:1.9. In some embodiments, the ratio of oil to surfactant is approximately 1:1.95. In some embodiments, the ratio of oil to surfactant is approximately 1:2. In some embodiments, the ratio of oil to surfactant is approximately 1:2.5. In some embodiments, the ratio of oil to surfactant is approximately 1:3. In some embodiments, the ratio of oil to surfactant is approximately 1:3.5. In some embodiments, the ratio of oil to surfactant is approximately 1:4. In some embodiments, the ratio of oil to surfactant is approximately 1:4.5. In some embodiments, the ratio of oil to surfactant is approximately 1:5.

In some embodiments, provided nanoparticle compositions comprise oil and surfactant at a ratio ranging between about 0.1:1 to about 2:1. In some embodiments, provided nanoparticle compositions comprise oil and surfactant at a ratio of about 0.1:1 to about 1:1. In some embodiments, provided nanoparticle compositions comprise oil and surfactant at a ratio of about 0.5:1 to about 1:1. In some embodiments, provided nanoparticle compositions comprise oil and surfactant at a ratio of about 0.1:1, about 0.15:1, about 0.2:1, about 0.25:1, about 0.3:1, about 0.35:1, about 0.4:1, about 0.45:1, about 0.5:1, about 0.5:1, about 0.55:1, about 0.6:1, about 0.65:1, about 0.7:1, about 0.75:1, about 0.8:1, about 0.85:1, about 0.9:1, about 0.95:1, or about 1:1 In some embodiments, provided nanoparticle compositions comprise oil and surfactant at a ratio of about 0.67:1.

In some embodiments, the aqueous dispersion medium (e*.g.*, water, buffer, salt solution, *etc.*) and surfactant are utilized at a ratio ranging between 0.01 and 20. In some embodiments, the aqueous dispersion medium (*e.g.*, water, buffer, salt solution, *etc.*) and surfactant are utilized at a ratio ranging between 0.1 and 20. In some embodiments, the aqueous dispersion medium (*e.g.*, water, buffer, salt solution, *etc.*) and surfactant are utilized at a ratio ranging between 0.5 and 10. In some embodiments, the aqueous dispersion medium (*e.g.*, water, buffer, salt solution, *etc.*) and surfactant are utilized at a ratio ranging between 0.5 and 1. In some embodiments, the ratio of aqueous dispersion medium (*e.g*., water, buffer, salt solution, *etc.*) to surfactant is approximately 0.01:1, approximately 0.02:1, approximately 0.03:1, approximately 0.04:1, approximately 0.05:1, approximately 0.06:1, approximately 0.07:1, approximately 0.08:1, approximately 0.0:1, approximately 0.1:1, approximately 0.2:1, approximately 0.3:1, approximately 0.4:1, approximately 0.5:1, approximately 1:1, approximately 2:1, approximately 3:1, approximately 4:1, approximately 5:1, approximately 6:1, approximately 7:1, approximately 8:1, approximately 9:1 or approximately 10:1. In some embodiments, the ratio of surfactant to water is approximately 0.5:1, approximately 1:1, approximately 2:1, approximately 3:1, approximately 4:1, approximately 5:1, approximately 6:1, approximately 7:1, approximately 8:1, approximately 9:1, approximately 10:1, approximately 11:1, approximately 12:1, approximately 13:1, approximately 14:1, approximately 15:1, approximately 16:1, approximately 17:1, approximately 18:1, approximately 19:1, or approximately 20:1. In some embodiments, aqueous dispersion medium (*e.g.*, water, buffer, salt solution, *etc.*) and surfactant are utilized at a ratio ranging between 0.5 and 2. In some embodiments, the ratio of aqueous dispersion medium (*e.g*., water, buffer, salt solution, *etc.*) to surfactant is approximately 0.5:1, approximately 1:1, or approximately 2:1. In some embodiments, the ratio of surfactant to aqueous dispersion medium (*e.g.*, water, buffer, salt solution, *etc.*) is approximately 0.5:1, approximately 1:1, or approximately 2:1. In some embodiments, the ratio of aqueous dispersion medium (*e.g*., water, buffer, salt solution, *etc.*) to surfactant is approximately 1:1. In some embodiments, compositions utilizing such ratios of aqueous dispersion medium (*e.g*., water, buffer, salt solution, *etc.*) to surfactant comprise water-in-oil emulsions.

In some embodiments, provided nanoparticle compositions comprise aqueous dispersion medium and surfactant at a ratio ranging between about 8:1 and about 9:1. In some embodiments, provided nanoparticle compositions comprise aqueous dispersion medium and surfactant at a ratio of about 8:1, about 8.1:1, about 8.2:1, about 8.3:1, about 8.4:1, about 8.5:1, about 8.6:1, about 8.7:1, about 8.8:1, about 8.9:1, about 9:1, *etc.* In some embodiments, provided nanoparticle compositions comprise aqueous dispersion medium and surfactant at a ratio of about 8.7:1. In some embodiments, provided nanoparticle compositions comprise aqueous dispersion medium and surfactant at a ratio of about 8.8:1.

In some embodiments, provided nanoparticle compositions comprise aqueous dispersion medium and surfactant at a ratio ranging between about 12:1 and about 14:1. In some embodiments, provided nanoparticle compositions comprise aqueous dispersion medium and surfactant at a ratio of about 12:1, about 12.1:1, about 12.2:1, about 12.3:1, about 12.4:1, about 12.5:1, about 12.6:1, about 12.7:1, about 12.8:1, about 12.9:1, about 13:1, about 13.1:1, about 13.2:1, about 13.3:1, about 13.4:1, about 13.5:1, about 13.6:1, about 13.7:1, about 13.8:1, about 13.9:1, about 14:1, *etc.* In some embodiments, provided nanoparticle compositions comprise aqueous dispersion medium and surfactant at a ratio of about 13.1:1.

In some embodiments, the percent of oil in the premix ranges between 0% and 50%. In some embodiments, the percent of oil in the premix ranges between 0% and 40%. In some embodiments, the percent of oil in the premix ranges between 0% and 30%. In some embodiments, the percent of oil in the premix ranges between 0% and 20%. In some embodiments, the percent of oil in the premix ranges between 0% and 10%. In some embodiments, the percent of oil in the premix ranges between 0% and 5%. In some embodiments, the percent of oil in the premix ranges between 5% and 10%, between 10% and 15%, between 15% and 20%, between 20% and 25%, between 25% and 30%, between 35% and 40%, or between 45% and 50%. In some embodiments, the percent of oil in the premix ranges between 10% and 20%, between 10% and 30%, between 10% and 40%, or between 10% and 50%. In some embodiments, the percent of oil in the premix ranges between 20% and 30%, between 20% and 40%, between 20% and 50%. In some embodiments, the percent of oil in the premix ranges between 30% and 40% or between 30% and 50%. In some embodiments, the percent of oil in the premix ranges between 40% and 50%.

In some embodiments the percent of oil in the premix is approximately 1%, approximately 2%, approximately 3%, approximately 4%, approximately 5%, approximately 6%, approximately 7%, approximately 9%, approximately 10%, approximately 11%, approximately 12%, approximately 13%, approximately 14%, approximately 15%, approximately 16%, approximately 17%, approximately 18%, approximately 19%, approximately 20%, approximately 21%, approximately 22%, approximately 23%, approximately 24%, approximately 25%, approximately 26%, approximately 27%, approximately 28%, approximately 29%, approximately 30%, approximately 31%, approximately 32%, approximately 33%, approximately 34%, approximately 35%, approximately 36%, approximately 37%, approximately 38%, approximately 39%, approximately 40%, approximately 41%, approximately 42%, approximately 43%, approximately 44%, approximately 45%, approximately 46%, approximately 47%, approximately 48%, approximately 49%, or approximately 50%. In some embodiments the percent of oil is approximately 10%. In some embodiments the percent of oil is approximately 9%. In some embodiments the percent of oil is approximately 8%. In some embodiments the percent of oil is approximately 7%. In some embodiments the percent of oil is approximately 6%. In some embodiments the percent of oil is approximately 5%. In some embodiments the percent of oil is approximately 4%. In some embodiments the percent of oil is approximately 3%. In some embodiments the percent of oil is approximately 2%. In some embodiments the percent of oil is approximately 1%.

In some embodiments, the percent of oil in provided nanoparticle compositions ranges between about 5% and about 8%. In some embodiments, the percent of oil in provided nanoparticle compositions is about 5%, about 5.1%, about 5.2%, about 5.3%, about 5.4%, about 5.5%, about 5.6%, about 5.7%, about 5.8%, about 5.9%, about 6%, about 6.1%, about 6.2%, about 6.3%, about 6.4%, about 6.5%, about 6.6%, about 6.7%, about 6.8%, about 6.9%, about 7%, about 7.1%, about 7.2%, about 7.3%, about 7.4%, about 7.5%, about 7.6%, about 7.7%, about 7.8%, about 7.9%, or about 8%. In some embodiments, the percent of oil in provided nanoparticle compositions is about 6.3%. In some embodiments, the percent of oil in provided nanoparticle compositions is about 6.4%.

The percent of aqueous dispersion medium (*e.g.*, water, buffer, salt solution, *etc.*) in the premix can range from 0% to 99%, from 10% to 99%, from 25% to 99%, from 50% to 99%, or from 75% to 99%. In some embodiments, the percent of aqueous dispersion medium (*e.g*., water, buffer, salt solution, *etc.*) in the premix can range from 0% to 75%, from 0% to 50%, from 0% to 25%, or from 0% to 10%. In some embodiments, the percent of aqueous dispersion medium (*e.g.*, water, buffer, salt solution, *etc.*) in the premix ranges between 0% and 30%. In some embodiments the percent of aqueous dispersion medium (e.g., water, buffer, salt solution, *etc*.)is approximately 1%, approximately 2%, approximately 3%, approximately 4%, approximately 5%, approximately 6%, approximately 7%, approximately 9%, approximately 10%, approximately 11%, approximately 12%, approximately 13%, approximately 14%, approximately 15%, approximately 16%, approximately 17%, approximately 18%, approximately 19%, approximately 20%, approximately 21%, approximately 22%, approximately 23%, approximately 24%, approximately 25%, approximately 26%, approximately 27%, approximately 28%, approximately 29%, approximately 30%, approximately 35%, approximately 40%, approximately 45%, approximately 50%, approximately 55%, approximately 60%, approximately 65%, approximately 70%, approximately 71%, approximately 72%, approximately 73%, approximately 74%, approximately 75%, approximately 76%, approximately 77%, approximately 78%, approximately 79%, approximately 80%, approximately 81%, approximately 82%, approximately 83%, approximately 84%, approximately 85%, approximately 86%, approximately 87%, approximately 88%, approximately 89%, approximately 90%, approximately 91%, approximately 92%, approximately 93%, approximately 94%, approximately 95%, approximately 96%, approximately 97%, approximately 98%, or approximately 99%,. In some embodiments the percent of water is approximately 83%. In some embodiments the percent of water is approximately 9%. In some embodiments the percent of water is approximately 5%.

In some embodiments, the percent of aqueous dispersion medium in provided nanoparticle compositions ranges between about 80% and about 85%. In some embodiments, the percent of aqueous dispersion medium in provided nanoparticle compositions is about 80, about 80.5%, about 81%, about 81.5%, about 82%, about 82.5%, about 83%, about 83.5%, about 84%, about 84.5%, or about 85%. In some embodiments, the percent of aqueous dispersion medium in provided nanoparticle compositions is about 83.5%. In some embodiments, the percent of oil in provided nanoparticle compositions is about 84%.

In some embodiments, the percent of surfactant in the premix ranges between 0% -30%. In some embodiments the percent of surfactant in the premix is approximately 1%, approximately 2%, approximately 3%, approximately 4%, approximately 5%, approximately 6%, approximately 7%, approximately 9%, approximately 10%, approximately 11%, approximately 12%, approximately 13%, approximately 14%, approximately 15%, approximately 16%, approximately 17%, approximately 18%, approximately 19%, approximately 20%, approximately 21%, approximately 22%, approximately 23%, approximately 24%, approximately 25%, approximately 26%, approximately 27%, approximately 28%, approximately 29%, approximately 30%, approximately 31%, approximately 32%, approximately 33%, approximately 34%, approximately 35%, approximately 36%, approximately 37%, approximately 38%, approximately 39%, approximately 40%, approximately 41%, approximately 42%, approximately 43%, approximately 44%, approximately 45%, approximately 46%, approximately 47%, approximately 48%, approximately 49%, or approximately 50%. In some embodiments the percent of surfactant is approximately 10%. In some embodiments the percent of surfactant is approximately 9%. In some embodiments the percent of surfactant is approximately 8%. In some embodiments the percent of surfactant is approximately 7%. In some embodiments the percent of surfactant is approximately 6%. In some embodiments the percent of surfactant is approximately 5%.

In some embodiments, the percent of surfactant in provided nanoparticle compositions ranges between about 8% and about 11%. In some embodiments, the percent of surfactant in provided nanoparticle compositions is about 8%, about 8.1%, about 8.2%, about 8.3%, about 8.4%, about 8.5%, about 8.6%, about 8.7%, about 8.8%, about 8.9%, about 9%, about 9.1%, about 9.2%, about 9.3%, about 9.4%, about 9.5%, about 9.6%, about 9.7%, about 9.8%, about 9.9%, about 10%, about 10.1%, about 10.2%, about 10.3%, about 10.4%, about 10.5%, about 10.6%, about 10.7%, about 10.8%, about 10.9%, or about 11%. In some embodiments, the percent of surfactant in provided nanoparticle compositions is about 9.5%. In some embodiments, the percent of surfactant in provided nanoparticle compositions is about 9.6%.

In some embodiments, the percent of excipient in provided nanoparticle compositions ranges between about 0.1% and about 1%. In some embodiments, the percent of excipient in provided nanoparticle compositions is about 0.1%, about 0.2%, about 0.3%, about 0.4%, about 0.5%, about 0.6%, about 0.7%, about 0.8%, about 0.9%, or about 1%. In some embodiments, the percent of excipient in provided nanoparticle compositions is about 0.4%.

In some embodiments, nanoparticle compositions comprise and/or consist essentially of the following proportions of ingredients:

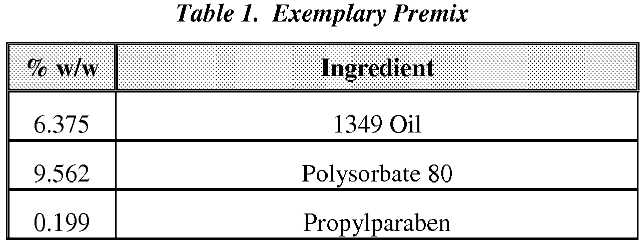

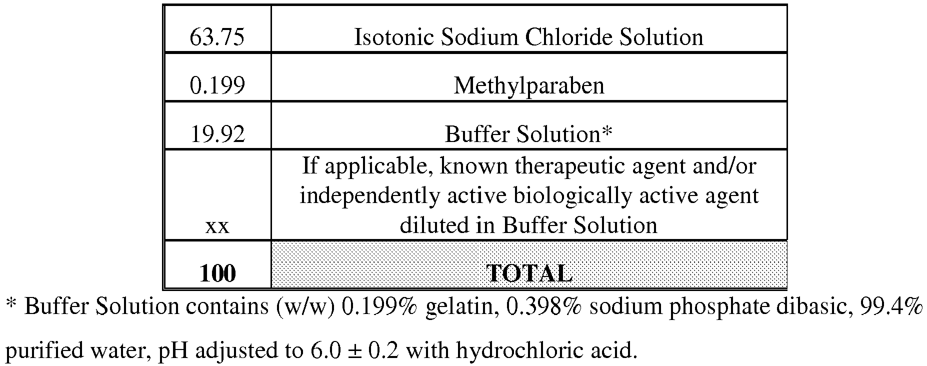

In some embodiments, a provided composition does not contain more than one oil. In some embodiments, a provided composition may comprise two or more oils (*e.g*., 2, 3, 4, 5, or more oils). In some embodiments, a provided composition does not contain more than one surfactant. In some embodiments, a provided composition may comprise two or more surfactants (*e.g*., 2, 3, 4, 5, or more surfactants).

In some embodiments, a provided composition consists essentially of an aqueous dispersion medium (*e.g.*, water, buffer, salt solution, *etc.*), an oil, and a surfactant. In some embodiments, a provided composition consists essentially of an aqueous dispersion medium (*e.g.*, water, buffer, salt solution, *etc.*), an oil, and a surfactant, and at least one substance used to produce and/or preserve the composition (*e.g.*, proteins, salts, *etc.*)*.*

In some embodiments, a provided composition consists of an aqueous dispersion medium (*e.g.*, water, buffer, salt solution, *etc.*), an oil, and a surfactant. In some embodiments, a provided composition consists of an aqueous dispersion medium (*e.g*., water, buffer, salt solution, *etc.*), an oil, a surfactant, and at least one substance used to produce and/or preserve the composition (*e.g.*, proteins, salts, *etc.*). In some embodiments, a provided composition consists of an aqueous dispersion medium (*e.g.*, water, buffer, salt solution, *etc.*), one or more oils, and one or more surfactants. In some embodiments, a provided composition consists of an aqueous dispersion medium (*e.g.*, water, buffer, salt solution, *etc.*), one or more oils, one or more surfactants, and at least one substance used to produce and/or preserve the composition (*e.g.*, proteins, salts, *etc.*)*.* In some embodiments, a provided composition does not contain an added preservative. In some embodiments, a provided composition does not contain parabens, such as methyparaben and propylparaben.

In some embodiments, any of the nanoparticle compositions described herein further comprise a known therapeutic agent and/or independently active biologically active agent (*e.g.*, a therapeutically effective amount of a known therapeutic agent and/or independently active biologically active agent). In some embodiments, any of the nanoparticle compositions described herein do not comprise a known therapeutic agent and/or independently active biologically active agent (*e.g*., a therapeutically effective amount of a known therapeutic agent and/or independently active biologically active agent).

In some embodiments, provided nanoparticle compositions do not require toxic solvents. In some embodiments, conventional strategies for inducing formation of nanoparticles in a composition utilize toxic (typically organic) solvents. In some embodiments, provided nanoparticle compositions do not require polymers. In some embodiments, strategies for preparing compositions that contain nanoparticle structures require polymers.

In some embodiments, provided nanoparticle compositions have better tissue absorption and/or better biocompatibility than other nanoparticle compositions. For example, in some embodiments, provided nanoparticle compositions have better tissue absorption and/or better biocompatibility than nanoparticle compositions that are not uniform, that utilize one or more toxic (*e.g*., organic) solvents, and/or that utilize one or more polymers.

### Provided Cream and/or Lotion Formulations

The present invention provides particular cream and/or lotion compositions that are particularly effective and/or useful in medical contexts, *e.g*., for therapeutic purposes. The present invention encompasses the recognition that certain cream and/or lotion formulations are particularly useful and/or effective for topical administration of agents to a subject in need thereof. In some embodiments, the present invention provides certain cream and/or lotion formulations.

In some embodiments, provided nanoparticle compositions are admixed with a cream and/or lotion formulation and/or a saline solution for preparation of a topical pharmaceutical composition. In some embodiments, known therapeutic agents and/or independently active biologically active agents (*e.g*., not associated with a nanoparticle composition) are admixed with a cream and/or lotion formulation for preparation of a topical pharmaceutical composition.

In some embodiments, a cream and/or lotion formulation comprises purified water. In some embodiments, a cream and/or lotion formulation comprises methylparaben. In some embodiments, a cream and/or lotion formulation comprises mineral oil. In some embodiments, a cream and/or lotion formulation comprises isopropyl myristate. In some embodiments, a cream and/or lotion formulation comprises white petrolatum. In some embodiments, a cream and/or lotion formulation comprises emulsifying wax. In some embodiments, a cream and/or lotion formulation comprises propylparaben. In some embodiments, a cream and/or lotion formulation comprises pemulen. In some embodiments, a cream and/or lotion formulation does not comprise pemulen.

In some embodiments, a cream and/or lotion formulation comprises purified water, methylparaben, mineral oil, isopropyl myristate, white petrolatum, emulsifying wax, and propylparaben. In some embodiments, a cream and/or lotion formulation consists essentially of purified water, methylparaben, mineral oil, isopropyl myristate, white petrolatum, emulsifying wax, and propylparaben. In some embodiments, a cream and/or lotion formulation consists of purified water, methylparaben, mineral oil, isopropyl myristate, white petrolatum, emulsifying wax, and propylparaben.

In some embodiments, white petrolatum is or comprises a petrolatum agent selected from the group consisting of white petrolatum, wool wax, lanolin, Vasoliment, VASELINE® brand petroleum jelly, Saxoline, petroleum jelly, mineral jelly, mineral fat, mineral wax, paraffin jelly, yellow petrolatum, 2,6,10,15,19,23-hexamethyltetracosane, dodecahydrosqualene, perhydrosqualene, squalane, white VASELINE®, White Protopet, Ultima White, Snow White, a substance corresponding to CAS number 8009-03-8, and/or combinations thereof.

In some embodiments, a mineral oil is or comprises a mineral oil agent selected from the group consisting of paraffinic oils (*e.g*., oils based on n-alkanes), naphthenic oils (*e.g.*, oils based on cycloalkanes), aromatic oils (*e.g.*, oils based on aromatic hydrocarbons), and/or combinations thereof.

In some embodiments, an emulsifying wax is or comprises emulsifying wax NF (*e.g*., Spectrum Chemical catalog number W1026).

In some embodiments, a cream and/or lotion formulation comprises and/or consists essentially of the following proportions of ingredients:

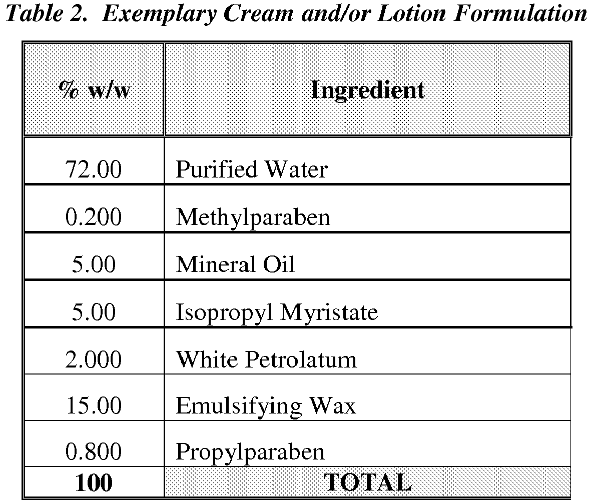

In some embodiments, a saline solution for bulk formulation comprises isotonic sodium chloride solution. In some embodiments, a saline solution for bulk formulation comprises methylparaben. In some embodiments, a saline solution for bulk formulation comprises isotonic sodium chloride solution and methylparaben. In some embodiments, a saline solution for bulk formulation consists essentially of isotonic sodium chloride solution and methylparaben. In some embodiments, a saline solution for bulk formulation consists of isotonic sodium chloride solution and methylparaben.

In some embodiments, a saline solution for bulk formulation comprises and/or consists essentially of the following proportions of ingredients:

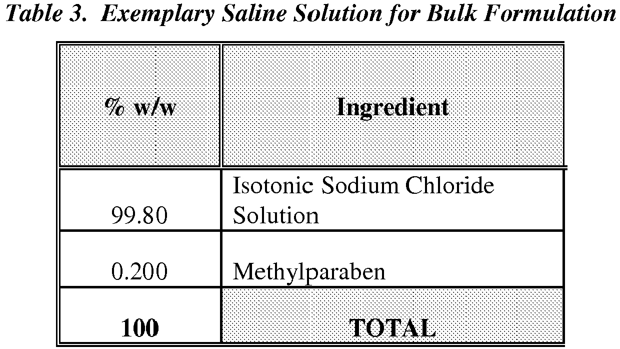

### Nanoparticle Compositions

Nanoparticle compositions are useful in a variety of contexts, and have proven to be particularly useful and/or effective in the context of medical applications, including administering therapeutic agents to patients in need thereof. Nanoparticle compositions have proven to be particularly useful and/or effective in the context of topical administration of therapeutic agents (see, *e.g*., PCT patent application number PCT US06/46236, filed December 1, 2006, published as WO 08/045107 on April 17, 2008, and entitled "BOTULINUM NANOEMULSIONS; in PCT patent application number PCT US07/86018, filed November 30, 2007, published as WO 08/070538 on June 12, 2008, and entitled "AMPHIPHILIC ENTITY NANOPARTICLES"; and/or in PCT patent application number PCT US09/48972, filed June 26, 2009, published as WO 09/158687 on December 30, 2009, and entitled "DERMAL DELIVERY"; the contents of all of which are incorporated herein by reference).

As described herein, the present invention provides, among other things, novel new and improved nanoparticle compositions. In some embodiments, provided nanoparticle compositions have particular components, and/or relative amounts of components, as described herein. In some embodiments, provided nanoparticle compositions have particular structural and/or functional attributes that distinguish and/or define them. In some embodiments, exemplary attributes (*e.g*., physical, structural, and/or functional attributes) that have been associated with nanoparticle compositions in general are described in the following paragraphs. In some embodiments, provided nanoparticle compositions have one or more of these attributes. In some embodiments, provided nanoparticle compositions do not have any of these attributes.

In general, a nanoparticle composition is any composition that includes at least one nanoparticle. In some embodiments, nanoparticle compositions comprise at least one known therapeutic agent and/or an independently active biologically active agent (*e.g*., botulinum toxin). A known therapeutic agent and/or an independently active biologically active agent may be encapsulated or completely surrounded by one or more nanoparticles; associated with the nanoparticle interface; and/or adsorbed to the outer surface of one or more nanoparticles. A known therapeutic agent and/or an independently active biologically active agent may or may not be covalently linked to the nanoparticles and/or nanoparticle compositions; a known therapeutic agent and/or an independently active biologically active agent may or may not be attached to nanoparticles and/or nanoparticle compositions by adsorption forces. In some embodiments, nanoparticle compositions comprise empty nanoparticles (*e.g.*, nanoparticles not containing any known therapeutic agents and/or independently active biologically active agents).

In some embodiments, nanoparticle compositions are stable. In some embodiments, nanoparticle compositions are uniform. For example, in some embodiments, the difference between the minimum diameter and maximum diameter of the nanoparticles in a nanoparticle composition does not exceed approximately 600 nm, approximately 550 nm, approximately 500 nm, approximately 450 nm, approximately 400 nm, approximately 350 nm, approximately 300 nm, approximately 250 nm, approximately 200 nm, approximately 150 nm, or approximately 100 nm, approximately 90 nm, approximately 80 nm, approximately 70 nm, approximately 60 nm, approximately 50 nm, or fewer nm.

In some embodiments, particles within nanoparticle compositions have diameters (*e.g.*, average and/or median diameters) that are smaller than about 1000 nm, about 600 nm, about 550 nm, about 500 nm, about 450 nm, about 400 nm, about 350 nm, about 300 nm, about 250 nm, about 200 nm, about 150 nm, about 130 nm, about 120 nm, about 115 nm, about 110 nm, about 100 nm, about 90 nm, about 80 nm, about 50 nm, or less.

In some embodiments, particles within nanoparticle compositions have diameters (*e.g.*, average and/or median diameters) within the range of about 10 nm and about 600 nm. In some embodiments, particles within nanoparticle compositions have diameters (*e.g.*, average and/or median diameters) within the range of about 10 nm to about 300 nm, about 10 nm to about 200 nm, about 10 nm to about 150 nm, about 10 nm to about 130 nm, about 10 nm to about 120 nm, about 10 nm to about 115 nm, about 10 nm to about 110 nm, about 10 nm to about 100 nm, or about 10 nm to about 90 nm. In some embodiments, particles within nanoparticle compositions have diameters (*e.g.*, average and/or median diameters) within the range of 1 nm to 1000 nm, 1 nm to 600 nm, 1 nm to 500 nm, 1 nm to 400 nm, 1 nm to 300 nm, 1 nm to 200 nm, 1 nm to 150 nm, 1 nm to 120 nm, 1 nm to 100 nm, 1 nm to 75 nm, 1 nm to 50 nm, or 1 nm to 25 nm. In some embodiments, particles within nanoparticle compositions have diameters (*e.g.*, average and/or median diameters) of 1 nm to 15 nm, 15 nm to 200 nm, 25 nm to 200 nm, 50 nm to 200 nm, or 75 nm to 200 nm.

In some embodiments, the total particle distribution is encompassed within the specified range of particle diameter size. In some embodiments, less than 50%, 25%, 10%, 5%, or 1% of the total particle distribution is outside of the specified range of particle diameter sizes. In some embodiments, less than 1% of the total particle distribution is outside of the specified range of particle diameter sizes. In certain embodiments, the nanoparticle composition is substantially free of particles having a diameter larger than 300 nm, 250 nm, 200 nm, 150 nm, 120 nm, 100 nm, 75 nm, 50 nm, or 25 nm. In some embodiments, less than 50%, 25%, 10%, 5%, or 1% of the total particle distribution have diameters larger than 300 nm, 250 nm, 200 nm, 150 nm, 120 nm, 100 nm, 75 nm, 50 nm, or 25 nm.

In some embodiments, particles within nanoparticle compositions have an average particle size that is under about 600 nm, about 550 nm, about 500 nm, about 450 nm, about 400 nm, about 350 nm, about 300 nm, about 250 nm, about 200 nm, about 150 nm, about 130 nm, about 120 nm, about 115 nm, about 110 nm, about 100 nm, about 90 nm, or about 50 nm. In some embodiments, the average particle size is within the range of about 10 nm and about 300 nm, about 50 nm and about 250, about 60 nm and about 200 nm, about 65 nm and about 150 nm, or about 70 nm and about 130 nm. In some embodiments, the average particle size is about 80 nm and about 110 nm. In some embodiments, the average particle size is about 90 nm and about 100 nm.

In some embodiments, a majority of the particles within nanoparticle compositions have diameters below a specified size or within a specified range. In some embodiments, the majority is more than 50%, 60%, 70%, 75%, 80%, 85%, 90%, 95%, 96%, 97%, 98%, 99%, 99.5%, 99.6%, 99.7%, 99.8%, 99.9% or more of the particles in the composition.

In some embodiments, nanoparticle compositions are substantially free of particles having a diameter in excess of 600 nm. Specifically, in some embodiments, fewer than 50% of the nanoparticles in nanoparticle compositions have a diameter in excess of 600 nm. In some embodiments, fewer than 25% of the particles have a diameter in excess of 600 nm. In some embodiments, fewer than 20%, 19%, 18%, 17%, 16%, 15%, 14%, 13%, 12%, 10%, 9%, 8%, 7%, 6%, 5%, 4%, 3%, 2%, 1%, 0.5% or less of the particles have a diameter in excess of 600 nm. Furthermore, in some embodiments, the nanoparticles in nanoparticle compositions have diameters within the range of 10 nm and 600 nm.

In some embodiments, nanoparticle compositions are substantially free of particles having a diameter in excess of 500 nm. Specifically, in some embodiments, fewer than 50% of the nanoparticles in nanoparticle compositions have a diameter in excess of 500 nm. In some embodiments, fewer than 25% of the particles have a diameter in excess of 500 nm. In some embodiments, fewer than 20%, 19%, 18%, 17%, 16%, 15%, 14%, 13%, 12%, 10%, 9%, 8%, 7%, 6%, 5%, 4%, 3%, 2%, 1%, 0.5% or less of the particles have a diameter in excess of 500 nm. Furthermore, in some embodiments, the nanoparticles in nanoparticle compositions have diameters within the range of 10 nm and 500 nm.

In some embodiments, nanoparticle compositions are substantially free of particles having a diameter in excess of 400 nm. Specifically, in some embodiments, fewer than 50% of the nanoparticles in nanoparticle compositions have a diameter in excess of 400 nm. In some embodiments, fewer than 25% of the particles have a diameter in excess of 400 nm. In some embodiments, fewer than 20%, 19%, 18%, 17%, 16%, 15%, 14%, 13%, 12%, 10%, 9%, 8%, 7%, 6%, 5%, 4%, 3%, 2%, 1%, 0.5% or less of the particles have a diameter in excess of 400 nm. Furthermore, in some embodiments, the nanoparticles in nanoparticle compositions have diameters within the range of 10 nm and 400 nm.

In some embodiments, nanoparticle compositions are substantially free of particles having a diameter in excess of 300 nm. Specifically, in some embodiments, fewer than 50%, of the nanoparticles in nanoparticle compositions have a diameter in excess of 300 nm. In some embodiments, fewer than 25% of the particles have a diameter in excess of 300 nm. In some embodiments, fewer than 20%, 19%, 18%, 17%, 16%, 15%, 14%, 13%, 12%, 10%, 9%, 8%, 7%, 6%, 5%, 4%, 3%, 2%, 1%, 0.5% or less of the particles have a diameter in excess of 300 nm. Furthermore, in some embodiments, the nanoparticles in nanoparticle compositions have diameters within the range of 10 nm and 300 nm.

In some embodiments, nanoparticle compositions are substantially free of particles having a diameter in excess of 200 nm. Specifically, in some embodiments, fewer than 50%, of the nanoparticles in nanoparticle compositions have a diameter in excess of 200 nm. In some embodiments, fewer than 25% of the particles have a diameter in excess of 200 nm. In some embodiments, fewer than 20%, 19%, 18%, 17%, 16%, 15%, 14%, 13%, 12%, 10%, 9%, 8%, 7%, 6%, 5%, 4%, 3%, 2%, 1%, 0.5% or less of the particles have a diameter in excess of 200 nm. Furthermore, in some embodiments, the nanoparticles in nanoparticle compositions have diameters within the range of 10 nm and 200 nm.

In some embodiments, provided compositions are substantially free of particles having a diameter in excess of 150 nm. Specifically, in some embodiments, fewer than 50% of the nanoparticles in provided compositions have a diameter in excess of 150 nm. In some embodiments, fewer than 25% of the particles have a diameter in excess of 150 nm. In some embodiments, fewer than 20%, 19%, 18%, 17%, 16%, 15%, 14%, 13%, 12%, 10%, 9%, 8%, 7%, 6%, 5%, 4%, 3%, 2%, 1%, 0.5% or less of the particles have a diameter in excess of 150 nm. Furthermore, in some embodiments, the nanoparticles in provided compositions have diameters within the range of 10 nm and 150 nm.

In some embodiments, nanoparticle compositions are substantially free of particles having a diameter in excess of 120 nm. Specifically, in some embodiments, fewer than 50%, of the nanoparticles in nanoparticle compositions have a diameter in excess of 120 nm. In some embodiments, fewer than 25% of the particles have a diameter in excess of 120 nm. In some embodiments, fewer than 20%, 19%, 18%, 17%, 16%, 15%, 14%, 13%, 12%, 10%, 9%, 8%, 7%, 6%, 5%, 4%, 3%, 2%, 1%, 0.5% or less of the particles have a diameter in excess of 120 nm. Furthermore, in some embodiments, the nanoparticles in nanoparticle compositions have diameters within the range of 10 nm and 120 nm.

In some embodiments, a majority of particles in a provided composition have diameters (*e.g.*, average and/or median diameters) between 10 nm and 150 nm. In some embodiments, a majority of particles in a provided composition have diameters (*e.g*., average and/or median diameters) between 10 nm and 120 nm. In some embodiments, a majority of particles in a provided composition have diameters (*e.g.*, average and/or median diameters) between 20 nm and 120 nm. In some embodiments, a majority of particles in a provided composition have diameters (*e.g*., average and/or median diameters) between 20 nm and 110 nm. In some embodiments, a majority of particles in a provided composition have diameters (*e.g.*, average and/or median diameters) between 20 nm and 100 nm. In some embodiments, a majority of particles in a provided composition have diameters (*e.g.*, average and/or median diameters) between 20 nm and 90 nm. In some embodiments, a majority of particles in a provided composition have diameters (*e.g.*, average and/or median diameters) between 20 nm and 80 nm. In some embodiments, a majority of particles in a provided composition have diameters (*e.g.*, average and/or median diameters) between 20 nm and 70 nm. In some embodiments, a majority of particles in a provided composition have diameters (*e.g.*, average and/or median diameters) between 20 nm and 60 nm. In some embodiments, a majority of particles in a provided composition have diameters (*e.g*., average and/or median diameters) between 20 nm and 50 nm. In some embodiments, a majority of particles in a provided composition have diameters (*e.g.*, average and/or median diameters) between 20 nm and 40 nm. In some embodiments, a majority of particles in a provided composition have diameters (*e.g.*, average and/or median diameters) between 20 nm and 30 nm.

In some embodiments, a majority of nanoparticles in a nanoparticle composition have diameters (*e.g.*, average and/or median diameters) between 10 nm and 120 nm. In some embodiments, a majority of nanoparticles in a nanoparticle composition have diameters (*e.g.*, average and/or median diameters) between 20 nm and 120 nm. In some embodiments, a majority of nanoparticles in a nanoparticle composition have diameters (*e.g.*, average and/or median diameters) between 20 nm and 110 nm. In some embodiments, a majority of nanoparticles in a nanoparticle composition have diameters (*e.g*., average and/or median diameters) between 20 nm and 100 nm. In some embodiments, a majority of nanoparticles in a nanoparticle composition have diameters between 20 nm and 90 nm. In some embodiments, a majority of nanoparticles in a nanoparticle composition have diameters (*e.g.*, average and/or median diameters) between 20 nm and 80 nm. In some embodiments, a majority of nanoparticles in a nanoparticle composition have diameters (*e.g.*, average and/or median diameters) between 20 nm and 70 nm. In some embodiments, a majority of nanoparticles in a nanoparticle composition have diameters (*e.g*., average and/or median diameters) between 20 nm and 60 nm. In some embodiments, a majority of nanoparticles in a nanoparticle composition have diameters (*e.g*., average and/or median diameters) between 20 nm and 50 nm. In some embodiments, a majority of nanoparticles in a nanoparticle composition have diameters (*e.g.*, average and/or median diameters) between 20 nm and 40 nm. In some embodiments, a majority of nanoparticles in a nanoparticle composition have diameters (*e.g*., average and/or median diameters) between 20 nm and 30 nm.

In some embodiments, about 50% of nanoparticles in a nanoparticle composition have diameters (*e.g.*, average and/or median diameters) between 10 nm and 40 nm. In some embodiments, about 90% of nanoparticles in a nanoparticle composition have diameters (*e.g.*, average and/or median diameters) between 10 nm and 80 nm. In some embodiments, about 90% of nanoparticles in a nanoparticle composition have diameters (*e.g.*, average and/or median diameters) between 10 nm and 90 nm. In some embodiments, about 95% of nanoparticles in a nanoparticle composition have diameters (*e.g*., average and/or median diameters) between 10 nm and 110 nm. In some embodiments, about 95% of nanoparticles in a nanoparticle composition have diameters (*e.g*., average and/or median diameters) between 10 nm and 120 nm. In some embodiments, about 95% of particles in a provided composition have diameters (*e.g.*, average and/or median diameters) between 10 nm and 150 nm.

In some embodiments, about 50% of the aggregate volume of all nanoparticles in a nanoparticle composition comprises or consists of nanoparticles having diameters between 10 nm and 40 nm. In some embodiments, about 90% of the aggregate volume of all nanoparticles in a nanoparticle composition comprises or consists of nanoparticles having diameters between 10 nm and 80 nm. In some embodiments, about 95% of the aggregate volume of all nanoparticles in a nanoparticle composition comprises or consists of nanoparticles having diameters between 10 nm and 110 nm. In some embodiments, about 95% of the aggregate volume of all nanoparticles in a nanoparticle composition comprises or consists of nanoparticles having diameters between 10 nm and 120 nm. In some embodiments, about 95% of the aggregate volume of all particles in a provided composition comprises or consists of nanoparticles having diameters between 10 nm and 150 nm.

Zeta potential is a measurement of the electric potential at a shear plane. A shear plane is an imaginary surface separating a thin layer of liquid bound to a solid surface (*e.g.*, nanoparticle surface) and showing elastic behavior from the rest of liquid (*e.g.*, liquid dispersion medium) showing normal viscous behavior. In some embodiments, nanoparticles have a zeta potential ranging between -80 mV and +80 mV. In some embodiments, nanoparticles have a zeta potential ranging between -50 mV and +50 mV. In some embodiments, nanoparticles have a zeta potential ranging between -25 mV and +25 mV. In some embodiments, nanoparticles have a zeta potential ranging between n -10 mV and +10 mV. In some embodiments, nanoparticles have a zeta potential of about -80 mV, about -70 mV, about -60 mV, about 50 mV, about -40 mV, about -30 mV, about -25 mV, about -20 mV, about -15 mV, about -10 mV, or about -5 mV. In some embodiments, nanoparticles have a zeta potential of about +50 mV, about +40 mV, about +30 mV, about +25 mV, about +20 mV, about +15 mV, about +10 mV, or about +5 mV. In some embodiments, nanoparticles have a zeta potential that is about 0 mV.

In some embodiments, nanoparticles have a zeta potential that is about -5 mV to about -80 mV. In some embodiments, nanoparticles have a zeta potential that is about -5 mV to about -70 mV. In some embodiments, nanoparticles have a zeta potential that is about -5 mV to about -60 mV. In some embodiments, nanoparticles have a zeta potential that is about -5 mV to about -50 mV. In some embodiments, nanoparticles have a zeta potential that is about -5 mV to about -40 mV. In some embodiments, nanoparticles have a zeta potential that is about -5 mV to about -30 mV. In some embodiments, nanoparticles have a zeta potential that is about -5 mV to about -20 mV.

In some embodiments, nanoparticles have a zeta potential that is about -10 mV to about -15 mV. In some embodiments, nanoparticles have a zeta potential that is about -10 mV to about -80 mV. In some embodiments, nanoparticles have a zeta potential that is about -10 mV to about -70 mV. In some embodiments, nanoparticles have a zeta potential that is about -10 mV to about -60 mV. In some embodiments, nanoparticles have a zeta potential that is about -10 mV to about -50 mV. In some embodiments, nanoparticles have a zeta potential that is about -10 mV to about -40 mV. In some embodiments, nanoparticles have a zeta potential that is about -10 mV to about -30 mV. In some embodiments, nanoparticles have a zeta potential that is about -10 mV to about -20 mV.

In some embodiments, nanoparticles have a zeta potential that is about -80 mV to about -70 mV. In some embodiments, nanoparticles have a zeta potential that is about -70 mV to about -60 mV. In some embodiments, nanoparticles have a zeta potential that is about -60 mV to about -50 mV. In some embodiments, nanoparticles have a zeta potential that is about -50 mV to about -40 mV. In some embodiments, nanoparticles have a zeta potential that is about -40 mV to about -30 mV. In some embodiments, nanoparticles have a zeta potential that is about -30 mV to about -20 mV. In some embodiments, nanoparticles have a zeta potential that is about -20 mV to about -10 mV. In some embodiments, nanoparticles have a zeta potential that is about -10 mV to about 0 mV.

In some embodiments, nanoparticles have a zeta potential that is about -15 mV to about -20 mV. In some embodiments, nanoparticles have a zeta potential that is about -5 mV, about -6 mV, about -7 mV, about -8 mV, about -9 mV, -10 mV, about -11 mV, about - 12 mV, about -13 mV, about -14 mV, about -15 mV, about 16 mV, about -17 mV, about -18 mV, about -19 mV, or about -20 mV.

In some embodiments, nanoparticle compositions are or comprise emulsions or dispersions. In some embodiments, nanoparticle compositions are "oil-in-water" dispersions (*i.e.*, dispersions in which oily particles are dispersed within an aqueous dispersion medium); in some embodiments, nanoparticle compositions are "water-in-oil" dispersions (*i.e.*, dispersions in which aqueous particles are dispersed within an oily dispersion medium).

In some embodiments, provided compositions do not require toxic solvents. By contrast, many conventional strategies for inducing formation of nanoparticles in a composition utilize toxic (typically organic) solvents. In some embodiments, provided compositions do not require polymers. By contrast, many conventional strategies for preparing compositions that contain nanoparticle structures require polymers.

In some embodiments, provided compositions have better tissue absorption and/or better biocompatibility than other nanoparticle compositions. For example, in some embodiments, provided compositions have better tissue absorption and/or better biocompatibility than nanoparticle compositions that are not uniform, that utilize one or more toxic (*e.g.*, organic) solvents, and/or that utilize one or more polymers.

In some embodiments, nanoparticle compositions are stable. In some embodiments, a stable nanoparticle composition is one for which the average particle size, the maximum particle size, the range of particle sizes, and/or the distribution of particle sizes (*i.e.*, the percentage of particles above a designated size and/or outside a designated range of sizes) is maintained for a period of time. In some embodiments, the period of time is at least about one hour; in some embodiments the period of time is about 5 hours, about 10 hours, about one (1) day, about one (1) week, about two (2) weeks, about one (1) month, about two (2) months, about three (3) months, about four (4) months, about five (5) months, about six (6) months, about eight (8) months, about ten (10) months, about twelve (12) months, about twenty-four (24) months, or longer. In some embodiments, the period of time is within the range of about one (1) day to about twenty-four (24) months, about two (2) weeks to about twelve (12) months, about two (2) months to about five (5) months, *etc.* For example, if a population of nanoemulsion particles is subjected to prolonged storage, temperature changes, and/or pH changes and a majority of the nanoparticles in the population maintain a diameter within a stated range (*i.e.*, for example, between approximately 10 nm and about 120 nm), the nanoparticle composition is stable. For some such populations, a majority is more than about 50%, about 60%, about 70%, about 80%, about 90%, about 95%, about 96%, about 97%, about 98%, about 99%, about 99.5%, about 99.6%, about 99.7%, about 99.8%, about 99.9%, or more than about 99.9% pure. In some embodiments, where a nanoparticle composition comprises at least one known therapeutic agent and/or an independently active biologically active agent, the nanoparticle composition is considered stable if the concentration of the known therapeutic agent and/or an independently active biologically active agent (*e.g.*, botulinum toxin) is maintained in the composition over the designated period of time under a designated set of conditions.

### Methods of Making Nanoparticle Compositions

In general, nanoparticle compositions (including provided nanoparticle compositions) may be prepared by a variety of methods. In some embodiments, nanoparticle compositions are prepared by chemical means (*e.g.*, via liquid phase chemistry, thermolysis, *etc.*). However, chemical means often require toxic (typically organic) solvents; in some embodiments, nanoparticle compositions are prepared in accordance with the present invention without utilizing such solvents.

To give but a few particular examples, exemplary methods known to be useful for preparing nanoparticle compositions are described below. In some embodiments, provided nanoparticle compositions are prepared using one or more of these methods. In some embodiments, provided nanoparticle compositions are not prepared using these methods.

### High Shear Force

In some embodiments, nanoparticle compositions self-assemble from a collection of combined components. In some embodiments, nanoparticle compositions are prepared by subjecting a combination of components (*i.e.*, a "premix") to high shear force. As used herein, the term "shear force" refers to a force that is parallel or tangential to the face of a material, as opposed to a force that is perpendicular to the face of a material. In some embodiments, high shear force is applied by high pressure, by cavitation, by homogenization, and/or by microfluidization. In some embodiments, combined nanoparticle-forming components are agitated, stirred, or otherwise mixed. In some such embodiments, the components are subjected to high shear force after having been mixed. In some embodiments, mixing may be performed for a period of time such as, for example, about 1 minute, about 3 minutes, about 5 minutes, about 10 minutes, about 15 minutes, about 30 minutes, about 45 minutes, about 1 hour, about 2 hours, about 3 hours, about 4 hours, about 5 hours, about 6 hours, about 7 hours, about 8 hours, about 9 hours, about 10 hours, about 11 hours, about 12 hours, about 13 hours, about 14 hours, or about 15 hours. In some embodiments, mixing may be performed for a period of time such as, for example, more than 15 minutes, more than 30 minutes, more than 45 minutes, more than 1 hour, more than 2 hours, more than 3 hours, more than 4 hours, more than 5 hours, more than 6 hours, more than 7 hours, more than 8 hours, more than 9 hours, more than 10 hours, more than 11 hours, more than 12 hours, more than 13 hours, more than 14 hours, or more than 15 hours. In some specific embodiments, mixing may be performed for a period of time such as, for example, less than 15 minutes, less than 30 minutes, less than 45 minutes, less than 1 hour, less than 2 hours, less than 3 hours, less than 4 hours, less than 5 hours, less than 6 hours, less than 7 hours, less than 8 hours, less than 9 hours, less than 10 hours, less than 11 hours, less than 12 hours, less than 13 hours, less than 14 hours, or less than 15 hours. In some embodiments, solubilization is achieved.

Any of a variety of methods known in the art can be used to generate high shear forces. In some embodiments, cavitation is used to generate high shear forces. According to the present invention, the use of mechanical energy (*i.e.*, high shear forces) can replace or minimize any requirement to use costly and/or toxic chemical solvents; can increase the speed at which nanoparticles assemble, can increase the yield of nanoparticles generated in a particular mix of components, and/or can greatly reduce the overall cost of preparing nanoparticle compositions. Furthermore, in embodiments in which a known therapeutic agent and/or an independently active biologically active agent (*e.g.*, botulinum toxin) is incorporated into nanoparticle compositions, the use of high shear force can increase the loading capacity of the nanoparticle as compared to traditional methods of forming nanoparticles. In traditional methods, loading of agents within or on the surface of nanoparticles typically relies on diffusion of the agent to the interior and/or to the surface of the nanoparticle. According to the present invention, the use of high shear force can allow for the manufacture of smaller particles (*e.g.*, on average) and/or a more narrow distribution of particle sizes in a nanoparticle composition.

In some embodiments, high shear forces are achieved by exposure to high pressure, for example by continuous turbulent flow at high pressure, for example about 15,000 psi. In some embodiments, such high pressure is within the range of about 18,000 psi to about 26,000 psi; in some embodiments, it is within the range of about 20,000 psi to about 25,000 psi; in some embodiments, it is within the range of about 25,000 psi to about 30,000 psi; in some embodiments, it is within the range of about 30,000 psi to about 35,000 psi; in some embodiments, it is within the range of about 30,000 psi to about 40,000 psi; in some embodiments, it is within the range of about 40,000 psi to about 50,000 psi.

In some embodiments, high shear force or high pressure may be administered by cavitation or high pressure homogenization.

In some embodiments, high shear force may be administered by passage through an instrument such as, for example, a Microfluidizer® Processor (Microfluidics Corporation/MFIC Corporation) or other like device. Microfluidizer® Processors provide high pressure and a resultant high shear rate by accelerating the product through microchannels to a high velocity for size reduction to the nanoscale range. The fluid is split in two and is pushed through microchannels with typical dimensions in the order of 75 microns at high velocities (in the range of 50 m/s to 300 m/s). As the fluid exits the microchannels it forms jets which collide with jets from opposing microchannels. In the channels the fluid experiences high shear (up to 10⁷ 1/s) which is orders of magnitude higher than that of conventional technologies. Jet collisions result in mixing in submicron level. Therefore, high shear and impact are responsible for particle size reduction and mixing of multiphase fluids in the Microfluidizer® technology.

More generally, a microfluidizer may be any device that powers a single acting intensifier pump. The intensifier pump amplifies the hydraulic pressure to a selected level which, in turn, imparts that pressure to the product stream. As the pump travels through its pressure stroke, it drives the product at constant pressure through the interaction chamber. Within the interaction chamber are specially designed fixed-geometry microchannels through which the product stream will accelerate to high velocities, creating high shear and impact forces that can generate a uniform nanoparticle composition (*e.g*., nanoemulsion) as the high velocity product stream impinges on itself and on wear-resistant surfaces.

As the intensifier pump completes its pressure stroke, it reverses direction and draws in a new volume of product. At the end of the intake stroke, it again reverses direction and drives the product at constant pressures, thereby repeating the process.

Upon exiting the interaction chamber, the product flows through an onboard heat exchanger which regulates the product to a desired temperature. At this point, the product may be recirculated through the system for further processing or directed externally to the next step in the process (U.S. Patents 4,533,254; and 4,908,154; both of which are incorporated herein by reference).

In some embodiments, a sample is "microfluidized" through exposure to high shear forces for a period of time less than about 10 minutes. In some embodiments, the period of time is less than about 9, about 8, about 7, about 6, about 5, about 4, about 3, about 2, or about 1 minute(s). In some embodiments, the period of time is within the range of about 1 to about 2 minutes or less; in some embodiments, the period of time is about 30 seconds.

In some embodiments, a sample is "microfluidized" through a single exposure to high shear forces; such embodiments are referred to herein as "single pass" microfluidization.

### Premix Composition

As is known in the nanoparticle field, subjecting a premix to high shear forces can generate a nanoparticle composition, and in particular can generate a nanoparticle composition, *e.g*., a uniform nanoparticle composition.

In some embodiments, provided nanoparticle compositions are prepared by subjecting a premix to high shear forces. In some embodiments, provided nanoparticle compositions are not prepared by subjecting a premix to high shear forces.

In general, a premix from which nanoparticle compositions may be prepared through the application of high shear force is expected to contain at least two immiscible materials, one of which will constitute the dispersion medium (*i.e.*, the liquid medium in which particles (*e.g.*, nanoparticles) are dispersed in the ultimate nanoparticle composition). An "oil-in-water" dispersion is one in which oily particles are dispersed within an aqueous dispersion medium. A "water-in-oil" dispersion is one in which aqueous particles are dispersed within an oily dispersion medium. Those of ordinary skill in the art will appreciate that a dispersion can be formed from any of a variety of two immiscible media and is not limited strictly to combinations of aqueous and oily media. The term "dispersion medium" therefore applies broadly to any of a variety of dispersion media notwithstanding that it is common to refer to "aqueous" and "oily" categories.

Thus, in some embodiments, a premix comprises an aqueous dispersion medium and an oily medium that becomes dispersed in nanoparticle form in the dispersion medium; in some embodiments, a premix comprises an oily dispersion medium and an aqueous medium that becomes dispersed in nanoparticle form in the oily dispersion medium.

Those of ordinary skill in the art will be well aware of suitable aqueous media that can be used as dispersion media or as media to be dispersed in accordance with the present invention. Representative such aqueous media include, for example, water, saline solutions (including phosphate buffered saline), water for injection, short chain alcohols, 5% dextrose, Ringer's solutions (lactated Ringer's injection, lactated Ringer's plus 5% dextrose injection, acylated Ringer's injection), Normosol-M, Isolyte E, and the like, and combinations thereof.

In some embodiments, a premix comprises an aqueous dispersion medium that comprises an isotonic sodium chloride solution. In some embodiments, a premix comprises an aqueous dispersion medium that consists essentially of an isotonic sodium chloride solution. In some embodiments, a premix comprises an aqueous dispersion medium that consists of an isotonic sodium chloride solution. In some embodiments, a premix comprises an aqueous dispersion medium that comprises gelatin. In some embodiments, a premix comprises an aqueous dispersion medium that comprises sodium phosphate. In some embodiments, a premix comprises an aqueous dispersion medium that comprises purified water. In some embodiments, a premix comprises an aqueous dispersion medium that comprises hydrochloric acid. In some embodiments, a premix comprises an aqueous dispersion medium that comprises gelatin, sodium phosphate, purified water, and hydrochloric acid. In some embodiments, a premix comprises an aqueous dispersion medium that consists essentially of gelatin, sodium phosphate, purified water, and hydrochloric acid. In some embodiments, a premix comprises an aqueous dispersion medium that consists of gelatin, sodium phosphate, purified water, and hydrochloric acid.

Those of ordinary skill in the art will also be well aware of suitable oily media that can be used as dispersion media or as media to be dispersed in accordance with the present invention. In some embodiments, oils may comprise one or more fatty acid groups or salts thereof. In some embodiments, a fatty acid group may comprise digestible, substituted or unsubstituted hydrocarbons. In some embodiments, a fatty acid group may be a C₆-C₅₀ fatty acid or salt thereof. In some embodiments, a fatty acid group may be a C₆-C₂₀ fatty acid or salt thereof. In some embodiments, a fatty acid group may be a C₆-C₁₆ fatty acid or salt thereof. In some embodiments, a fatty acid group may be a C₆-C₁₂ fatty acid or salt thereof. In some embodiments, a fatty acid group may be a C₆ fatty acid or salt thereof. In some embodiments, a fatty acid group may be a C₈ fatty acid or salt thereof. In some embodiments, a fatty acid group may be a C₁₀ fatty acid or salt thereof. In some embodiments, a fatty acid group may be a C₁₂ fatty acid or salt thereof. In some embodiments, a fatty acid group may be unsaturated. In some embodiments, a fatty acid group may be monounsaturated. In some embodiments, a fatty acid group may be polyunsaturated. In some embodiments, a double bond of an unsaturated fatty acid group may be in the *cis* conformation. In some embodiments, a double bond of an unsaturated fatty acid may be in the *trans* conformation. In some embodiments, a fatty acid group may be one or more of butyric, caproic, caprylic, capric, lauric, myristic, palmitic, stearic, arachidic, behenic, lignoceric acid, and/or combinations thereof. In some embodiments, a fatty acid group may be one or more of palmitoleic, oleic, vaccenic, linoleic, alpha-linolenic, gamma-linoleic, arachidonic, gadoleic, arachidonic, eicosapentaenoic, docosahexaenoic, erucic acid, and/or combinations thereof.

In some embodiments, a premix comprises an oily dispersion medium that comprises, consists essentially of, or consists of a medium chain triglyceride (*e.g.*, fatty acids containing 6-12 carbons atoms, such as caprylic acid, octanoic acid, capric acid, decanoic acid, lauric acid, *etc.*, which, in some embodiments, may be obtained from coconut oil or palm kernel oil or camphor tree fruit extracts). Exemplary medium chain triglycerides include monounsaturated, and/or polyunsaturated soybean oil, coconut oil, canola oil, safflower oil, olive oil, corn oil, cottonseed oil, linseed oil, safflower oil, palm oil, peanut oil, flaxseed oil, sunflower oil, rice bran oil, sesame oil, rapeseed oil, cocoa butter, almond oil, cashew oil, hazelnut oil, mongongo nut oil, acai oil, borage seed oil, evening primrose oil, carob pod oil, amaranth oil, apple seed oil, artichoke oil, avocado oil, babassu oil, ben oil, borneo tallow nut oil, cocoa butter, cocklebur oil, cohune oil, dika oil, grape seed oil, hemp oil, kapok seed oil, kenaf seed oil, lallemantia oil, marula oil, meadowfoam seed oil, mustard oil, papaya seed oil, perilla seed oil, pequi oil, poppyseed oil, prune kernel oil, quinoa oil, tea seed oil, thistle oil, tigernut oil, tomato seed oil, wheat germ oil, Labrafac™ Lipophile WL 1349 oil, a silicone oil, a mineral oil, a lauroyl macrogol-6 glyceride, a lauroyl polyoxyl-6 glyceride, an oleoyl macrogol-6 glyceride, an oleoyl polyoxyl-6 glyceride, a linoleoyl macrogol-6 glyceride, a linoleoyl polyoxyl-6 glyceride, propylene glycol monocaprylate, propylene glycol monolaurate, propylene glycol monolaurate, polglyceryl-3 dioleate, propylene glycol dicaprylocaprate, diethylene glycol monethyl ether, a caprylocaproyl macrogol-8 glyceride, a caprylocaproyl polyoxyl-8 glyceride, and/or combinations thereof.

In some embodiments, an oil is or comprises saturated, monounsaturated, and/or polyunsaturated short-chain fatty acids, medium-chain fatty acids, long-chain fatty acids, very-long-chain fatty acids, and/or combinations thereof. In some embodiments, exemplary very-long-chain fatty acids include, but are not limited to, myristoleic acid, palmitoleic acid, sapienic acid, oleic acid, linoleic acid, alpha linolenic acid, arachidonic acid, eicosapentaenoic acid, erucic acid, docoshexaenoic acid, lauric acid, myristic acid, palmitic acid, stearic acid, arachidic acid, behenic acid, lignoceric acid, cerotic acid, and/or combinations thereof.

In some embodiments, an oil is selected from the group consisting of short-chain triglycerides, medium-chain triglycerides, long-chain triglycerides, and/or combinations thereof. In some embodiments, a short-chain triglyceride, a medium-chain triglyceride, and/or a long-chain triglyceride selected from the group consisting of saturated, monounsaturated, and/or polyunsaturated soybean oil, coconut oil, canola oil, safflower oil, olive oil, corn oil, cottonseed oil, linseed oil, safflower oil, palm oil, peanut oil, flaxseed oil, sunflower oil, rice bran oil, sesame oil, rapeseed oil, cocoa butter, almond oil, cashew oil, hazelnut oil, macadamia oil, mongongo nut oil, pecan oil, pine nut oil, pistachio oil, sachainchi oil, walnut oil, bottle gourd oil, buffalo gourd oil, butternut squash seed oil, pumpkin seed oil, watermelon seed oil, acai oil, blackcurrant seed oil, borage seed oil, evening primrose oil, carob pod oil, amaranth oil, apricot oil, apricot kernel oil, apple seed oil, argan oil, artichoke oil, avocado oil, babassu oil, ben oil, borneo tallow nut oil, cape chestnut oil, cassia oil, cocoa butter, cocklebur oil, cohune oil, coriander seed oil, dika oil, grape seed oil, hemp oil, kapok seed oil, kenaf seed oil, lallemantia oil, marula oil, meadowfoam seed oil, mustard oil, nutmeg butter, okra seed oil, papaya seed oil, perilla seed oil, pequi oil, poppyseed oil, prune kernel oil, quinoa oil, ramtil oil, royle oil, tea seed oil, thistle oil, tigernut oil, tomato seed oil, wheat germ oil, radish oil, salicornia oil, tung oil, algae oil, copaiba oil, honge oil, jatropha oil, petroleum nut oil, WL 1349 oil, a silicone oil, a mineral oil, a lauroyl macrogol-6 glyceride, a lauroyl polyoxyl-6 glyceride, an oleoyl macrogol-6 glyceride, an oleoyl polyoxyl-6 glyceride, a linoleoyl macrogol-6 glyceride, a linoleoyl polyoxyl-6 glyceride, propylene glycol monocaprylate, propylene glycol monolaurate, propylene glycol monolaurate, polglyceryl-3 dioleate, propylene glycol dicaprylocaprate, diethylene glycol monethyl ether, a caprylocaproyl macrogol-8 glyceride, a caprylocaproyl polyoxyl-8 glyceride, and/or combinations thereof.

In some embodiments, an oil agent is or comprises saturated, monounsaturated, and/or polyunsaturated soybean oil, coconut oil, canola oil, safflower oil, olive oil, corn oil, cottonseed oil, linseed oil, safflower oil, palm oil, peanut oil, flaxseed oil, sunflower oil, rice bran oil, sesame oil, rapeseed oil, cocoa butter, almond oil, cashew oil, hazelnut oil, macadamia oil, mongongo nut oil, pecan oil, pine nut oil, pistachio oil, sachainchi oil, walnut oil, bottle gourd oil, buffalo gourd oil, butternut squash seed oil, pumpkin seed oil, watermelon seed oil, acai oil, blackcurrant seed oil, borage seed oil, evening primrose oil, carob pod oil, amaranth oil, apricot oil, apricot kernel oil, apple seed oil, argan oil, artichoke oil, avocado oil, babassu oil, ben oil, borneo tallow nut oil, cape chestnut oil, cassia oil, cocoa butter, cocklebur oil, cohune oil, coriander seed oil, dika oil, grape seed oil, hemp oil, kapok seed oil, kenaf seed oil, lallemantia oil, marula oil, meadowfoam seed oil, mustard oil, nutmeg butter, okra seed oil, papaya seed oil, perilla seed oil, pequi oil, poppyseed oil, prune kernel oil, quinoa oil, ramtil oil, royle oil, tea seed oil, thistle oil, tigernut oil, tomato seed oil, wheat germ oil, radish oil, salicornia oil, tung oil, algae oil, copaiba oil, honge oil, jatropha oil, petroleum nut oil, WL 1349 oil, a silicone oil, a mineral oil, a lauroyl macrogol-6 glyceride, a lauroyl polyoxyl-6 glyceride, an oleoyl macrogol-6 glyceride, an oleoyl polyoxyl-6 glyceride, a linoleoyl macrogol-6 glyceride, a linoleoyl polyoxyl-6 glyceride, propylene glycol monocaprylate, propylene glycol monolaurate, propylene glycol monolaurate, polglyceryl-3 dioleate, propylene glycol dicaprylocaprate, diethylene glycol monethyl ether, a caprylocaproyl macrogol-8 glyceride, a caprylocaproyl polyoxyl-8 glyceride, bergamot, cade, camomile, caraway, carnauba, castor, cinnamon, cod liver, coffee, emu, eucalyptus, fish, geraniol, hyssop, jojoba, kukui nut, lavandin, lavender, lemon, litsea cubeba, mallow, mango seed, mink, orange, orange roughy, palm kernel, peach kernel, rosemary, sandalwood, sasquana, savoury, sea buckthorn, shea butter, tea tree, tsubaki, vetiver, butyl stearate, caprylic triglyceride, capric triglyceride, cyclomethicone, diethyl sebacate, dimethicone 360, isopropyl myristate, octyldodecanol, oleyl alcohol, and/or combinations thereof.

In some embodiments, a premix comprises an oily dispersion medium that comprises 1349 oil. In some embodiments, a premix comprises an oily dispersion medium that consists essentially of 1349 oil. In some embodiments, a premix comprises an oily dispersion medium that consists of 1349 oil.

In some embodiments, a premix comprises an oily dispersion medium that comprises soybean oil. In some embodiments, a premix comprises an oily dispersion medium that consists essentially of soybean oil. In some embodiments, a premix comprises an oily dispersion medium that consists of soybean oil.

In addition to the two immiscible media, a premix according to the present invention may include, for example, one or more surfactants or emulsifying agents. In some embodiments, a surfactant is or comprises an amphiphilic entity in that it contains a hydrophilic moiety and a hydrophobic moiety, typically at opposing ends of the entity. In some embodiments, an amphiphilic entity is said to have a hydrophilic head and a hydrophobic tail. In some embodiments, an amphiphilic entity has a charged (anionic, cationic, or zwitterionic) head group; in some embodiments, an amphiphilic entity has an uncharged head group.

Suitable such surfactants or emulsifying agents include, but are not limited to, pemulen; phosphoglycerides; phosphatidylcholines; dipalmitoyl phosphatidylcholine (DPPC); dioleylphosphatidyl ethanolamine (DOPE); dioleyloxypropyltriethylammonium (DOTMA); dioleoylphosphatidylcholine; cholesterol; cholesterol ester; diacylglycerol; diacylglycerolsuccinate; diphosphatidyl glycerol (DPPG); hexanedecanol; fatty alcohols such as polyethylene glycol (PEG); polyoxyethylene-9-lauryl ether; a surface active fatty acid, such as palmitic acid or oleic acid; fatty acids; fatty acid monoglycerides; fatty acid diglycerides; fatty acid amides; sorbitan trioleate (SPAN®85) glycocholate; sorbitan monolaurate (SPAN®20); polyoxyethylene monostearate; surfactin; a poloxomer; a sorbitan fatty acid ester such as sorbitan trioleate; lecithin; lysolecithin; phosphatidylserine; phosphatidylinositol; sphingomyelin; phosphatidylethanolamine (cephalin); cardiolipin; phosphatidic acid; cerebrosides; dicetylphosphate; dipalmitoylphosphatidylglycerol; stearylamine; dodecylamine; hexadecyl-amine; acetyl palmitate; glycerol ricinoleate; hexadecyl stearate; isopropyl myristate; tyloxapol; poly(ethylene glycol)5000-phosphatidylethanolamine; poly(ethylene glycol)400-monostearate; phospholipids; synthetic and/or natural detergents having high surfactant properties; deoxycholates; cyclodextrins; chaotropic salts; ion pairing agents; sodium dodecyl sulfate; pemulen; an amphiphilic entity having a head group based on a polyoxyethylene glycol sorbitan alkyl ester (*e.g.*, as in a polysorbate (TWEEN®), a super-refined polysorbate (TWEEN®), and/or combination thereof; including, but not limited to, polysorbate 20 (TWEEN®20); polysorbate 60 (TWEEN®60); polysorbate 65 (TWEEN®65); polysorbate 80 (TWEEN®80); polysorbate 85 (TWEEN®85); super-refined polysorbate 20 (SR TWEEN®20); super-refined polysorbate 60 (SR TWEEN®60); super-refined polysorbate 65 (SR TWEEN®65); super-refined polysorbate 80 (SR TWEEN®80); super-refined polysorbate 85 (SR TWEEN®85); and/or combinations thereof); an amphiphilic entity having a sulfate-based head group (*e.g.*, as in ammonium lauryl sulfate, sodium lauryl sulfate, sodium laureth sulfate, sodium myreth sulfate, *etc.*); an amphiphilic entity having a sulfonate-based head group (*e.g.*, as in dioctyl sodium sulfosuccinate, perfluorooctanesulfonate [PFOS], perfluorobutanesulfonate, alkyl benzene sulfonates, CHAPS (3-[(3-Cholamidopropyl)dimethylammonio]-1-propanesulfonate, cocamidopropyl hydroxysultaine, *etc.*); an amphiphilic entity having a phosphate-based head group (*e.g.*, as in alkyl aryl ether phosphate, alkyl ether phosphate, lecithin, *etc.*); an amphiphilic entity having a carboxylate-based head group (*e.g.*, as in fatty acids, sodium stearate, sodium lauroyl sarcosinate, carboxylate fluorosurfactants, perfluorononanoate, perfluorooctanoate [PFOA or PFO], amino acids, imino acids, cocamidopropyl betaine, *etc.*); an amphiphilic entity having an amine-based head group (*e.g.*, a primary, secondary, or tertiary amine, as in octenideine dihydrochloride); an amphiphilic entity having a head group comprising a quaternary ammonium ion (*e.g.*, as in cetyl trimethylammonium bromide [CTAB] a.k.a. hexadecyl trimethyl ammonium bromide, cetyl trimethylammonium chloride [CTAC], cetylpyridinium chloride [CPC], polyethoxylated tallow amine [POEA], benzalkonium chloride [BAC], Benzethonium chloride [BZT], 5-Bromo-5-nitro-1,3-dioxane, Dimethyldioctadecylammonium chloride, Dioctadecyldimethylammonium bromide [DODAB]); an amphiphilic entity having a head group based on a fatty alcohol (*e.g.*, as in cetyl alcohol, stearyl alcohol, cetostearyl alcohol, oleyl alcohol, *etc.*); an amphiphilic entity having a head group based on a polyoxyethylene glycol alkyl ether (*e.g.*, as in octaethylene glycol monododecyl ether, pentaethylene glycol monododecyl ether); an amphiphilic entity having a head group based on polyoxypropylene glycol alkyl ether; an amphiphilic entity having a head group based on a glucoside alkyl ether (*e.g.*, as in decyl glucoside, lauryl glucoside, octyl glucoside, *etc.*); an amphiphilic entity having a head group based on a polyoxyethylene glycol octylphenol ether (*e.g.*, as in Triton X-100); an amphiphilic entity having a head group based on a polyoxyethylene glycol alkylphenol ether (*e.g.*, as in nonosynol-9); an amphiphilic entity having a head group based on a glycerol alkyl ester (*e.g.*, as in glyceryl laurate); an amphiphilic entity having a head group based on a sorbitan alkyl ester (*e.g.*, spans); an amphiphilic entity that is or comprises cocamide MEA, cocamide DEA< dodecyl dimethylamine oxide; a block copolymer of polyethylene glycol and polypropylene glycol (*i.e.*, a poloxamer); an amphiphilic entity having a tail group based on or containing a hydrocarbon chain; an amphiphilic entity having a tail group based on or containing an alkyl ether chain; an amphiphilic entity having a tail group based on or containing a polyethylene; an amphiphilic entity having a tail group based on or containing polypropylene oxide; an amphiphilic entity having a tail group based on or containing a fluorocarbon chain; an amphiphilic entity having a tail group based on or containing a siloxane chain; and/or combinations thereof.

In some embodiments, a premix comprises a surfactant that comprises a polysorbate (TWEEN®) substance. In some embodiments, a premix comprises a surfactant that comprises a super-refined polysorbate (SR TWEEN®) substance. In some embodiments, a premix comprises a surfactant that comprises a polysorbate selected from the group consisting of polysorbate 20 (TWEEN®20); polysorbate 60 (TWEEN®60); polysorbate 65 (TWEEN®65); polysorbate 80 (TWEEN®80); polysorbate 85 (TWEEN®85); super-refined polysorbate 20 (SR TWEEN®20); super-refined polysorbate 60 (SR TWEEN®60); super-refined polysorbate 65 (SR TWEEN®65); super-refined polysorbate 80 (SR TWEEN®80); super-refined polysorbate 85 (SR TWEEN®85); and combinations thereof. In some embodiments, a premix comprises a surfactant that comprises polysorbate 80 (TWEEN®80). In some embodiments, a premix comprises a surfactant that comprises super-refined polysorbate 80 (SR TWEEN®80). In some embodiments, a premix comprises a surfactant that consists essentially of a polysorbate (TWEEN®) substance. In some embodiments, a premix comprises a surfactant that consists essentially of a super-refined polysorbate (SR TWEEN®) substance. In some embodiments, a premix comprises a surfactant that consists essentially of a polysorbate selected from the group consisting of polysorbate 20 (TWEEN®20); polysorbate 60 (TWEEN®60); polysorbate 65 (TWEEN®65); polysorbate 80 (TWEEN®80); polysorbate 85 (TWEEN®85); super-refined polysorbate 20 (SR TWEEN®20); super-refined polysorbate 60 (SR TWEEN®60); super-refined polysorbate 65 (SR TWEEN®65); super-refined polysorbate 80 (SR TWEEN®80); super-refined polysorbate 85 (SR TWEEN®85); and combinations thereof. In some embodiments, a premix comprises a surfactant that consists essentially of polysorbate 80 (TWEEN®80). In some embodiments, a premix comprises a surfactant that consists essentially of super-refined polysorbate 80 (SR TWEEN®80). In some embodiments, a premix comprises a surfactant that consists of a polysorbate (TWEEN®) substance. In some embodiments, a premix comprises a surfactant that consists of a super-refined polysorbate (SR TWEEN®) substance. In some embodiments, a premix comprises a surfactant that consists of a polysorbate selected from the group consisting of polysorbate 20 (TWEEN®20); polysorbate 60 (TWEEN®60); polysorbate 65 (TWEEN®65); polysorbate 80 (TWEEN®80); polysorbate 85 (TWEEN®85); super-refined polysorbate 20 (SR TWEEN®20); super-refined polysorbate 60 (SR TWEEN®60); super-refined polysorbate 65 (SR TWEEN®65); super-refined polysorbate 80 (SR TWEEN®80); super-refined polysorbate 85 (SR TWEEN®85); and combinations thereof. In some embodiments, a premix comprises a surfactant that consists of polysorbate 80 (TWEEN®80). In some embodiments, a premix comprises a surfactant that consists of super-refined polysorbate 80 (SR TWEEN®80). In some embodiments, a premix comprises a surfactant that comprises pemulen. In some embodiments, a premix comprises a surfactant that consists essentially of pemulen. In some embodiments, a premix comprises a surfactant that consists of pemulen.

In some embodiments, a surfactant is a mixture of different surfactants. Surfactants may be extracted and purified from a natural source or may be prepared synthetically in a laboratory. In some embodiments, surfactants are commercially available.

In some embodiments, a premix comprises a gelatin agent selected from the group consisting of a hydrolyzed collagen protein, including, but not limited to, gelatin agents selected from the group consisting of Gelatine, Gelfoam, Puragel, Galfoam, a substance corresponding to CAS number 9000-70-8, other forms of gelatin, and/or combinations thereof.

In light of the teachings provided herein, those of ordinary skill in the art will readily be able to identify alternative or additional gelatin agents. In general, as is known in the art, a gelatin is a protein substance that is produced by partial, typically irreversible hydrolysis of collagen extracted from the boiled bones, connective tissues, organs and some intestines of animals such as domesticated cattle, pigs, and horses.

One of ordinary skill in the art would readily appreciate that gelatin itself may not be the only agent with desirable attributes, such as those described herein, and could readily test a variety of agents, particularly peptide agents, to identify additional agents having similar attributes and/or functions. Exemplary peptide agents that could be tested for attributes and/or functions similar to those exhibited by gelatin include, but are not limited to, proteins derived from blood and/or plasma, including, but not limited to, albumin, fibrin, thrombin, prothrombin, and/or combinations thereof.

In addition to the two immiscible media, and optionally a surfactant, a premix according to the present invention may include, for example, one or more excipients. In some embodiments, a premix comprises an excipient that comprises methylparaben. In some embodiments, a premix comprises an excipient that consists essentially of methylparaben. In some embodiments, a premix comprises an excipient that consists of methylparaben. In some embodiments, a premix comprises an excipient that comprises propylparaben. In some embodiments, a premix comprises an excipient that consists essentially of propylparaben. In some embodiments, a premix comprises an excipient that consists of propylparaben. In some embodiments, a premix comprises an excipient that comprises propylparaben and methylparaben. In some embodiments, a premix comprises an excipient that consists essentially of propylparaben and methylparaben. In some embodiments, a premix comprises an excipient that consists of propylparaben and methylparaben. In some embodiments, a premix is substantially or completely free of parabens.

In some embodiments, all of the components present in the final nanoparticle composition are present in the premix and are subjected to high shear force to produce the nanoparticle composition. In some embodiments, one or more of the components that are present in the final nanoparticle composition is/are missing from the premix or is/are present in the premix in a smaller amount than in the final nanoparticle composition. That is, in some embodiments, one or more materials are added to the nanoparticle composition after the premix is subjected to high shear force.

In some embodiments, the premix is prepared as a solution prior to application of high shear force. In some embodiments, for nanoparticle compositions that include at least one known therapeutic agent and/or an independently active biologically active agent (*e.g.*, botulinum toxin), it is often desirable for the therapeutic agent to be dissolved in the premix before the high shear force is applied. Thus, in many embodiments, the therapeutic agent is soluble in at least one of the media (or in a combination of media utilized in the premix). In some embodiments, such dissolution requires heating; in other embodiments it does not.

In some embodiments, the premix components may assemble into particles before the application of high shear force. At least some of such particles may be microparticles or even nanoparticles. In some embodiments, a nanoparticle composition is prepared from a premix, wherein the premix is selected from the group comprising a suspension or a microemulsion. In some embodiments, however, particle structures do not form in the premix before application of high shear force.

In certain embodiments, relative amount of premix components are selected or adjusted to generate nanoparticles having desired characteristics.

In some embodiments, the premix comprises oil and surfactant at a ratio ranging between 0.5 - 10. In some embodiments, the ratio of oil to surfactant is approximately 0.5:1, approximately 1:1, approximately 2:1, approximately 3:1, approximately 4:1, approximately 5:1, approximately 6:1, approximately 7:1, approximately 8:1, approximately 9:1 or approximately 10:1. In some embodiments, the ratio of surfactant to oil is approximately 0.5:1, approximately 1:1, approximately 2:1, approximately 3:1, approximately 4:1, approximately 5:1, approximately 6:1, approximately 7:1, approximately 8:1, approximately 9:1 or approximately 10:1.
In some embodiments, oil and surfactant are utilized at a ratio ranging between 0.1 and 2. In some embodiments, the ratio of oil to surfactant is approximately 0.1:1. In some embodiments, the ratio of oil to surfactant is approximately 0.15:1. In some embodiments, the ratio of oil to surfactant is approximately 0.2:1. In some embodiments, the ratio of oil to surfactant is approximately 0.25:1. In some embodiments, the ratio of oil to surfactant is approximately 0.3:1. In some embodiments, the ratio of oil to surfactant is approximately 0.35:1. In some embodiments, the ratio of oil to surfactant is approximately 0.4:1. In some embodiments, the ratio of oil to surfactant is approximately 0.45:1. In some embodiments, the ratio of oil to surfactant is approximately 0.5:1. In some embodiments, the ratio of oil to surfactant is approximately 0.55:1. In some embodiments, the ratio of oil to surfactant is approximately 0.6:1. In some embodiments, the ratio of oil to surfactant is approximately 0.65:1. In some embodiments, the ratio of oil to surfactant is approximately 0.7:1. In some embodiments, the ratio of oil to surfactant is approximately 0.75:1. In some embodiments, the ratio of oil to surfactant is approximately 0.8:1. In some embodiments, the ratio of oil to surfactant is approximately 0.85:1. In some embodiments, the ratio of oil to surfactant is approximately 0.9:1. In some embodiments, the ratio of oil to surfactant is approximately 0.95:1. In some embodiments, the ratio of oil to surfactant is approximately 1:1. In some embodiments, the ratio of oil to surfactant is approximately 1:1.05. In some embodiments, the ratio of oil to surfactant is approximately 1:1.1. In some embodiments, the ratio of oil to surfactant is approximately 1:1.15. In some embodiments, the ratio of oil to surfactant is approximately 1:1.2. In some embodiments, the ratio of oil to surfactant is approximately 1:1.25. In some embodiments, the ratio of oil to surfactant is approximately 1:1.3. In some embodiments, the ratio of oil to surfactant is approximately 1:1.35. In some embodiments, the ratio of oil to surfactant is approximately 1:1.4. In some embodiments, the ratio of oil to surfactant is approximately 1:1.45. In some embodiments, the ratio of oil to surfactant is approximately 1:1.5. In some embodiments, the ratio of oil to surfactant is approximately 1:1.55. In some embodiments, the ratio of oil to surfactant is approximately 1:1.6. In some embodiments, the ratio of oil to surfactant is approximately 1:1.65. In some embodiments, the ratio of oil to surfactant is approximately 1:1.7. In some embodiments, the ratio of oil to surfactant is approximately 1:1.75. In some embodiments, the ratio of oil to surfactant is approximately 1:1.8. In some embodiments, the ratio of oil to surfactant is approximately 1:1.85. In some embodiments, the ratio of oil to surfactant is approximately 1:1.9. In some embodiments, the ratio of oil to surfactant is approximately 1:1.95. In some embodiments, the ratio of oil to surfactant is approximately 1:2. In some embodiments, the ratio of oil to surfactant is approximately 1:2.5. In some embodiments, the ratio of oil to surfactant is approximately 1:3. In some embodiments, the ratio of oil to surfactant is approximately 1:3.5. In some embodiments, the ratio of oil to surfactant is approximately 1:4. In some embodiments, the ratio of oil to surfactant is approximately 1:4.5. In some embodiments, the ratio of oil to surfactant is approximately 1:5.

In some embodiments, the premix comprises oil and surfactant at a ratio ranging between about 0.1:1 to about 2:1. In some embodiments, the premix comprises oil and surfactant at a ratio of about 0.1:1 to about 1:1. In some embodiments, the premix comprises oil and surfactant at a ratio of about 0.5:1 to about 1:1. In some embodiments, the premix comprises oil and surfactant at a ratio of about 0.1:1, about 0.15:1, about 0.2:1, about 0.25:1, about 0.3:1, about 0.35:1, about 0.4:1, about 0.45:1, about 0.5:1, about 0.5:1, about 0.55:1, about 0.6:1, about 0.65:1, about 0.7:1, about 0.75:1, about 0.8:1, about 0.85:1, about 0.9:1, about 0.95:1, or about 1:1 In some embodiments, the premix comprises oil and surfactant at a ratio of about 0.67:1.

In some embodiments, the aqueous dispersion medium (*e.g*., water, buffer, salt solution, *etc.*) and surfactant are utilized at a ratio ranging between 0.01 and 20. In some embodiments, the aqueous dispersion medium (*e.g.*, water, buffer, salt solution, *etc.*) and surfactant are utilized at a ratio ranging between 0.1 and 20. In some embodiments, the aqueous dispersion medium (*e.g.*, water, buffer, salt solution, *etc.*) and surfactant are utilized at a ratio ranging between 0.5 and 10. In some embodiments, the aqueous dispersion medium (*e.g.*, water, buffer, salt solution, *etc.*) and surfactant are utilized at a ratio ranging between 0.5 and 1. In some embodiments, the ratio of aqueous dispersion medium (*e.g.*, water, buffer, salt solution, *etc.*) to surfactant is approximately 0.01:1, approximately 0.02:1, approximately 0.03:1, approximately 0.04:1, approximately 0.05:1, approximately 0.06:1, approximately 0.07:1, approximately 0.08:1, approximately 0.0:1, approximately 0.1:1, approximately 0.2:1, approximately 0.3:1, approximately 0.4:1, approximately 0.5:1, approximately 1:1, approximately 2:1, approximately 3:1, approximately 4:1, approximately 5:1, approximately 6:1, approximately 7:1, approximately 8:1, approximately 9:1 or approximately 10:1. In some embodiments, the ratio of surfactant to water is approximately 0.5:1, approximately 1:1, approximately 2:1, approximately 3:1, approximately 4:1, approximately 5:1, approximately 6:1, approximately 7:1, approximately 8:1, approximately 9:1, approximately 10:1, approximately 11:1, approximately 12:1, approximately 13:1, approximately 14:1, approximately 15:1, approximately 16:1, approximately 17:1, approximately 18:1, approximately 19:1, or approximately 20:1. In some embodiments, aqueous dispersion medium (*e.g.*, water, buffer, salt solution, *etc.*) and surfactant are utilized at a ratio ranging between 0.5 and 2. In some embodiments, the ratio of aqueous dispersion medium (*e.g*., water, buffer, salt solution, *etc.*) to surfactant is approximately 0.5:1, approximately 1:1, or approximately 2:1. In some embodiments, the ratio of surfactant to aqueous dispersion medium (*e.g.*, water, buffer, salt solution, *etc.*) is approximately 0.5:1, approximately 1:1, or approximately 2:1. In some embodiments, the ratio of aqueous dispersion medium (*e.g*., water, buffer, salt solution, *etc.*) to surfactant is approximately 1:1. In some embodiments, compositions utilizing such ratios of aqueous dispersion medium (*e.g*., water, buffer, salt solution, *etc.*) to surfactant comprise water-in-oil emulsions.

In some embodiments, aqueous dispersion media and surfactant are utilized at a ratio ranging between about 8:1 and about 9:1. In some embodiments, aqueous dispersion media and surfactant are utilized at a ratio of about 8:1, about 8.1:1, about 8.2:1, about 8.3:1, about 8.4:1, about 8.5:1, about 8.6:1, about 8.7:1, about 8.8:1, about 8.9:1, about 9:1, *etc.* In some embodiments, aqueous dispersion media and surfactant are utilized at a ratio of about 8.7:1. In some embodiments, aqueous dispersion media and surfactant are utilized at a ratio of about 8.8:1.

In some embodiments, aqueous dispersion media and oil are utilized at a ratio ranging between about 12:1 and about 14:1. In some embodiments, aqueous dispersion media and surfactant are utilized at a ratio of about 12:1, about 12.1:1, about 12.2:1, about 12.3:1, about 12.4:1, about 12.5:1, about 12.6:1, about 12.7:1, about 12.8:1, about 12.9:1, about 13:1, about 13.1:1, about 13.2:1, about 13.3:1, about 13.4:1, about 13.5:1, about 13.6:1, about 13.7:1, about 13.8:1, about 13.9:1, about 14:1, *etc.* In some embodiments, aqueous dispersion media and surfactant are utilized at a ratio of about 13.1:1.

In some embodiments, the percent of oil in the premix ranges between 0% and 50%. In some embodiments, the percent of oil in the premix ranges between 0% and 40%. In some embodiments, the percent of oil in the premix ranges between 0% and 30%. In some embodiments, the percent of oil in the premix ranges between 0% and 20%. In some embodiments, the percent of oil in the premix ranges between 0% and 10%. In some embodiments, the percent of oil in the premix ranges between 0% and 5%. In some embodiments, the percent of oil in the premix ranges between 5% and 10%, between 10% and 15%, between 15% and 20%, between 20% and 25%, between 25% and 30%, between 35% and 40%, or between 45% and 50%. In some embodiments, the percent of oil in the premix ranges between 10% and 20%, between 10% and 30%, between 10% and 40%, or between 10% and 50%. In some embodiments, the percent of oil in the premix ranges between 20% and 30%, between 20% and 40%, between 20% and 50%. In some embodiments, the percent of oil in the premix ranges between 30% and 40% or between 30% and 50%. In some embodiments, the percent of oil in the premix ranges between 40% and 50%.

In some embodiments the percent of oil in the premix is approximately 1%, approximately 2%, approximately 3%, approximately 4%, approximately 5%, approximately 6%, approximately 7%, approximately 9%, approximately 10%, approximately 11%, approximately 12%, approximately 13%, approximately 14%, approximately 15%, approximately 16%, approximately 17%, approximately 18%, approximately 19%, approximately 20%, approximately 21%, approximately 22%, approximately 23%, approximately 24%, approximately 25%, approximately 26%, approximately 27%, approximately 28%, approximately 29%, approximately 30%, approximately 31%, approximately 32%, approximately 33%, approximately 34%, approximately 35%, approximately 36%, approximately 37%, approximately 38%, approximately 39%, approximately 40%, approximately 41%, approximately 42%, approximately 43%, approximately 44%, approximately 45%, approximately 46%, approximately 47%, approximately 48%, approximately 49%, or approximately 50%. In some embodiments the percent of oil is approximately 10%. In some embodiments the percent of oil is approximately 9%. In some embodiments the percent of oil is approximately 8%. In some embodiments the percent of oil is approximately 7%. In some embodiments the percent of oil is approximately 6%. In some embodiments the percent of oil is approximately 5%. In some embodiments the percent of oil is approximately 4%. In some embodiments the percent of oil is approximately 3%. In some embodiments the percent of oil is approximately 2%. In some embodiments the percent of oil is approximately 1%.

In some embodiments, the percent of oil in the premix ranges between about 5% and about 8%. In some embodiments, the percent of oil in the premix is about 5%, about 5.1%, about 5.2%, about 5.3%, about 5.4%, about 5.5%, about 5.6%, about 5.7%, about 5.8%, about 5.9%, about 6%, about 6.1%, about 6.2%, about 6.3%, about 6.4%, about 6.5%, about 6.6%, about 6.7%, about 6.8%, about 6.9%, about 7%, about 7.1%, about 7.2%, about 7.3%, about 7.4%, about 7.5%, about 7.6%, about 7.7%, about 7.8%, about 7.9%, or about 8%. In some embodiments, the percent of oil in the premix is about 6.3%. In some embodiments, the percent of oil in the premix is about 6.4%.The percent of aqueous dispersion medium (*e.g.*, water, buffer, salt solution, *etc.*) in the premix can range from 0% to 99%, from 10% to 99%, from 25% to 99%, from 50% to 99%, or from 75% to 99%. In some embodiments, the percent of aqueous dispersion medium (*e.g.*, water, buffer, salt solution, *etc.*) in the premix can range from 0% to 75%, from 0% to 50%, from 0% to 25%, or from 0% to 10%. In some embodiments, the percent of aqueous dispersion medium (*e.g.*, water, buffer, salt solution, *etc.*) in the premix ranges between 0% and 30%. In some embodiments the percent of aqueous dispersion medium (*e.g.*, water, buffer, salt solution, *etc.*) is approximately 1%, approximately 2%, approximately 3%, approximately 4%, approximately 5%, approximately 6%, approximately 7%, approximately 9%, approximately 10%, approximately 11%, approximately 12%, approximately 13%, approximately 14%, approximately 15%, approximately 16%, approximately 17%, approximately 18%, approximately 19%, approximately 20%, approximately 21%, approximately 22%, approximately 23%, approximately 24%, approximately 25%, approximately 26%, approximately 27%, approximately 28%, approximately 29%, approximately 30%, approximately 35%, approximately 40%, approximately 45%, approximately 50%, approximately 55%, approximately 60%, approximately 65%, approximately 70%, approximately 71%, approximately 72%, approximately 73%, approximately 74%, approximately 75%, approximately 76%, approximately 77%, approximately 78%, approximately 79%, approximately 80%, approximately 81%, approximately 82%, approximately 83%, approximately 84%, approximately 85%, approximately 86%, approximately 87%, approximately 88%, approximately 89%, approximately 90%, approximately 91%, approximately 92%, approximately 93%, approximately 94%, approximately 95%, approximately 96%, approximately 97%, approximately 98%, or approximately 99%,. In some embodiments the percent of water is approximately 83%. In some embodiments the percent of water is approximately 9%. In some embodiments the percent of water is approximately 5%.

In some embodiments, the percent of aqueous dispersion medium in the premix ranges between about 80% and about 85%. In some embodiments, the percent of aqueous dispersion medium in the premix is about 80, about 80.5%, about 81%, about 81.5%, about 82%, about 82.5%, about 83%, about 83.5%, about 84%, about 84.5%, or about 85%. In some embodiments, the percent of aqueous dispersion medium in the premix is about 83.5%. In some embodiments, the percent of oil in the premix is about 84%.

In some embodiments, the percent of surfactant in the premix ranges between 0% -30%. In some embodiments the percent of surfactant in the premix is approximately 1%, approximately 2%, approximately 3%, approximately 4%, approximately 5%, approximately 6%, approximately 7%, approximately 9%, approximately 10%, approximately 11%, approximately 12%, approximately 13%, approximately 14%, approximately 15%, approximately 16%, approximately 17%, approximately 18%, approximately 19%, approximately 20%, approximately 21%, approximately 22%, approximately 23%, approximately 24%, approximately 25%, approximately 26%, approximately 27%, approximately 28%, approximately 29%, approximately 30%, approximately 31%, approximately 32%, approximately 33%, approximately 34%, approximately 35%, approximately 36%, approximately 37%, approximately 38%, approximately 39%, approximately 40%, approximately 41%, approximately 42%, approximately 43%, approximately 44%, approximately 45%, approximately 46%, approximately 47%, approximately 48%, approximately 49%, or approximately 50%. In some embodiments the percent of surfactant is approximately 10%. In some embodiments the percent of surfactant is approximately 9%. In some embodiments the percent of surfactant is approximately 8%. In some embodiments the percent of surfactant is approximately 7%. In some embodiments the percent of surfactant is approximately 6%. In some embodiments the percent of surfactant is approximately 5%.

In some embodiments, the percent of surfactant in the premix ranges between about 8% and about 11%. In some embodiments, the percent of surfactant in the premix is about 8%, about 8.1%, about 8.2%, about 8.3%, about 8.4%, about 8.5%, about 8.6%, about 8.7%, about 8.8%, about 8.9%, about 9%, about 9.1%, about 9.2%, about 9.3%, about 9.4%, about 9.5%, about 9.6%, about 9.7%, about 9.8%, about 9.9%, about 10%, about 10.1%, about 10.2%, about 10.3%, about 10.4%, about 10.5%, about 10.6%, about 10.7%, about 10.8%, about 10.9%, or about 11%. In some embodiments, the percent of oil in the premix is about 9.5%. In some embodiments, the percent of surfactant in the premix is about 9.6%.

In some embodiments, the percent of excipient in the premix ranges between about 0.1% and about 1%. In some embodiments, the percent of excipient in the premix is about 0.1%, about 0.2%, about 0.3%, about 0.4%, about 0.5%, about 0.6%, about 0.7%, about 0.8%, about 0.9%, or about 1%. In some embodiments, the percent of excipient in the premix is about 0.4%.

In some embodiments, a premix consists essentially of the following proportions of ingredients:

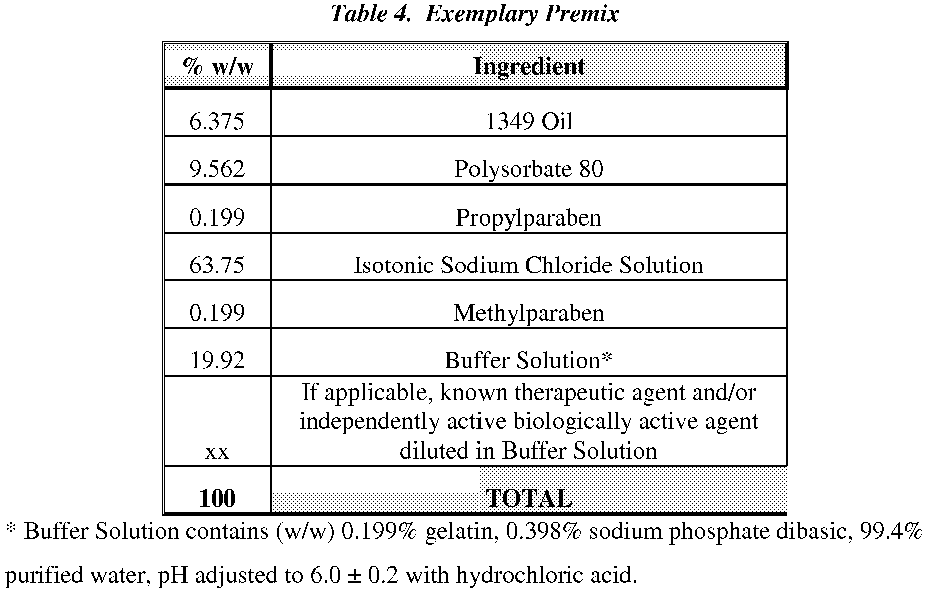

In some embodiments, a provided composition does not contain more than one oil. In some embodiments, a provided composition may comprise two or more oils (*e.g.*, 2, 3, 4, 5, or more oils). In some embodiments, a provided composition does not contain more than one surfactant. In some embodiments, a provided composition may comprise two or more surfactants (*e.g*., 2, 3, 4, 5, or more surfactants).

In some embodiments, a provided composition consists essentially of an aqueous dispersion medium (*e.g.*, water, buffer, salt solution, *etc.*), an oil, and a surfactant. In some embodiments, a provided composition consists essentially of an aqueous dispersion medium (*e.g.*, water, buffer, salt solution, *etc.*), an oil, and a surfactant, and at least one substance used to produce and/or preserve the composition (*e.g.*, proteins, salts, *etc.*).

In some embodiments, a provided composition consists of an aqueous dispersion medium (*e.g.*, water, buffer, salt solution, *etc.*), an oil, and a surfactant. In some embodiments, a provided composition consists of an aqueous dispersion medium (*e.g*., water, buffer, salt solution, *etc.*), an oil, a surfactant, and at least one substance used to produce and/or preserve the composition (*e.g.*, proteins, salts, *etc.*). In some embodiments, a provided composition consists of an aqueous dispersion medium (*e.g.*, water, buffer, salt solution, *etc.*), one or more oils, and one or more surfactants. In some embodiments, a provided composition consists of an aqueous dispersion medium (*e.g.*, water, buffer, salt solution, *etc.*), one or more oils, one or more surfactants, and at least one substance used to produce and/or preserve the composition (*e.g.*, proteins, salts, *etc.*)*.* In some embodiments, a provided composition does not contain an added preservative. In some embodiments, a provided composition does not contain parabens, such as methyparaben and propylparaben.

In some embodiments, any of the nanoparticle compositions described herein further comprise a known therapeutic agent and/or independently active biologically active agent. In some embodiments, any of the nanoparticle compositions described herein do not comprise a known therapeutic agent and/or independently active biologically active agent.

### Identification and/or Characterization of Biologically Active Components

As described herein, the present invention encompasses the finding that certain provided compositions, not containing any agent previously known to have relevant biological activity, nonetheless can achieve biological effects. The present invention further encompasses the recognition that such effects may result from and/or require nanoparticle structure, and in particular may result from and/or require certain embodiments of nanoparticle structure described herein. Alternatively or additionally, the present invention encompasses the recognition that one or more components of described provided compositions may contribute to or provide the biological effects observed with the nanoparticle composition, partially or wholly independent of nanoparticle structure.

The present invention therefore provides systems for identifying and/or characterizing biologically active agents by assaying individual components, or combinations of components, of provided compositions as described herein. According to certain embodiments of the present invention, one or more such components, alone or in combination with others, may have biological activity independent of nanoparticle structure (*e.g.*, in the context of a composition that is not a nanoparticle composition, and particularly is not a nanoemulsion, or a uniform nanoparticle composition, as described herein). Such embodiments of the present invention provide both (i) the identification/characterization of such components, and (ii) compositions containing such components, in amounts appropriate to achieve the relevant biological effects when administered as part of a dosing regimen, *e.g.*, as described herein. Such component-containing compositions are "provided compositions" herein, whether or not they contain nanoparticle structure. The present invention also provides uses for such provided compositions, as described herein.

### Therapeutic Agents

In some embodiments, provided compositions comprise one or more therapeutic agents. In some embodiments, provided compositions do not comprise any known therapeutic agents and/or independently active biologically active agents.

Any therapeutic agent can be utilized in accordance with the compositions described herein, including, but not limited to, nucleic acids (*e.g.*, DNA, RNA, DNA-RNA hybrids, siRNAs, shRNAs, miRNAs, RNAi-inducing entities, aptamers, *etc.*), polypeptides, proteins (including multimeric proteins, protein complexes, *etc.*), peptides, antibodies, glycoproteins, small molecules, carbohydrates, lipids, organometallic compounds, hormones, metals, radioactive elements and compounds, vaccines, immunological agents, fragments thereof, and/or combinations thereof. In some embodiments, therapeutic agents include any of those described in the section entitled "Dermatologic Conditions." Exemplary possible therapeutic agents are described in more detail in the following paragraphs.

In some embodiments, a therapeutic agent is present in a premix at a concentration of about 0.1%, about 0.5%, about 0.75%, 1%, about 2%, about 2.5%, about 5%, about 7.5%, about 10%, about 15%, about 20%, about 25%, about 30%, about 35%, about 40%, or about 50%. In some embodiments, a therapeutic agent is present in a provided composition at a concentration of about 0.1%, about 0.5%, about 0.75%, 1%, about 2%, about 2.5%, about 5%, about 7.5%, about 10%, about 15%, about 20%, or about 25%.

### Nucleic Acid Agents

Any of a variety of nucleic acids may be incorporated in provided compositions according to the present invention. In some embodiments, a nucleic acid to be incorporated in a provided composition is a polynucleotide. In some embodiments, a nucleic acid to be incorporated in a provided composition is a nucleotide. In some embodiments, a nucleic acid to be incorporated in a provided composition is a nucleoside. In some embodiments, a nucleic acid to be incorporated into a provided composition is set forth in PCT application serial number PCT/US08/65329, filed May 30, 2008, published as PCT publication WO 08/151022 on December 11, 2008, and entitled "NUCLEIC ACID NANOPARTICLES AND USES THEREFOR"; incorporated herein by reference.

In some embodiments, a nucleic acid agent is less than about 50 nucleotides in length. In some embodiments, a nucleic acid agent is less than about 90, about 80, about 70, about 65, about 60, about 55, about 50, about 45, about 40, about 35, about 30, about 25, about 20, about 15, about 13, about 12, about 10, about 9, about 8, about 7, about 6, about 5, about 4, about 3, or about 2 nucleotides in length. In some embodiments, a nucleic acid agent is a single nucleic acid residue (*e.g.*, a single nucleotide or a single nucleoside). In some embodiments, a nucleic acid agent to be incorporated in a provided composition comprises only naturally-occurring nucleic acid residues (*e.g.*, nucleotides, nucleosides). In some embodiments, a nucleic acid agent comprises one or more non-naturally occurring nucleic acid residues.

In general, a nucleoside comprises a pentose sugar (*e.g*., ribose or deoxyribose) and a heterocyclic nitrogenous base (*e.g.*, purines: cytosine, thymidine, and uracil; and pyrimidines: guanine and adenine) covalently linked by a glycosidic bond. In general, a nucleotide comprises a nucleoside and one to three 5' phosphate groups.

In some embodiments, a nucleic acid agent may be DNA, RNA, or combinations thereof. In some embodiments, a nucleic acid agent may be an oligonucleotide and/or polynucleotide. As used herein, the terms "oligonucleotide" and "polynucleotide" may be used interchangeably. In some embodiments, a nucleic acid agent may be an oligonucleotide and/or modified oligonucleotide (including, but not limited to, modifications through phosphorylation); an antisense oligonucleotide and/or modified antisense oligonucleotide (including, but not limited to, modifications through phosphorylation). In some embodiments, a nucleic acid agent may comprise cDNA and/or genomic DNA. In some embodiments, a nucleic acid agent may comprise non-human DNA and/or RNA (*e.g.*, viral, bacterial, or fungal nucleic acid sequences). In some embodiments, a nucleic acid agent may be a plasmid, cosmid, gene fragment, artificial and/or natural chromosome (*e.g.*, a yeast artificial chromosome), and/or a part thereof. In some embodiments, a nucleic acid agent may be a functional RNA (*e.g.*, mRNA, a tRNA, an rRNA and/or a ribozyme). In some embodiments, a nucleic acid agent may be an RNAi-inducing agent, small interfering RNA (siRNA), short hairpin RNA (shRNA), and/or microRNA (miRNA). In some embodiments, a nucleic acid agent may be a peptide nucleic acid (PNA). In some embodiments, a nucleic acid agent may be a polynucleotide comprising synthetic analogues of nucleic acids, which may be modified or unmodified. In some embodiments, a nucleic acid agent may comprise various structural forms of DNA including single-stranded DNA, double-stranded DNA, supercoiled DNA and/or triple -helical DNA; Z-DNA; and/or combinations thereof.

In some embodiments, nucleic acid agents may be prepared according to any of a variety of available techniques including, but not limited to chemical synthesis, enzymatic synthesis, enzymatic or chemical cleavage of a longer precursor, *etc.* Methods of synthesizing nucleic acid polymers (*e.g.*, polynucleotides) are known in the art (see, *e.g.*, Gait, M.J. (ed.) Oligonucleotide synthesis: a practical approach, Oxford [Oxfordshire], Washington, DC: IRL Press, 1984; and Herdewijn, P. (ed.) Oligonucleotide synthesis: methods and applications, Methods in molecular biology, v. 288 (Clifton, N.J.) Totowa, N.J.: Humana Press, 2005; both of which are incorporated herein by reference).

In some embodiments, nucleic acid agents may comprise naturally-occurring nucleic acid residues. In some embodiments, naturally- occurring nucleic acid residues include nucleosides, modified nucleosides, naturally- occurring nucleosides with hydrocarbon linkers (*e.g.*, an alkylene) or a polyether linker (*e.g.*, a PEG linker) inserted between one or more nucleosides, modified nucleosides with hydrocarbon or PEG linkers inserted between one or more nucleosides, or a combination of thereof. In some embodiments, nucleic acid residues or modified nucleic acid residues can be replaced with a hydrocarbon linker or a polyether linker provided that the binding affinity, selectivity, and/or other functional characteristics of the nucleic acid residues are not substantially reduced by the substitution.

In some embodiments, nucleic acid agents may comprise nucleic acid residues entirely of the types found in naturally occurring nucleic acids, or may instead include one or more nucleic acid residue analogs or have a structure that otherwise differs from that of a naturally occurring nucleic acid. U.S. Patents 6,403,779; 6,399,754; 6,225,460; 6,127,533; 6,031,086; 6,005,087; 5,977,089; and references therein disclose a wide variety of specific nucleic acid residue analogs and modifications that may be used. See Crooke, S. (ed.) Antisense Drug Technology: Principles, Strategies, and Applications (1^{st} ed), Marcel Dekker; ISBN: 0824705661; 1st edition (2001), incorporated by reference, and references therein. For example, 2'-modifications include halo, alkoxy and allyloxy groups. In some embodiments, the 2'-OH group is replaced by a group selected from H, OR, R, halo, SH, SRi, NH₂, NH_{R}, NR₂ or CN, wherein R is C₁-C₆ alkyl, alkenyl, or alkynyl, and halo is F, Cl, Br, or I. Examples of modified linkages include phosphorothioate and 5'-N- phosphoramidite linkages.

In some embodiments, nucleic acid agents comprise a variety of different nucleic acid residue (*e.g.*, nucleotide, nucleoside) analogs, modified backbones, or non-naturally occurring internucleoside linkages. In some embodiments, nucleic acid agents may include natural nucleosides (*i.e.*, adenosine, thymidine, guanosine, cytidine, uridine, deoxyadenosine, deoxythymidine, deoxyguanosine, and deoxycytidine) or modified nucleosides. Examples of modified nucleotides include base modified nucleoside (*e.g*., aracytidine, inosine, isoguanosine, nebularine, pseudouridine, 2,6-diaminopurine, 2-aminopurine, 2-thiothymidine, 3-deaza- 5-azacytidine, 2'-deoxyuridine, 3-nitorpyrrole, 4-methylindole, 4-thiouridine, A-thiothymidine, 2-aminoadenosine, 2-thiothymidine, 2-thiouridine, 5-bromocytidine, 5-iodouridine, inosine, 6-azauridine, 6-chloropurine, 7-deazaadenosine, 7-deazaguanosine, 8-azaadenosine, 8-azidoadenosine, benzimidazole, Ml-methyladenosine, pyrrolo- pyrimidine, 2-amino-6-chloropurine, 3-methyl adenosine, 5-propynylcytidine, 5- propynyluridine, 5-bromouridine, 5-fluorouridine, 5-methylcytidine, 7-deazaadenosine, 7-deazaguanosine, 8-oxoadenosine, 8-oxoguanosine, O(6)-methylguanine, and 2- thiocytidine), chemically or biologically modified bases (*e.g.*, methylated bases), modified sugars (*e.g.*, 2'-fluororibose, 2'-aminoribose, 2'-azidoribose, T-O- methylribose, L-enantiomeric nucleosides arabinose, and hexose), modified phosphate groups (*e.g.*, phosphorothioates and 5'-iV-phosphoramidite linkages), and combinations thereof. Natural and modified nucleic acid residue monomers for the chemical synthesis of nucleic acids are readily available. In some cases, nucleic acids comprising residues having such modifications display improved properties relative to nucleic acids consisting only of naturally occurring residues. In some embodiments, nucleic acid modifications described herein are utilized to reduce and/or prevent digestion by nucleases (*e.g.*, exonucleases, endonucleases, *etc.*). For example, the structure of a polynucleotide may be stabilized by including nucleotide analogs at the 3' end of one or both strands order to reduce digestion.

Modified nucleic acids (*e.g.*, modified polynucleotides) need not be uniformly modified along the entire length of the polynucleotide. Different nucleic acid residue modifications and/or backbone structures may exist at various positions in the polynucleotide. One of ordinary skill in the art will appreciate that the residue analogs or other modification(s) may be located at any position(s) of a polynucleotide such that the function of the polynucleotide is not substantially affected. The modified region may be at the 5 '-end and/or the 3 '-end of one or both strands. For example, modified polynucleotides in which approximately 1 to approximately 5 residues at the 5 ' and/or 3 ' end of either of both strands are residue analogs and/or have a backbone modification have been employed. The modification may be a 5 ' or 3 ' terminal modification. One or both nucleic acid strands may comprise at least 50% unmodified residues, at least 80% unmodified residues, at least 90% unmodified residues, or 100% unmodified residues.

In some embodiments, nucleic acid agents comprise a modification to a sugar, residue, or internucleoside linkage such as those described in U.S. Patent Publications 2003/0175950, 2004/0192626, 2004/0092470, 2005/0020525, and 2005/0032733 (all of which are incorporated herein by reference). In some embodiments, nucleic acid agents are nucleic acid agents having any one or more of the modification described therein. For example, a number of terminal conjugates, *e.g.*, lipids such as cholesterol, lithocholic acid, aluric acid, or long alkyl branched chains have been reported to improve cellular uptake. Analogs and modifications may be tested using, *e.g.*, using any appropriate assay known in the art. In some embodiments, nucleic acid agents may comprise one or more non-natural nucleoside linkages. In some embodiments, one or more internal nucleotides at the 3 '-end, 5 '-end, or both 3'- and 5 '-ends of the nucleic acid are inverted to yield linkages such as a 3' - 3' linkage or a 5' - 5' linkage.

In some embodiments, nucleic acid agents are not synthetic, but are naturally-occurring entities that have been isolated from their natural environments.

In some embodiments, nucleic acid agents comprise only naturally-occurring nucleic acid residues (*e.g.*, nucleotides and/or nucleosides). In some embodiments, nucleic acid agents comprise only unmodified residues. Provided compositions are capable of delivery of both modified and unmodified nucleic acids.

In some embodiments, nucleic acid agents, generally, are ones that have biological activity in the skin (*e.g.*, epidermis and dermis), subcutaneous tissue (*e.g.*, adipose tissue), contiguous muscles, and/or distant tissues (*e.g.*, organs such as lungs, liver, *etc.*).

In some embodiments, nucleic acid agents are or comprise a functional RNA (*e.g.*, antisense oligonucleotide, ribozyme, RNA that participates in forming triple helical structures, *etc.*). In certain embodiments, nucleic acid agents are or comprise an antisense molecule that binds to a translational start site, transcriptional start site, and/or splice junction. In some embodiments, antisense oligonucleotides prevent translation or post-transcriptional processing of RNA. Alternatively or additionally, antisense oligonucleotides may alter transcription of a target gene by binding to DNA of a target gene, such as, for example, a regulatory element.

Typically, antisense RNAs exhibit sufficient complementarity to a target transcript to allow hybridization of the antisense RNA to the target transcript. Mismatches are tolerated, as long as hybridization to the target can still occur. In general, antisense RNAs can be of any length, as long as hybridization can still occur. In some embodiments, antisense RNAs are about 20 nt, about 30 nt, about 40 nt, about 50 nt, about 75 nt, about 100 nt, about 150 nt, about 200 nt, about 250 nt, about 500 nt, or longer. In some embodiments, antisense RNAs comprise an inhibitory region that hybridizes with a target transcript of about 20 nt, about 30 nt, about 40 nt, about 50 nt, about 75 nt, about 100 nt, about 150 nt, about 200 nt, about 250 nt, about 500 nt, or longer.

In some embodiments, nucleic acid agents are or comprise an agent that mediates RNA interference (RNAi). RNAi is a mechanism that inhibits expression of specific genes. RNAi typically inhibits gene expression at the level of translation, but can function by inhibiting gene expression at the level of transcription. RNAi targets include any RNA that might be present in cells, including but not limited to, cellular transcripts, pathogen transcripts (*e.g.*, from viruses, bacteria, fungi, *etc.*), transposons, vectors, *etc.*

In some embodiments, RNAi agents may target any portion of a transcript. In some embodiments, a target transcript is located within a coding sequence of a gene. In some embodiments, a target transcript is located within non-coding sequence. In some embodiments, a target transcript is located within an exon. In some embodiments, a target transcript is located within an intron. In some embodiments, a target transcript is located within a 5' untranslated region (UTR) or 3' UTR of a gene. In some embodiments, a target transcript is located within an enhancer region. In some embodiments, a target transcript is located within a promoter.

For any particular gene target, design of RNAi agents and/or RNAi-inducing agents typically follows certain guidelines. In general, it is desirable to avoid sections of target transcript that may be shared with other transcripts whose degradation is not desired. In some embodiments, RNAi agents and/or RNAi-inducing entities target transcripts and/or portions thereof that are highly conserved. In some embodiments, RNAi agents and/or RNAi-inducing entities target transcripts and/or portions thereof that are not highly conserved.

As used herein, a "small interfering RNA" or "siRNA" refers to an RNAi agent comprising an RNA duplex (referred to herein as a "duplex region") that is approximately 19 basepairs (bp) in length and optionally further comprises one or two single-stranded overhangs. In some embodiments, an siRNA comprises a duplex region ranging from 15 bp to 29 bp in length and optionally further comprising one or two single-stranded overhangs. An siRNA is typically formed from two RNA molecules (*i.e.*, two strands) that hybridize together. One strand of an siRNA includes a portion that hybridizes with a target transcript. In some embodiments, siRNAs mediate inhibition of gene expression by causing degradation of target transcripts.

As used herein, a "short hairpin RNA" or "shRNA" refers to an RNAi agent comprising an RNA having at least two complementary portions hybridized or capable of hybridizing to form a double-stranded (duplex) structure sufficiently long to mediate RNAi (typically at least approximately 19 bp in length), and at least one single-stranded portion, typically ranging between approximately 1 nucleotide (nt) and approximately 10 nt in length that forms a loop. In some embodiments, an shRNA comprises a duplex portion ranging from 15 bp to 29 bp in length and at least one single-stranded portion, typically ranging between approximately 1 nt and approximately 10 nt in length that forms a loop. In some embodiments, the single-stranded portion is approximately 1 nt, approximately 2 nt, approximately 3 nt, approximately 4 nt, approximately 5 nt, approximately 6 nt, approximately 7 nt, approximately 8 nt, approximately 9 nt, or approximately 10 nt in length. In some embodiments, shRNAs are processed into siRNAs by cellular RNAi machinery (*e.g.*, by Dicer). Thus, in some embodiments, shRNAs may be precursors of siRNAs. Regardless, siRNAs in general are capable of inhibiting expression of a target RNA, similar to siRNAs. As used herein, the term "short RNAi agent" is used to refer to siRNAs and shRNAs, collectively.

Short RNAi agents typically include a base-paired region ("duplex region") between approximately 15 nt and approximately 29 nt long, *e.g*., approximately 19 nt long, and may optionally have one or more free or looped ends. RNAi-inducing agents and/or short RNAi agents typically include a region (the "duplex region"), one strand of which contains an inhibitory region between 15 nt to 29 nt in length that is sufficiently complementary to a portion of the target transcript (the "target portion"), so that a hybrid (the "core region") can form in vivo between this strand and the target transcript. The core region is understood not to include overhangs.

In some embodiments, siRNAs comprise 3' overhangs at one or both ends of the duplex region. In some embodiments, an shRNA comprises a 3' overhang at its free end. In some embodiments, siRNAs comprise a single nucleotide 3' overhang. In some embodiments, siRNAs comprise a 3' overhang of 2 nt. In some embodiments, siRNAs comprise a 3' overhang of 1 nt. Overhangs, if present, may, but need not be, complementary to the target transcript. siRNAs with 2 nt - 3 nt overhangs on their 3' ends are frequently efficient in reducing target transcript levels than siRNAs with blunt ends.

In some embodiments, the inhibitory region of a short RNAi agent is 100% complementary to a region of a target transcript. However, in some embodiments, the inhibitory region of a short RNAi agent is less than 100% complementary to a region of a target transcript. The inhibitory region need only be sufficiently complementary to a target transcript such that hybridization can occur, *e.g.*, under physiological conditions in a cell and/or in an *in vitro* system that supports RNAi (*e.g.*, a *Drosophila* extract system).

One of ordinary skill in the art will appreciate that short RNAi agent duplexes may tolerate a mismatches and/or bulges, particularly mismatches within the central region of the duplex, while still leading to effective silencing. One of skill in the art will also recognize that it may be desirable to avoid mismatches in the central portion of the short RNAi agent/target transcript core region (see, *e.g.*, Elbashir et al., EMBO J. 20:6877, 2001; incorporated herein by reference). For example, the 3' nucleotides of the antisense strand of the siRNA often do not contribute significantly to specificity of the target recognition and may be less critical for target cleavage.

Micro RNAs (miRNAs) are genomically encoded non-coding RNAs of about 21 - 23 nucleotides in length that help regulate gene expression, particularly during development (see, *e.g.,* Bartel, 2004, Cell, 116:281; Novina and Sharp, 2004, Nature, 430:161; and U.S. Patent Publication 2005/0059005; also reviewed in Wang and Li, 2007, Front. Biosci., 12:3975; and Zhao, 2007, Trends Biochem. Sci., 32:189; each of which are incorporated herein by reference). The phenomenon of RNA interference, broadly defined, includes the endogenously induced gene silencing effects of miRNAs as well as silencing triggered by foreign dsRNA. Mature miRNAs are structurally similar to siRNAs produced from exogenous dsRNA, but before reaching maturity, miRNAs first undergo extensive post-transcriptional modification. An miRNA is typically expressed from a much longer RNA-coding gene as a primary transcript known as a pri-miRNA, which is processed in the cell nucleus to a 70-nucleotide stem-loop structure called a pre- miRNA by the microprocessor complex. This complex consists of an RNase III enzyme called Drosha and a dsRNA-binding protein Pasha. The dsRNA portion of this pre- miRNA is bound and cleaved by dicer to produce the mature miRNA molecule that can
be integrated into the RISC complex; thus, miRNA and siRNA share the same cellular machinery downstream of their initial processing (Gregory et al, 2006, *Meth. Mol. Biol.,* 342:33; incorporated herein by reference). In general, miRNAs are not perfectly complementary to their target transcripts.

In some embodiments, miRNAs can range between 18 nt - 26 nt in length. Typically, miRNAs are single-stranded. However, in some embodiments, miRNAs may be at least partially double-stranded. In certain embodiments, miRNAs may comprise an RNA duplex (referred to herein as a "duplex region") and may optionally further comprises one or two single-stranded overhangs. In some embodiments, an RNAi agent comprises a duplex region ranging from 15 bp to 29 bp in length and optionally further comprising one to three single-stranded overhangs. An miRNA may be formed from two RNA molecules that hybridize together, or may alternatively be generated from a single RNA molecule that includes a self-hybridizing portion. The duplex portion of an miRNA usually, but does not necessarily, comprise one or more bulges consisting of one or more unpaired nucleotides. One strand of an miRNA includes a portion that hybridizes with a target RNA. In certain embodiments, one strand of the miRNA is not precisely complementary with a region of the target RNA, meaning that the miRNA hybridizes to the target RNA with one or more mismatches. In some embodiments, one strand of the miRNA is precisely complementary with a region of the target RNA, meaning that the miRNA hybridizes to the target RNA with no mismatches. Typically, miRNAs are thought to mediate inhibition of gene expression by inhibiting translation of target transcripts. However, in some embodiments, miRNAs may mediate inhibition of gene expression by causing degradation of target transcripts.

In certain embodiments, nucleic acid agents are or comprise a ribozyme designed to catalytically cleave target mRNA transcripts may be used to prevent translation of a target mRNA and/or expression of a target (see, *e.g.*, PCT publication WO 90/11364; and Sarver et al., 1990, Science 247:1222; both of which are incorporated herein by reference).

In certain embodiments, nucleic acid agents are or comprise a nucleic acid that participates in forming triple helical structures. Endogenous target gene expression may be reduced by targeting deoxyribonucleotide sequences complementary to the regulatory region of the target gene (*e.g.*, the target gene's promoter and/or enhancers) to form triple helical structures that prevent transcription of the target gene in target muscle cells in the body (see generally, Helene, 1991, *Anticancer Drug Des.,* 6:569; Helene *et al.*, 1992, *Ann K Y. Acad. Sci.,* 660:27; and Maher, 1992, *Bioassays* 14:807; all of which are incorporated herein by reference).

In some instances, the mechanism by which a particular nucleic acid agent exerts a therapeutic effect and/or affects a biological activity is unknown. The present invention contemplates the use of such nucleic acid agents. In some embodiments, the methods of the present invention may be used for delivery (*e.g.*, transdermal delivery) of biologically active nucleic acid agents (*e.g.*, polynucleotides and/or nucleic acid residues) that have not yet been identified or characterized.

In some embodiments, nucleic acid agents improve wound healing. Such nucleic acid agents include DNA coding for keratinocyte growth factor-1 (KGF-I) (Lin et al., 2006, Wound Repair Regen., 14:618; incorporated herein by reference). In some embodiments, nucleic acids coding for KGF-I are characterized by all or a portion of a sequence as set forth in GenBank sequence NC 000015.

In some embodiments, nucleic acid agents may promote skin wound healing. Such nucleic acid agents include Connexin43 (Cx43) antisense oligonucleotides, RNAi agents, siRNAs, shRNAs, and/or miRNAs (Mori et al., 2006, J. Cell Sci., 119:5193; incorporated herein by reference). In some embodiments, antisense oligonucleotides, RNAi agents, siRNAs, shRNAs, and/or miRNAs may target one or more regions and/or characteristic portions of a nucleic acid having a sequence such as that set forth in GenBank sequence AK312324.

In some embodiments, nucleic acid agents may suppress abnormal skin cell proliferation and enhance skin wound healing. Such nucleotides may include nucleotides and monophosphate nucleosides, diphosphate nucleosides, and triphosphate nucleosides. In some embodiments, such nucleic acid agents include adenine nucleotides and adenosine and adenosine triphosphate nucleosides (Brown et al., 2000, J. Invest. Dermatol., 115:849; and Wang et al., 1990, Biochem. Biophys. Res. Commun., 166:251; all of which are incorporated herein by reference).

In some embodiments, nucleic acid agents stimulate interferon production which inhibits collagen synthesis in the skin and has the potential to treat scleroderma, a tissue connective disease. Such nucleic acid agents include those that encode interferon gamma (IFNγ) (Badea et al., 2005, J. Gene Med., 7:1200; and Gray and Goeddel, 1983, Proc. Natl. Acad. Sci., USA, 80:5842; both of which are incorporated herein by reference). In some embodiments, nucleic acids coding for IFNγ are characterized by all or a portion of a sequence as set forth in GenBank sequence NM OOl 127598, NM 000612, NM OO 1007139, or NC 000076.

In some embodiments, nucleic acid agents treat the skin disorder pachyonychia congenita. Such nucleic acid agents include siRNAs which target genes encoding keratin in the skin (Hickerson et al, 2006, Ann. N. Y. Acad. Sci., 1082:56; incorporated herein by reference). In specific embodiments, such siRNAs may comprise one or more of any one of the following antisense sequences: AAACUUGUUUUUGAGGGUCU (SEQ ID NO.: 1); UUUUUGAGGGUCUUGAUCU (SEQ ID NO.: 2); UUUUGAGGGUCUUGAUCUGU (SEQ ID NO.: 3); UUUGAGGGUCUUGAUCUGUU (SEQ ID NO.: 4); AAGGAGGCAAACUUGUUUUU (SEQ ID NO.: 5); AACUUGUUGAGGGUCUUGAU (SEQ ID NO.: 6); AAACUUGUUGAGGGUCUUGU (SEQ ID NO.: 7); and CAAACUUGUUGAGGGUCUUU (SEQ ID NO.: 8) (Hickerson et al., 2006, Ann. N.Y. Acad. Sci., 1082:56; incorporated herein by reference).

In some embodiments, nucleic acid agents suppress molecular pathways that cause skin inflammation. Such nucleic acid agents include cytidine-phosphate-guanosine oligodeoxynucleotides, including two cytidine- phosphate-guanosine (CpG) motifs (CpG- 1-phosphorothioate (PTO)) and a poly-cytidine motif (Non-CpG-5 -PTO) (Pivarcsi, 2007, J. Invest. Dermatol., 127:846; and Dorn et al., 2007, J. Invest. Dermatol., 127:746; both of which are incorporated herein by reference). In some embodiments, such nucleic acid agents may comprise phosphorothioate linkages. In some embodiments, such nucleic acid agents may comprise phosphodiester linkages. In specific embodiments, such nucleic acid agents may comprise one or more of any one of the following nucleotide sequences:
TCCATGACGTTCCTGACGTT (SEQ ID NO.: 9); TCCATGACGTTCCTGACGTT (SEQ ID NO.: 10); TCCATGACGTTCCTGACGT (SEQ ID NO.: 11);
TCCATGACGTTCCTGACG (SEQ ID NO.: 12); TCCTCGACGTCCCTGA(SEQ ID NO.: 13); CATGACGTTCCT (SEQ ID NO.: 14); GACGTT (SEQ ID NO.: 15); and
AACGTCAGGAACGTCATGGA (SEQ ID NO.: 16) (Pivarcsi, 2007, J. Invest. Dermatol, 127:846; and Dorn *et al.*, 2007, J. Invest. Dermatol, 127:746; both of which are incorporated herein by reference).

In some embodiments, nucleic acid agents may reduce VEGF production in the skin, which could have anti-angiogenesis therapeutic effects for conditions such as psoriasis. Such nucleic acid agents include a 19-mer antisense phosphorothioate oligodeoxynucleotide (complementary to bases 6-24 relative to the translational start site of the VEGF/VPF mRNA) (Smyth *et al.*, 1997, *J. Invest. Dermatol.,* 108:523; incorporated herein by reference). In specific embodiments, such nucleic acid agents may comprise one or more of any one of the following antisense nucleotide sequences: CACCCAAGACAGCAGAAAG (SEQ ID NO.: 17); CTCCCAAGAC AGCAGAAAG (SEQ ID NO.: 18); CTGCCAAGACAGCAGAAAG (SEQ ID NO.: 19); CACCCAACTCTCCAGAAAG (SEQ ID NO.: 20); CACCCAAGACAGCAGAATG (SEQ ID NO.: 21); and CACCCAAGACAGCAGATTG (SEQ ID NO.: 22) (Smyth et al., 1997, J. Invest. Dermatol., 108:523; incorporated herein by reference).

In some embodiments, nucleic acid agents may improve the DNA repair rate of skin damaged by UV radiation (*e.g.*, UV radiation from sun exposure and/or sun burn). In some embodiments, nucleic acid agents may prevent or delay the onset of skin cancer. In some embodiments, nucleic acid agents may stimulate melanocyte production to result in tanning of the skin. In some embodiments, nucleic acid agents may achieve the appearance of tanning of the skin. Such nucleic acid agents may include dipyrimidine sequences (*e.g.*, TT, TC, CT, CC). In some embodiments, such nucleic acid agents include thymidine dinucleotide (pTT), DNA oligonucleotides substantially homologous to the telomere 3' overhang sequence, and a 5'-phosphorylated 9 base oligonucleotide (p9mer). In some embodiments, nucleic acid agents to be used in accordance with the present invention may treat various kinds of cancer (*e.g.*, breast cancer, pancreatic cancer, ovarian cancer, melanoma, gliomas, *etc.*). Such nucleic acid agents include oligonucleotides substantially homologous to the telomere 3' overhang sequence. In specific embodiments, the telomere 3' overhang sequence comprises one or more of the following sequences: TTAGGG (SEQ ID NO.: 23); GTTAGGGTT AG (SEQ ID NO.: 24); GGTTAGGTGTAGGTTT (SEQ ID NO.: 25); GTTAGGGTT (SEQ ID NO.: 26); TTAGGGTTA (SEQ ID NO.: 27); GTTAGGTTTAAGGTT (SEQ ID NO.: 28); GGTAGGTGTAGGGTG (SEQ ID NO.: 29); GGTCGGTGTCGGGTG (SEQ ID NO.: 30); GGCAGGCGCAGGGCG (SEQ ID NO.: 31); GTTAGGGTTAGGGTT (SEQ ID NO.: 32); GATAAGGGATTGGGAT (SEQ ID NO.: 33); GAGTATGAG (SEQ ID NO.: 34); GGGTTAGGG (SEQ ID NO.: 35); GTT AGGGTTAG (SEQ ID NO.: 36); GGTAGGTGTAGGATT (SEQ ID NO.: 37); GGTAGGTGTAGGATTT (SEQ ID NO.: 38); GGTTAGGTGTAGGTTT (SEQ ID NO.: 39); GGTTAGGTGGAGGTTT (SEQ ID NO.: 40); GGTTAGGTTTAGGTTT (SEQ ID NO.: 41); GGTTAGGTTAAGGTTA (SEQ ID NO.: 42); GGTAGGTGTAGGGTG (SEQ ID NO.: 43); GTTAGGGTTAGGGTTA (SEQ ID NO.: 44); GGTTGGTTGGTTGGTT (SEQ ID NO.: 45); CCTTGGTTGGTTGGTTGGTT (SEQ ID NO.: 46); and GGTTGGTTGGTTGGTTGGTT (SEQ ID NO.: 47). In specific embodiments, the p9mer comprises the following nucleotide sequence: pGAGTATGAG (SEQ ID NO.: 34) (Goukassian et al., 2004, Proc. Natl. Acad. Sci., USA, 101 :3933; Arad et al., 2006, FASEB J., 20:1895; Yaar et al., 2007, Breast Cancer Res., 9:R13; Goukassian et al., 2002, FASEB J., 16:754; and Ohashi et al., 2007, J. Cell Physiol., 210:582; all of which are incorporated herein by reference).

In some embodiments, nucleic acid agents may treat melanoma skin cancer (*e.g.*, metastatic melanoma skin cancer). Such nucleic acid agents include a toll-like receptor 9-activating oligonucleotide (Pashenkov et al., 2006, J. Clin. Oncol., 24:5716; incorporated herein by reference). In specific embodiments, such nucleic acids may comprise the following nucleotide sequence: TCGTCGTTTTGTCGTTTTGTCGTT (SEQ ID NO.: 48) (Pashenkov et al., 2006, J. Clin. Oncol., 24:5716; incorporated herein by reference).

In some embodiments, a nucleic acid sequence that is homologous to any of the nucleic acids described herein may be used in accordance with the present invention. In some embodiments, nucleic acid sequences are considered to be "homologous" to one another if they comprise fewer than 30, 25, 20, 15, 10, 5, 4, 3, 2, or 1 nucleic acid substitutions relative to one another. In some embodiments, nucleic acid sequences are considered to be "homologous" to one another if their sequences are at least 25%, 30%, 35%, 40%, 45%, 50%, 55%, 60%, 65%, 70%, 75%, 80%, 85%, 90%, 95%, or 99% identical. In some embodiments, nucleic acid sequences are considered to be "homologous" to one another if their sequences are at least 25%, 30%, 35%, 40%, 45%, 50%, 55%, 60%, 65%, 70%, 75%, 80%, 85%, 90%, 95%, or 99% similar.

### Protein Agents

Any of a variety of protein agents may be incorporated in provided compositions. In some embodiments, protein agents are peptide agents. In some embodiments, it a peptide is less than about 100 amino acids in length; in some embodiments, a peptide is less than about 90, about 80, about 70, about 65, about 60, about 55, about 50, about 45, about 40, about 35, about 30, about 25, about 20, about 15, about 13, about 12, about 10, about 9, about 8, about 7, about 6, or about 5 amino acids in length. In some specific embodiments, the peptide agent is a penta peptide. In some embodiments, a peptide agent is comprised solely of naturally occurring amino acids. In some embodiments, a peptide agent comprises one or more non-naturally occurring amino acid.

Unmodified short peptides for use in accordance with the present invention, generally, are ones that have biological activity in the skin (including epidermis and dermis), sub-cutaneous tissue (including adipose tissue) and/or contiguous muscles. Such peptides include, but are not limited to, peptides to promote extra-cellular matrix production (*e.g.*, KTTKS, SEQ ID NO.: 49; EYKTTKSSRL, SEQ ID NO.: 50; VIEYKTTK, SEQ ID NO.: 51; KTTK, SEQ ID NO.: 52; GKTVIEYKTTKS, SEQ ID NO.: 53; GKTVIEYKTTKSSRL, SEQ ID NO.: 54; WGKTVIEYKTTKSSRLPIID, SEQ ID NO.: 55; CTSHTGAWGKTVIEYKTTKS, SEQ ID NO.: 56; TTKS, SEQ ID NO.: 57), peptides that may decrease wrinkles (*e.g.*, EEMQRR, SEQ ID NO.: 58), peptides to improve wound healing (*e.g.*, gastrin-releasing peptide, VGVAPG, SEQ ID NO.: 59; YYRADA, SEQ ID NO.: 60; GHK, SEQ ID NO.: 61, interferon, interferon inducer), and peptides (*e.g.*, P144; TSLDASIIWAMMQN, SEQ ID NO.: 62) to treat excessive accumulation of extra-cellular matrix that are result in conditions such as hypertrophic scarring, keloids, and localized or systemic sclerosis (scleroderma) (Katayama, et al. 1993, J. Biol. Chem., 268:9941; Lupo, 2005, Dermatol. Surg., 31:832; Robinson et al., International J. Cosmetic Science, 27:155; Bhartiya et al., 1992, J. Cell. Physiol, 150:312; and Santiago et al., 2005, J. Investigative Dermatology, 125:450; all of which are incorporated herein by reference). See Table 5 below for definitions of peptide abbreviations.

**Table 5: Peptide Abbreviations**

| **Trivial name^{a}** | **Symbols^{b}** | | **Systematic Name^{c}** | **Formula** |
|---|---|---|---|---|
| Alanine | Ala | A | 2-Aminopropanoic acid | CH₃-CH(NH₂)-COOH |
| Arginine | Arg | R | 2-Amino-5-guanidinopentanoic acid | H₂N-C(=NH)-NH-[CH₂]₃-CH(NH₂)-COOH |
| Asparagine | Asn^{d} | N^{d} | 2-Amino-3-carbamoylpropanoic acid | H₂N-CO-CH₂-CH(NH₂)-COOH |
| Aspartic acid | Asp^{d} | D^{d} | 2-Aminobutanedioic acid | HOOC-CH₂-CH(NH₂)-COOH |
| Cysteine | Cys | C | 2-Amino-3-mercaptopropanoic acid | HS-CH₂-CH(NH₂)-COOH |
| Glutamine | Gln^{d} | Q^{d} | 2-Amino-4-carbamoylbutanoic acid | H₂N-CO-[CH₂]₂-CH(NH₂)-COOH |
| Glutamic acid | acidGlu^{d} | E^{d} | 2-Aminopentanedioic acid | HOOC-[CH₂]₂-CH(NH₂)-COOH |
| Glycine | Gly | G | Aminoethanoic acid | CH₂(NH₂)-COOH |
| Histidine | His | H | 2-Amino-3-(1*H*-imidazol-4-yl)-propanoic acid | 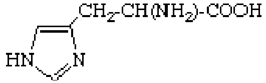 |
| Isoleucine | Ile | I | 2-Amino-3-methylpentanoic acid^{e} | C₂H₅-CH(CH₃)-CH(NH₂)-COOH |
| Leucine | Leu | L | 2-Amino-4-methylpentanoic acid | (CH₃)₂CH-CH₂-CH(NH₂)-COOH |
| Lysine | Lys | K | 2,6-Diaminohexanoic acid | H₂N-[CH₂]₄-CH(NH₂)-COOH |
| Methionine | Met | M | 2-Amino-4-(methylthio)butanoic acid | CH₃-S-[CH₂]₂-CH(NH₂)-COOH |
| Phenylalanine | Phe | F | 2-Amino-3-phenylpropanoic acid | C₆H₅-CH₂-CH(NH₂)-COOH |
| Proline | Pro | P | Pyrrolidine-2-carboxylic acid | 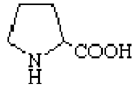 |
| Serine | Ser | S | 2-Amino-3-hydroxypropanoic acid | HO-CH₂-CH(NH₂)-COOH |
| Threonine | Thr | T | 2-Amino-3-hydroxybutanoic acid | CH₃-CH(OH)-CH(NH₂)-COOH |
| Tryptophan | Trp | W | 2-Amino-3-(1*H*-indol-3-yl)-propanoic acid | 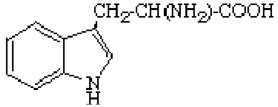 |
| Tyrosine | Tyr | Y | 2-Amino-3-(4-hydroxyphenyl)-propanoic acid | 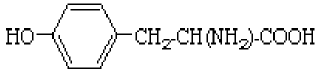 |
| Valine | Val | V | 2-Amino-3-methylbutanoic acid | (CH₃)₂CH-CH(NH₂)-COOH |

### Botulinum Toxin

In some embodiments, a protein agent is botulinum toxin. Botulinum toxin (BTX) BTX is produced in nature by the anaerobic, gram positive bacillus *Clostridium botulinum* and is a potent polypeptide neurotoxin. Most notably, BTX causes a neuroparalytic illness in humans and animals referred to as botulism. BTX can apparently pass through the lining of the gut and attack peripheral motor neurons. Symptoms of botulinum toxin intoxication can progress from difficulty walking, swallowing, and speaking to paralysis of the respiratory muscles, and death.

BTX-A is the most lethal natural biological agent known to man. The LD₅₀ in female Swiss Webster mice (18 g - 20 g) for commercially available BTX-A is about 50 picograms; this amount is defined as 1 Unit of BTX-A. On a molar basis, BTX-A is about 1.8 billion times more lethal than diphtheria, about 600 million times more lethal than sodium cyanide, about 30 million times more lethal than cobra toxin and about 12 million times more lethal than cholera (Singh, et al., ed., "Critical Aspects of Bacterial Protein Toxins" Natural Toxins II, pp. 63-84, Plenum Press, New York, 1996).

The different serotypes of botulinum toxin vary in the animal species that they affect and in the severity and duration of the paralysis they evoke. For example, it has been determined that BTX-A is 500 times more potent than is BTX-B, as measured by the rate of paralysis produced in the rat. Additionally, BTX-B has been determined to be non-toxic in primates at a dose of 480 U/kg, which is about 12 times the primate LD₅₀ for BTX-A. Furthermore, it is known that botulinum toxin type B has, upon intramuscular injection, a shorter duration of activity and is also less potent than BTX-A at the same dose level.

Botulinum toxin apparently binds with high affinity to cholinergic motor neurons, is translocated into the neuron and blocks the release of acetylcholine and other pre-formed mediators and transmitters. For example, *in vitro* studies performed on neurons other than motor neurons revealed that botulinum toxin not only blocks acetylcholine release, but can also prevent liberation of other neurotransmitters (*e.g*., neurotransmitters stored in vesicles), including small organic molecules and neuropeptides (*e.g*., adrenaline; noradrenaline; dopamine; glutamate; aspartate; glycine; GABA; ATP that is co-stored with neurotransmitters such as acetylcholine and/or glutamate; substance P; and/or CGRP) (Poulain, 2008, Botulinum J., 1:14; incorporated herein by reference).

Botulinum toxins have been used in clinical settings for the treatment of certain neuromuscular disorders. In particular, BTX-A has been approved by the U.S. Food and Drug Administration for the treatment of cervical dystonia in adults to decrease the severity of abnormal head position and neck pain associated with cervical dystonia; the treatment of severe primary axillary hyperhidrosis that is inadequately managed with topical agents; the treatment of strabismus and blepharospasm associated with dystonia, including benign essential blepharospasm or VII nerve disorders in patients 12 years of age and above; and for the temporary improvement in the appearance of moderate to severe glabellar lines associated with corrugator and/or procerus muscle activity in adult patients ≤ 65 years of age.

Clinical effects of peripheral intramuscular BTX-A are usually seen within one week of injection. The typical duration of symptomatic relief from a single intramuscular injection of BTX-A averages about three months.

Although all the botulinum toxins serotypes apparently inhibit release of the neurotransmitter acetylcholine at the neuromuscular junction, they do so by affecting different neurosecretory proteins and/or cleaving these proteins at different sites. For example, botulinum types A and E both cleave the 25 kilodalton (kD) synaptosomal associated protein (SNAP-25), but they target different amino acid sequences within this protein. Botulinum toxin types B, D, F and G act on vesicle-associated membrane protein (VAMP, also called synaptobrevin), with each serotype cleaving the protein at a different site. Finally, botulinum toxin type C₁ has been shown to cleave both syntaxin and SNAP-25. These differences in mechanism of action may affect the relative potency and/or duration of action of the various botulinum toxin serotypes. The cytosol of pancreatic islet B cells contains at least SNAP-25 (Gonelle-Gispert et al., 1999, Biochem. J., 339 (pt 1): 159-65; incorporated herein by reference), and synaptobrevin (1995, Mov. Disord., 10: 376; incorporated herein by reference).

The molecular weight of a botulinum toxin protein molecule, for all seven of the known botulinum toxin serotypes, is about 150 kD. Botulinum toxins are released by the *Clostridium* bacterium as complexes comprising the 150 kD botulinum toxin protein molecule along with associated non-toxin proteins. Thus, the BTX-A complex can be produced by the *Clostridium* bacterium as 900 kD, 500 kD and 360 kD forms. Botulinum toxin types B and C₁ are apparently produced as only a 500 kD complex. Botulinum toxin type D is produced as both 300 kD and 500 kD complexes. Finally, botulinum toxin types E and F are produced as only approximately 300 kD complexes.

BTX complexes (*i.e.*, those compositions having molecular weights greater than about 150 kD) are believed to contain a non-toxin hemagglutinin protein and a non-toxin and non-toxic non-hemagglutinin protein. These two non-toxin proteins (which along with the botulinum toxin molecule comprise the relevant neurotoxin complex) may act to provide stability against denaturation to the botulinum toxin molecule and protection against digestive acids when toxin is ingested.

Either BTX proteins or BTX complexes may be utilized as known therapeutic agents and/or independently active biologically active agents in accordance with the present invention. Indeed, it will be appreciated by those of ordinary skill in the art that any portion or fragment of a BTX protein or complex that retains the appropriate activity may be utilized as described herein.

*In vitro* studies have indicated that botulinum toxin inhibits potassium cation induced release of both acetylcholine and norepinephrine from primary cell cultures of brainstem tissue. Additionally, it has been reported that botulinum toxin inhibits the evoked release of both glycine and glutamate in primary cultures of spinal cord neurons and that in brain synaptosome preparations botulinum toxin inhibits the release of each of the neurotransmitters acetylcholine, dopamine, norepinephrine, CGRP and glutamate.

As noted above, botulinum toxin for use in accordance with the present invention can be derived from any source. For purposes of completeness, however, we note that a variety of sources, including commercial sources, for certain botulinum toxin preparations are readily available.

In some embodiments, botulinum toxin is selected from the group consisting of type A, type Ab, type Af, type B, type Bf, type C1, type C2, type D, type E, type F, and type G; mutants thereof; variants thereof; fragments thereof; characteristic portions thereof; and/or fusions thereof. In some embodiments, botulinum toxin is present as any of the subtypes described in Sakaguchi, 1982, Pharmacol. Ther., 19:165; and/or Smith et al., 2005, Infect. Immun., 73:5450; both of which are incorporated herein by reference.

For example, BTX or BTX complex can be obtained by establishing and growing cultures of *Clostridium botulinum* in a fermenter and then harvesting and purifying the fermented mixture in accordance with known procedures. All the botulinum toxin serotypes are initially synthesized as inactive single chain proteins which must be cleaved or nicked by proteases to become neuroactive. The bacterial strains that make botulinum toxin serotypes A and G possess endogenous proteases. Therefore, serotypes A and G can be recovered from bacterial cultures in predominantly their active form. In contrast, botulinum toxin serotypes C₁, D and E are synthesized by nonproteolytic strains and are therefore typically unactivated when recovered from culture. Serotypes B and F are produced by both proteolytic and nonproteolytic strains and therefore can be recovered in either the active or inactive form. However, even the proteolytic strains that produce, for example, the BTX-A serotype typically only cleave a portion of the toxin produced. The exact proportion of nicked to unnicked molecules can depend on the length of incubation and the temperature of the culture. Therefore, a certain percentage of any preparation of, for example BTX-A, is likely to be inactive. The presence of inactive botulinum toxin molecules in a clinical preparation will contribute to the overall protein load of the preparation, which has been linked in some commercially available botulinum toxin preparations to increased antigenicity, without contributing to its clinical efficacy.

High quality crystalline botulinum toxin type A can be produced from the Hall A strain of *Clostridium botulinum* with characteristics of ≥ 3 × 10⁷ U/mg, an A₂₆₀/A₂₇₈ of less than 0.60 and a distinct pattern of banding on gel electrophoresis. The known Schantz process can be used to obtain crystalline botulinum toxin including type A (Shantz et al., 1992, Microbiol. Rev., 56:80; incorporated herein by reference).

Generally, the botulinum toxin complex can be isolated and purified from an anaerobic fermentation by cultivating *Clostridium botulinum* (*e.g.*, type A) in a suitable medium. The known process can be used, upon separation out of the non-toxin proteins, to obtain pure botulinum toxins, such as for example: purified botulinum toxin type A with an approximately 150 kD molecular weight with a specific potency of 1-2 × 10⁸ LD₅₀ U/mg or greater; purified botulinum toxin type B with an approximately 156 kD molecular weight with a specific potency of 1-2 × 10⁸ LD₅₀ U/mg or greater, and; purified botulinum toxin type F with an approximately 155 kD molecular weight with a specific potency of 1-2 × 10⁷ LD₅₀ U/mg or greater.

Alternatively or additionally, already prepared and purified botulinum toxins and toxin complexes can be obtained from, for example, List Biological Laboratories, Inc., Campbell, CA; the Centre for Applied Microbiology and Research, Porton Down, U.K.; Wako (Osaka, Japan) as well as from Sigma Chemicals of St. Louis, MO.

Pure botulinum toxin, when administered as a free solution, is so labile that it is generally not used to prepare a pharmaceutical composition. Furthermore, the botulinum toxin complexes, such the toxin type A complex can also be susceptible to denaturation due to surface denaturation, heat, and alkaline conditions. In some cases, inactivated toxin forms toxoid proteins which may be immunogenic. Resulting antibodies can render a patient refractory to toxin injection.

In some embodiments, the present invention provides botulinum toxin compositions, including, but not limited to, botulinum toxin nanoparticle compositions (*e.g.*, nanoemulsions) in which the botulinum toxin has improved stability when compared to currently administered free solutions. That is, in some embodiments, botulinum toxin present in a nanoparticle composition is protected, at least in part, from at least one adverse condition such as heat, alkaline conditions, acidic conditions, degradative enzymes, host organism antibodies, *etc.* Alternatively or additionally, botulinum toxin present in nanoparticle compositions may show less surface denaturation than an otherwise comparable preparation of botulinum toxin in free solution. Surface denaturation refers to protein degradation that results from interactions of proteins with surfaces (*e.g.*, walls of a container in which proteins are stored) or with air (*e.g.*, at the interface between a nanoparticle composition and air).

Indeed, one surprising aspect of the present invention encompasses the recognition that botulinum toxin may be stabilized by incorporation into a nanoparticle composition. Those of ordinary skill in the art will readily appreciate that a nanoparticle composition according to this aspect of the present invention may be prepared by any available means. In some embodiments, the present invention allows use of isolated botulinum toxin rather than botulinum toxin complex, at least in part due to the additional stability imparted by incorporation into a nanoparticle composition.

The present invention further provides botulinum toxin compositions, including, but not limited to, botulinum toxin nanoparticle compositions (*e.g*., nanoemulsions) in which the botulinum toxin has improved ability to permeate skin when compared to currently administered free solutions. In some embodiments, the minimal time between administration and intracellular accumulation results in a method of administration having improved efficacy and decreased side effects.

Moreover, as demonstrated herein, the present invention provides botulinum toxin compositions, including, but not limited to, botulinum toxin nanoparticle compositions from which botulinum toxin can cross the skin without requiring alteration or disruption of skin structures. For example, commercially available technologies for transdermal administration of biologically active agents traditionally require chemical, physical, electrical or other disruption of at least the outer layer of skin. Such disruption can cause irritation, undesirable medical side-effects, and/or unwanted aesthetic outcomes. The present invention provides botulinum toxin compositions, including, but not limited to, botulinum toxin nanoparticle compositions that, when administered to skin, do not significantly or noticeably irritate the skin and/or erode the stratum corneum, and yet allow botulinum toxin to permeate the skin to have its biological effects.

In some embodiments, provided compositions comprising botulinum toxin (*e.g*., botulinum nanoemulsions) are useful for topical and/or transdermal (*e.g*., by lotions, creams, liniments, ointments, powders, gels, drops, *etc.*) routes of administration.

As with proteins generally, the biological activities of the botulinum toxins (which are intracellular peptidases) can be affected by changes in three dimensional conformation. Thus, botulinum toxin type A can be detoxified by heat, various chemicals, surface stretching and surface drying. Additionally, it is known that dilution of the toxin complex obtained by the known culturing, fermentation and purification to the much, much lower toxin concentrations used for pharmaceutical composition formulation results in rapid detoxification of the toxin unless a suitable stabilizing agent is present. Dilution of the toxin from milligram quantities to a solution containing nanograms per milliliter presents significant difficulties because of the rapid loss of specific toxicity upon such great dilution. Since the toxin may be used months or years after the toxin containing pharmaceutical composition is formulated, solution preparations of the toxin may be formulated with a stabilizing agent, such as gelatin, albumin, and/or combinations thereof.

As noted above, the present invention may provide stabilized preparations of botulinum toxin. Notwithstanding the additional stability that may be imparted by the formulation itself, in some embodiments, use of additional stabilizers is contemplated. For example, in some embodiments, at least one additional protein is used together with the botulinum toxin. In some embodiments, this additional protein comprises gelatin. In some embodiments, this additional protein comprises albumin. In some embodiments, this additional protein comprises one or more of the proteins naturally found in a botulinum toxin complex. Indeed, in some embodiments, a complete botulinum toxin complex is employed. In some such embodiments, gelatin and/or albumin is also utilized. Thus, in some embodiments, the present invention provides a botulinum nanoemulsion (*e.g.*, microfluidized nanoemulsion) comprising albumin. In some embodiments, the present invention provides a botulinum nanoemulsion (*e.g*., microfluidized nanoemulsion) that does not comprise albumin. In some embodiments, the present invention provides a botulinum nanoemulsion (*e.g*., microfluidized nanoemulsion) comprising gelatin. In some embodiments, the present invention provides a botulinum nanoemulsion (*e.g*., microfluidized nanoemulsion) that does not comprise gelatin.

In some embodiments, the botulinum toxin utilized is BOTOX® (Allergan, Inc.). BOTOX® consists of a purified botulinum toxin type A complex, albumin and sodium chloride packaged in sterile, vacuum-dried form.

The botulinum toxin type A present in BOTOX® is made from a culture of the Hall strain of *Clostridium botulinum* grown in a medium containing N-Z amine and yeast extract. The botulinum toxin type A complex is purified from the culture solution by a series of acid precipitations to a crystalline complex (see, *e.g.,* Shantz et al., 1992, Microbiol. Rev., 56:80; incorporated herein by reference) consisting of the active high molecular weight toxin protein and at least one associated hemagglutinin protein. The crystalline complex is redissolved in a solution containing saline and albumin and sterile filtered (0.2 microns) prior to vacuum-drying. BOTOX® can be reconstituted with sterile, non-preserved saline prior to intramuscular injection. Each vial of BOTOX® contains about 100 units (U) of *Clostridium botulinum* toxin type A purified neurotoxin complex, 0.5 milligrams of human serum albumin, and 0.9 milligrams of sodium chloride in a sterile, vacuum-dried form without a preservative.

Currently, BOTOX® is typically reconstituted with 0.9% sodium chloride for administration by injection. Since there is a concern that BOTOX® can be denatured by bubbling or similar violent agitation, it is recommended that the diluent be gently injected into the vial. BOTOX®, as a free solution, is recommended to be administered within four hours after reconstitution. Further, between reconstitution and injection, it is further recommended that reconstituted BOTOX® be stored in a refrigerator (*i.e.*, for example, between 2° to 8° C). Reconstituted BOTOX® is clear, colorless and free of particulate matter.

It has been reported that BOTOX® has been used in clinical settings as follows (for a review, see, *e.g.,* Poulain, 2008, *Botulinum J.,* 1:14; incorporated herein by reference):
(1) about 75 U - 125 U of BOTOX® per intramuscular injection (multiple muscles) to treat cervical dystonia;
(2) 5 U - 10 U of BOTOX® per intramuscular injection to treat glabellar lines (brow furrows) (5 units injected intramuscularly into the procerus muscle and 10 units injected intramuscularly into each corrugator supercilli muscle);
(3) about 30 U - 80 U of BOTOX® to treat constipation by intrasphincter injection of the puborectalis muscle;
(4) about 1 U - 5 U per muscle of intramuscularly injected BOTOX® to treat blepharospasm by injecting the lateral pre-tarsal orbicularis oculi muscle of the upper lid and the lateral pre-tarsal orbicularis oculi of the lower lid.
(5) to treat strabismus, extraocular muscles have be in injected intramuscularly with between about 1 U -5 U of BOTOX®, the amount injected varying based upon both the size of the muscle to be injected and the extent of muscle paralysis desired (*i.e*., amount of diopter correction desired).
(6) to treat upper limb spasticity following stroke by intramuscular injections of BOTOX® into five different upper limb flexor muscles, as follows:
   (a) flexor digitorum profundus: 7.5 U to 30 U
   (b) flexor digitorum sublimus: 7.5 U to 30 U
   (c) flexor carpi ulnaris: 10 U to 40 U
   (d) flexor carpi radialis: 15 U to 60 U
   (e) biceps brachii: 50 U to 200 U
   Each of the five indicated muscles has been injected at the same treatment session, so that the patient receives from 90 U to 360 U of upper limb flexor muscle BOTOX® by intramuscular injection at each treatment session.
(7) to treat migraine, pericranial injected (injected symmetrically into glabellar, frontalis and temporalis muscles) injection of 25 U of BOTOX® has showed significant benefit as a prophylactic treatment of migraine compared to vehicle as measured by decreased measures of migraine frequency, maximal severity, associated vomiting and acute medication use over the three month period following the 25 U injection.

The present invention demonstrates that a botulinum nanoparticle composition, when incorporated into a cream that is applied to the skin for transdermal delivery of the toxin, achieves biological results (*i.e*., reduction of wrinkles, treatment of sweat gland disorders, *etc.*) comparable to those historically observed with injection of a botulinum toxin solution containing approximately the same amount of BOTOX®.

In some embodiments, provided botulinum nanoparticle compositions are distinguishable from other botulinum-toxin-containing compositions that have been described. For example, Donovan has described a preparation in which botulinum toxin has been incorporated into a lipid vesicle for transdermal delivery (U.S. Patent Publication 2004/0009180; incorporated herein by reference). Such vesicles also require the incorporation of an enhancing agent, such as an alcohol, to facilitate the absorption of botulinum toxin through the skin. Donovan also describes a neurotoxin that is incorporated into a transfersome, which are deformable carriers containing lipids and membrane softeners (Hofer et al., 2000, World J. Surg., 24:1187; and U.S. Patent 6,165,500; both of which are incorporated herein by reference). Donovan specifically describes the preparation of phosphatidyl choline + sodium cholate liposomes incorporating botulinum toxin.

The positive clinical responses of botulinum toxin type A has led to interest in other botulinum toxin serotypes. A study of two commercially available botulinum type A preparations (BOTOX® and DYSPORT®) and preparations of botulinum toxins type B and F (both obtained from Wako Chemicals, Japan) has been carried out to determine local muscle weakening efficacy, safety and antigenic potential in mice. Botulinum toxin preparations were injected into the head of the right gastrocnemius muscle (0.5 to 200.0 U/kg) and muscle weakness was assessed using the mouse digit abduction scoring assay (DAS). ED₅₀ values were calculated from dose response curves.

Additional mice were given intramuscular or peritoneal injections to determine LD₅₀ doses. The therapeutic index was calculated as LD₅₀/ED₅₀. Separate groups of mice received hind limb injections of BOTOX® (5.0 to 10.0 U/kg) or botulinum toxin type B (50.0 to 400.0 U/kg), and were tested for muscle weakness and increased water consumption, the later being a putative model for dry mouth. Peak muscle weakness and duration were dose related for all serotypes.

DAS ED₅₀ values (U/kg) were as follows: BOTOX®: 6.7, DYSPORT®: 24.7, botulinum toxin type B: 27.0 to 244.0, botulinum toxin type F: 4.3. BOTOX® had a longer duration of action than botulinum toxin type B or botulinum toxin type F. Therapeutic index values were as follows: BOTOX®: 10.5, DYSPORT®: 6.3, botulinum toxin type B: 3.2. Water consumption was greater in mice injected with botulinum toxin type B than with BOTOX®, although botulinum toxin type B was less effective at weakening muscles. DAS results indicate relative peak potencies of botulinum toxin type A being equal to botulinum toxin type F, and botulinum toxin type F being greater than botulinum toxin type B. With regard to duration of effect, botulinum toxin type A was greater than botulinum toxin type B, and botulinum toxin type B duration of effect was greater than botulinum toxin type F. As shown by the therapeutic index values, the two commercial preparations of botulinum toxin type A (BOTOX® and DYSPORT®) are different. The increased water consumption behavior observed following hind limb injection of botulinum toxin type B indicates that clinically significant amounts of this serotype entered the murine systemic circulation. The results also indicate that in order to achieve efficacy comparable to botulinum toxin type A, it may be necessary to increase doses of the other serotypes examined. Increased dosage, however, can compromise safety.

Antigenic potential was assessed by monthly intramuscular injections in rabbits (1.5 or 6.5 ng/kg for botulinum toxin type B or 0.15 ng/kg for BOTOX®). After four months of injections, 2 of 4 rabbits treated with 1.5 ng/kg and 4 of 4 animals treated with 6.5 ng/kg developed antibodies against botulinum toxin type B. In a separate study, 0 of 9 BOTOX® treated rabbits demonstrated antibodies against botulinum toxin type A. Therefore, in rabbits, botulinum toxin type B was more antigenic than was BOTOX®, possibly because of the higher protein load injected to achieve an effective dose of botulinum toxin type B (Aoki, 1999, Eur. J. Neurol., 6:S3-S10).

As indicated herein, the present invention contemplates use of botulinum toxin of any serotype. Those of ordinary skill in the art will readily be able to assess the appropriateness of a particular serotype for a particular use and, according to the teachings herein, will be able to prepare nanoparticle compositions containing such botulinum toxin. Thus, the present invention provides nanoparticle compositions containing botulinum toxin of any serotype, including compositions containing only botulinum toxin proteins and compositions containing one or more other proteins. In some embodiments, such other proteins comprise or consist of gelatin. In some embodiments, such other proteins comprise or consist of albumin. In some embodiments, botulinum toxin complexes are employed.

Commercially available sources of botulinum toxin that may be utilized in accordance with the present invention include, but are not limited to, BOTOX®, DYSPORT® (*Clostridium botulinum* type A toxin hemagglutinin complex with human serum albumin and lactose; Ispen Limited, Berkshire U.K.), Xeomin®, PurTox®, Medy-Tox, NT-201 (Merz Pharmaceuticals), and/or MYOBLOC® (an injectable solution consisting of botulinum toxin type B, human serum albumin, sodium succinate, and sodium chloride, pH 5.6, Elan Pharmaceuticals, Dublin, Ireland), *etc.*

In some embodiments, a provided composition containing both a nanoparticle composition and a cream and/or lotion formulation contain between about 1 to about 20,000 Units botulinum toxin per ml. In some embodiments, a provided composition containing both a nanoparticle composition and a cream and/or lotion formulation contain between about 50 to about 1,000 Units botulinum toxin per ml. In some embodiments, a provided composition containing both a nanoparticle composition and a cream and/or lotion formulation contain between about 50 to about 500 Units botulinum toxin per ml.

In some embodiments, a nanoparticle composition contains between about 2 to about 40,000 Units botulinum toxin per ml. In some embodiments, a nanoparticle composition contains between about 2 to about 12,000 Units botulinum toxin per ml. In some embodiments, a nanoparticle composition contains between about 100 to about 2,000 Units botulinum toxin per ml. In some embodiments, a nanoparticle composition contains between about 50 to about 1,000 Units botulinum toxin per ml.

### Small Molecule Agents

Any of a variety of small molecule agents may be incorporated in provided compositions according to the present invention. In some embodiments, a small molecule agent includes, but is not limited to, an anti-cancer agent, antibiotic, anti-viral agent, anti-HIV agent, anti-parasite agent, anti-protozoal agent, anesthetic, anticoagulant, inhibitor of an enzyme, steroidal agent, steroidal or non-steroidal anti-inflammatory agent, antihistamine, immunosuppressant agent, anti-neoplastic agent, antigen, vaccine, antibody, decongestant, sedative, opioid, analgesic, anti-pyretic, birth control agent, hormone, prostaglandin, progestational agent, anti-glaucoma agent, ophthalmic agent, anti-cholinergic, analgesic, antidepressant, anti-psychotic, neurotoxin, hypnotic, tranquilizer, anti-convulsant, muscle relaxant, anti-Parkinson agent, anti-spasmodic, muscle contractant, channel blocker, miotic agent, anti-secretory agent, anti-thrombotic agent, anticoagulant, anti-cholinergic, β-adrenergic blocking agent, diuretic, cardiovascular active agent, vasoactive agent, vasodilating agent, anti-hypertensive agent, angiogenic agent, modulators of cell-extracellular matrix interactions (*e.g.*, cell growth inhibitors and anti-adhesion molecules), inhibitors of DNA, RNA, or protein synthesis, *etc.*), and/or combinations thereof.

In certain embodiments, a small molecule agent can be any drug. In some embodiments, a drug is one that has already been deemed safe and effective for use in humans or animals by the appropriate governmental agency or regulatory body. For example, drugs approved for human use are listed by the FDA under 21 C.F.R. §§ 330.5, 331 through 361, and 440 through 460, incorporated herein by reference; drugs for veterinary use are listed by the FDA under 21 C.F.R. §§ 500 through 589, incorporated herein by reference. All listed drugs are considered acceptable for use in accordance with the present invention.

A more complete listing of classes and specific drugs suitable for use in the present invention may be found in Pharmaceutical Drugs: Syntheses, Patents, Applications by Axel Kleemann and Jurgen Engel, Thieme Medical Publishing, 1999 and the Merck Index: An Encyclopedia of Chemicals, Drugs and Biologicals, Ed. by Budavari et al., CRC Press, 1996, both of which are incorporated herein by reference.

### Antibody Agents

Any of a variety of antibody agents may be incorporated in provided compositions according to the present invention. In some embodiments, antibody agents include, but are not limited to, polyclonal, monoclonal, chimeric (*i.e*. "humanized"), single chain (recombinant) antibodies. In some embodiments, antibodies may have reduced effector functions and/or bispecific molecules. In some embodiments, antibodies may include Fab fragments and/or fragments produced by a Fab expression library.

### Lipid Agents

Any of a variety of lipid agents may be incorporated in provided compositions according to the present invention. In some embodiments, lipid agents include, but are not limited to, oils, fatty acids, saturated fatty acid, unsaturated fatty acids, essential fatty acids, *cis* fatty acids, *trans* fatty acids, glycerides, monoglycerides, diglycerides, triglycerides, hormones, steroids (*e.g.*, cholesterol, bile acids), vitamins (*e.g.* vitamin E), phospholipids, sphingolipids, and lipoproteins.

### Diagnostic Agents

Any of a variety of diagnostic agents may be incorporated in provided compositions according to the present invention. In some embodiments, diagnostic agents include, but are not limited to, gases; commercially available imaging agents used in positron emissions tomography (PET), computer assisted tomography (CAT), single photon emission computerized tomography, x-ray, fluoroscopy, and magnetic resonance imaging (MRI); and contrast agents. Examples of suitable materials for use as contrast agents in MRI include gadolinium chelates, as well as iron, magnesium, manganese, copper, and chromium. Examples of materials useful for CAT and x-ray imaging include iodine-based materials.

### Prophylactic Agents

Any of a variety of prophylactic agents may be incorporated in provided compositions according to the present invention. In some embodiments, prophylactic agents include, but are not limited to, vaccines. Vaccines may comprise isolated proteins or peptides, inactivated organisms and viruses, dead organisms and virus, genetically altered organisms or viruses, and cell extracts. Prophylactic agents may be combined with interleukins, interferon, cytokines, and adjuvants such as cholera toxin, alum, Freund's adjuvant, *etc.* Prophylactic agents may include antigens of such bacterial organisms as *Streptococccus pnuemoniae, Haemophilus influenzae, Staphylococcus aureus, Streptococcus pyrogenes, Corynebacterium diphtheriae, Listeria monocytogenes, Bacillus anthracis, Clostridium tetani, Clostridium botulinum, Clostridium perfringens, Neisseria meningitidis, Neisseria gonorrhoeae, Streptococcus mutans, Pseudomonas aeruginosa, Salmonella typhi, Haemophilus parainfluenzae, Bordetella pertussis, Francisella tularensis, Yersinia pestis, Vibrio cholerae, Legionella pneumophila, Mycobacterium tuberculosis, Mycobacterium leprae, Treponema pallidum, Leptospirosis interrogans*, *Borrelia burgdorferi, Camphylobacter jejuni,* and the like; antigens of such viruses as smallpox, influenza A and B, respiratory syncytial virus, parainfluenza, measles, HIV, varicella-zoster, herpes simplex 1 and 2, cytomegalovirus, Epstein-Barr virus, rotavirus, rhinovirus, adenovirus, papillomavirus, poliovirus, mumps, rabies, rubella, coxsackieviruses, equine encephalitis, Japanese encephalitis, yellow fever, Rift Valley fever, hepatitis A, B, C, D, and E virus, and the like; antigens of fungal, protozoan, and parasitic organisms such as *Cryptococcus neoformans, Histoplasma capsulatum, Candida albicans, Candida tropicalis, Nocardia asteroides*, *Rickettsia ricketsii, Rickettsia typhi, Mycoplasma pneumoniae, Chlamydial psittaci, Chlamydial trachomatis, Plasmodium falciparum, Trypanosoma brucei, Entamoeba histolytica, Toxoplasma gondii, Trichomonas vaginalis*, *Schistosoma mansoni,* and the like. These antigens may be in the form of whole killed organisms, peptides, proteins, glycoproteins, carbohydrates, or combinations thereof.

Those skilled in the art will recognize that the preceding paragraphs provide an exemplary, not comprehensive, list of agents that can be delivered using compositions and methods in accordance with the present invention. Any agent may be associated with provided compositions in accordance with the present invention.

### Dermatologic Conditions

The present invention provides methods and compositions for the treatment and/or prevention of any of a variety of dermatologic conditions. In some embodiments, the present invention provides methods and compositions for the treatment and/or prevention of diseases, disorders, or conditions associated with activity of sweat and/or sebaceous glands. In some embodiments, the present invention provides methods and compositions for the treatment and/or prevention of diseases, disorders or conditions associated with the epidermal and/or dermal level of the skin.

In some embodiments, the present invention provides methods and compositions for the treatment and/or prevention of one or more of acne, unwanted sweating, body odor, hyperhidrosis, bromhidrosis, chromhidrosis, rosacea, hair loss, psoriasis, actinic keratosis, eczematous dermatitis (*e.g.*, atopic dermatitis, *etc.*), excess sebum-producing disorders (*e.g.*, seborrhea, seborrheic dermatitis, *etc.*), burns, Raynaud's phenomenon, lupus erthythematosus, hyperpigmentation disorders (*e.g.*, melasma, *etc.*), hypopigmentation disorders (*e.g.*, vitiligo, *etc.*), skin cancer (*e.g.*, squamous cell skin carcinoma, basal cell skin carcinoma, *etc.*), dermal infection (*e.g.*, bacterial infection, viral infection, fungal infection, *etc.*), facial wrinkles, (*e.g.*, wrinkles involving the forehead, glabellar, rhytids and/or periorbital regions), headache, unsightly facial expressions (*e.g.*, due to overactivity of underlying facial musculature), neck lines, hyperfunctional facial lines, hyperkinetic facial lines, platysma bands, neuromuscular disorders and conditions involving muscular spasm and/or contracture (including various forms of facial palsy, cerebral palsy, blepharospasm, facial contracture), dystonia, prostate hyperplasia, headache, strabismus, hemifacial spasm, tremor, spasticity such as that resulting from multiple sclerosis, retroorbital muscle, various ophthalmologic conditions, and/or combinations thereof.

In some embodiments, the present invention involves administration of at least one provided composition according to a dosing regimen sufficient to achieve a reduction in the degree and/or prevalence of a relevant dermatologic condition of at least about 20%; in some embodiments according to a dosing regimen sufficient to achieve a of at least about 25%; in some embodiments according to a dosing regimen sufficient to achieve a reduction of at least about 30%; in some embodiments according to a dosing regimen sufficient to achieve a reduction of at least about 31%, about 32%, about 33%, about 34%, about 35%, about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, about 59%, about 60%, about 61%, about 62%, about 63%, about 64%, about 65%, about 66%, about 67%, about 68%, about 69%, about 70%, about 71%, about 72%, about 73%, about 74%, about 75%, about 76%, about 77%, about 78%, about 79%, about 80%, about 81%, about 82%, about 83%, about 84%, about 85%, about 86%, about 87%, about 88%, about 89%, about 90%, or more.

In some embodiments, the present invention involves administration of at least one provided composition according to a dosing regimen sufficient to achieve a reduction in the degree and/or prevalence of a relevant dermatologic condition of at least about 20% in a specified percentage of a population of patients to which the composition was administered; in some embodiments according to a dosing regimen sufficient to achieve a of at least about 25% in a specified percentage of a population of patients to which the composition was administered; in some embodiments according to a dosing regimen sufficient to achieve a reduction of at least about 30% in a specified percentage of a population of patients to which the composition was administered; in some embodiments according to a dosing regimen sufficient to achieve a reduction of at least about 31%, about 32%, about 33%, about 34%, about 35%, about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, about 59%, about 60%, about 61%, about 62%, about 63%, about 64%, about 65%, about 66%, about 67%, about 68%, about 69%, about 70%, about 71%, about 72%, about 73%, about 74%, about 75%, about 76%, about 77%, about 78%, about 79%, about 80%, about 81%, about 82%, about 83%, about 84%, about 85%, about 86%, about 87%, about 88%, about 89%, about 90% or more in a specified percentage of a population of patients to which the composition was administered. In some embodiments, the specified percentage of population of patients to which the composition was administered is at least about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100%. To give but a few illustrative examples, in some embodiments, the present invention involves administration of at least one provided composition according to a dosing regimen sufficient to achieve a reduction in the degree and/or prevalence of a relevant dermatologic condition of at least about 20% in at least about 50% of the population of patients to which the composition was administered. In some embodiments, the present invention involves administration of at least one provided composition according to a dosing regimen sufficient to achieve a reduction in the degree and/or prevalence of a relevant dermatologic condition of at least about 30% in at least about 50% of the population of patients to which the composition was administered.

The present invention provides methods of treating and/or preventing a dermatologic condition comprising administration of a provided composition to a subject suffering from, susceptible to, and/or displaying symptoms the dermatologic condition. In some embodiments, provided compositions for treatment of a dermatologic condition as described herein are formulated for any route of administration described herein. In some embodiments, provided compositions are formulated for topical administration. In some embodiments, provided compositions are formulated into a cream, liniment, lotion, gel, shampoo, conditioner, sunscreen, deodorant, and/or antiperspirant (*e.g.*, as a roll-on, solid stick, gel, cream, aerosol, *etc.*), *etc.*, as appropriate to the condition being treated.

In some embodiments, provided compositions are formulated for injection, *e.g.*, into an affected site. In some embodiments, provided compositions are formulated for systemic delivery.

In some embodiments, such a provided composition is administered locally to an affected site (*e.g.*, axillae, hands, feet, scalp, hair follicle, face, neck, back, arms, chest, *etc.*, as appropriate to the particular condition being treated). In some embodiments, local administration is achieved by topical administration and/or by injection. In some embodiments, a provided composition is administered systemically (*e.g.*, orally, topically, via injection, *etc.*).

Further considerations for formulation and administration are described in further detail in the sections entitled "Compositions and Formulations" and "Administration."

More detailed discussion of certain of these conditions and their treatment and/or prevention in accordance with the present invention is provided below.

### Unwanted Sweating

In some embodiments, provided compositions are useful for treating and/or preventing unwanted sweating (or perspiration). In some embodiments, unwanted sweating is a symptom of a clinically diagnosed condition such as hyperhidrosis. In some embodiments, unwanted sweating is not associated with a clinical diagnosis such as hyperhidrosis, but is simply any sweating (perspiration) which is unwanted by the patient. In some embodiments, sweating which is unwanted by the patient includes all sweating.

In some embodiments, administration of a provided composition according to a dosing regimen sufficient to achieve sweat reduction upon administration of provided compositions to individuals who are not suffering from a clinical sweating condition, but nonetheless desire sweat reduction. As a further discovery, in some embodiments, the present invention achieves such levels to individuals who suffer from a sweat-related clinical disorder, for example hyperhidrosis, chromhidrosis, bromhidrosis, *etc.*

In some embodiments, provided compositions for treatment and/or prevention of unwanted sweating are formulated into a cream, liniment, lotion, gel, sunscreen, deodorant, and/or antiperspirant (*e.g.*, as a roll-on, solid stick, gel, cream, aerosol, *etc.*), *etc..*

In some embodiments, provided compositions for treatment and/or prevention of unwanted sweating are administered locally to an affected site (*e.g*., axillae, hands, feet, etc.).

Current therapies useful in the treatment of unwanted sweating include, but are not limited to, botulinum toxin; antiperspirants (*e.g.*, aluminum chloride, aluminum chlorohydrate, aluminum-zirconium compounds, aluminum zirconium tetrachlorohydrex gly, aluminum zirconium trichlorohydrex gly, ammonium alum, *etc.*); aluminum chlorohydrex compounds; aluminum dichlorohydrate; aluminum dichlorohydrex compounds; aluminum sesquichlorohydrate; aluminum sesquichlorohydrex compounds; oral medication (*e.g.*, diphenhydramine hydrochloride, hydroxyzine, glycopyrrolate, *etc.*); anticholinergic drugs (*e.g.*, oxybutynin, glycopyrrolate, propantheline bromide, benztropine, *etc.*); beta-blockers; antidepressants; anxiolytics; talc and/or baby powder; and/or combinations thereof.

Alternative or additional current treatments for unwanted sweating include, but are not limited to, surgery (*e.g.*, endoscopic thoracic sympathectomy, lumbar sympathectomy, sweat gland suction, percutaneous sympathectomy, *etc.*); iontophoresis; weight loss; relaxation and/or meditation; hypnosis; use of shoe inserts; and/or combinations thereof.

### Hyperhidrosis

In some embodiments, provided compositions are useful for treating hyperhidrosis. Hyperhidrosis is a medical condition in which a person sweats excessively and unpredictably. People with hyperhidrosis can sweat even when the temperature is cool, and when they are at rest. Sweating helps the body stay cool and is perfectly natural. People sweat more in warm temperatures, when they exercise, or in response to situations that make them nervous, angry, embarrassed, or afraid. Uncontrollable sweating can lead to significant discomfort, both physical and emotional.

Hyperhidrosis occurs without normal sweat triggers, and refers to the condition characterized by perspiration in excess of that required for regulation of body temperature. Those with hyperhidrosis appear to have overactive sweat glands. Hyperhidrosis can either be generalized or localized to specific parts of the body. Hands, feet, axillae, forehead, and the groin area are among the most active regions of perspiration due to the relatively high concentration of sweat glands; however, any part of the body may be affected. Excessive sweating that affects hands, feet, and armpits and has no other identifiable cause is referred to as "primary" or "focal hyperhidrosis." Primary hyperhidrosis affects 2% - 3% of the population, yet less than 40% of patients with this condition seek medical advice. There may be a genetic component involved in primary hyperhidrosis. One theory is that hyperhidrosis results from an overactive sympathetic nervous system. Primary hyperhidrosis is found to start during adolescence or even before.

If sweating occurs as a result of another medical condition, it is called secondary hyperhidrosis. Sweating may be all over one's body, or it may be localized to one area. Secondary hyperhidrosis can start at any point in life. For some, it can seem to come on unexpectedly. Conditions that cause secondary hyperhidrosis include but are not limited to, acromegaly, hyperthyroidism, glucose control disorders (including diabetes), pheochromocytoma, carcinoid syndrome, cancer, tuberculosis, infections, menopause, spinal cord injury, stroke, thyroid gland disorder, pituitary gland disorder, gout, mercury poisoning, Parkinson's disease, heart disease, lung disease, certain medications, substance abuse, or anxiety conditions.

Hyperhidrosis can be categorized as "palmar" (*i.e.*, excessive sweating of the hands), "axillary" (*i.e*., excessive sweating of the armpits), "plantar" (*i.e*., excessive sweating of the feet), "facial" (*i.e*., excessive sweating of the face), "cranial" (*i.e*., excessive sweating of the head, especially noted around the hairline), or "general" (*i.e*., overall excessive sweating).

In some embodiments, provided compositions for treatment and/or prevention of hyperhidrosis are formulated into a cream, liniment, lotion, gel, sunscreen, deodorant, and/or antiperspirant (*e.g.*, as a roll-on, solid stick, gel, cream, aerosol, etc.), etc..

In some embodiments, provided compositions for treatment and/or prevention of hyperhidrosis are administered locally to an affected site (*e.g.*, axillae, hands, feet, *etc*).

Current therapies for the treatment of hyperhidrosis include, but are not limited to, botulinum toxin, antiperspirants (*e.g.*, aluminum chloride, aluminum chlorohydrate, aluminum-zirconium compounds, aluminum zirconium tetrachlorohydrex gly, aluminum zirconium trichlorohydrex gly, ammonium alum, *etc.*); oral medication (*e.g.*, diphenhydramine hydrochloride, hydroxyzine, glycopyrrolate, *etc.*); anticholinergic drugs (*e.g.*, oxybutynin, glycopyrrolate, propantheline bromide, benztropine, *etc.*); beta-blockers; antidepressants; anxiolytics; talc and/or baby powder; and/or combinations thereof.

Alternative or additional current therapies for the treatment of hyperhidrosis include, but are not limited to, surgery (*e.g*., endoscopic thoracic sympathectomy [ETS], lumbar sympathectomy, sweat gland suction, percutaneous sympathectomy, *etc.*); iontophoresis; weight loss; relaxation and/or meditation; hypnosis; use of shoe inserts; and/or combinations thereof.

In ETS procedures, select sympathetic nerves or nerve ganglia in the chest are either excised, cut, burned, or clamped. The procedure causes relief of excessive hand sweating in about 85% - 95% of patients. However, compensatory sweating is seen in about 20% to 80% of patients. While ETS can be helpful to treat axillary hyperhidrosis, palmar hyperhidrosis patients frequently have better results.

Lumbar sympathectomy can be useful for patients for whom endoscopic thoracic sympathectomy did not relieve their excessive plantar sweating. With this procedure, the sympathetic chain in the lumbar region is being clipped or divided in order to relieve the severe or excessive feet sweating. The success rate is about 90%.

Sweat gland suction is a technique adapted and modified from liposuction (Bieniek et al., 2005, Acta dermatovenerologica Croatica: ADC/Hrvatsko dermatolosko drustvo, 13:212-8; incorporated herein by reference). Approximately 30% of the sweat glands are removed with a proportionate reduction in sweat.

Iontophoresis was originally described in the 1950s, and its exact mode of action remains elusive to date (Kreyden, 2004, J. Cosmetic Dermatol., 3:211-4; incorporated herein by reference). An affected area is placed in a device that has two pails of water with a conductor in each one. The hand or foot acts like a conductor between the positively- and negatively-charged pails. As the low current passes through the area, the minerals in the water clog the sweat glands, limiting the amount of sweat released. The device is usually used for the hands and feet, but there has been a device created for the axillae area and for the stump region of amputees.

Percutaneous sympathectomy is a minimally invasive procedure in which nerves are blocked by injection of phenol (Wang et al., 2001, Neurosurgery, 49:628-34; incorporated herein by reference).

In some subjects, weight loss can help alleviate one or more symptoms of hyperhidrosis, as hyperhidrosis can be aggravated by obesity.

Relaxation, meditation, and/or hypnosis therapies are sometimes utilized in the treatment and/or prevention of hyperhidrosis. For example, hypnosis has been used with some success in improving the process of administering injections for the treatment of hyperhidrosis (Maillard et al., 2007, Annales de dermatologie et de venereologie, 134:653-4; incorporated herein by reference).

### Body Odor

In some embodiments, provided compositions are useful for treating and/or preventing body odor. In some embodiments, body odor is a symptom of a clinically diagnosed condition such as bromhidrosis. In some embodiments, body odor is not associated with a clinical diagnosis such as bromhidrosis, but is simply any body odor (*e.g.*, unwanted body odor) of a subject. In some embodiments, therapies effective for treating unwanted sweating and/or hyperhidrosis are also effective for treating body odor.

In some embodiments, provided compositions for treatment and/or prevention of body odor are formulated into a cream, liniment, lotion, gel, sunscreen, deodorant, and/or antiperspirant (*e.g.*, as a roll-on, solid stick, gel, cream, aerosol, *etc.*), *etc.*

In some embodiments, provided compositions for treatment and/or prevention of body odor are administered locally to an affected site (*e.g.*, axillae, hands, feet, *etc.*).

### Bromhidrosis

In some embodiments, provided compositions may be useful for treating bromhidrosis (also called osmidrosis, ozochrotia, body odor, and B.O.) is the smell of bacteria growing on a body. Bacteria multiply rapidly in the presence of sweat, but sweat itself is almost completely odorless. Body odor is associated with the hair, feet, groin, anus, skin in general, armpits, genitals, pubic hair, and mouth.

Apocrine bromhidrosis is the most prevalent form, whereas eccrine bromhidrosis is less common. Several factors contribute to the pathogenesis of apocrine bromhidrosis. Bacterial decomposition of apocrine secretion yields ammonia and short-chain fatty acids, with their characteristic strong odors. The most abundant of these acids is (E)-3-methyl-2-hexanoic acid (E-3M2H), which is brought to the skin surface bound by two apocrine secretion odor-binding proteins (ASOB1 and ASOB2). One of these binding proteins, ASOB2, has been identified as apolipoprotein D (apoD), a known member of the lipocalin family of carrier proteins.

Axillary bacterial florae have been shown to produce the distinctive axillary odor by transforming nonodiferous precursors in sweat to more odiferous volatile acids. The most common of these are E-3M2H and (RS)-3-hydroxy-3-methlyhexanoic acid (HMHA), which are released through the action of a specific zinc-dependent N-alpha-acyl-glutamine aminoacylase (*N*-AGA) from *Corynebacterium* species. This aminoacylase has recently been demonstrated to also release other odiferous acids from glutamine conjugates in sweat, which may be the basis of individual body odor.

In certain circumstances, eccrine secretion, which is typically odorless, assumes an offensive aroma and causes eccrine bromhidrosis. When eccrine sweat softens keratin, bacterial degradation of the keratin yields a foul smell. Ingestion of some foods, including garlic, onion, curry, alcohol, certain drugs (*e.g.*, penicillin, bromides), and toxins may cause eccrine bromhidrosis. Eccrine bromhidrosis may result from underlying metabolic or endogenous causes.

The role of excessive eccrine secretion, or hyperhidrosis, in the pathogenesis of bromhidrosis is unclear. Hyperhidrosis may promote the spread of apocrine sweat and contribute further to bromhidrosis by creating a moist environment, one ripe for bacterial overgrowth. Conversely, eccrine hyperhidrosis may cause a decrease in odor because the eccrine sweat flushes away the more odiferous apocrine sweat.

In some embodiments, therapies effective for treating unwanted sweating and/or hyperhidrosis are also effective for treating bromhidrosis.

In some embodiments, provided compositions for treatment and/or prevention of bromhidrosis are formulated into a cream, liniment, lotion, gel, sunscreen, deodorant, and/or antiperspirant (*e.g.*, as a roll-on, solid stick, gel, cream, aerosol, *etc.*), *etc.*

In some embodiments, provided compositions for treatment and/or prevention of bromhidrosis are administered locally to an affected site (*e.g.*, axillae, hands, feet, *etc.*).

### Chromhidrosis

In some embodiments, provided compositions are useful for treating and/or preventing chromhidrosis, a rare condition characterized by the secretion of colored sweat. Chromhidrosis is caused by the deposition of lipofuscin in the sweat glands. Approximately 10% of people without the disease have colored sweat that is regarded as acceptable and within the normal range. Usually chromhidrosis affects the apocrine glands, mainly on the face and underarms. Lipofuscin pigment is produced in the apocrine gland, and its various oxidative states account for the characteristic yellow, green, blue, or black secretions observed in apocrine chromhidrosis. Chromhidrosis of the eccrine glands is rare, occurring mainly after the ingestion of certain dyes or drugs. Pseudochromhidrosis occurs when clear eccrine sweat becomes colored on the surface of the skin as a result of extrinsic dyes, paints, or chromogenic bacteria.

In some embodiments, therapies effective for treating unwanted sweating and/or hyperhidrosis are also effective for treating chromhidrosis.

In some embodiments, provided compositions for treatment and/or prevention of chromhidrosis are formulated into a cream, liniment, lotion, gel, sunscreen, deodorant, and/or antiperspirant (*e.g.*, as a roll-on, solid stick, gel, cream, aerosol, *etc*).

In some embodiments, provided compositions for treatment and/or prevention of chromhidrosis are administered locally to an affected site (*e.g.*, axillae, hands, feet, *etc.*).

### Rosacea

In some embodiments, provided compositions may be useful for treating and/or preventing rosacea, a condition that is estimated to affect over 45 million people worldwide. Rosacea affects both sexes, but is almost three times more common in women, and has a peak age of onset between 30 and 60. It begins as erythema (*i.e*., flushing and redness) on the central face and across the cheeks, nose, and/or forehead but can also less commonly affect the neck and chest. As rosacea progresses, other symptoms can develop such as one or more of semi-permanent erythema, telangiectasia (*i.e*., dilation of superficial blood vessels on the face), red domed papules and pustules, red gritty eyes, burning and stinging sensations, and/or rhinophyma (*i.e.*, a red lobulated nose).

There are four main subtypes of rosacea. "Erythematotelangiectatic rosacea" is characterized by permanent redness with a tendency to flush and blush easily. It is also common to have small blood vessels visible near the surface of the skin (*i.e.*, telangiectasias) and/or burning or itching sensations. "Papulopustular rosacea" is characterized by some permanent redness with papules and/or pustules, which typically last 1 to 4 days. This subtype is commonly confused with acne. "Phymatous rosacea" is most commonly associated with rhinophyma, an enlargement of the nose. Symptoms include thickening skin, irregular surface nodularities, and enlargement. Phymatous rosacea can also affect the chin (gnatophyma), forehead (metophyma), cheeks, eyelids (blepharophyma), and/or ears (otophyma) (see, *e.g.*, Jansen and Plewig, 1998, Facial Plast. Surg., 14:241; incorporated herein by reference). Small blood vessels visible near the surface of the skin (*i.e.*, telangiectasias) may be present. "Ocular rosacea" is characterized by red, dry, irritated eyes and/or eyelids. Other symptoms may include foreign body sensations, itching, and/or burning.

Rosacea can be triggered by any of a variety of stimuli. Triggers that cause episodes of flushing and blushing play a part in the development of rosacea, such as exposure to temperature extremes, strenuous exercise, heat from sunlight, severe sunburn, stress, anxiety, cold wind, and/or moving to a warm or hot environment from a cold one. Some foods and drinks can trigger flushing, such as alcohol, foods and beverages containing caffeine (*e.g.*, hot tea, coffee), foods high in histamines, and spicy foods. Certain medications and topical irritants can quickly progress rosacea (*e.g.*, steroids, benzoyl peroxide, isotretinoin, *etc.*).

In some embodiments of the present invention, different subtypes of rosacea are treated differently from other subtypes of rosacea (Cohen and Tiemstra, 2002, J. Am. Board Fam. Pract., 15:214; incorporated herein by reference). In some embodiments, different subtypes of rosacea are not treated differently from other subtypes of rosacea.

Current therapies utilized in the treatment of rosacea include, for example, botulinum toxin, oral antibiotics (*e.g.*, tetracycline, doxycycline, minocycline, metronidazole, macrolide antibiotics, *etc.*), and/or combinations thereof. In some embodiments, oral antibiotics may be administered at anti-inflammatory doses (*e.g.*, about 40 mg/day) or at higher doses. In some embodiments, such agents include oral isotretinoin. In some embodiments, such agents include topical antibiotics (*e.g.*, metronidazole, clindamycin, erythromycin, *etc*.); topical azelaic acid (*e.g.*, FINACEA™, AZELEX™, FINEVIN®, SKINOREN, *etc*.); topical sulfacetamide; topical sulfur; topical calcineurin inhibitor (*e.g.*, tacrolimus, pimecrolimus, *etc.*); topical benzoyl peroxide; topical permethrin; a combination of plant-sourced methylsulfonylmethane (MSM) and Silymarin; and/or combinations thereof.

Alternative or additional current therapies for the treatment of rosacea include, but are not limited to, use of a gentle skin cleansing regimen using non-irritating cleansers; protecting skin from the sun by covering skin with clothing; applying sunscreen to exposed skin; dermatological vascular laser (single wavelength); intense pulsed light (broad spectrum); carbon dioxide lasers; low level light therapies; and/or combinations thereof.

Rosacea may be treated via dermatological vascular laser (single wavelength) and/or intense pulsed light (broad spectrum) (Angermeier, 1999, J. Cutan. Laser Ther., 1:95; incorporated herein by reference). These methods use light to penetrate the epidermis to target the capillaries in the dermis. Light is absorbed by oxy-hemoglobin, thereby causing capillary walls to heat up to 70 °C, damaging them, which causes them to be absorbed by the body's natural defense mechanism. These methods may be successful for eliminating redness altogether, though additional periodic treatments might be necessary to remove newly-formed capillaries. Alternatively or additionally, a 595 nm long pulse-duration pulsed-dye laser may be useful for the treatment of rosacea (Kligman and Bernstein, 2008, Lasers Surg. Med., 40:233; incorporated herein by reference).

Alternatively or additionally, carbon dioxide lasers can be used to remove excess tissue, for example, caused by phymatous rosacea. Carbon dioxide lasers emit a wavelength that is absorbed directly by the skin. The laser beam can be focused into a thin beam and used as a scalpel or defocused and used to vaporize tissue.

In some embodiments, rosacea can be treated using low level light therapies.

In some embodiments, provided compositions for treatment and/or prevention of rosacea are formulated into a cream, liniment, lotion, gel, sunscreen, deodorant, and/or antiperspirant (*e.g*., as a roll-on, solid stick, gel, cream, aerosol, etc.), etc..

In some embodiments, provided compositions for treatment and/or prevention of rosacea are administered locally to an affected site (*e.g.*, axillae, hands, feet, scalp, face, neck, back, arms, chest, *etc.*).

### Hair Loss

In some embodiments, provided compositions are useful for treating and/or preventing hair loss. Baldness involves the state of lacking hair where it often grows, especially on the head. The most common form of baldness is a progressive hair thinning condition called androgenic alopecia or "male pattern baldness" that occurs in adult male humans and other species. The amount and patterns of baldness can vary greatly; it ranges from male and female "pattern alopecia" (androgenic alopecia, also called androgenetic alopecia or alopecia androgenetica); "alopecia areata," which involves the loss of some of the hair from the head; "alopecia totalis," which involves the loss of all head hair; to the most extreme form, "alopecia universalis," which involves the loss of all hair from the head and the body.

Current therapies used in the treatment of hair loss include, but are not limited to, botulinum toxin, aza-steroids, such as finasteride (PROPECIA®; PROSCAR®; *etc.*) or dutasteride (AVODART®); topically applied minoxidil, a vasodilator (ROGAINE®); antiandrogens (*e.g*., ketoconazole, fluconazole, spironolactone, *etc.*); saw palmetto; caffeine; copper peptides; nitroxide spin labels TEMPO and TEMPOL; unsaturated fatty acids (*e.g.*, gamma linolenic acid); hedgehog agonists; azelaic acid and zinc in combination; Chinese knotweed; pumpkin seed; spironolactone; tretinoin; zinc; stinging nettle; and/or combinations thereof.

In some embodiments, provided compositions for treatment and/or prevention of hair loss are formulated into a cream, liniment, lotion, gel, shampoo, conditioner, *etc.*

In some embodiments, provided compositions for treatment and/or prevention of hair loss are administered locally to an affected site (*e.g.*, scalp, hair follicle, face, neck, back, arms, chest, *etc.*).

### Acne

In some embodiments, provided compositions are useful for treating and/or preventing acne vulgaris (commonly referred to as "acne"), a skin disease caused by changes in the pilosebaceous units (*i.e.*, skin structures comprising a hair follicle and its associated sebaceous gland). In some embodiments, acne is inflammatory. In some embodiments, acne is noninflammatory. While not life-threatening, acne vulgaris can cause significant problems for affected individuals. Depending on its severity and other factors, recalcitrant acne can be psychologically debilitating, and can impose significant financial and emotional costs on those whom it afflicts. Despite some recent successes in acne therapy, treatment failures are still common, especially in adult women. While many adults "outgrow" this disease, there are some who continue to be afflicted during much of adulthood, despite continued medical advances. Unfortunately, the most potent acne medication in current use is administered systemically via a treatment that is teratogenic, an important issue for many women. There is an unfilled need for a more localized and effective treatment for acne, one with minimal side effects.

In general, acne develops as a result of blockages in follicles. The pathology centers on the pilosebaceous units, comprising a sebaceous gland, a follicle (*i.e*., pore), and a vellus hair. Among the first events leading to acne are hyperkeratinization and formation of a plug of keratin and sebum (a "microcomedo"), obstructing the upper region of a follicle. Enlargement of sebaceous glands and an increase in sebum production occur with increased androgen production at adrenarche. A microcomedo may enlarge to form an open comedo (a "blackhead") or closed comedo (a "whitehead"). In these conditions the naturally occurring largely commensual bacteria *Propionibacterium acnes* can cause inflammation, leading to inflammatory lesions (papules, infected pustules, or nodules) in the dermis around the microcomedo or comedo, which results in redness and may result in scarring or hyperpigmentation.

Adolescence is marked by an increase in levels of circulating androgens, particularly dehydroepiandrosterone sulfate (DHEAS). Increased androgen levels are thought to cause sebaceous glands to enlarge and to increase sebum production. While most acne patients have normal hormone levels, there are reasons to conclude that increased sebum production plays a role in acne. For example, there may be a correlation between the rate of sebum production and the severity of acne. In addition, acne patients typically produce sebum that is deficient in linoleic acid, which is a potential cause of abnormal keratinization and follicular obstruction.

In response to increased sebum levels, *Propionibacterium acnes,* a relatively slow growing, typically aerotolerant anaerobic gram positive, diphtheroid bacterium, often colonizes the sebaceous follicles. *P. acnes* exacerbates acne by acting as a chemo-attractant for neutrophils. Neutrophils ingest *P. acnes,* and in doing so release various hydrolytic enzymes that damage the follicular wall. Released follicular contents then invade the dermis and cause an inflammatory reaction, manifesting as pustules, erythematous papules, or nodules. In a separate route, *P. acnes* can hydrolyze triglycerides to free fatty acids, which also increase inflammation and follicular obstruction. *P. acnes* may also activate the complement components of the immune system, which can also lead to follicular obstruction.

Follicles are lined with squamous epithelium, a layer of cells that is contiguous with the skin surface. In an acne-prone individual, the shedding of cells from this lining is often impeded, perhaps due to an increased level of intercellular adhesion that promotes the retention of cells. Retained cells can obstruct follicles, resulting in comedones. Such inhibited shedding may be related to abnormalities in epidermal differentiation and/or to abnormal sebum composition (*e.g.*, a deficiency in linoleic acid). It has also been demonstrated that increased sebum levels can irritate keratinocytes, causing the release of interleukin-1, which in turn can cause follicular hyperkeratinization. In general, each of these acne-causing routes, which are not mutually exclusive, is associated with follicular obstruction.

Several factors are known to be linked to acne, including, but not limited to, family and/or genetic history (see, *e.g.*, Ballanger et al., 2006, Dermatology, 212:145-149; incorporated herein by reference); hormonal activity (*e.g*., menstrual cycles, puberty, *etc.);* stress (*e.g.*, through increased output of hormones from the adrenal glands); hyperactive sebaceous glands; accumulation of dead skin cells; bacteria in the pores (*e.g.*, *P. acnes*); skin irritation or scratching; use of anabolic steroids; use of medications containing halogens (*e.g.*, iodides, chlorides, bromides), lithium, barbiturates, or androgens; exposure to certain chemical compounds (*e.g.*, dioxins such as chlorinated dioxins); exposure to testosterone, dihydrotestosterone (DHT), dehydroepiandrosterone sulfate (DHEAS), and/or insulin-like growth factor 1 (IGF-I); diet including milk and/or high levels of carbohydrate; low levels of vitamins A and/or E; poor hygiene; or any combinations thereof.

In some embodiments, acne treatments work via one or more of the following mechanisms: (1) normalizing shedding into the pore to prevent blockage; (2) killing *P. acnes;* (3) having antinflammatory activity; and/or (4) manipulating hormone levels.

The present invention provides methods of treating and/or preventing acne comprising administration of a provided composition to a subject suffering from, susceptible to, and/or displaying symptoms of acne. In some embodiments, such a provided composition is administered locally to an affected site (*e.g.*, face, neck, back, arms, chest, *etc.*).

In some embodiments, provided compositions for treatment of acne are formulated into a cream, liniment, lotion, gel, sunscreen, *etc.*

Exemplary current treatments for acne include, but are not limited to, botulinum toxin, cleansers or soaps; topical bactericidals (*e.g.*, benzoyl peroxide, triclosan, chlorhexidine gluconate, *etc.*); topical antibiotics (*e.g.*, externally-applied erythromycin, clindamycin, tetracycline, *etc.*); oral antibiotics (*e.g.*, erythromycin, tetracycline, oxytetracycline, doxycycline, minocycline, lymecycline, trimethoprim, *etc.*); hormonal treatments (*e.g.*, estrogen/progestogen oral contraceptives, low dose spironolactone, cortisone, *etc*.); topical retinoids (*e.g*., tretinoin [RETIN-A®], adapalene [DIFFERIN®], tazarotene [TAZORAC®], retinol, isotretinoin, *etc.);* oral retinoids (*e.g*., isotretinoin [ACCUTANE®, AMNESTEEM™, SOTRET™, CLARAVIS™]); herbs (*e.g*., aloe vera; aruna, haldi [turmeric], papaya, *etc*.); azelaic acid; anti-inflammatory agents (*e.g*., naproxen, ibuprofen, rofecoxib [Tehrani and Dharmalingam, 2004, Indian J. Dermatol. Venereol. Leprol., 70:345-348; incorporated herein by reference], *etc.);* nicotinamide [vitamin B3]; tea tree oil [melaleuca oil]; rofecoxib; zinc (Dreno et al., 1989, Acta Derm. Venereol., 69:541-3; and Dreno et al., 2001, Dermatology, 203:135-40; both of which are incorporated herein by reference); and/or combinations thereof.

Alternative or additional current therapies for the treatment and/or prevention of acne include, but are not limited to, phototherapy (*e.g.*, alternating blue and red light); photodynamic therapy (*e.g.*, intense blue/violet light); laser treatment (*e.g.*, to burn away the follicle sac from which the hair grows; to burn away the sebaceous gland which produces the oil; and/or to induce formation of oxygen in the bacteria, killing them); local heating; and/or combinations thereof.

It is known in the art that short-term improvement of acne can be achieved with sunlight, but studies have shown that sunlight worsens acne long-term. More recently, visible light has been successfully employed to treat acne (*i.e.*, "phototherapy") - in particular, intense violet light (405 nm - 420 nm) generated by purpose-built fluorescent lighting, dichroic bulbs, LEDs, and/or lasers. Used twice weekly, this has been shown to reduce the number of acne lesions by about 64% (Kawada et al., 2002, J. Dermatol. Sci., 30:129-35; incorporated herein by reference) and is even more effective when applied daily. Without wishing to be bound by any one theory, a porphyrin (Coproporphyrin III) produced within *P*. *acnes* generates free radicals when irradiated by 420 nm and shorter wavelengths of light (Kjeldstad, 1984, Z. Naturforsch [C], 39:300-2; incorporated herein by reference). Particularly when applied over several days, these free radicals ultimately kill bacteria (Ashkenazi et al., 2003, FEMS Immunol. Med. Microbiol., 35:17-24; incorporated herein by reference). Since porphyrins are not otherwise present in skin, and no ultraviolet (UV) light is employed, it appears to be safe, and has been licensed by the U.S. FDA. The treatment apparently works even better if used with red visible light (about 660 nm), resulting in a 76% reduction of lesions after 3 months of daily treatment for 80% of the patients (Papageorgiou et al., 2000, Br. J. Dermatol., 142:973-8; incorporated herein by reference). Unlike most of other treatments, few negative side effects are typically experienced, and development of bacterial resistance to the treatment seems very unlikely. After treatment, clearance can be longer lived than is typical with topical or oral antibiotic treatments (*e.g.*, may be up to several months).

There is some evidence that photodynamic therapy (*e.g.*, therapy with intense blue/violet light (405 nm - 425 nm)) can decrease the number of inflammatory acne lesion by 60% - 70% in 4 weeks of therapy, particularly when *P. acnes* is pretreated with delta-aminolevulinic acid (ALA), which increases the production of porphyrins.

Laser surgery has been in use for some time to reduce the scars left behind by acne, but research has been done on lasers for prevention of acne formation itself. In general, laser is used to burn away the follicle sac from which the hair grows, to burn away the sebaceous gland which produces the oil, and/or to induce formation of oxygen in the bacteria, thereby killing them.

Local heating therapies are sometimes used, for example, to kill bacteria in a developing pimple, thereby expediting healing.

In some embodiments, provided compositions for treatment and/or prevention of acne are formulated into a cream, liniment, lotion, gel, sunscreen, *etc.*

In some embodiments, provided compositions for treatment and/or prevention of acne are administered locally to an affected site (*e.g.*, axillae, hands, feet, face, neck, back, arms, chest, *etc.*).

### Psoriasis

In some embodiments, provided compositions are useful for treating psoriasis and/or preventing, a disorder which affects the skin and joints. Psoriasis commonly causes red scaly patches to appear on the skin. The scaly patches caused by psoriasis, called "psoriatic plaques," are areas of inflammation and excessive skin production. Skin rapidly accumulates at these sites and takes a silvery-white appearance. Plaques frequently occur on the skin of the elbows and knees, but can affect any area including the scalp and genitals. Psoriasis is hypothesized to be immune-mediated and is not contagious.

Psoriasis is a chronic recurring condition which varies in severity from minor localized patches to complete body coverage. Fingernails and toenails are frequently affected ("psoriatic nail dystrophy"). Psoriasis can also cause inflammation of the joints, which is known as "psoriatic arthritis." Ten to fifteen percent of people with psoriasis have psoriatic arthritis.

The cause of psoriasis is not known, but it is believed to have a genetic component. Several factors are thought to aggravate psoriasis, including stress, excessive alcohol consumption, and smoking. Individuals with psoriasis may suffer from depression and loss of self-esteem. As such, quality of life is an important factor in evaluating the severity of the disease.

Current therapies utilized in the treatment and/or prevention of psoriasis include, but are not limited to, botulinum toxin; coal tar; dithranol (anthralin); a corticosteroid such as desoximetasone (TOPICORT®); a vitamin D3 analog (*e.g*., calcipotriol); a retinoid; argan oil; topical administration of psoralen with exposure to ultraviolet A light (PUVA); milk thistle; methotrexate; cyclosporine; the antimetabolite tioguanine; hydroxyurea; sulfasalazine; mycophenolate mofetil; azathioprine; tacrolimus; and/or antibody-based therapeutics (*e.g.*, alefacept [AMEVIEVE®], etanercept [EMBREL®], infliximab [REMICADE®], efalizumab [RAPTIVA®], *etc*).

In some embodiments, provided compositions for treatment and/or prevention of psoriasis are formulated into a cream, liniment, lotion, gel, sunscreen, *etc.*

In some embodiments, provided compositions for treatment and/or prevention of psoriasis are administered locally to an affected site (*e.g.*, axillae, hands, feet, scalp, face, neck, back, arms, chest, *etc.*).

### Dermal Infections

In some embodiments, provided compositions are useful for treating and/or preventing dermal infections (*e.g.*, bacterial, viral, and/or fungal infections).

In some embodiments, diseases, disorders, or conditions associated with infection of the dermis are associated with bacterial infection, for example caused by or correlated with infection by one or more of *Staphylococcus aureus, Streptococcus pyogenes,* group B and C streptococci, anaerobic bacteria (*e.g.*, *Clostridium* species), *Corynebacterium* species (*e.g.*, *Corynebacterium minutissimum, Corynebacterium tenuis, etc.*), *Dermatophilus congolensis,* and/or combinations thereof. Diseases, disorders, or conditions associated with bacterial infection of the dermis, include, but are not limited to, impetigo, folliculitis, furunculosis, carbunculosis, hidradenitis suppurativa (*i.e.*, bacterial infection of sweat glands and/or hair follicles), skin abscesses, cat scratch disease, cellulitis, erysipelas, ecthyma, necrotizing fasciitis, erythrasma, pitted keratolysis, trichomycosis axillaris, staphylococcal scalded skin syndrome, acute paronychia, and/or combinations thereof.

In some embodiments, diseases, disorders, or conditions associated with infection of the dermis are associated with viral infection, for example caused by or correlated with infection by one or more of herpes simplex virus (*e.g.*, type 1 and/or type 2), varicella-zoster virus, human papillomavirus, poxvirus, *etc.* Diseases, disorders, or conditions associated with viral infection of the dermis include, but are not limited to, herpes labialis, genital herpes, shingles, molluscum contagiosum, warts, and/or combinations thereof.

In some embodiments, diseases, disorders, or conditions associated with infection of the dermis are associated with fungal infection, for example caused by or correlated with infection by one or more of *Trichophyton* species (*e.g.*, *Trichophyton rubrum*), *Microsporum* species, *Epidermophyton* species, *Candida* species (*e.g.*, *Candida albicans*), *Pityrosporum ovale,* and/or combinations thereof. Diseases, disorders, or conditions associated with fungal infection of the dermis, include, but are not limited to, dermatophytosis, tinea pedis ("athlete's foot"), candidal intertrigo, thrush, paronychia, angular cheilitis, candidal vulvovaginitis, balanitis, tinea versicolor, chronic paronychia, and/or combinations thereof.

Current therapies for treatment and/or prevention of bacterial infection of the dermis include, but are not limited to, botulinum toxin; antibiotics (*e.g.*, penicillin, dicloxacillin, cephalexin, erythromycin, clindamycin, gentamicin, *etc.*), topical antibiotics (*e.g.* clindamycin, erythromycin, mupirocin *etc.*), topical mixture of bacitracin and polymyxin (*e.g*., NEOSPORIN®, POLYSPORIN®), topical fusidic acid cream, and combinations thereof.

Current therapies for treatment and/or prevention of diseases, disorders, or conditions associated with viral infection of the dermis include, but are not limited to, botulinum toxin, antiviral therapeutics (*e.g.*, acyclovir, famciclovir, valacyclovir, *etc.),* topical treatments (*e.g.*, trichloroacetic acid, salicylic acid, podophyllin, canthacur, imiquimod cream, *etc.*), and/or combinations thereof.

Current therapies for treatment and/or prevention of diseases, disorders, or conditions associated with fungal infection of the dermis include, but are not limited to, botulinum toxin, topical therapeutics (*e.g*., terbinafine [LAMISIL®], clotrimazole [LOTRIMIN®, MYCELEX®], or econazole [SPECTAZOLE®], selenium sulfide shampoo, ketoconazole shampoo, *etc*.), oral therapeutics (*e.g*., itraconazole [SPORANOX®], terbinafine, *etc*.), and/or combinations thereof.

Alternative or additional current therapies utilized in the treatment and/or prevention of one or more symptoms and/or causes of dermal infection include, but are not limited to, surgical removal of affected skin, amputation, *etc.*

In some embodiments, provided compositions for treatment and/or prevention of dermal infections are formulated into a cream, liniment, lotion, gel, shampoo, conditioner, sunscreen, deodorant, and/or antiperspirant (*e.g.*, as a roll-on, solid stick, gel, cream, aerosol, etc.), etc..

In some embodiments, provided compositions for treatment and/or prevention of dermal infections are administered locally to an affected site (*e.g.*, on axillae, hands, feet, scalp, hair follicle, face, neck, back, arms, chest, *etc.*).

### Actinic Keratosis

In some embodiments, provided compositions may are useful for treating and/or preventing actinic keratosis. Actinic keratosis (also called "solar keratosis," or "AK") is a premalignant condition of thick, scaly, or crusty patches of skin. Actinic keratosis is most common in fair-skinned people who are frequently exposed to the sun. When skin is exposed to the sun constantly, thick, scaly, or crusty bumps appear. The scaly or crusty part of the bump is dry and rough. A growth starts out as flat scaly areas, and later grows into a tough, wart-like area.

An actinic keratosis site commonly ranges between 2 mm and 6 mm in size, and can be dark or light, tan, pink, red, a combination of all these, or have the same pigment as the surrounding skin. It may appear on any sun-exposed area, such as the face, ears, neck, scalp, chest, backs of hands, forearms, or lips.

Current therapies utilized for treatment and/or prevention of diseases, disorders, or conditions associated with actinic keratosis include, but are not limited to, botulinum toxin, 5-fluorouracil, imiquimod, diclofenac, crocodile oil, and/or combinations thereof.

Alternative or additional current therapies utilized to treat and/or prevent one or more symptoms and/or causes of actinic keratosis include, but are not limited to, cryosurgery, photodynamic therapy, laser treatment, electrocautery, surgery, *etc.*

In some embodiments, provided compositions for treatment and/or prevention of actinic keratosis are formulated into a cream, liniment, lotion, gel, shampoo, conditioner, sunscreen, deodorant, and/or antiperspirant (*e.g*., as a roll-on, solid stick, gel, cream, aerosol, etc.), etc..

In some embodiments, provided compositions for treatment and/or prevention of actinic keratosis are administered locally to an affected site (*e.g.*, on axillae, hands, feet, scalp, hair follicle, face, neck, back, arms, chest, *etc.*).

### Eczematous Dermatitis

In some embodiments, provided compositions are useful for treating and/or preventing eczematous dermatitis, a skin condition characterized by local inflammatory reactions that are erythematous with indistinct margins. In the acute phase, lesions may exhibit edema, vesiculation, oozing, and in some cases bullae. Most chronic lesions are dry and scaly and may exhibit secondary lichenification. These lesions frequently get secondary bacterial infections, which may also cause crusting. These lesions are frequently pruritic. Sometimes, this condition may be secondary to exposure to an allergen.

Atopic dermatitis is a more generalized form of eczematous dermatitis which typically involves many areas of the skin and intense prurititis. This condition is often associated with a personal or family history of asthma, hay fever, or other allergies. Lesions are frequently distributed on the antecubital andpopliteal fosse, and on the wrist and neck. Eczematous dermatitis and atopic dermatitis are also known in the art as "eczema."

Current therapies utilized for treating and/or preventing one or more symptoms and/or causes of eczematous dermatitis include botulinum toxin, glucocorticosteroids, coal tar, calcineurin inhibitors (*e.g.*, tacrolimus, pimecrolimus, *etc.*), antihistamines (*e.g.*, diphenhydramine, *etc.*), cyclosporine, interferon, omalizumab, rituximab, mycophenolate mofetil, AMG 157, JNJ-26113100, CD 2027, SUN13834, S-777469, GW842470X, TS022, roflumilast, calcipotriol, pitrakinra, and/or combinations thereof.

In some embodiments, provided compositions for treatment and/or prevention of eczematous dermatitis are formulated into a cream, liniment, lotion, gel, shampoo, conditioner, sunscreen, deodorant, and/or antiperspirant (*e.g.*, as a roll-on, solid stick, gel, cream, aerosol, *etc.*), *etc.*

In some embodiments, provided compositions for treatment and/or prevention of eczematous dermatitis are administered locally to an affected site (*e.g.*, on axillae, hands, feet, scalp, face, neck, back, arms, chest, *etc.*).

### Excess Sebum-Producing Disorders

In some embodiments, provided compositions are useful for treating and/or preventing excess sebum-producing disorders (*e.g.*, seborrhea, seborrheic dermatitis, *etc.*), disorders affecting the areas of the skin that are rich in sebum glands, which typically include the scalp, face, and/or trunk. Patients with these conditions typically have scaly, flaky, erythematous, and often pruritic skin. Involvement of the scalp can result in hair loss. In some cases, the skin is also oily.

Current therapies utilized for treating and/or preventing one or more symptoms and/or causes of excess sebum-producing disorders include botulinum toxin, salicylic acid, azelaic acid, selenium sulfide, imidazoles (*e.g.*, ketoconazole, miconazole, fluconazole, econazole, bifonazole, climazole, ciclopirox, ciclopiroxolamine, *etc.*), itraconazole, terbinafine, zinc pyrithione, benzoyl peroxide, coal tar, juniper tar, glucocorticosteroids (*e.g.*, hydrocortisone, *etc.*), metronidazole, lithium, calcineurin inhibitors (*e.g.*, tacrolimus, pimecrolimus, *etc.*), Vitamin D3, isotretinoin, and/or combinations thereof.

In some embodiments, provided compositions for treatment and/or prevention of one or more excess sebum-producing disorders are formulated into a cream, liniment, lotion, gel, sunscreen, deodorant, and/or antiperspirant (*e.g*., as a roll-on, solid stick, gel, cream, aerosol, *etc.*), *etc.*

In some embodiments, provided compositions for treatment and/or prevention of one or more excess sebum-producing disorders are administered locally to an affected site (*e.g.*, on axillae, hands, feet, scalp, face, neck, back, arms, chest, *etc.*).

### Burns

In some embodiments, provided compositions are useful for treating burns, a type of injury to flesh caused by heat, electricity, chemicals, light, radiation or friction. Many burns affect only the skin, but sometimes burns can injure deeper tissues, such as muscle, bone, and blood vessels. Burns can be classified as either first-degree, second-degree, third-degree, or fourth-degree.

First-degree burns are usually limited to redness (erythema), a white plaque and minor pain at the site of injury. These burns generally involve only the epidermis. Most sunburns can be included as first-degree burns.

Second-degree burns manifest as erythema with superficial blistering of the skin, and can involve more or less pain depending on the level of nerve involvement. Second-degree burns typically involve the superficial (papillary) dermis and may also involve the deep (reticular) dermis layer. Burns that require more than three weeks to heal are often excised and skin grafted for best result.

Third-degree burns occur when the epidermis is lost with damage to the subcutaneous tissue. Burn victims will typically exhibit charring and extreme damage of the epidermis, and sometimes hard eschar will be present. Third-degree burns result in scarring and victims will also exhibit the loss of hair shafts and keratin. These burns may require grafting. These burns are not painful, as all the nerves have been damaged by the burn and are not sending pain signals; however, all third-degree burns are surrounded by first and second-degree burns, which are painful.

Fourth-degree burns involve muscle, tendon, and bone. When extremities are involved, this often leads to amputation or significant functional impairment.

Current therapies utilized for treating and/or preventing one or more symptoms and/or causes of burns include botulinum toxin, antibiotics, analgesics, and/or combinations thereof.

In some embodiments, provided compositions for treatment and/or prevention of burns are formulated into a cream, liniment, lotion, gel, sunscreen, *etc.*

In some embodiments, provided compositions for treatment of burns are administered locally to an affected site (*e.g.*, on axillae, hands, feet, scalp, face, neck, back, arms, chest, *etc.*).

### Raynaud's Phenomenon

In some embodiments, provided compositions are for treating and/or preventing Raynaud's phenomenon, a vasospastic condition of the fingers and toes. Typically in response to cold or emotional stress, the skin of the fingers become discolored (white, blue, and/or red, often in this sequence) and painful. Severe Raynaud's can result in necrosis of the skin and ultimately the fingers and/or toes, resulting in "auto-amputation." Nails of Raynaud's patients may become brittle. This condition is frequently associated with connective tissue diseases such as scleroderma and/or rheumatoid arthritis.

Current therapies for treatment and/or prevention of one or more symptoms and/or causes of Raynaud's phenomenon include botulinum toxin, calcium channel blockers (*e.g.*, nifedipine, *etc.*), alpha blockers (*e.g.*, hydralazine, *etc.*), nitroglycerin, angiotensin II receptor antagonists (*e.g.*, losartan, *etc.*), selective serotonin reuptake inhibitors (*e.g.*, fluoxetine, *etc.*), glyceryl trinitrate, tadalafil, *Ginkgo biloba* extract, SLx-2101, St. John's Wort, fasudil, cilostazol, iloprost, relaxin, treprostinil diethanolamine, sildenafil, atorvastatin, imatinib mesylate, treprostinil diethanolamine, and/or combinations thereof.

In some embodiments, provided compositions for treatment and/or prevention of Raynaud's phenomenon are formulated into a cream, liniment, lotion, gel, sunscreen, *etc.*

In some embodiments, provided compositions for treatment and/or prevention of Raynaud's phenomenon are administered locally to an affected site (*e.g.*, on axillae, hands, feet, *etc.*).

### Lupus Erthythematosus

In some embodiments, provided compositions are useful for treating and/or preventing lupus erthythematosus, an autoimmune condition that may involve the skin as well as disease of multiple organ systems, including the brain and nervous system, kidneys, liver, and/or blood vessels. A lupus rash often involves the malar region of the face and is described as a "butterfly rash". Some patients exhibit thick, red, scaly patches of skin referred to as discoid lupus. Hair loss can also be a manifestation of the disease. Mouth, nasal and vaginal ulcers are also possible.

Current therapies for the treatment and/or prevention of one or more symptoms and/or causes of lupus erthythematosus include botulinum toxin, nonsteroidal anti-inflammatory medications (*e.g.*, ibuprofen, *etc.*), aspirin, antimalarial drugs (*e.g.*, chloroquine, hydroxychloroquine, *etc.*), corticosteroids (*e.g.*, hydroxycortisone, *etc.*), immunosupressive medications (*e.g*., azathioprine, cyclophosphamide, cyclosporine, mycophenolate mofetil, methotrexate, therapeutic antibodies, *etc.*), and/or combinations thereof.

In some embodiments, provided compositions for treatment and/or prevention of lupus erythematosus are formulated into a cream, liniment, lotion, gel, sunscreen, *etc.*

In some embodiments, provided compositions for treatment and/or prevention of lupus erythematosus are administered locally to an affected site (*e.g.*, on axillae, hands, feet, scalp, face, neck, back, arms, chest, *etc.*).

### Hyperpigmentation Disorders

In some embodiments, provided compositions are useful for treating and/or preventing one or more hyperpigmentation disorders (*e.g.*, melasma, *etc.*), that result in focal or generalized abnormal darkening of the skin. Hyperpigmentation is often due to skin damage due to sun exposure, medications, and/or inflammation (including inflammation due to acne vulgaris). Melasma is a condition of dark, irregular patches of skin found most usually on the upper cheek, nose, lips, upper lip, and/or forehead. Melasma is often associated with pregnancy.

Current therapies utilized for the treatment and/or prevention of one or more symptoms and/or causes of hyperpigmentation disorders include botulinum toxin, phenols (*e.g.*, hydroxyquinone, mequinol, *etc.*), retinoids (*e.g.*, tretinoin, isotretinoin, *etc.*), alpha-hydroxy acids (*e.g.*, glycolic acid, salicylic acid, azelaic acid, *etc.*) and/or combinations thereof.

In some embodiments, provided compositions for treatment and/or prevention of one or more hyperpigmentation disorders are formulated into a cream, liniment, lotion, gel, sunscreen, *etc.*

In some embodiments, provided compositions for treatment and/or prevention of one or more hyperpigmentation disorders are administered locally to an affected site (*e.g.*, on axillae, hands, feet, scalp, hair follicle, face, neck, back, arms, chest, *etc.*).

### Hypopigmentation Disorders

In some embodiments, provided compositions may are for treating and/or preventing one or more hypopigmentation disorders (*e.g.*, vitiligo, *etc.*), which are characterized by focal and/or generalized abnormal lightening of the skin. Vitiligo is characterized by a chronic focal loss of skin pigment and hence lightening of the skin. When skin lesions occur, they are most prominent on the face, hands and wrists. Depigmentation is particularly noticeable around body orifices, such as the mouth, eyes, nostrils, genitalia, and/or umbilicus.

Current therapies utilized for the treatment and/or prevention of one or more symptoms and/or causes of hypopigmentation disorders include botulinum toxin, corticosteroids, calcineurin inhibitors (*e.g.*, tacrolimus, pimecrolimus, *etc.*), calcipotriol, psoralen, and/or combinations thereof.

In some embodiments, provided compositions for treatment and/or prevention of one or more hypopigmentation disorders are formulated into a cream, liniment, lotion, gel, sunscreen, *etc.*

In some embodiments, provided compositions for treatment and/or prevention of one or more hypopigmentation disorders are administered locally to an affected site (*e.g.*, on axillae, hands, feet, scalp, face, neck, back, arms, chest, *etc.*).

### Skin Cancer

In some embodiments, provided compositions are useful for treating and/or preventing skin cancer (*e.g.*, squamous cell skin carcinoma, basal cell skin carcinoma, *etc.*), a malignant growth of skin tissue, often resulting in a visible tumor. Skin cancer may exhibit skin growths, changes in the skin that do not heal, ulceration of the skin, discolored skin, and/or changes to existing moles, such as the appearance of irregular edges to the mole and/or or an enlargement of the mole. Basal cell carcinoma usually looks like a raised, smooth, pearly bump on the sun-exposed skin of the head, neck, and/or shoulders. Occasionally, small blood vessels can be seen within these tumors. Crusting and bleeding in the center of these tumors are frequently exhibited. Squamous cell carcinoma is commonly a red, scaling, thickened patch on sun-exposed skin. Ulceration and bleeding may be exhibited and when untreated, this form of skin cancer may develop into a large mass.

Current therapies utilized for treatment and/or prevention of squamous cell skin carcinoma include botulinum toxin, 5-aminolevulinic acid, 5-fluorouracil, acitretin, afamelanotide, API 31510, API 31510, cetuximab, dasatinib, eflornithine, erlotinib, GDC-0449, efitinib, HPPH, imiquinod, methyl aminolevulinate, PEG-interferon alfa-2a, PEP005, silicon phthalocyanine 4, tazarotene, tretinoin, verteporfin, and/or combinations thereof.

Current therapies utilized for treatment and/or prevention of basal cell skin carcinoma include botulinum toxin, 5-aminolevulinic acid, 5-fluorouracil, acitretin, afamelanotide, API 31510, API 31510, cetuximab, dasatinib, eflornithine, erlotinib, GDC-0449, gefitinib, HPPH, imiquinod, methyl aminolevulinate, PEG-interferon alfa-2a, PEP005, silicon phthalocyanine 4, tazarotene, Tretinoin, verteporfin, and/or combinations thereof.

In some embodiments, provided compositions for treatment and/or prevention of skin cancer are formulated into a cream, liniment, lotion, gel, sunscreen, deodorant, and/or antiperspirant (*e.g.*, as a roll-on, solid stick, gel, cream, aerosol, *etc.*), *etc.*

In some embodiments, provided compositions for treatment and/or prevention of skin cancer are administered locally to an affected site (*e.g.*, on axillae, hands, feet, scalp, face, neck, back, arms, chest, *etc.*).

### Treatment of Wrinkles

In some embodiments, provided compositions are useful for treating and/or preventing wrinkles (*e.g.*, facial wrinkles). Facial wrinkles involving the forehead, glabellar, rhytids and/or periorbital regions are a common aesthetic problem and are believed related to overactivity of the underlying facial musculature. For instance, the development of glabellar wrinkles is related, at least in part, to the dynamics of the underlying procerus, corrugator supercilii, and orbicularis oculi muscles. Facial lines are considered problematic because they produce the appearance of aging. In some cases, they can also be misinterpreted as manifestations of negative emotions (*e.g.*, anger, anxiety, sadness, *etc.*), fatigue, or stress.

Current therapies utilized in the treatment and/or prevention of wrinkles include, but are not limited to, botulinum toxin; tretinoin (RETIN-A®); epidermal growth factor; and/or glycosaminoglycans.

In recent years, injections of botulinum toxin solutions have become one of the most popular therapies for the treatment of hyperfunctional facial lines. After injection, the toxin acts to paralyze or weaken facial mimetic muscles. This apparently reduces or eliminates the appearance of wrinkles. Sadick, 2004, Clin. Dermatol. 22:29-33 (incorporated herein by reference).

The initial cosmetic use of a botulinum toxin solution was for treatment of forehead frown lines (Carruthers et al., 1992, J. Dermatol. Surg Oncol., 18:17; incorporated herein by reference). It has also been noted that injection of botulinum toxin solution into the platysma produces an uplift of the mouth (Brandt et al., 1998, Dermatol. Surg., 24:1232; incorporated herein by reference). Injection of botulinum toxin solution into the point of the chin has also been done for treatment of prominent mental crease (Carruthers et al., "Cosmetic Uses of Botulinum A Exotoxin," pp. 325-48, Advances in Dermatology, James, et al., eds., Mosby-Yearbook, Chicago, 1997; incorporated herein by reference).

It has been recently been suggested that the onset of facial wrinkles and/or lines can be delayed by the long-term use of botulinum type A toxin treatment via repeated injections (Binder, 2006, Arch. Facial Plast. Surg., 8:426). However, repeated injections are painful to the patient, and there is a risk of injecting unintended muscle groups, potentially causing adverse side-effects (*e.g*. ptosis).

In some embodiments, provided compositions for treatment and/or prevention of wrinkles are formulated into a cream, liniment, lotion, gel, sunscreen, *etc.*

In some embodiments, provided compositions for treatment and/or prevention of wrinkles are administered locally to an affected site (*e.g.*, face, neck, *etc.*).

### Headache

In some embodiments, provided compositions are useful for treating and/or preventing headache. In some embodiments, headache includes, but is not limited to, migraine headache, essential headache, cervicogenic headache, and/or tension headache.

Current therapies utilized for treatment and/or prevention of headache include botulinum toxin, analgesics (*e.g*., paracetamol, acetaminophen, non-steroidal anti-inflammatory drugs, such as aspirin, ibuprofen, diclofenac, naproxen), amitriptyline, fluoxetine, gabapentin, tizanidine, topiramate, anti-epileptics (*e.g.*, valproate), muscle relaxants such as any of those described herein, opiates (*e.g.*, morphine, codeine, thebaine, papaverine, oxycodone, hydrocodone, *etc.*), and/or combinations thereof.

In some embodiments, provided compositions for treatment and/or prevention of headache are formulated into a cream, liniment, lotion, gel, sunscreen, *etc.*

In some embodiments, provided compositions for treatment and/or prevention of headache are administered locally to an affected site (*e.g.*, face, neck, *etc.*).

### Compositions and Formulations

As noted herein, the present invention provides compositions comprising nanoparticle compositions, cream and/or lotion formulations, and/or individual components thereof. Provided compositions may be formulated for topical and/or transdermal delivery (*e.g.*, by lotions, creams, liniments, ointments, powders, gels, drops, *etc.*).

Formulations of provided compositions may be prepared by any appropriate method, for example as known or hereafter developed in the art of pharmacology. In general, such preparatory methods include the step of bringing an provided composition into association with one or more excipients, and then, if necessary and/or desirable, shaping and/or packaging the product into an appropriate form for administration, for example as or in a single- or multi-dose unit.

In some embodiments, compositions may be prepared, packaged, and/or sold in bulk, as a single unit dose, and/or as a plurality of single unit doses. As used herein, a "unit dose" is a discrete amount of the pharmaceutical composition comprising a predetermined amount of the provided composition. The amount of the provided composition is generally equal to the dosage of the provided composition which would be administered to a subject and/or a convenient fraction of such a dosage such as, for example, one-half or one-third of such a dosage.

Appropriate excipients for use in compositions (*e.g.*, pharmaceutically and/or cosmetically acceptable compositions) may, for example, include one or more excipients such as solvents, dispersion media, granulating media, diluents, or other liquid vehicles, dispersion or suspension aids, surface active agents and/or emulsifiers, isotonic agents, thickening or emulsifying agents, preservatives, solid binders, lubricants, disintegrating agents, binding agents, preservatives, buffering agents and the like, as suited to the particular dosage form desired. Alternatively or additionally, excipients such as cocoa butter and/or suppository waxes, coloring agents, coating agents, sweetening, flavoring, and/or perfuming agents can be utilized. Remington's The Science and Practice of Pharmacy, 21st Edition, A. R. Gennaro (Lippincott, Williams & Wilkins, Baltimore, MD, 2005; incorporated herein by reference) discloses various excipients used in formulating pharmaceutical compositions and known techniques for the preparation thereof.

In some embodiments, an appropriate excipient (*e.g.*, a pharmaceutically and/or cosmetically acceptable excipient) is at least 95%, at least 96%, at least 97%, at least 98%, at least 99%, or 100% pure. In some embodiments, an excipient is approved by United States Food and Drug Administration. In some embodiments, an excipient is pharmaceutical grade. In some embodiments, an excipient meets the standards of the United States Pharmacopoeia (USP), the European Pharmacopoeia (EP), the British Pharmacopoeia, and/or other International Pharmacopoeia.

In some embodiments, provided compositions are formulated as a cream, liniment, ointment, oil, foam, spray, lotion, liquid, powder, thickening lotion, or gel (*e.g.*, formulated for transdermal delivery as described herein). Particular exemplary such formulations may be prepared, for example, as cosmetic formulation products such as skin softeners, nutritional lotion type emulsions, cleansing lotions, cleansing creams, skin milks, emollient lotions, massage creams, emollient creams, make-up bases, lipsticks, facial packs or facial gels, cleaner formulations such as shampoos, rinses, body cleansers, hair-tonics, or soaps, or dermatological compositions such as lotions, ointments, gels, creams, liniments, patches, deodorants, or sprays.

In some embodiments, provided nanoparticle compositions are admixed with a cream formulation (*e.g.*, a provided cream formulation) and/or a saline solution for preparation of a pharmaceutical composition.

In some embodiments, provided compositions are formulated with cosmetically acceptable components. For example, in some embodiments, provided compositions are formulated with water and also any cosmetically acceptable solvent, in particular, monoalcohols, such as alkanols having 1 to 8 carbon atoms (like ethanol, isopropanol, benzyl alcohol and phenylethyl alcohol), polyalcohols, such as alkylene glycols (like glycerine, ethylene glycol and propylene glycol), and glycol ethers, such as mono-, di-, and tri-ethylene glycol monoalkyl ethers, for example, ethylene glycol monomethyl ether and diethylene glycol monomethyl ether, used singly or in a mixture. Such components can be present, for example, in proportions of up to as much as 60%, 70%, 80%, or 90% by weight, relative to the weight of the total composition.

In some embodiments, provided compositions for topical administration include one or more cosmetically acceptable components that impart appearance attributes desirable or appropriate to the subject to which the composition is to be applied (*e.g.*, a matte appearance, which may be particularly desirable or appropriate for administration to subjects having greasy skin).

In some embodiments, provided compositions are formulated with at least one cosmetically acceptable filler material, for example, in order to obtain a matte product, which may be especially desired for individuals with greasy skin.

Those of ordinary skill in the art will appreciate that provided compositions may be incorporated into a device such as, for example, a patch. A variety of transdermal patch structures are known in the art; those of ordinary skill will appreciate that provided compositions may readily be incorporated into any of a variety of such structures. In some embodiments, a transdermal patch may further comprise a plurality of needles extending from one side of the patch that is applied to the skin, wherein needles extend from the patch to project through the stratum corneum of the skin. In some embodiments, needles do not rupture a blood vessel.

In some embodiments, a transdermal patch includes an adhesive. Some examples of adhesive patches are well known (for example, see U.S. Design Patent 296,006; and U.S. Patents 6,010,715; 5,591,767; 5,008,110; 5,683,712; 5,948,433; and 5,965,154; all of which are incorporated herein by reference). Adhesive patches are generally characterized as having an adhesive layer, which will be applied to a patient's skin, a depot or reservoir for holding a provided composition, and an exterior surface that prevents leakage of the provided composition from the depot. The exterior surface of a patch is typically non-adhesive.

In accordance with the present invention, a provided composition is incorporated into the patch so that it remains stable for extended periods of time. For example, a provided composition may be incorporated into a polymeric matrix that stabilizes the agent, and permits the agent to diffuse from the matrix and the patch. A provided composition may also be incorporated into the adhesive layer of the patch so that once the patch is applied to the skin, the provided composition may diffuse through the skin. In some embodiments, an adhesive layer may be heat-activated where temperatures of about 37 °C cause the adhesive to slowly liquefy so that the agent diffuses through the skin. The adhesive may remain tacky when stored at less than 37 °C, and once applied to the skin, the adhesive loses its tackiness as it liquefies.

In some embodiments, a provided composition can be provided in a depot in the patch so that pressure applied to the patch causes the provided composition to be directed out of the patch (optionally through needles) and through the stratum corneum.

Suitable devices for use in administering provided compositions intradermally include short needle devices such as those described in U.S. Patents 4,886,499; 5,190,521; 5,328,483; 5,527,288; 4,270,537; 5,015,235; 5,141,496; and 5,417,662. Intradermal compositions may be administered by devices which limit the effective penetration length of a needle into the skin, such as those described in PCT publication WO 99/34850 and functional equivalents thereof. Jet injection devices which deliver provided compositions to the dermis via a liquid jet injector and/or via a needle which pierces the stratum corneum and produces a jet which reaches the dermis are suitable. Jet injection devices are described, for example, in U.S. Patents 5,480,381; 5,599,302; 5,334,144; 5,993,412; 5,649,912; 5,569,189; 5,704,911; 5,383,851; 5,893,397; 5,466,220; 5,339,163; 5,312,335; 5,503,627; 5,064,413; 5,520,639; 4,596,556; 4,790,824; 4,941,880; 4,940,460; and PCT publications WO 97/37705 and WO 97/13537. Ballistic powder/particle delivery devices which use compressed gas to accelerate provided compositions in powder form through the outer layers of the skin to the dermis are suitable. Alternatively or additionally, conventional syringes may be used in the classical mantoux method of intradermal administration.

In order to prolong the effect of a provided composition, it may be desirable to slow the absorption of the provided composition from subcutaneous or intramuscular injection. This may be accomplished by the use of a liquid suspension of crystalline or amorphous material with poor water solubility. The rate of absorption of the provided composition then depends upon its rate of dissolution which, in turn, may depend upon crystal size and crystalline form. Depending upon the ratio of provided composition to polymer and the nature of the particular polymer employed, the rate of provided composition release can be controlled. Examples of other biodegradable polymers include poly(orthoesters) and poly(anhydrides).

### Administration

As described herein, the present invention provides methods of administering provided compositions to a subject for various applications including, for example, cosmetic and/or medical applications. In some embodiments, the present invention provides methods of treating and/or preventing diseases, disorders, and/or conditions associated with activity of epidermal and/or dermal structures (*e.g.*, sweat glands, sebaceous glands, hair follicles, *etc.*) by administering provided compositions to a subject in need thereof.

In some embodiments, provided methods involve topical, transdermal, or intradermal administration of provided compositions to the skin of a subject. In some embodiments, such routes achieve local delivery. In some embodiments, such routes achieve systemic delivery.

### Transdermal Administration

Human skin comprises the dermis and the epidermis. The epidermis has several layers of tissue, namely, stratum corneum, stratum lucidum, stratum granulosum, stratum spinosum, and stratum basale (identified in order from the outer surface of the skin inward).

The stratum corneum presents the most significant hurdle in traditional methods of transdermal delivery of medications. The stratum corneum is typically about 10 µm - 15 µm thick, and it comprises flattened, keratised cells (corneocytes) arranged in several layers. The intercellular space between the corneocytes is filled with lipidic structures, and may play a role in the permeation of substances through skin (Bauerova et al., 2001, Eur. J. Drug Metabolism Pharmacokinetics, 26:85; incorporated herein by reference).

The rest of the epidermis below the stratum corneum is approximately 150 µm thick. The dermis is about 1 mm - 2 mm thick and is located below the epidermis. The dermis is supported by various tissues, such as connective tissue, capillaries neuronal processes, *etc.*

Transdermal administration of pharmaceuticals generally has been the subject of research in an attempt to provide an alternative route of administration of medications without undesirable consequences associated with injections and oral delivery. For example, needles often cause localized pain, bleeding and bruising, and potentially expose patients to transmissible diseases; oral administration can suffer from poor bioavailability of medications due to the extremely acidic environment of the patient's stomach. In some embodiments, transdermal delivery has a more even, regular, and/or consistent pharmacokinetic profile as compared with other routes of administration.

Efforts have been made to develop transdermal administration delivery systems for certain pharmaceuticals. It is generally desirable with transdermal administration to minimize damage to a patient's skin. Among other beneficial features, transdermal administration of medication may reduce or eliminate pain associated with injections and/or reduce the likelihood of infection.

Traditionally, attempts at transdermal administration of medication have been focused on increasing the permeability of the stratum corneum. Some attempts have included using chemical penetration enhancing agents that increase the permeability of molecules through the skin. Some attempts have included using mechanical apparatus to bypass or ablate portions of the stratum corneum. In addition, attempts have included use of ultrasound or iontophoresis to facilitate the permeation of pharmaceuticals through the skin. In some instances, the goal has been to deliver a pharmaceutical agent, typically a small molecule, through the skin, for example so that an agent may pass to the capillary bed in the dermis where the agent may be systemically incorporated into the subject to achieve a therapeutic effect. In some instances, the goal has been to achieve local and/or non-systemic effects.

In some embodiments, the present invention achieves transdermal delivery with provided compositions without use of abrasive or other disrupting agents (whether chemical, mechanical, electrical, magnetic, *etc.*). In some embodiments, the present invention achieves transdermal delivery of provided compositions without affirmative steps to permeabilize or disrupt the stratum corneum.

In some embodiments, the present invention contemplates transdermal delivery of provided compositions to achieve systemic delivery and/or effects. In some embodiments, the present invention contemplates transdermal delivery of provided compositions to achieve local delivery and/or effects, for example without achieving systemic delivery and/or effects.

In some embodiments, a provided composition is applied directly to the skin. In some embodiments, an applied composition is absorbed through the epidermal layers. In some embodiments, a provided composition can penetrate the top layer of the skin, including the stratum corneum, dermal pores, and/or dermal glands, without the use of chemical or mechanical skin permeation enhancers or other agents that cause abrasion.

In some embodiments, the present invention provides methods and compositions for specific delivery of provided compositions to epidermal and/or dermal structures. In some embodiments, provided compositions are specifically delivered to epidermal and/or dermal structures without significant delivery to subdermal structures. In some embodiments, greater than about 50%, greater than about 60%, greater than about 70%, greater than about 80%, greater than about 85%, greater than about 90%, greater than about 95%, greater than about 96%, greater than about 97%, greater than about 98%, greater than about 99%, greater than about 99.5%, or about 100% of a provided composition administered to the skin of a subject is delivered specifically to the epidermis and/or dermis. In some embodiments, less than about 50%, less than about 40%, less than about 30%, less than about 20%, less than about 10%, less than about 5%, less than about 4%, less than about 3%, less than about 2%, less than about 1%, less than about 0.5%, or less than about 0.1% of a provided composition administered to the skin of a subject is delivered to subdermal structures.

In some embodiments, specific delivery to epidermal and/or dermal structures is achieved through application of a dose of provided composition that is lower than a dose per area used to achieve delivery to subdermal structures. For example, in some embodiments, a volume of provided composition is applied to a larger surface area; in some embodiments, a provided composition containing a reduced amount of provided composition per unit volume of composition is utilized than would be utilized to achieve delivery to subdermal structures; in some embodiments, penetration of provided composition into the skin is reduced (*e.g.*, through combination with penetration inhibitors and/or adjustment of provided composition characteristics such as component ratios, component identity, *etc.*, and combinations thereof). In some embodiments, such a lower dose is at least about 2-fold, about 3-fold, about 4-fold, about 5-fold, about 10-fold, about 20-fold, about 30-fold, about 40-fold, about 50-fold, about 100-fold, or greater than about 100-fold lower than a dose per area used to achieve delivery to subdermal structures.

### Combination Therapy

According to the present invention, provided compositions may be administered in combination with one or more other active agents and/or therapeutic modalities, such as known therapeutic agents and/or independently active biologically active agents. In some embodiments, provided compositions include one or more such other active agents; in some embodiments, such other active agents are provided as part of distinct compositions. In some embodiments, combination therapy involves simultaneous administration of one or more doses or units of two or more different active agents and/or therapeutic modalities; in some embodiments, combination therapy involves simultaneous exposure to two or more different active agents and/or therapeutic modalities, for example through overlapping dosing regimens.

In some embodiments, provided compositions include or are administered in combination with one or more other active agents useful for the treatment of the relevant dermatologic or other disease, disorder and/or condition, for example as discussed herein in context of the relevant disease, disorder, and/or condition.

### Kits

In some embodiments, the present invention provides pharmaceutical packs or kits including provided compositions according to the present invention. In certain embodiments, pharmaceutical packs or kits include preparations or pharmaceutical compositions containing provided compositions in one or more containers filled with optionally one or more additional ingredients of pharmaceutical compositions. In certain embodiments, the pharmaceutical pack or kit includes an additional approved therapeutic agent (*e.g.*, benzoyl peroxide for treatment of acne; aluminum compounds for treatment of hyperhidrosis; *etc.*) for use in combination therapies. Optionally associated with such container(s) can be a notice in the form prescribed by a governmental agency regulating the manufacture, use or sale of pharmaceutical products, which notice reflects approval by the agency of manufacture, use, or sale for human administration.

Kits are provided that include therapeutic reagents. As but one non-limiting example, provided compositions can be provided as topical formulations and administered as therapy. Pharmaceutical doses or instructions therefor may be provided in a kit for administration to an individual suffering from or at risk for conditions or disorders, e.g., those associated with the dermal level of the skin.

In some embodiments, a kit may comprise (i) a provided composition; and (ii) at least one pharmaceutically acceptable excipient; and optionally (iii) at least one syringe, spatula, swab for administration to skin; and (iv) instructions for use.

### Exemplification

The representative examples that follow are intended to help illustrate the invention, and are not intended to, nor should they be construed to, limit the scope of the invention. Indeed, various modifications of the invention and many further embodiments thereof, in addition to those shown and described herein, will become apparent to those skilled in the art from the full contents of this document, including the examples which follow and the references to the scientific and patent literature cited herein. The following examples contain information, exemplification and guidance, which can be adapted to the practice of this invention in its various embodiments and the equivalents thereof.

### Example 1: Exemplary Botulinum Nanoparticle Composition

This example presents an exemplary botulinum toxin nanoparticle composition for use in accordance with the present invention.

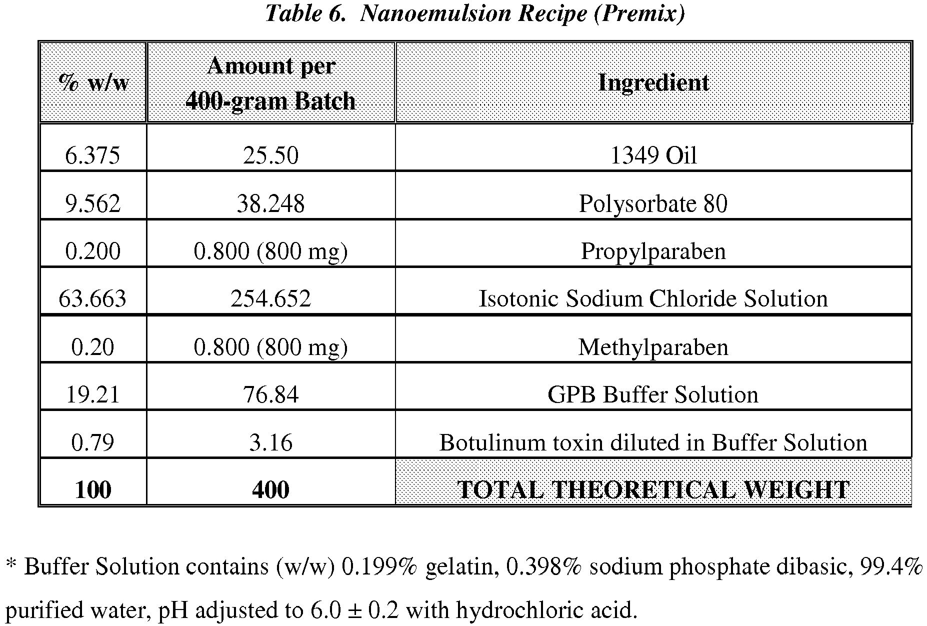

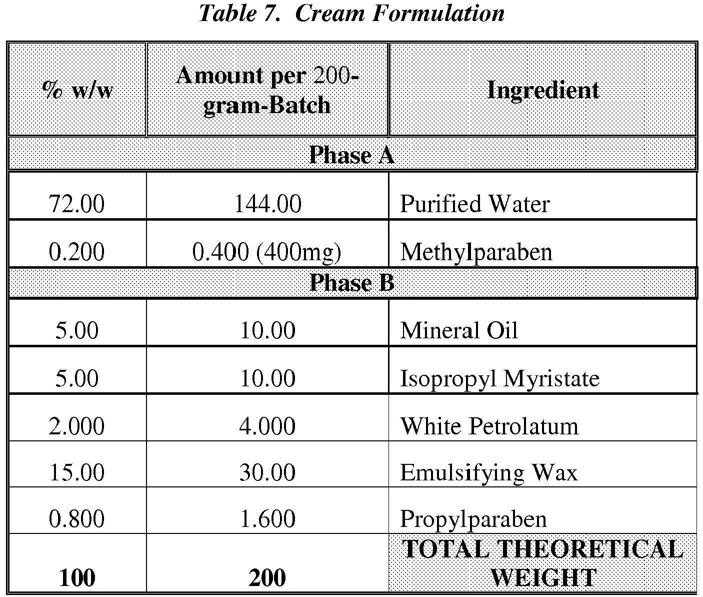

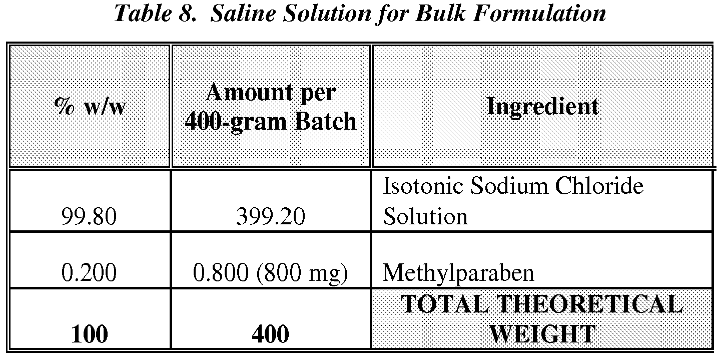

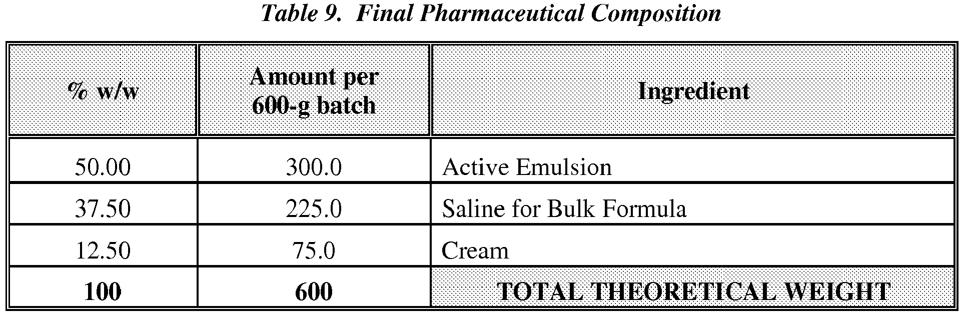

### Example 2: Exemplary Empty Nanoparticle Composition

This example presents an exemplary botulinum toxin nanoparticle composition for use in accordance with the present invention.

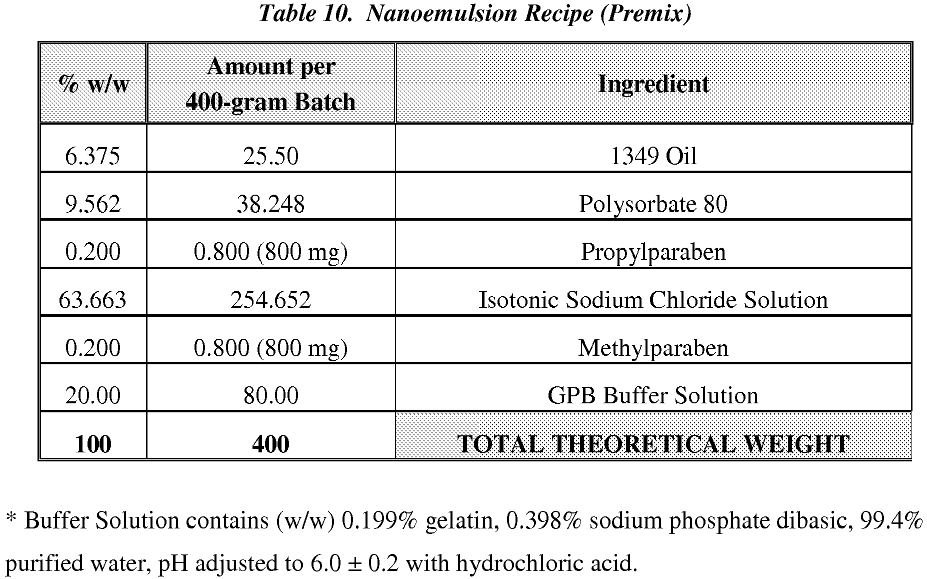

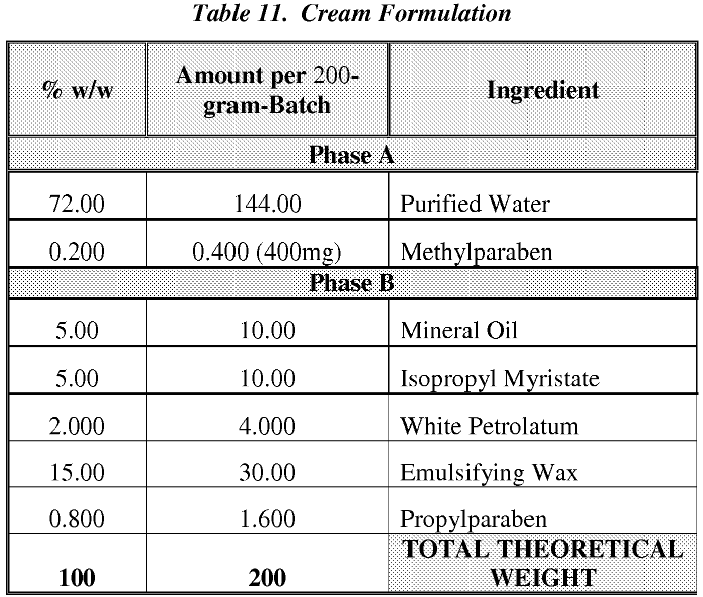

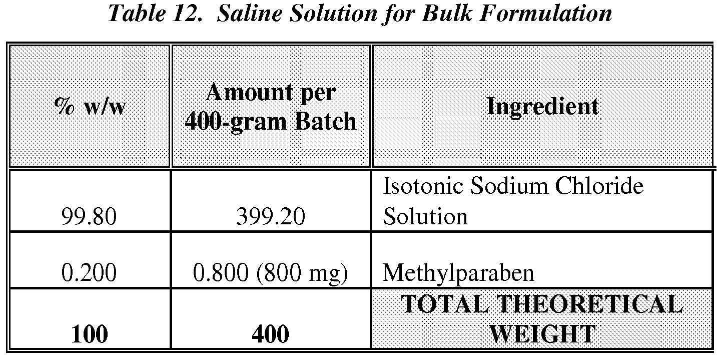

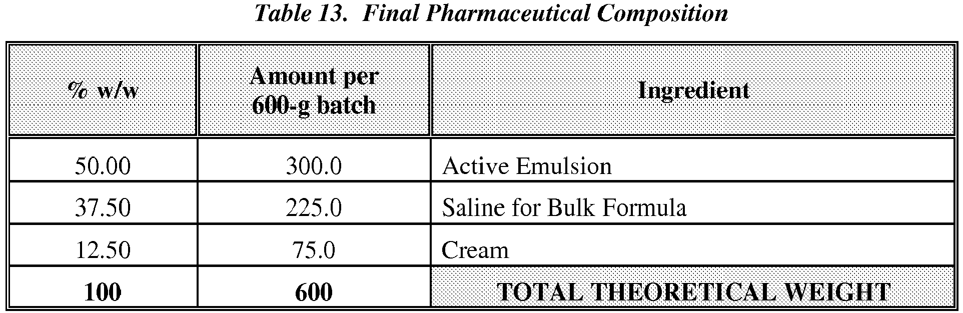

### Example 3: Exemplary Botulinum Nanoparticle Composition

This example presents an exemplary botulinum toxin nanoparticle composition for use in accordance with the present invention.

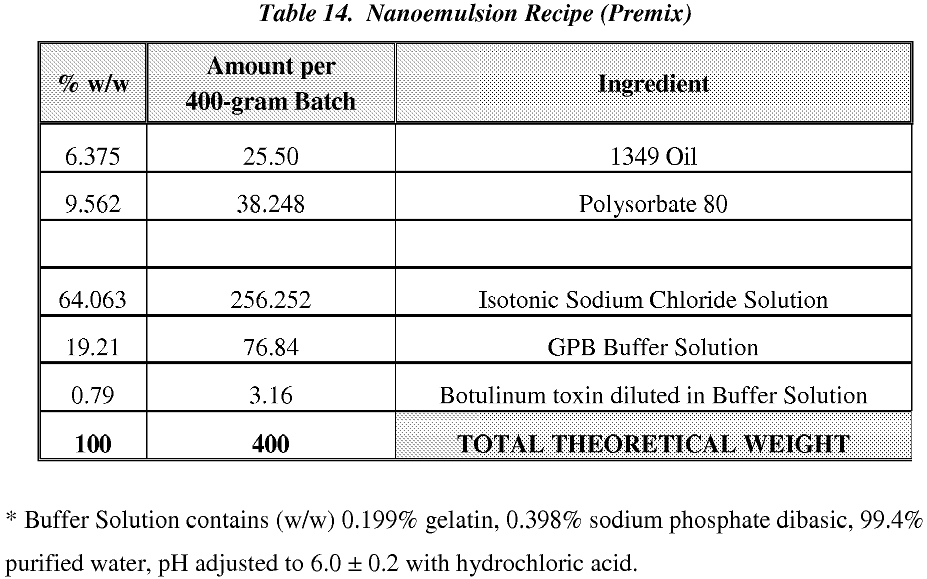

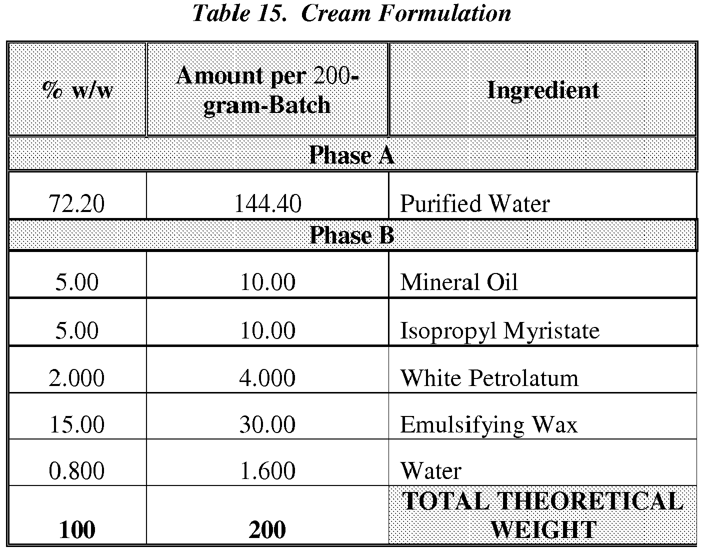

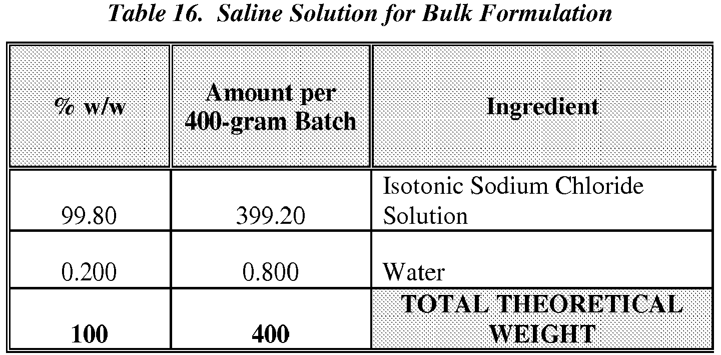

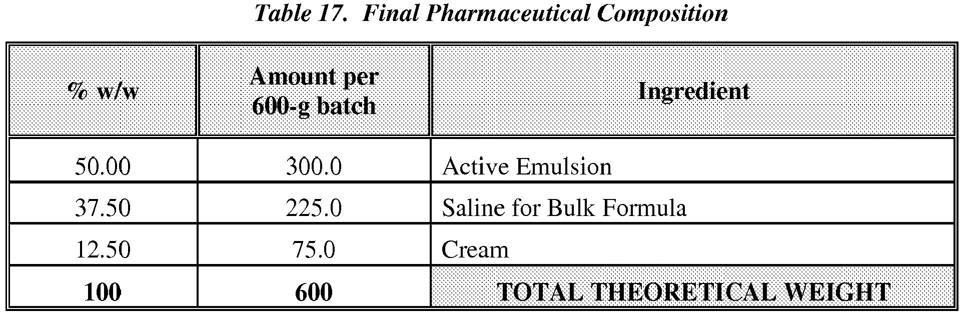

### Example 4: Exemplary Empty Nanoparticle Composition

This example presents an exemplary empty nanoparticle composition for use in accordance with the present invention.

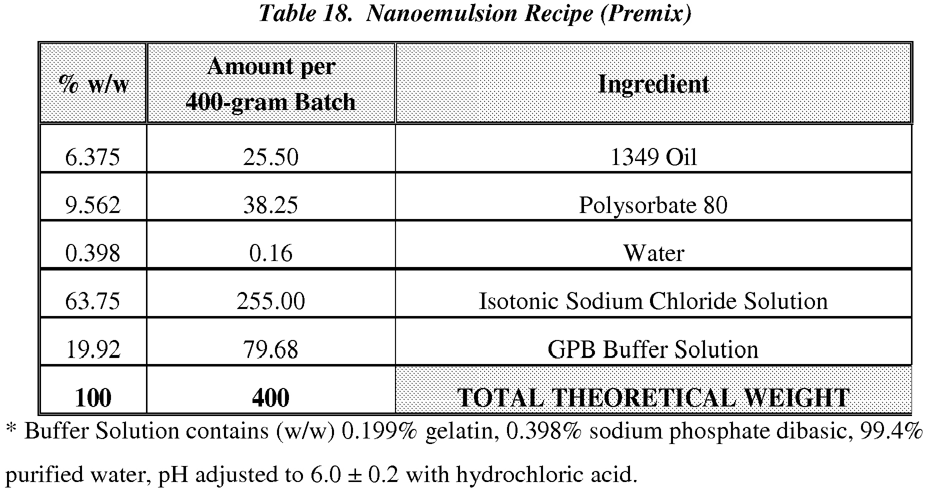

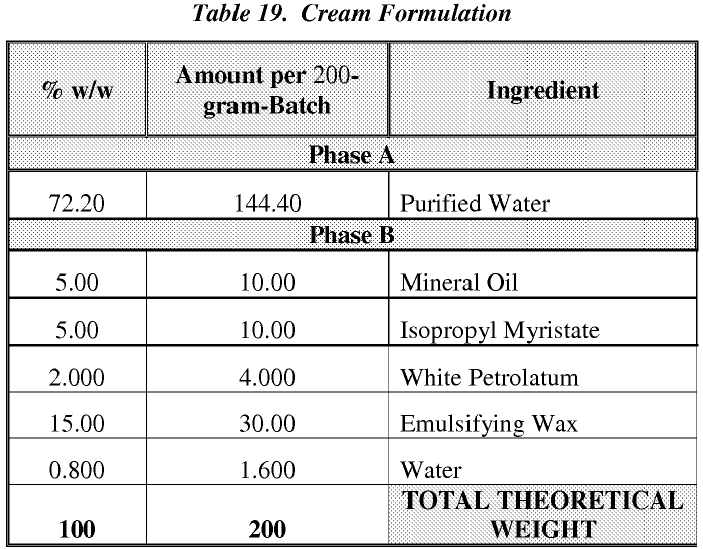

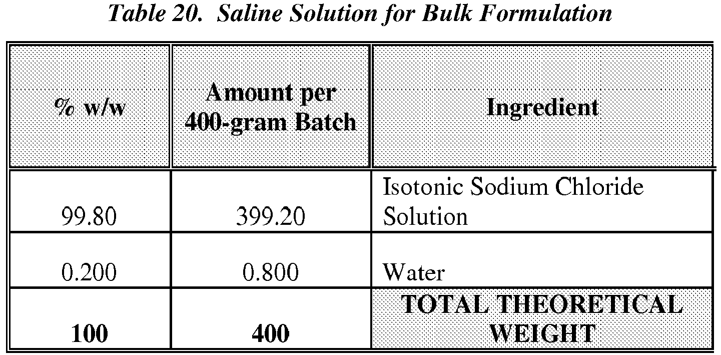

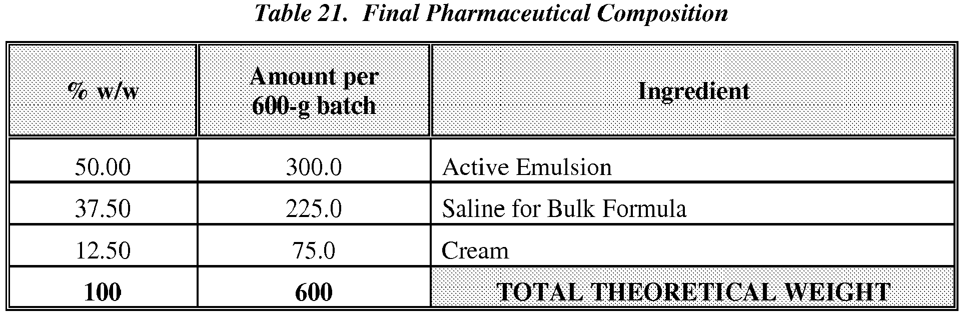

### Example 5: Surfactant to Oil to Ratio in Nanoparticle Compositions

This example demonstrates the discovery that the degree of penetration of a provided composition and/or individual component thereof across skin may be controlled by varying the ratio of surfactant to oil used for nanoparticle composition formation. Exemplary results of topical application of botulinum toxin type A in a nanoparticle composition in mouse models are presented below.

Treatments included:
(A) treatment wherein the surfactant:oil ratio of 1.0: nanoparticle composition comprising 3.2 g 1349 oil, 3.2 g Tween-80, 25.8 g 0.9% Saline, 6.0 g Gelatin Phosphate Buffer, and 1.8 g botulinum toxin solution
(B) treatment wherein the surfactant:oil ratio of 1.5: nanoparticle composition comprising 2.56 g 1349 oil, 3.84 g Tween-80, 25.8 g 0.9% Saline, 6.0 g Gelatin Phosphate Buffer, and 1.8 g botulinum toxin solution and
(C) treatment wherein the surfactant:oil ratio of 1.75: nanoparticle composition comprising 2.32 g 1349 oil, 4.08 g Tween-80, 25.8 g 0.9% Saline, 6.0 g Gelatin Phosphate Buffer, and 1.8 g botulinum toxin solution.
Each treatment was microfluidized at 22,000 PSI to create nanoparticles and then combined with an equal volume of pemulen prior to application to mice.

Mice (18 - 24 grams) were employed for each treatment group (n=9). Mice were randomly assigned to each treatment group. Mice were anesthetized using ketamine and xylazine. The right calf muscle was shaved. A total treatment volume of 50 µL was applied to the skin of the shaved calf and rubbed into the skin until no longer visible.

11 days following treatment mice were sacrificed and the gastrocnemius muscle of the treated leg was dissected out and preserved in chilled isopentane. Tissue was stored at -80 degrees centigrade until stained for the presence of acetylcholinesterase. Nerve terminals that stained positively for acetylcholinesterase were counted for each tissue sample. Positive staining indicated that the botulinum had a pharmacologic effect on the nerve terminals.

The results demonstrated that the number of counted nerve terminals for Treatment B (67) was 91% greater than Treatment A (35) and 34% greater than Treatment C (50). Thus, in some embodiments, a surfactant to oil ratio of 1.5 is desirable for transdermal penetration of a provided composition and/or individual component thereof as compared with a lower or higher ratio.

### Example 6: Identity of Oil in Nanoparticle Compositions

This example demonstrates the discovery that the degree of penetration of a provided composition and/or individual component thereof across skin may be controlled by varying the type of oil used for nanoparticle composition formation. It was found that certain oils used in a nanoemulsion composition result in a higher degree of penetration than other oils, as evidenced by significant increase of stained acetylcholine esterase (ACE) from topical application of botulinum toxin type A in mouse models. Exemplary results of topical application of botulinum toxin type A in mouse models are presented below.

Treatments included:
(A) treatment wherein the oil comprises soybean oil: nanoparticle composition comprising 1.6 g soybean oil, 1.6 g Tween-80, 13.8 g 0.9% Saline with 0.05% Albumin, 2.125 g Gelatin Phosphate Buffer, and 0.875 g botulinum toxin solution
(B) treatment wherein the oil comprises 1349 oil: nanoparticle composition comprising 1.6 g 1349 oil, 1.6 g Tween-80, 13.8 g 0.9% Saline with 0.05% Albumin, 2.125 g Gelatin Phosphate Buffer, and 0.875 g botulinum toxin.
Each treatment was microfluidized at 22,000 PSI to create nanoparticles and then combined with an equal volume of pemulen prior to application on mice.

Mice (18 - 24 grams) were employed for each treatment group (n=8). Mice were randomly assigned to each treatment group. Mice were anesthetized using ketamine and xylazine. The right calf muscle was shaved. A total treatment volume of 50 µL was applied to the skin of the shaved calf and rubbed into the skin until no longer visible.

11 days following treatment mice were sacrificed and the gastrocnemius muscle of the treated leg was dissected out and preserved in chilled isopentane. Tissue was stored at -80 degrees centigrade until stained for the presence of acetylcholinesterase. Nerve terminals that stained positively for acetylcholinesterase were counted for each tissue sample. Positive staining indicated that the botulinum had a pharmacologic effect on the nerve terminals.

The results demonstrated that the number of counted nerve terminals for Treatment B (36) was 29% greater than Treatment A (28). Thus, in some embodiments, 1349 oil is desirable for transdermal penetration of a provided composition and/or individual component thereof as compared with soybean oil.

### Example 7: Identity of Surfactant in Nanoparticle Compositions

This example demonstrates the discovery that the degree of penetration of a provided composition and/or individual component thereof across skin may be controlled by varying the type of surfactant used for nanoparticle composition formation. It was found that certain surfactants used in a nanoemulsion result in a higher degree of penetration than other surfactants, as evidenced by significant increase of stained acetylcholine esterase (ACE) from topical application of botulinum toxin type A in mouse models. Exemplary results of topical application of botulinum toxin type A in mouse models are presented below.

Treatments included:
(A) treatment wherein the surfactant comprises Tween 80: nanoparticle composition comprising 2.4 g 1349 oil, 2.4 g Tween-80, 22.68 g 0.9% Saline with 0.05% Albumin and 0.675 g botulinum toxin solution
(B) treatment wherein the surfactant comprises Super Refined Tween-80: nanoparticle composition comprising 2.4 g 1349 oil, 2.4 g Super Refined Tween-80, 22.68 g 0.9% Saline with 0.05% Albumin and 0.675 g botulinum toxin solution.
Each treatment was microfluidized at 22,000 PSI to create nanoparticles and then combined with an equal volume of pemulen prior to application on mice.

Mice (18 - 24 grams) were employed for each treatment group (n=10). Mice were randomly assigned to each treatment group. Mice were anesthetized using ketamine and xylazine. The right calf muscle was shaved. A total treatment volume of 50 µL was applied to the skin of the shaved calf and rubbed into the skin until no longer visible.

11 days following treatment mice were sacrificed and the gastrocnemius muscle of the treated leg was dissected out and preserved in chilled isopentane. Tissue was stored at -80 degrees centigrade until stained for the presence of acetylcholinesterase. Nerve terminals that stained positively for acetylcholinesterase were counted for each tissue sample. Positive staining indicated that the botulinum had a pharmacologic effect on the nerve terminals.

The results demonstrated that the number of counted nerve terminals for Treatment B (22) was 29% greater than Treatment A (17). Thus, in some embodiments, Super Refined Tween-80 is a desirable surfactant for transdermal penetration of a nanoparticle composition and/or individual component thereof as compared with Tween-80.

### Example 8: Identity of Excipient in Provided Compositions

This example demonstrates the discovery that the degree of penetration of a provided composition and/or individual component thereof across skin may be controlled by varying the type of excipient that is mixed with a nanoparticle composition (in other words, an excipient that is mixed with a nanoparticle composition that was formed prior to mixture with the excipient). It was found that certain excipients used in combination with a nanoemulsion result in a higher degree of penetration than other excipients, as evidenced by significant increase of stained acetylcholine esterase (ACE) from topical application of botulinum toxin type A in mouse models. Exemplary results of topical application of botulinum toxin type A in mouse models are presented below.

Treatments included:
(A) treatment comprising (i) an excipient comprising Pemulen, and (ii) a nanoparticle composition comprising 3.2 g 1349 oil, 3.2 g Tween-80, 25.8 g 0.9% Saline, 6.0 g Gelatin Phosphate Buffer and 1.8 g botulinum toxin solution
(B) treatment comprising (i) an excipient comprising deionized water, mineral oil, isopropyl myristate, white petrolatum, emulsifying wax, EDTA, and cetostearyl alcohol), and (ii) a nanoparticle composition comprising 3.2 g 1349 oil, 3.2 g Tween-80, 25.8 g 0.9% Saline, 6.0 g Gelatin Phosphate Buffer and 1.8 g botulinum toxin solution.
Each treatment was microfluidized at 22,000 PSI to create nanoparticles and then combined with an equal volume of excipient prior to application on the mice.

Mice (18 - 24 grams) were employed for each treatment group (n=9). Mice were randomly assigned to each treatment group. Mice were anesthetized using ketamine and xylazine. The right calf muscle was shaved. A total treatment volume of 50 µL was applied to the skin of the shaved calf and rubbed into the skin until no longer visible.

11 days following treatment mice were sacrificed and the gastrocnemius muscle of the treated leg was dissected out and preserved in chilled isopentane. Tissue was stored at -80 degrees centigrade until stained for the presence of acetylcholinesterase. Nerve terminals that stained positively for acetylcholinesterase were counted for each tissue sample. Positive staining indicated that the botulinum had a pharmacologic effect on the nerve terminals.

The results demonstrated that the number of counted nerve terminals for Treatment B (72) was 33% greater than Treatment A (54). Thus, in some embodiments, a Cream-based excipient (*e.g*., with EDTA & Cetostearyl Alcohol) is desirable for transdermal penetration of a provided composition and/or individual component thereof as compared with Pemulen.

### Example 9: Concentration of Excipient in Provided Compositions

This example demonstrates the discovery that the degree of penetration of a provided composition and/or individual component thereof across skin may be controlled by varying the concentration of excipient that is mixed with a nanoparticle composition (in other words, an excipient that is mixed with a nanoparticle composition that was formed prior to mixture with the excipient). It was found that certain concentrations of excipients used in combination with a nanoemulsion result in a higher degree of penetration than other concentrations, as evidenced by significant increase of stained acetylcholine esterase (ACE) from topical application of botulinum toxin type A in mouse models. Exemplary results of topical application of botulinum toxin type A in mouse models are presented below.

Treatments included:
(A) treatment comprising (i) an excipient comprising a cream:water ratio of 3:1, and (ii) a nanoparticle composition comprising 3.2 g 1349 oil, 3.2 g Tween-80, 25.8 g 0.9% Saline, 6.0 g Gelatin Phosphate Buffer, and 1.8 g botulinum toxin solution
(B) treatment comprising (i) an excipient comprising a cream:water ratio of 1:1, and (ii) a nanoparticle composition comprising 3.2 g 1349 oil, 3.2 g Tween-80, 25.8 g 0.9% Saline, 6.0 g Gelatin Phosphate Buffer, and 1.8 g botulinum toxin solution
(C) treatment comprising (i) an excipient comprising a cream:deionized water ratio of 1:3, and (ii) a nanoparticle composition comprising 3.2 g 1349 oil, 3.2 g Tween-80, 25.8 g 0.9% Saline, 6.0 g Gelatin Phosphate Buffer, and 1.8 g botulinum toxin solution.
Each treatment was microfluidized at 22,000 PSI to create nanoparticles and then combined with an equal volume of cream/water mixture prior to application on the mice.

Mice (18 - 24 grams) were employed for each treatment group (n=9). Mice were randomly assigned to each treatment group. Mice were anesthetized using ketamine and xylazine. The right calf muscle was shaved. A total treatment volume of 50 µL was applied to the skin of the shaved calf and rubbed into the skin until no longer visible.

11 days following treatment mice were sacrificed and the gastrocnemius muscle of the treated leg was dissected out and preserved in chilled isopentane. Tissue was stored at -80 degrees centigrade until stained for the presence of acetylcholinesterase. Nerve terminals that stained positively for acetylcholinesterase were counted for each tissue sample. Positive staining indicated that the botulinum had a pharmacologic effect on the nerve terminals.

The results demonstrated that the number of counted nerve terminals for Treatment C (91) was 75% greater than Treatment B (52) and 66% greater than Treatment A (55). Thus, in some embodiments, lower concentrations of excipients result in a higher degree of transdermal penetration of a provided composition and/or individual component thereof as compared with higher concentrations of excipients.

### Example 10: Identity of Preservative in Excipients in Provided Compositions

This example demonstrates the discovery that the degree of penetration of a provided composition and/or individual component thereof across skin may be controlled by varying the type of preservative that is mixed with a nanoparticle composition (in other words, a preservative that is mixed with a nanoparticle composition that was formed prior to mixture with the preservative). It was found that certain preservatives used in combination with a nanoemulsion result in a higher degree of penetration than other preservatives, as evidenced by significant increase of stained acetylcholine esterase (ACE) from topical application of botulinum toxin type A in mouse models. Exemplary results of topical application of botulinum toxin type A in mouse models are presented below.

Treatments included:
(A) treatment comprising a mixture of (i) an excipient comprising Pemulen and no added preservatives, and (ii) a nanoparticle composition comprising 3.2 g 1349 oil, 3.2 g Tween-80, 23.35 g 0.9% Saline, 6.0 g Gelatin Phosphate Buffer, and 1.8 g botulinum toxin solution
(B) treatment comprising a mixture of (i) an excipient comprising Pemulen with added preservatives (*e.g.*, benzyl alcohol), and (ii) nanoparticle composition comprising 3.2 g 1349 oil, 3.2 g Tween-80, 23.35 g 0.9% Saline, 6.0 g Gelatin Phosphate Buffer, and 1.8 g botulinum toxin solution
(C) treatment comprising a mixture of (i) an excipient comprising Pemulen, with added preservatives (*e.g.*, parabens), and (ii) a nanoparticle composition comprising 3.2 g 1349 oil, 3.2 g Tween-80, 23.35 g 0.9% Saline, 6.0 g Gelatin Phosphate Buffer, and 1.8 g botulinum toxin solution.
Each treatment was microfluidized at 22,000 PSI to create nanoparticles and then combined with an equal volume of Pemulen excipient (as described in (i) above) prior to application on mice.

Mice (18 - 24 grams) were employed for each treatment group (n=9). Mice were randomly assigned to each treatment group. Mice were anesthetized using ketamine and xylazine. The right calf muscle was shaved. A total treatment volume of 50 µL was applied to the skin of the shaved calf and rubbed into the skin until no longer visible.

11 days following treatment mice were sacrificed and the gastrocnemius muscle of the treated leg was dissected out and preserved in chilled isopentane. Tissue was stored at -80 degrees centigrade until stained for the presence of acetylcholinesterase. Nerve terminals that stained positively for acetylcholinesterase were counted for each tissue sample. Positive staining indicated that the botulinum had a pharmacologic effect on the nerve terminals.

The results demonstrated that the number of counted nerve terminals for Treatment C (72) was 20% greater than Treatment B (60) and 4% greater than Treatment A (69). Thus, in some embodiments, excipients with a paraben as a preservative result in a higher degree of transdermal penetration of a provided composition and/or individual component thereof as compared with excipients without a paraben as a preservative. In some embodiments, excipients with a paraben as a preservative result in a higher degree of transdermal penetration of a provided composition and/or individual component thereof as compared with benzyl alcohol as a preservative.

### Example 11: Parabens in Nanoparticle Compositions

This example demonstrates the discovery that the degree of penetration of a provided composition and/or individual component thereof across skin may be enhanced by including a paraben in the nanoparticle composition. It was found that parabens used in a nanoemulsion result in a higher degree of penetration than when parabens are not included, as evidenced by significant increase of stained acetylcholine esterase (ACE) from topical application of botulinum toxin type A in mouse models. Exemplary results of topical application of botulinum toxin type A in mouse models are presented below.

Treatments included:
(A) treatment comprising a nanoparticle composition comprising 3.2 g 1349 oil, 3.2 g Tween-80, 25.8 g 0.9% Saline, 6.0 g Gelatin Phosphate Buffer and 1.8 g botulinum toxin solution
(B) treatment comprising a nanoparticle composition comprising 3.2 g 1349 oil, 3.2 g Tween-80 with 0.2% parabens, 25.8 g 0.9% Saline with 0.2% parabens, 6.0 g Gelatin Phosphate Buffer and 1.8 g botulinum toxin solution.
Each treatment was microfluidized at 22,000 PSI to create nanoparticles and then mixed with an equal volume of pemulen as an excipient prior to application on the mice.

Mice (18 - 24 grams) were employed for each treatment group (n=9). Mice were randomly assigned to each treatment group. Mice were anesthetized using ketamine and xylazine. The right calf muscle was shaved. A total treatment volume of 50 µL was applied to the skin of the shaved calf and rubbed into the skin until no longer visible.

11 days following treatment mice were sacrificed and the gastrocnemius muscle of the treated leg was dissected out and preserved in chilled isopentane. Tissue was stored at -80 degrees centigrade until stained for the presence of acetylcholinesterase. Nerve terminals that stained positively for acetylcholinesterase were counted for each tissue sample. Positive staining indicated that the botulinum had a pharmacologic effect on the nerve terminals.

The results demonstrated that the number of counted nerve terminals for Treatment B (93) was 72% greater than Treatment A (54). Thus, in some embodiments, nanoparticle compositions comprising parabens result in a higher degree of transdermal penetration of a provided composition and/or individual component thereof as compared with nanoparticle compositions without parabens.

### Example 12: Botulinum Holotoxin in Nanoparticle Compositions

This example demonstrates the discovery that the degree of penetration of botulinum toxin across skin may be enhanced by using the holotoxin form of botulinum toxin (as opposed to the complexed form of botulinum toxin) in a nanoparticle composition. It was found that botulinum holotoxin used in a nanoemulsion result in a higher degree of penetration of the toxin, as evidenced by better and quicker penetration response of measured Digit Abduction Scores (DAS) in topical skin application of the toxin formulations in mouse models. Exemplary results of topical application of botulinum toxin in mouse models are presented below.

Treatments included:
(A) treatment comprising nanoparticle compositions comprising comprised of 1.92 g 1349 oil, 2.88 g Tween-80, 19.2 g 0.9% Saline with 0.2% parabens, 0.81 g Gelatin Phosphate Buffer, and 5.19 g botulinum toxin type A complex solution (2.13 x 10⁻⁴ nM)
(B) treatment comprising nanoparticle compositions comprising 1.92 g 1349 oil, 2.88 g Tween-80, 19.2 g 0.9% Saline with 0.2% parabens, 0.81 g Gelatin Phosphate Buffer, and 5.19 g botulinum toxin type A holotoxin solution (2.13 x 10⁻⁴ nM).
Each treatment was microfluidized at 22,000 PSI to create nanoparticles and then combined with an equal volume of cream prior to application on the mice.

Mice (18 - 24 grams) were employed for each treatment group (n=8). Mice were randomly assigned to each treatment group. Mice were anesthetized using ketamine and xylazine. The right calf muscle was shaved. A total treatment volume of 50 µL was applied to the skin of the shaved calf and rubbed into the skin until no longer visible. Each day after treatment for a period of eleven days, DAS was observed and recorded for the treated leg.

The results demonstrated that peak DAS for the mice treated with holotoxin-containing Treatment B (DAS = 4) was over three times greater than the toxin-complex-containing Treatment A (DAS = 1.5). Thus, in some embodiments, treatment preparations containing botulinum type A holotoxin are superior in achieving transdermal penetration as compared with preparations containing botulinum type A complex.

### Example 13: Gelatin in Nanoparticle Compositions

This example demonstrates the discovery that the degree of penetration of a provided composition and/or individual component thereof across skin may be enhanced by using nanoparticle compositions made with gelatin at certain concentrations rather than nanoparticle compositions made with bovine serum albumin (BSA) and/or nanoparticle compositions made with no additional protein in the nanoparticle composition. When botulinum toxin was formulated in the nanoparticle compositions, it was found that use of gelatin resulted in a higher degree of penetration of the toxin, as evidenced by an increase of measured Digit Abduction Scores (DAS) in topical skin application of the toxin-containing nanoparticle compositions in mouse models. Exemplary results of topical application of botulinum toxin in mouse models are presented below.

Treatments included:
(A) treatment comprising gelatin at 0.026%: nanoparticle compositions comprising 2.56 g 1349 oil, 3.84 g Tween-80, 25.6 g 0.9% Saline with 0.2% parabens, 6.4 g Gelatin Phosphate Buffer, and 1.6 g botulinum type A holotoxin solution
(B) treatment comprising gelatin at 0.036%: nanoparticle compositions comprising 2.56 g 1349 oil, 3.84 g Tween-80, 25.6 g 0.9% Saline with 0.2% parabens, 6.4 g Gelatin Phosphate Buffer (with 1.38 times the gelatin concentration in the Buffer in Treatment A), and 1.6 g botulinum type A holotoxin solution
(C) treatment comprising gelatin at 0.056%: nanoparticle compositions comprising 2.56 g 1349 oil, 3.84 g Tween-80, 25.6 g 0.9% Saline with 0.2% parabens, 6.4 g Gelatin Phosphate Buffer (with 2.15 times the gelatin concentration in the Buffer in Treatment A), and 1.6 g botulinum type A holotoxin solution
(D) treatment comprising gelatin at 0.00%: nanoparticle compositions comprising 2.56 g 1349 oil, 3.84 g Tween-80, 25.6 g 0.9% Saline with 0.2% parabens, 6.4 g Phosphate Buffer, and 1.6 g botulinum type A holotoxin solution
(E) treatment comprising gelatin at 0.00%: nanoparticle compositions comprising 2.56 g 1349 oil, 3.84 g Tween-80, 25.6 g 0.9% Saline with 0.2% parabens and 0.02% BSA, 6.4 g Phosphate Buffer, and 1.6 g botulinum type A holotoxin solution.
Each treatment was microfluidized at 22,000 PSI to create nanoparticles and then combined with an equal volume of cream prior to application on the mice.

Mice (18 - 24 grams) were employed for each treatment group (n=15). Mice were randomly assigned to each treatment group. Mice were anesthetized using ketamine and xylazine. The right calf muscle was shaved. A total treatment volume of 50 µL was applied to the skin of the shaved calf and rubbed into the skin until no longer visible. Each day after treatment for a period of eleven days, DAS was observed and recorded for the treated leg.

The results demonstrated that peak DAS for the mice treated with holotoxin-containing Treatment A (DAS = 3.5) was 52%, 59% and 35% greater than Treatment B, C or D (DAS = 2.3, 2.2 and 2.6), respectively. Treatment A was also 4 times greater than Treatment E (DAS = 3.5 vs. 0.8). Thus, in some embodiments, treatment preparations containing gelatin at 0.026% are desirable to achieve transdermal penetration as compared with preparations containing higher amounts of gelatin and/or no gelatin. In some embodiments, treatment preparations containing gelatin are desirable to achieve transdermal penetration as compared with preparations containing BSA.

### Example 14: Nanoparticle Compositions for Storage of Botulinum Toxin

This example demonstrates the discovery that bioactivity of botulinum toxin can be preserved by storing it as part of a nanoparticle composition (*e.g.*, nanoemulsion). It was found that nanoemulsions resulted in stability as evidenced by preserving biological activity of toxin at refrigerated temperature (2 °C to 8 °C) over a long period of time as measured by mouse i.v. (intravenous injection) potency method. Exemplary results are presented below.

Treatments included:
(A) nanoparticle compositions comprising 3.2 g 1349 oil, 3.2 g Tween-80, 23.35 g 0.9% Saline, 6.0 g Gelatin Phosphate Buffer, and 1.8 g botulinum toxin solution.
Each treatment was microfluidized at 22,000 PSI to create nanoparticles and then stored at 2 °C to 8 °C for a number of months.

Mice (18 - 24 grams) were employed for each treatment group (n=9). Mice were randomly assigned to a treatment group. A total treatment volume of 200 µL was injected to the tail vein of the mice. Mice were observed over the next 8 hours and time of death was recorded.

The results demonstrated that over a 12 month period the nanoemulsion is active and has not lost any potency. Thus, in some embodiments, storing a nanoemulsion for 12 months at 2 °C to 8 °C keeps botulinum toxin stable.

### Example 15: Nanoparticle Compositions for Frozen Storage of Botulinum Toxin

This example demonstrates the discovery that bioactivity of botulinum toxin can be preserved by storing it as part of a nanoparticle composition (*e.g.*, nanoemulsion) that can be stored in a frozen state. It was found that nanoemulsions were able to be frozen and still retain bioactivity and transdermal penetration properties of unfrozen nanoemulsions, as evidenced by maintaining unchanged DAS scores from topical skin applications after 1, 2 and 3 cycles of freeze/thaw process. Exemplary results are presented below.

Treatments included:
(A - D) nanoparticle compositions comprising 5.76 g 1349 oil, 8.64 g Tween-80 with 0.2% parabens, 57.6 g 0.9% Saline with 0.2% parabens, 13.5 g Gelatin Phosphate Buffer, and 4.5 g botulinum type A holotoxin solution. Each treatment was microfluidized at 22,000 PSI to create nanoparticles and then combined with an equal volume of cream prior to application on the mice.
Treatment A had no cycle of freeze/thaw cycles
Treatment B had 1 cycle of freeze/thaw cycle
Treatment C had 2 cycles of freeze/thaw cycles
Treatment D had 3 cycles of freeze/thaw cycles

Mice (18 - 24 grams) were employed for each treatment group (n=10). Mice were randomly assigned to each treatment group. Mice were anesthetized using ketamine and xylazine. The right calf muscle was shaved. A total treatment volume of 50 µL was applied to the skin of the shaved calf and rubbed into the skin until no longer visible. Each day after treatment for a period of eleven days, DAS was observed and recorded for the treated leg.

The results demonstrated peak DAS for the mice treated with holotoxin-containing Treatment A, no cycle of freeze/thaw (DAS = 4.0), Treatment B, 1 cycle of freeze/thaw (DAS = 4.0), Treatment C, 2 cycles of freeze/thaw (DAS = 3.9) and Treatment D, 3 cycles of freeze/thaw (DAS = 4.0). Thus, in some embodiments, treatment preparations after 0, 1, 2 and 3 cycles of the freeze/thaw process does not affect DAS scores and/or retain comparable bioactivity and transdermal penetration.

Moreover, it was found that certain freezing techniques for nanoparticle compositions were more effective than others in preserving the bioactivity of the nanoemulsion, as evidenced by measured DAS scores from topical skin applications in mouse models between treatments that were frozen gradually versus those flash frozen. Exemplary results are presented below.

All treatments comprised nanoparticle compositions comprising 2.56 g 1349 oil, 3.84 g Tween-80 with 0.2% parabens, 25.6 g 0.9% Saline with 0.2% parabens, 6.4 g Gelatin Phosphate Buffer, and 1.6 g botulinum type A holotoxin solution. Each treatment was microfluidized at 22,000 PSI to create nanoparticles and then combined with an equal volume of cream prior to application on the mice. Treatment A was gradually frozen at -70 °C; Treatment B was flash frozen to -196 °C and then stored at -70 °C.

Mice (18 - 24 grams) were employed for each treatment group (n=20). Mice were randomly assigned to each treatment group. Mice were anesthetized using ketamine and xylazine. The right calf muscle was shaved. A total treatment volume of 50 µL was applied to the skin of the shaved calf and rubbed into the skin until no longer visible. Each day after treatment for a period of eleven days, DAS was observed and recorded for the treated leg.

The results demonstrated that peak DAS for the mice treated with Treatment A (DAS = 1.6) was 78% greater than mice treated with Treatment B (DAS = 0.9). Thus, in some embodiments, gradually freezing a nanoemulsion preparation is desirable in preserving bioactivity of the nanoemulsion as compared with flash freezing the treatment.

### Example 16: Nanoemulsion Delivery System

This example demonstrates the discovery that certain provided compositions had better tissue penetrating capabilities than others. Table 22 presents an exemplary nanoemulsion formulation having excellent tissue penetrating capabilities. The components in the nanoemulsion formulation presented in Table 22 have been normalized by weight (g) and percentage (%).

**Table 22: Nanoemulsion Formulation**

| **Component** | **Weight (g)** | **Percent (by weight)** |
|---|---|---|
| 1349 oil | 3.2 | 3.19 |
| Tween-80 | 4.8 | 4.79 |
| Methylparaben | 0.2 | 0.2 |
| Propylparaben | 0.2 | 0.2 |
| Sodium chloride (a) | 0.63 | 0.63 |
| Sodium phosphate dibasic | 0.04 | 0.04 |
| Gelatin | 0.02 | 0.02 |
| Toxin (b) | 0.0 | 0.0 |
| Mineral oil | 0.63 | 0.63 |
| Isopropyl myristate | 0.62 | 0.62 |
| White petrolatum | 0.25 | 0.25 |
| Emulsifying wax | 1.87 | 1.87 |
| Purified water (c) | 87.76 | 87.57 |
| Total | 100.22 | 100.00 |

### Example 17: Subcutaneous DAS Method for Assessing Potency of a Topical Chemodenervating Agent

The present example presents discovery of a method for testing potency of a provided composition (*e.g*., topical botulinum formulation) by injecting the composition into subcutaneous (s.c.) tissue of a rodent limb and measuring the degree of limb paralysis using an observed digital abduction score (DAS).

Evaluating biologic potency of a topical chemodenervating agent presents unique challenges. The present invention encompasses methods in which very small doses (*e.g.*, less than 1 LD50 unit) of botulinum toxin sample can be measured by mouse s.c. method with a standard curve. To the best of the inventors' knowledge, this method is the most sensitive method available to date to measure the toxin potency *in vivo* and in non-solution matrix samples (*e.g.*, toxin in nanoemulsion/lotion formulations). This method is useful for determining toxin potency, optimizing formulations of provided compositions, and assessing stability of provided compositions in various contexts, including, but not limited to, solution, emulsion and creams.

All treatments comprised botulinum type A holotoxin diluted with 0.9% Saline containing 20% Gelatin Phosphate Buffer. Treatment A = 0.100 U/dose, Treatment B = 0.170 U/dose, Treatment C = 0.290 U/dose, Treatment D = 0.500 U/dose and Treatment E = 0.850 U/dose.

Mice (18 - 24 grams) were employed for each treatment group (n=8). Mice were randomly assigned to each treatment group. Mice were anesthetized using ketamine and xylazine. A total treatment volume of 50 µL was injected into the s.c. tissue of the mouse hind limb to generate a DAS response. Each day after treatment for a period of four days, DAS was observed and recorded for the treated leg.

The results demonstrated that peak DAS for the mice treated with holotoxin-containing treatments. A dose/response curve was generated from the injections. Treatments A (DAS = 0.63), Treatment B (DAS = 0.63), Treatment C (DAS = 2.13), Treatment D (DAS = 3.75) and Treatment E (DAS = 4.0). Thus, in some embodiments, s.c. injections can be useful determining toxin potency in various formulations, such as solution, emulsion and creams.

### Example 18: Topical DAS Method for Assessing Potency of a Topical Chemodenervating Agent

The present example presents discovery of a method for testing potency of a topical botulinum preparation by applying the preparation onto the skin of a mouse limb and measuring the degree of limb paralysis using an observed digital abduction score (DAS).

Evaluating biologic potency of a topical chemodenervating agent presents unique challenges. The present invention provides methods for measuring penetration of botulinum toxin molecules across skin. A measured amount of nanoemulsion delivery system sample was applied to the mouse skin (*e.g*., skin overlying the mouse's calf muscle), and DAS values were measured over a period of up to 2 weeks. DAS values of mice limbs reflect effect of toxin molecules penetrated across the skin, thus demonstrating the effectiveness of topical delivery system. This method is useful for determining toxin potency, optimizing formulations of provided compositions, and assessing stability of provided compositions in various contexts, including, but not limited to, solution, emulsion and creams.

All treatments comprised 6.4 g 1349 oil, 9.6 g Tween-80 with 0.2% parabens, 64.0 g 0.9% Saline with 0.2% parabens, 17.24 g Gelatin Phosphate Buffer, and 2.76 g botulinum type A holotoxin. Each treatment was microfluidized at 22,000 PSI to create nanoparticles and then combined with an equal volume of cream prior to application on the mice. Treatment A was stored at +5°C; Treatment B was stored at -20°C; Treatment C was stored at -80°C; and Treatment D was stored at -80°C with 1 cycle of freeze/thaw.

Mice (18 - 24 grams) were employed for each treatment group (n=10). Mice were randomly assigned to each treatment group. Mice were anesthetized using ketamine and xylazine. The right calf muscle was shaved. A total treatment volume of 50 µL was applied to the skin of the shaved calf and rubbed into the skin until no longer visible. Each day after treatment for a period of eleven days, DAS was observed and recorded for the treated leg.

The results demonstrated peak DAS for the mice treated with holotoxin-containing after being stored for 5 months as follows: Treatment A, stored at +5°C (DAS = 3.9), Treatment B, stored at -20°C (DAS = 3.8), Treatment C, stored at -80°C (DAS = 4.0) and Treatment D, stored at -80°C with 1 cycles of freeze/thaw (DAS = 4.0). Thus, in some embodiments, after being stored for 5 months at either +5°C, -20°C, -80°C and -80°C with 1 cycle of freeze/thaw, there was no change in DAS effect from day 0 (in other words, all treatment preparations retained bioactivity and transdermal penetration capabilities).

### Example 19: Intravenous Time-to-Death Method for Assessing Potency of a Topical Chemodenervating Agent

The present example demonstrates the discovery that potency of a provided composition (e.g., topical botulinum preparation) can be tested by injecting the composition into a vein of a rodent and measuring the average and/or median time to death.

Evaluating biologic potency of a topical chemodenervating agent presents unique challenges. The present invention provides methods to measure toxin potency via mouse tail vein IV (intravenous) injection and TTD (time-to-death) of animals. In accordance with these methods, a standard curve of toxin reference standard is established with a series of dose levels. Sample potency is calculated from standard curve with TTD of sample group. These methods are useful in toxin fermentation, purification and process development, stability testing and release potency testing.

A linear plot of dose response curve between TTD (minutes) and Log (dose) or Ln (dose) can yield linear regression for good potency determination. In some embodiments, both Log (TTD) and Log (dose) and Ln (TTD) and Ln (dose) yield a better linear regression (*i.e.*, better R²) as compared with a non-Log / Ln (TTD) and (dose).

All treatments comprised botulinum type A holotoxin diluted with 0.9% Saline containing 20% Gelatin Phosphate Buffer. Treatment A = 40 U/dose, Treatment B = 151 U/dose, Treatment C = 452 U/dose, Treatment D = 1395 U/dose, Treatment E = 4147 U/dose, Treatment F = 12441 U/dose and Treatment G = 37700 U/dose.

Mice (18 - 24 grams) were employed for each treatment group (n=5). Mice were randomly assigned to each treatment group. A total treatment volume of 200 µL was injected into the intravenous tail vein of each mouse to measure the time of death. Each mouse was observed for 4 hours after injection.

The results demonstrated that the Log standard curve had a linear regression R² correlation of 0.9696 and the Ln standard curve had a liner regression correlation of 0.9778 while the non-Log / Ln standard curve had an inferior linear regression correlation of 0.3574. Using the Log or Ln linear regression, a dose response curve can be plotted to determine the potency of a sample and that this method is superior to a non-logarithmically transformed dose-response curve. Thus, in some embodiments, this method can be very useful in toxin fermentation, purification and process development, stability testing and release potency testing.

### Example 20: Intraperitoneal LD50 Method for Assessing the Potency of a Topical Chemodenervating Agent

The present example demonstrates the discovery that potency of a provided composition (*e.g.*, topical botulinum preparation) can be tested by injecting the composition into the peritoneum of a mouse and measuring the average or median time it takes 50% of treated animals to die.

Evaluating biologic potency of a topical chemodenervating agent presents unique challenges. The present invention provides methods for evaluating potency by measuring IP (intraperitoneal) injection, *e.g.*, directly (*i.e.*, without a reference standard). In accordance with these methods, a series of toxin samples at different dilutions with unknown potency are injected intraperitoneally to mice and the total number of mice dead and alive over a period of 72 hours is recorded. A plot of % death and dilutions can produce the potency, LD50 unit, of the sample. The 1 LD50 is the lethal dose (of toxin) that caused 50% animal to die within 72 hours. To reduce the large variability of regular plot of % death vs. Log (dilutions), the Reed and Muench method can be used for more consistent and accurate LD50 value.

All treatments comprised 6.4 g 1349 oil, 9.6 g Tween-80 with 0.2% parabens, 64.0 g 0.9% Saline with 0.2% parabens, 17.24 g Gelatin Phosphate Buffer, and 2.76 g botulinum type A holotoxin solution. Each treatment was microfluidized at 22,000 PSI to create nanoparticles and then mixed with an equal volume of cream excipient prior to application on mice. The treatment was diluted from its original concentration in five concentrations (Treatments A - E) to perform the study: Treatment A had a 1:7.5 dilution factor; Treatment B had a 1:10.0 dilution factor; Treatment C had a 1:13.5 dilution factor; Treatment D had a 1:18.0 dilution factor; and Treatment E had a 1:24.3 dilution factor.

Mice (18 - 24 grams) were employed for each treatment group (n=8). Mice were randomly assigned to each treatment group. A total treatment volume of 200 µL was injected into the intraperitoneal of each mouse. Each mouse was observed for 3 days after injection to record the death and survival from the five treatment groups.

Results were calculated to determine potency of the original treatment. The nominal potency of the treatment was 8000 U/mL; and after calculating the potency from the experimental data using the Reed and Muench IP method, the actual potency came to be 7828 U/mL. In conclusion, the Reed and Muench IP method can generate an accurate potency measurement even in the absence a reference standard.

### Example 21: Clinical Study to Evaluate Effect of Empty Nanoemulsion Formulation ("Composition H") on Axillary Sweating

### Study Design Summary

The purpose of the study was to determine if Emulsion H is biologically active in reducing sweating. Subjects were selected who believed they sweated excessively and who demonstrated excessive sweating by gravimetric sweat measurement. Some subjects received treatment with the potentially biologically active formulation and some subjects received treatment with a placebo, i.e. water. Neither the subject nor the investigator knew which treatment the subject was receiving.

Two weeks after a single treatment, subjects were re-assessed by gravimetric sweat measurement to determine the degree of sweat reduction. A comparison of posttreatment sweat production between the treatment groups was made to determine the degree of sweat reduction by the potentially biologically active formulation.

### Study Subject Inclusion / Exclusion Criteria

The study used the following criteria to enroll subjects:

### Inclusion Criteria

- able to understand and give written informed consent
- ages 18 - 70 years of age
- diagnosis of moderate to severe primary axillary hyperhidrosis
- Hyperhidrosis Disease Severity Scale score of ≥3 (the HDSS scale is described below)
- ≥50 mg of sweat production/axilla in 5 minutes as measured gravimetrically
- willingness to use only over-the-counter deodorants during the course of the study
- willingness to shave underarms prior to each study visit
- female subjects must have a negative urine pregnancy test and be non-lactating at the initial ("Baseline") study site visit
- patients should be in good general health as determined by the investigator and free of any disease that may interfere with study evaluations

### Exclusion Criteria

- diagnosis of secondary hyperhidrosis (that is, hyperhidrosis due to another medical condition such as hyperthyroidism, cancer, tuberculosis, malaria, or other infection)
- signs of infection in the axilla
- skin affliction in the axilla requiring medical treatment
- application of topical medication to the treatment area within 14 days prior to treatment
- 20% aluminum hydrochloride, e.g. Drysol®, in 2 weeks prior of Baseline
- oral anticholinergic treatment (e.g., Benadryl, Atarax, Chlortrimeton, and Robinul) in prior 2 weeks
- use of antiperspirants, deodorants, powders or lotions in the 2 days prior to Baseline
- botulinum toxin treatment in prior 9 months
- history of surgery for axillary hyperhidrosis
- participation in another investigational drug trial or receiving any investigational treatment(s) within 30 days of Baseline
- alcohol or drug abuse within the past 3 years
- female subjects who are pregnant or are nursing a child
- psychiatric disease interfering with the patient's ability to give informed consent
- use of axillary depilatories, e.g Nair®, Veet®
- use of axillary epilation (waxing, laser, electrolysis) within 1 week of Baseline
- refusal or inability to comply with the requirements of the protocol for any reason

### Treatment And Assessment Methods

### Clinical Visits

Prior to scheduling an initial visit to the investigator's study site, potential participants were queried with regards to their use of anti-perspirants, topical medications, or depilatory products in the axilla. Subjects who met Exclusion Criteria were not scheduled. Potential participants were instructed not to use such products and to shave his or her underarms prior to the Baseline study visit.

At the Baseline study visit, prior to participating in any aspect of the study, each subject was fully informed, both verbally and in writing, of the conduct and consequences of the study. Each subject signed the written Informed Consent Form prior to the conduct of the screening evaluation to determine whether the subject was potentially eligible for the study. A verbal screening evaluation and gravimetric sweat measurement were performed to determine if the subject met the Inclusion Criteria but did not meet the Exclusion Criteria.

### The Hyperhidrosis Disease Severity Scale

The subject was asked to rate the perceived severity of the subject's disease by selecting the one sentence that best describes the current level to which subject's underarm sweating interferes with the subject's life:
0 = My underarm sweating is not noticeable and never interferes with my daily activities.
1 = My underarm sweating is noticeable but rarely interferes with my daily activities.
2 = My underarm sweating is tolerable but sometimes interferes with my daily activities.
3 = My underarm sweating is barely tolerable and frequently interferes with my daily activities.
4 = My underarm sweating is barely tolerable and always interferes with my daily activities.
5 = My underarm sweating is intolerable and always interferes with my daily activities.

### Gravimetric Sweat Measurement Method

The sweat production of the subject is measured gravimetrically by the following procedure:
- The subject was placed in a room with relatively constant temperature and humidity for at least 30 min.
- The subject was placed in a semi-reclining position with the axilla fully exposed and the arm resting comfortably above the head.
- The subject's axilla was dried gently with a cotton gauze pad.
- The investigator used a forceps to place one filter paper (90 mm diameter) on a balance sensitive to 0.1 mg and recorded its weight.
- The investigator used a forceps to place the measured filter paper on the axilla, covered it with plastic and taped the edges of the bag against the subject's skin with hypoallergenic tape to form a seal around the plastic bag.
- After 5 minutes, the investigator gently removed the tape and plastic from the subject's axilla and then, using forceps, immediately placed the filter paper onto the scale to record its weight. The scale was then dried and zero balanced.
- This measurement was then repeated as described above with the other axilla.

### Treatment application

If the subject was eligible for treatment on this basis, the subject was then treated. For treatment, one of the study preparations (0.3 mL/axilla) was applied topically with a gloved finger by the investigator to the subject's skin of the axilla. Emulsion H contained 19.2 mg Labrafac Lipophile WL 1349 and 28.8 mg Polysorbate 80, NF, in addition to 0.9% Sodium Chloride Irrigation, USP, and Gelatin Phosphate Buffer. The average diameter (e.g., particle size) of nanoparticles contained in the Emusion H empty nanoparticle composition was approximately 80.1 nm. The preparation was administered in small increments to avoid run-off. The liquid was rubbed-in until vanished.

Following treatment, the subject was instructed to shower on the day of treatment immediately prior to going to bed and, in so doing, wash the axilla with soap and water. The subject was instructed not to use any of the following medications:
- Botulinum Toxin containing products applied to the axilla for the course of the study
- Aluminum hydrochloride topical, e.g. Drysol® for the course of the study
- Oral anticholinergic treatment (e.g., Benadryl, Atarax, Chlortrimeton, and Robinul) for the course of the study
- Use of antiperspirants, deodorants, powders, or lotions in the 2 days prior to the Baseline visit and 2 days prior to the office visit two weeks following treatment when gravimetric sweat measurement would be conducted.
- Use of antiperspirants, deodorants, powders or lotions for 1 day after the treatment
- Topical medications applied to the treatment area for 5 days following treatment
- Investigational Medications or treatments within 30 days of Baseline and during the course of the study.

The subject was scheduled for a follow-up office visit two weeks after the treatment. At the follow-up office visit, the subject was questioned as to their compliance with the instructions regarding which medications not to use between treatment and the two week follow-up office visit. If the subject was non-compliant, the subject was disqualified from the study. If the subject was compliant, the subject was re-assessed using the gravimetric sweat measurement procedure.

### Treatment Results and Conclusion

The study was conducted at multiple study sites and conducted in compliance with Good Clinical Practice standards. Ten subjects were treated with Emulsion H. Two weeks after the treatment, each subject was re-assessed by gravimetric sweat measurement.

On average, subjects in the Emulsion H group had a reduction in sweat production of 151 mg two weeks after treatment as measured by gravimetric sweat measurement. In contrast, subjects treated with the placebo had a 53 mg reduction in sweat production as measured by gravimetric sweat measurement. Therefore, subjects treated with Emulsion H had a 286% greater reduction in sweat production than the subjects in the control group.

It was also determined what percent of study subjects receiving either Emulsion H or placebo experienced at least a 30% reduction in sweat production when compared to levels measured at the Baseline visit. It was found that 60% of subjects treated with Emulsion H had at least a 30% reduction in sweat production when compared to levels at the Baseline visit. This contrasts with only 29% of subjects in the control group that had at least a 30% reduction in sweat production when compared to levels at the Baseline visit. Therefore, by this assessment subjects treated with Emulsion H had a 210% greater effectiveness in reducing sweat production than those subjects treated with placebo.

Given these data, it is concluded that Emulsion H is (i) biologically active in reducing sweat production, (ii) is an anti-perspirant formulation, and (iii) may be used effectively in treating hyperhidrosis.

### Example 22: Clinical Study to Evaluate Effect of "Emulsion V" Nanoparticle Composition on Axillary Sweating

### Study Design Summary

The purpose of the study was to determine if Emulsion V is biologically active in reducing sweating. Subjects were selected who believed they sweated excessively and who demonstrated excessive sweating by gravimetric sweat measurement. Some subjects received treatment with the potentially biologically active formulation and some subjects received treatment with a placebo, i.e. water. Neither the subject nor the investigator knew which treatment the subject was receiving.

Two weeks after a single treatment, subjects were re-assessed by gravimetric sweat measurement to determine the degree of sweat reduction. A comparison of posttreatment sweat production between the treatment groups was made to determine the degree of sweat reduction by the potentially biologically active formulation.

### Study Subject Inclusion / Exclusion Criteria

The study used the following criteria to enroll subjects:

### Inclusion Criteria

- able to understand and give written informed consent
- ages 18 - 70 years of age
- diagnosis of moderate to severe primary axillary hyperhidrosis
- Hyperhidrosis Disease Severity Scale score of ≥3 (the HDSS scale is described below)
- ≥50 mg of sweat production/axilla in 5 minutes as measured gravimetrically
- willingness to use only over-the-counter deodorants during the course of the study
- willingness to shave underarms prior to each study visit
- female subjects must have a negative urine pregnancy test and be non-lactating at the initial ("Baseline") study site visit
- patients should be in good general health as determined by the investigator and free of any disease that may interfere with study evaluations

### Exclusion Criteria

- diagnosis of secondary hyperhidrosis (that is, hyperhidrosis due to another medical condition such as hyperthyroidism, cancer, tuberculosis, malaria, or other infection)
- signs of infection in the axilla
- skin affliction in the axilla requiring medical treatment
- application of topical medication to the treatment area within 14 days prior to treatment
- 20% aluminum hydrochloride, e.g. Drysol®, in 2 weeks prior of Baseline
- oral anticholinergic treatment (e.g., Benadryl, Atarax, Chlortrimeton, and Robinul) in prior 2 weeks
- use of antiperspirants, deodorants, powders or lotions in the 2 days prior to Baseline
- botulinum toxin treatment in prior 9 months
- history of surgery for axillary hyperhidrosis
- participation in another investigational drug trial or receiving any investigational treatment(s) within 30 days of Baseline
- alcohol or drug abuse within the past 3 years
- female subjects who are pregnant or are nursing a child
- psychiatric disease interfering with the patient's ability to give informed consent
- use of axillary depilatories, e.g Nair®, Veet®
- use of axillary epilation (waxing, laser, electrolysis) within 1 week of Baseline
- refusal or inability to comply with the requirements of the protocol for any reason

### Treatment and Assessment Methods

### Clinical Visits

Prior to scheduling an initial visit to the investigator's study site, potential participants were queried with regards to their use of anti-perspirants, topical medications, or depilatory products in the axilla. Subjects who met Exclusion Criteria were not scheduled. Potential participants were instructed not to use such products and to shave his or her underarms prior to the Baseline study visit.

At the Baseline study visit, prior to participating in any aspect of the study, each subject was fully informed, both verbally and in writing, of the conduct and consequences of the study. Each subject signed the written Informed Consent Form prior to the conduct of the screening evaluation to determine whether the subject was potentially eligible for the study. A verbal screening evaluation and gravimetric sweat measurement were performed to determine if the subject met the Inclusion Criteria but did not meet the Exclusion Criteria.

### The Hyperhidrosis Disease Severity Scale

The subject was asked to rate the perceived severity of the subject's disease by selecting the one sentence that best describes the current level to which subject's underarm sweating interferes with the subject's life:
0 = My underarm sweating is not noticeable and never interferes with my daily activities.
1 = My underarm sweating is noticeable but rarely interferes with my daily activities.
2 = My underarm sweating is tolerable but sometimes interferes with my daily activities.
3 = My underarm sweating is barely tolerable and frequently interferes with my daily activities.
4 = My underarm sweating is barely tolerable and always interferes with my daily activities.
5 = My underarm sweating is intolerable and always interferes with my daily activities.

### Gravimetric Sweat Measurement Method

The sweat production of the subject is measured gravimetrically by the following procedure:
- The subject was placed in a room with relatively constant temperature and humidity for at least 30 min.
- The subject was placed in a semi-reclining position with the axilla fully exposed and the arm resting comfortably above the head.
- The subject's axilla was dried gently with a cotton gauze pad.
- The investigator used a forceps to place one filter paper (90 mm diameter) on a balance sensitive to 0.1 mg and recorded its weight.
- The investigator used a forceps to place the measured filter paper on the axilla, covered it with plastic and taped the edges of the bag against the subject's skin with hypoallergenic tape to form a seal around the plastic bag.
- After 5 minutes, the investigator gently removed the tape and plastic from the subject's axilla and then, using forceps, immediately placed the filter paper onto the scale to record its weight. The scale was then dried and zero balanced.
- This measurement was then repeated as described above with the other axilla.

### Treatment application

If the subject was eligible for treatment on this basis, the subject was then treated. For treatment, one of the study preparations (0.3 mL/axilla) was applied topically with a gloved finger by the investigator to the subject's skin of the axilla. Emulsion V contained Emulsifying Wax, Gelatin Phosphate Buffer Solution, Isopropyl Myristate, Labrafac Lipophile, Methylparaben, Mineral Oil Heavy Viscosity Range, Polysorbate 80, Propylparaben, Purified Water, Sodium Chloride Injection, and White Petrolatum. All ingredients are either NF or USP grade. The average diameter (e.g., particle size) of nanoparticles contained in the Emusion V empty nanoparticle composition was approximately 77.1 nm. The preparation was administered in small increments to avoid run-off. The liquid was rubbed-in until vanished.

Following treatment, the subject was instructed to shower on the day of treatment immediately prior to going to bed and, in so doing, wash the axilla with soap and water. The subject was instructed not to use any of the following medications:
- Botulinum Toxin containing products applied to the axilla for the course of the study
- Aluminum hydrochloride topical, e.g. Drysol® for the course of the study
- Oral anticholinergic treatment (e.g., Benadryl, Atarax, Chlortrimeton, and Robinul) for the course of the study
- Use of antiperspirants, deodorants, powders, or lotions in the 2 days prior to the Baseline visit and 2 days prior to the office visit two weeks following treatment when gravimetric sweat measurement would be conducted.
- Use of antiperspirants, deodorants, powders or lotions for 1 day after the treatment
- Topical medications applied to the treatment area for 5 days following treatment
- Investigational Medications or treatments within 30 days of Baseline and during the course of the study.

The subject was scheduled for a follow-up office visit two weeks after the treatment. At the follow-up office visit, the subject was questioned as to their compliance with the instructions regarding which medications not to use between treatment and the two week follow-up office visit. If the subject was non-compliant, the subject was disqualified from the study. If the subject was compliant, the subject was re-assessed using the gravimetric sweat measurement procedure.

### Treatment Results and Conclusion

The study was conducted at multiple study sites and conducted in compliance with Good Clinical Practice standards. Ten subjects were treated with Emulsion V. Two weeks after the treatment, each subject was re-assessed by gravimetric sweat measurement.

On average, subjects in the Emulsion V group had a reduction in sweat production of 151 mg two weeks after treatment as measured by gravimetric sweat measurement. In contrast, subjects treated with the placebo had a 53 mg reduction in sweat production as measured by gravimetric sweat measurement. Therefore, subjects treated with Emulsion V had a 286% greater reduction in sweat production than the subjects in the control group.

It was also determined what percent of study subjects receiving either Emulsion V or placebo experienced at least a 30% reduction in sweat production when compared to levels measured at the Baseline visit. It was found that 60% of subjects treated with Emulsion V had at least a 30% reduction in sweat production when compared to levels at the Baseline visit. This contrasts with only 29% of subjects in the control group that had at least a 30% reduction in sweat production when compared to levels at the Baseline visit. Therefore, by this assessment subjects treated with Emulsion V had a 210% greater effectiveness in reducing sweat production than those subjects treated with placebo.

Given these data, it is concluded that Emulsion V is (i) biologically active in reducing sweat production, (ii) is an anti-perspirant formulation, and (iii) may be used effectively in treating hyperhidrosis.

### Example 23: Clinical Study to Evaluate Effect of Polysorbate 80 on Axillary Sweating

### Study Design Summary

The purpose of the study was to determine if Polysorbate 80 is biologically active in reducing sweating. Subjects were selected who believed they sweated excessively and who demonstrated excessive sweating by gravimetric sweat measurement. Some subjects received treatment with the potentially biologically active substance and some subjects received treatment with a placebo, i.e. water. Neither the subject nor the investigator knew which treatment the subject was receiving.

Two weeks after a single treatment, subjects were re-assessed by gravimetric sweat measurement to determine the degree of sweat reduction. A comparison of posttreatment sweat production between the treatment groups was made to determine the degree of sweat reduction by the potentially biologically active substance.

### Study Subject Inclusion / Exclusion Criteria

The study used the following criteria to enroll subjects:

### Inclusion Criteria

- able to understand and give written informed consent
- ages 18 - 70 years of age
- diagnosis of moderate to severe primary axillary hyperhidrosis
- Hyperhidrosis Disease Severity Scale score of ≥3 (the HDSS scale is described below)
- ≥50 mg of sweat production/axilla in 5 minutes as measured gravimetrically
- willingness to use only over-the-counter deodorants during the course of the study
- willingness to shave underarms prior to each study visit
- female subjects must have a negative urine pregnancy test and be non-lactating at the initial ("Baseline") study site visit
- patients should be in good general health as determined by the investigator and free of any disease that may interfere with study evaluations

### Exclusion Criteria

- diagnosis of secondary hyperhidrosis (that is, hyperhidrosis due to another medical condition such as hyperthyroidism, cancer, tuberculosis, malaria, or other infection)
- signs of infection in the axilla
- skin affliction in the axilla requiring medical treatment
- application of topical medication to the treatment area within 14 days prior to treatment
- 20% aluminum hydrochloride, e.g. Drysol®, in 2 weeks prior of Baseline
- oral anticholinergic treatment (e.g., Benadryl, Atarax, Chlortrimeton, and Robinul) in prior 2 weeks
- use of antiperspirants, deodorants, powders or lotions in the 2 days prior to Baseline
- botulinum toxin treatment in prior 9 months
- history of surgery for axillary hyperhidrosis
- participation in another investigational drug trial or receiving any investigational treatment(s) within 30 days of Baseline
- alcohol or drug abuse within the past 3 years
- female subjects who are pregnant or are nursing a child
- psychiatric disease interfering with the patient's ability to give informed consent
- use of axillary depilatories, e.g Nair®, Veet®
- use of axillary epilation (waxing, laser, electrolysis) within 1 week of Baseline
- refusal or inability to comply with the requirements of the protocol for any reason

### Treatment and Assessment Methods

### Clinical Visits

Prior to scheduling an initial visit to the investigator's study site, potential participants were queried with regards to their use of anti-perspirants, topical medications, or depilatory products in the axilla. Subjects who met Exclusion Criteria were not scheduled. Potential participants were instructed not to use such products and to shave his or her underarms prior to the Baseline study visit.

At the Baseline study visit, prior to participating in any aspect of the study, each subject was fully informed, both verbally and in writing, of the conduct and consequences of the study. Each subject signed the written Informed Consent Form prior to the conduct of the screening evaluation to determine whether the subject was potentially eligible for the study. A verbal screening evaluation and gravimetric sweat measurement were performed to determine if the subject met the Inclusion Criteria but did not meet the Exclusion Criteria.

### The Hyperhidrosis Disease Severity Scale

The subject was asked to rate the perceived severity of the subject's disease by selecting the one sentence that best describes the current level to which subject's underarm sweating interferes with the subject's life:
0 = My underarm sweating is not noticeable and never interferes with my daily activities.
1 = My underarm sweating is noticeable but rarely interferes with my daily activities.
2 = My underarm sweating is tolerable but sometimes interferes with my daily activities.
3 = My underarm sweating is barely tolerable and frequently interferes with my daily activities.
4 = My underarm sweating is barely tolerable and always interferes with my daily activities.
5 = My underarm sweating is intolerable and always interferes with my daily activities.

### Gravimetric Sweat Measurement Method

The sweat production of the subject is measured gravimetrically by the following procedure:
- The subject was placed in a room with relatively constant temperature and humidity for at least 30 min.
- The subject was placed in a semi-reclining position with the axilla fully exposed and the arm resting comfortably above the head.
- The subject's axilla was dried gently with a cotton gauze pad.
- The investigator used a forceps to place one filter paper (90 mm diameter) on a balance sensitive to 0.1 mg and recorded its weight.
- The investigator used a forceps to place the measured filter paper on the axilla, covered it with plastic and taped the edges of the bag against the subject's skin with hypoallergenic tape to form a seal around the plastic bag.
- After 5 minutes, the investigator gently removed the tape and plastic from the subject's axilla and then, using forceps, immediately placed the filter paper onto the scale to record its weight. The scale was then dried and zero balanced.
- This measurement was then repeated as described above with the other axilla.

### Treatment application

If the subject was eligible for treatment on this basis, the subject was then treated. For treatment, one of the study preparations (0.3 mL/axilla) was applied topically with a gloved finger by the investigator to the subject's skin of the axilla. The preparation was administered in small increments to avoid run-off. The liquid was rubbed-in until vanished. Each subject who was selected to have a treatment with the potentially biologically active substance had 14.34 mg of Polysorbate 80 applied to each axilla.

Following treatment, the subject was instructed to shower on the day of treatment immediately prior to going to bed and, in so doing, wash the axilla with soap and water. The subject was instructed not to use any of the following medications:
- Botulinum Toxin containing products applied to the axilla for the course of the study
- Aluminum hydrochloride topical, e.g. Drysol® for the course of the study
- Oral anticholinergic treatment (e.g., Benadryl, Atarax, Chlortrimeton, and Robinul) for the course of the study
- Use of antiperspirants, deodorants, powders, or lotions in the 2 days prior to the Baseline visit and 2 days prior to the office visit two weeks following treatment when gravimetric sweat measurement would be conducted.
- Use of antiperspirants, deodorants, powders or lotions for 1 day after the treatment
- Topical medications applied to the treatment area for 5 days following treatment
- Investigational Medications or treatments within 30 days of Baseline and during the course of the study.

The subject was scheduled for a follow-up office visit two weeks after the treatment. At the follow-up office visit, the subject was questioned as to their compliance with the instructions regarding which medications not to use between treatment and the two week follow-up office visit. If the subject was non-compliant, the subject was disqualified from the study. If the subject was compliant, the subject was re-assessed using the gravimetric sweat measurement procedure.

### Treatment Results and Conclusion

The study was conducted at multiple study sites and conducted in compliance with Good Clinical Practice standards. Ten subjects were treated with Polysorbate 80. Two weeks after the treatment, each subject was re-assessed by gravimetric sweat measurement.

On average, subjects in the Polysorbate 80 group had a reduction in sweat production of 159 mg two weeks after treatment as measured by gravimetric sweat measurement. In contrast, subjects treated with the placebo had a 53 mg reduction in sweat production as measured by gravimetric sweat measurement. Therefore, subjects treated with Polysorbate 80 had a 300% greater reduction in sweat production than the subjects in the control group.

It was also determined what percent of study subjects receiving either Polysorbate 80 or placebo experienced at least a 30% reduction in sweat production when compared to levels measured at the Baseline visit. It was found that 80% of subjects treated with Polysorbate 80 had at least a 30% reduction in sweat production when compared to levels at the Baseline visit. This contrasts with only 29% of subjects in the control group that had at least a 30% reduction in sweat production when compared to levels at the Baseline visit. Therefore, by this assessment subjects treated with Polysorbate 80 had a 280% greater effectiveness in reducing sweat production than those subjects treated with placebo.

Given these data, it is concluded that Polysorbate 80 is (i) biologically active in reducing sweat production, (ii) is an anti-perspirant substance, and (iii) may be used effectively in treating hyperhidrosis.

### Example 24: Clinical Study to Evaluate Effect of Labrafac Lipophile WL 1349 on Axillary Sweating

### Study Design Summary

The purpose of the study was to determine if Labrafac Lipophile WL 1349 is biologically active in reducing sweating. Subjects were selected who believed they sweated excessively and who demonstrated excessive sweating by gravimetric sweat measurement. Some subjects received treatment with the potentially biologically active substance and some subjects received treatment with a placebo, i.e. water. Neither the subject nor the investigator knew which treatment the subject was receiving.

Two weeks after a single treatment, subjects were re-assessed by gravimetric sweat measurement to determine the degree of sweat reduction. A comparison of posttreatment sweat production between the treatment groups was made to determine the degree of sweat reduction by the potentially biologically active substance.

### Study Subject Inclusion / Exclusion Criteria

The following criteria were used to enroll subject:

### Inclusion Criteria

- able to understand and give written informed consent
- ages 18 - 70 years of age
- diagnosis of moderate to severe primary axillary hyperhidrosis
- Hyperhidrosis Disease Severity Scale score of ≥3 (the HDSS scale is described below)
- ≥50 mg of sweat production/axilla in 5 minutes as measured gravimetrically
- willingness to use only over-the-counter deodorants during the course of the study
- willingness to shave underarms prior to each study visit
- female subjects must have a negative urine pregnancy test and be non-lactating at the initial ("Baseline") study site visit
- patients should be in good general health as determined by the investigator and free of any disease that may interfere with study evaluations

### Exclusion Criteria

- diagnosis of secondary hyperhidrosis (that is, hyperhidrosis due to another medical condition such as hyperthyroidism, cancer, tuberculosis, malaria, or other infection)
- signs of infection in the axilla
- skin affliction in the axilla requiring medical treatment
- application of topical medication to the treatment area within 14 days prior to treatment
- 20% aluminum hydrochloride, e.g. Drysol®, in 2 weeks prior of Baseline
- oral anticholinergic treatment (e.g., Benadryl, Atarax, Chlortrimeton, and Robinul) in prior 2 weeks
- use of antiperspirants, deodorants, powders or lotions in the 2 days prior to Baseline
- botulinum toxin treatment in prior 9 months
- history of surgery for axillary hyperhidrosis
- participation in another investigational drug trial or receiving any investigational treatment(s) within 30 days of Baseline
- alcohol or drug abuse within the past 3 years
- female subjects who are pregnant or are nursing a child
- psychiatric disease interfering with the patient's ability to give informed consent
- use of axillary depilatories, e.g Nair®, Veet®
- use of axillary epilation (waxing, laser, electrolysis) within 1 week of Baseline
- refusal or inability to comply with the requirements of the protocol for any reason

### Treatment and Assessment Methods

### Clinical Visits

Prior to scheduling an initial visit to the investigator's study site, potential participants were queried with regards to their use of anti-perspirants, topical medications, or depilatory products in the axilla. Subjects who met Exclusion Criteria were not scheduled. Potential participants were instructed not to use such products and to shave his or her underarms prior to the Baseline study visit.

At the Baseline study visit, prior to participating in any aspect of the study, each subject was fully informed, both verbally and in writing, of the conduct and consequences of the study. Each subject signed the written Informed Consent Form prior to the conduct of the screening evaluation to determine whether the subject was potentially eligible for the study. A verbal screening evaluation and gravimetric sweat measurement were performed to determine if the subject met the Inclusion Criteria but did not meet the Exclusion Criteria.

### The Hyperhidrosis Disease Severity Scale

The subject was asked to rate the perceived severity of the subject's disease by selecting the one sentence that best describes the current level to which subject's underarm sweating interferes with the subject's life:
0 = My underarm sweating is not noticeable and never interferes with my daily activities.
1 = My underarm sweating is noticeable but rarely interferes with my daily activities.
2 = My underarm sweating is tolerable but sometimes interferes with my daily activities.
3 = My underarm sweating is barely tolerable and frequently interferes with my daily activities.
4 = My underarm sweating is barely tolerable and always interferes with my daily activities.
5 = My underarm sweating is intolerable and always interferes with my daily activities.

### Gravimetric Sweat Measurement Method

The sweat production of the subject is measured gravimetrically by the following procedure:
- The subject was placed in a room with relatively constant temperature and humidity for at least 30 min.
- The subject was placed in a semi-reclining position with the axilla fully exposed and the arm resting comfortably above the head.
- The subject's axilla was dried gently with a cotton gauze pad.
- The investigator used a forceps to place one filter paper (90 mm diameter) on a balance sensitive to 0.1 mg and recorded its weight.
- The investigator used a forceps to place the measured filter paper on the axilla, covered it with plastic and taped the edges of the bag against the subject's skin with hypoallergenic tape to form a seal around the plastic bag.
- After 5 minutes, the investigator gently removed the tape and plastic from the subject's axilla and then, using forceps, immediately placed the filter paper onto the scale to record its weight. The scale was then dried and zero balanced.
- This measurement was then repeated as described above with the other axilla.

### Treatment application

If the subject was eligible for treatment on this basis, the subject was then treated. For treatment, one of the study preparations (0.3 mL/axilla) was applied topically with a gloved finger by the investigator to the subject's skin of the axilla. The preparation was administered in small increments to avoid run-off. The liquid was rubbed-in until vanished. Each subject who was selected to have a treatment with the potentially biologically active substance had 9.57 mg of Labrafac Lipophile WL 1349 applied to each axilla.

Following treatment, the subject was instructed to shower on the day of treatment immediately prior to going to bed and, in so doing, wash the axilla with soap and water. The subject was instructed not to use any of the following medications:
- Botulinum Toxin containing products applied to the axilla for the course of the study
- Aluminum hydrochloride topical, e.g. Drysol® for the course of the study
- Oral anticholinergic treatment (e.g., Benadryl, Atarax, Chlortrimeton, and Robinul) for the course of the study
- Use of antiperspirants, deodorants, powders, or lotions in the 2 days prior to the Baseline visit and 2 days prior to the office visit two weeks following treatment when gravimetric sweat measurement would be conducted.
- Use of antiperspirants, deodorants, powders or lotions for 1 day after the treatment
- Topical medications applied to the treatment area for 5 days following treatment
- Investigational Medications or treatments within 30 days of Baseline and during the course of the study.

The subject was scheduled for a follow-up office visit two weeks after the treatment. At the follow-up office visit, the subject was questioned as to their compliance with the instructions regarding which medications not to use between treatment and the two week follow-up office visit. If the subject was non-compliant, the subject was disqualified from the study. If the subject was compliant, the subject was re-assessed using the gravimetric sweat measurement procedure.

### Treatment Results and Conclusion

The study was conducted at multiple study sites and conducted in compliance with Good Clinical Practice standards. Ten subjects were treated with Labrafac Lipophile WL 1349. Two weeks after the treatment, each subject was re-assessed by gravimetric sweat measurement.

On average, subjects in the Labrafac Lipophile WL 1349 group had a reduction in sweat production of 165 mg two weeks after treatment as measured by gravimetric sweat measurement. In contrast, subjects treated with the placebo had a 53 mg reduction in sweat production as measured by gravimetric sweat measurement. Therefore, subjects treated with Labrafac Lipophile WL 1349 had a 313% greater reduction in sweat production than the subjects in the control group.

It was also determined what percent of study subjects receiving either Labrafac Lipophile WL 1349 or placebo experienced at least a 30% reduction in sweat production when compared to levels measured at the Baseline visit. It was found that 80% of subjects treated with Labrafac Lipophile WL 1349 had at least a 30% reduction in sweat production when compared to levels at the Baseline visit. This contrasts with only 29% of subjects in the control group that had at least a 30% reduction in sweat production when compared to levels at the Baseline visit. Therefore, by this assessment subjects treated with Labrafac Lipophile WL 1349 had a 280% greater effectiveness in reducing sweat production than those subjects treated with placebo.

Given these data, it is concluded that Labrafac Lipophile WL 1349 is (i) biologically active in reducing sweat production, (ii) is an anti-perspirant substance, and (iii) may be used effectively in treating hyperhidrosis.

### Example 25: Clinical Study to Evaluate Effect of Isopropyl Myristate on Axillary Sweating

### Study Design Summary

The purpose of the study was to determine if Isopropyl Myristate is biologically active in reducing sweating. Subjects were selected who believed they sweated excessively and who demonstrated excessive sweating by gravimetric sweat measurement. Some subjects received treatment with the potentially biologically active substance and some subjects received treatment with a placebo, i.e. water. Neither the subject nor the investigator knew which treatment the subject was receiving.

Two weeks after a single treatment, subjects were re-assessed by gravimetric sweat measurement to determine the degree of sweat reduction. A comparison of posttreatment sweat production between the treatment groups was made to determine the degree of sweat reduction by the potentially biologically active substance.

### Study Subject Inclusion / Exclusion Criteria

The following criteria were used to enroll subjects:

### Inclusion Criteria

- able to understand and give written informed consent
- ages 18 - 70 years of age
- diagnosis of moderate to severe primary axillary hyperhidrosis
- Hyperhidrosis Disease Severity Scale score of ≥3 (the HDSS scale is described below)
- ≥50 mg of sweat production/axilla in 5 minutes as measured gravimetrically
- willingness to use only over-the-counter deodorants during the course of the study
- willingness to shave underarms prior to each study visit
- female subjects must have a negative urine pregnancy test and be non-lactating at the initial ("Baseline") study site visit
- patients should be in good general health as determined by the investigator and free of any disease that may interfere with study evaluations

### Exclusion Criteria

- diagnosis of secondary hyperhidrosis (that is, hyperhidrosis due to another medical condition such as hyperthyroidism, cancer, tuberculosis, malaria, or other infection)
- signs of infection in the axilla
- skin affliction in the axilla requiring medical treatment
- application of topical medication to the treatment area within 14 days prior to treatment
- 20% aluminum hydrochloride, e.g. Drysol®, in 2 weeks prior of Baseline
- oral anticholinergic treatment (e.g., Benadryl, Atarax, Chlortrimeton, and Robinul) in prior 2 weeks
- use of antiperspirants, deodorants, powders or lotions in the 2 days prior to Baseline
- botulinum toxin treatment in prior 9 months
- history of surgery for axillary hyperhidrosis
- participation in another investigational drug trial or receiving any investigational treatment(s) within 30 days of Baseline
- alcohol or drug abuse within the past 3 years
- female subjects who are pregnant or are nursing a child
- psychiatric disease interfering with the patient's ability to give informed consent
- use of axillary depilatories, e.g Nair®, Veet®
- use of axillary epilation (waxing, laser, electrolysis) within 1 week of Baseline
- refusal or inability to comply with the requirements of the protocol for any reason

### Treatment and Assessment Methods

### Clinical Visits

Prior to scheduling an initial visit to the investigator's study site, potential participants were queried with regards to their use of anti-perspirants, topical medications, or depilatory products in the axilla. Subjects who met Exclusion Criteria were not scheduled. Potential participants were instructed not to use such products and to shave his or her underarms prior to the Baseline study visit.

At the Baseline study visit, prior to participating in any aspect of the study, each subject was fully informed, both verbally and in writing, of the conduct and consequences of the study. Each subject signed the written Informed Consent Form prior to the conduct of the screening evaluation to determine whether the subject was potentially eligible for the study. A verbal screening evaluation and gravimetric sweat measurement were performed to determine if the subject met the Inclusion Criteria but did not meet the Exclusion Criteria.

### The Hyperhidrosis Disease Severity Scale

The subject was asked to rate the perceived severity of the subject's disease by selecting the one sentence that best describes the current level to which subject's underarm sweating interferes with the subject's life:
0 = My underarm sweating is not noticeable and never interferes with my daily activities.
1 = My underarm sweating is noticeable but rarely interferes with my daily activities.
2 = My underarm sweating is tolerable but sometimes interferes with my daily activities.
3 = My underarm sweating is barely tolerable and frequently interferes with my daily activities.
4 = My underarm sweating is barely tolerable and always interferes with my daily activities.
5 = My underarm sweating is intolerable and always interferes with my daily activities.

### Gravimetric Sweat Measurement Method

The sweat production of the subject is measured gravimetrically by the following procedure:
- The subject was placed in a room with relatively constant temperature and humidity for at least 30 min.
- The subject was placed in a semi-reclining position with the axilla fully exposed and the arm resting comfortably above the head.
- The subject's axilla was dried gently with a cotton gauze pad.
- The investigator used a forceps to place one filter paper (90 mm diameter) on a balance sensitive to 0.1 mg and recorded its weight.
- The investigator used a forceps to place the measured filter paper on the axilla, covered it with plastic and taped the edges of the bag against the subject's skin with hypoallergenic tape to form a seal around the plastic bag.
- After 5 minutes, the investigator gently removed the tape and plastic from the subject's axilla and then, using forceps, immediately placed the filter paper onto the scale to record its weight. The scale was then dried and zero balanced.
- This measurement was then repeated as described above with the other axilla.

### Treatment application

If the subject was eligible for treatment on this basis, the subject was then treated. For treatment, one of the study preparations (0.3 mL/axilla) was applied topically with a gloved finger by the investigator to the subject's skin of the axilla. The preparation was administered in small increments to avoid run-off. The liquid was rubbed-in until vanished. Each subject who was selected to have a treatment with the potentially biologically active substance had 1.89 mg of Isopropyl Myristate applied to each axilla.

Following treatment, the subject was instructed to shower on the day of treatment immediately prior to going to bed and, in so doing, wash the axilla with soap and water. The subject was instructed not to use any of the following medications:
- Botulinum Toxin containing products applied to the axilla for the course of the study
- Aluminum hydrochloride topical, e.g. Drysol® for the course of the study
- Oral anticholinergic treatment (e.g., Benadryl, Atarax, Chlortrimeton, and Robinul) for the course of the study
- Use of antiperspirants, deodorants, powders, or lotions in the 2 days prior to the Baseline visit and 2 days prior to the office visit two weeks following treatment when gravimetric sweat measurement would be conducted.
- Use of antiperspirants, deodorants, powders or lotions for 1 day after the treatment
- Topical medications applied to the treatment area for 5 days following treatment
- Investigational Medications or treatments within 30 days of Baseline and during the course of the study.

The subject was scheduled for a follow-up office visit two weeks after the treatment. At the follow-up office visit, the subject was questioned as to their compliance with the instructions regarding which medications not to use between treatment and the two week follow-up office visit. If the subject was non-compliant, the subject was disqualified from the study. If the subject was compliant, the subject was re-assessed using the gravimetric sweat measurement procedure.

### Treatment Results and Conclusion

The study was conducted at multiple study sites and conducted in compliance with Good Clinical Practice standards. Ten subjects were treated with Isopropyl Myristate. Two weeks after the treatment, each subject was re-assessed by gravimetric sweat measurement.

On average, subjects in the Isopropyl Myristate group had a reduction in sweat production of 103 mg two weeks after treatment as measured by gravimetric sweat measurement. In contrast, subjects treated with the placebo had a 53 mg reduction in sweat production as measured by gravimetric sweat measurement. Therefore, subjects treated with Isopropyl Myristate had a 195% greater reduction in sweat production than the subjects in the control group.

It was also determined what percent of study subjects receiving either Isopropyl Myristate or placebo experienced at least a 30% reduction in sweat production when compared to levels measured at the Baseline visit. It was found that 55% of subjects treated with Isopropyl Myristate had at least a 30% reduction in sweat production when compared to levels at the Baseline visit. This contrasts with only 29% of subjects in the control group that had at least a 30% reduction in sweat production when compared to levels at the Baseline visit. Therefore, by this assessment subjects treated with Isopropyl Myristate had a 191% greater effectiveness in reducing sweat production than those subjects treated with placebo.

Given these data, it is concluded that Isopropyl Myristate is (i) biologically active in reducing sweat production, (ii) is an anti-perspirant substance, and (iii) may be used effectively in treating hyperhidrosis.

### Example 26: Clinical Study to Evaluate Effect of Propylparaben on Axillary Sweating

### Study Design Summary

The purpose of the study was to determine if Propylparaben is biologically active in reducing sweating. Subjects were selected who believed they sweated excessively and who demonstrated excessive sweating by gravimetric sweat measurement. Some subjects received treatment with the potentially biologically active substance and some subjects received treatment with a placebo, i.e. water. Neither the subject nor the investigator knew which treatment the subject was receiving.

Two weeks after a single treatment, subjects were re-assessed by gravimetric sweat measurement to determine the degree of sweat reduction. A comparison of posttreatment sweat production between the treatment groups was made to determine the degree of sweat reduction by the potentially biologically active substance.

### Study Subject Inclusion / Exclusion Criteria

The study enrolled subjects based on the following criteria:

### Inclusion Criteria

- able to understand and give written informed consent
- ages 18 - 70 years of age
- diagnosis of moderate to severe primary axillary hyperhidrosis
- Hyperhidrosis Disease Severity Scale score of ≥3 (the HDSS scale is described below)
- ≥50 mg of sweat production/axilla in 5 minutes as measured gravimetrically
- willingness to use only over-the-counter deodorants during the course of the study
- willingness to shave underarms prior to each study visit
- female subjects must have a negative urine pregnancy test and be non-lactating at the initial ("Baseline") study site visit
- patients should be in good general health as determined by the investigator and free of any disease that may interfere with study evaluations

### Exclusion Criteria

- diagnosis of secondary hyperhidrosis (that is, hyperhidrosis due to another medical condition such as hyperthyroidism, cancer, tuberculosis, malaria, or other infection)
- signs of infection in the axilla
- skin affliction in the axilla requiring medical treatment
- application of topical medication to the treatment area within 14 days prior to treatment
- 20% aluminum hydrochloride, e.g. Drysol®, in 2 weeks prior of Baseline
- oral anticholinergic treatment (e.g., Benadryl, Atarax, Chlortrimeton, and Robinul) in prior 2 weeks
- use of antiperspirants, deodorants, powders or lotions in the 2 days prior to Baseline
- botulinum toxin treatment in prior 9 months
- history of surgery for axillary hyperhidrosis
- participation in another investigational drug trial or receiving any investigational treatment(s) within 30 days of Baseline
- alcohol or drug abuse within the past 3 years
- female subjects who are pregnant or are nursing a child
- psychiatric disease interfering with the patient's ability to give informed consent
- use of axillary depilatories, e.g Nair®, Veet®
- use of axillary epilation (waxing, laser, electrolysis) within 1 week of Baseline
- refusal or inability to comply with the requirements of the protocol for any reason

### Treatment and Assessment Methods

### Clinical Visits

Prior to scheduling an initial visit to the investigator's study site, potential participants were queried with regards to their use of anti-perspirants, topical medications, or depilatory products in the axilla. Subjects who met Exclusion Criteria were not scheduled. Potential participants were instructed not to use such products and to shave his or her underarms prior to the Baseline study visit.

At the Baseline study visit, prior to participating in any aspect of the study, each subject was fully informed, both verbally and in writing, of the conduct and consequences of the study. Each subject signed the written Informed Consent Form prior to the conduct of the screening evaluation to determine whether the subject was potentially eligible for the study. A verbal screening evaluation and gravimetric sweat measurement were performed to determine if the subject met the Inclusion Criteria but did not meet the Exclusion Criteria.

### The Hyperhidrosis Disease Severity Scale

The subject was asked to rate the perceived severity of the subject's disease by selecting the one sentence that best describes the current level to which subject's underarm sweating interferes with the subject's life:
0 = My underarm sweating is not noticeable and never interferes with my daily activities.
1 = My underarm sweating is noticeable but rarely interferes with my daily activities.
2 = My underarm sweating is tolerable but sometimes interferes with my daily activities.
3 = My underarm sweating is barely tolerable and frequently interferes with my daily activities.
4 = My underarm sweating is barely tolerable and always interferes with my daily activities.
5 = My underarm sweating is intolerable and always interferes with my daily activities.

### Gravimetric Sweat Measurement Method

The sweat production of the subject is measured gravimetrically by the following procedure:
- The subject was placed in a room with relatively constant temperature and humidity for at least 30 min.
- The subject was placed in a semi-reclining position with the axilla fully exposed and the arm resting comfortably above the head.
- The subject's axilla was dried gently with a cotton gauze pad.
- The investigator used a forceps to place one filter paper (90 mm diameter) on a balance sensitive to 0.1 mg and recorded its weight.
- The investigator used a forceps to place the measured filter paper on the axilla, covered it with plastic and taped the edges of the bag against the subject's skin with hypoallergenic tape to form a seal around the plastic bag.
- After 5 minutes, the investigator gently removed the tape and plastic from the subject's axilla and then, using forceps, immediately placed the filter paper onto the scale to record its weight. The scale was then dried and zero balanced.
- This measurement was then repeated as described above with the other axilla.

### Treatment application

If the subject was eligible for treatment on this basis, the subject was then treated. For treatment, one of the study preparations (0.3 mL/axilla) was applied topically with a gloved finger by the investigator to the subject's skin of the axilla. The preparation was administered in small increments to avoid run-off. The liquid was rubbed-in until vanished. Each subject who was selected to have a treatment with the potentially biologically active substance had 0.20 mg of Propylparaben applied to each axilla.

Following treatment, the subject was instructed to shower on the day of treatment immediately prior to going to bed and, in so doing, wash the axilla with soap and water. The subject was instructed not to use any of the following medications:
- Botulinum Toxin containing products applied to the axilla for the course of the study
- Aluminum hydrochloride topical, e.g. Drysol® for the course of the study
- Oral anticholinergic treatment (e.g., Benadryl, Atarax, Chlortrimeton, and Robinul) for the course of the study
- Use of antiperspirants, deodorants, powders, or lotions in the 2 days prior to the Baseline visit and 2 days prior to the office visit two weeks following treatment when gravimetric sweat measurement would be conducted.
- Use of antiperspirants, deodorants, powders or lotions for 1 day after the treatment
- Topical medications applied to the treatment area for 5 days following treatment
- Investigational Medications or treatments within 30 days of Baseline and during the course of the study.

The subject was scheduled for a follow-up office visit two weeks after the treatment. At the follow-up office visit, the subject was questioned as to their compliance with the instructions regarding which medications not to use between treatment and the two week follow-up office visit. If the subject was non-compliant, the subject was disqualified from the study. If the subject was compliant, the subject was re-assessed using the gravimetric sweat measurement procedure.

### Treatment Results and Conclusion

The study was conducted at multiple study sites and conducted in compliance with Good Clinical Practice standards. Ten subjects were treated with Propylparaben. Two weeks after the treatment, each subject was re-assessed by gravimetric sweat measurement.

On average, subjects in the Propylparaben group had a reduction in sweat production of 177 mg two weeks after treatment as measured by gravimetric sweat measurement. In contrast, subjects treated with the placebo had a 53 mg reduction in sweat production as measured by gravimetric sweat measurement. Therefore, subjects treated with Propylparaben had a 337% greater reduction in sweat production than the subjects in the control group.

It was also determined what percent of study subjects receiving either Propylparaben or placebo experienced at least a 30% reduction in sweat production when compared to levels measured at the Baseline visit. It was found that 70% of subjects treated with Propylparaben had at least a 30% reduction in sweat production when compared to levels at the Baseline visit. This contrasts with only 29% of subjects in the control group that had at least a 30% reduction in sweat production when compared to levels at the Baseline visit. Therefore, by this assessment subjects treated with Propylparaben had a 245% greater effectiveness in reducing sweat production than those subjects treated with placebo.

Given these data, it is concluded that Propylparaben is (i) biologically active in reducing sweat production, (ii) is an anti-perspirant substance, and (iii) may be used effectively in treating hyperhidrosis.

### Example 27: Anti-wrinkle effects of "Emulsion V" Nanoparticle Composition

### Study Design Summary

The purpose of the study was to determine if Emulsion V is biologically active in reducing Lateral Canthal Lines (Crow's Feet Wrinkles). Subjects were selected who demonstrated moderate to severe Lateral Canthal Lines on contraction (i.e., while smiling) as assessed by the investigator. All subjects received treatment with the potentially biologically active formulation.

After a single treatment at Baseline subjects were re-assessed by the investigator using an Investigator's Global Assessment ("IGA") score to determine the severity of the subject's Crows Feet at Week 1, 2, 4, 8, and Week 12, respectively. A comparison of posttreatment wrinkle severity to the Baseline score was made to determine the degree of wrinkle reduction by the potentially biologically active formulation.

### Study Subject Inclusion / Exclusion Criteria

The study enrolled adult male and female subjects diagnosed with moderate to severe Crow's Feet wrinkles on contraction based on the following criteria:

### Inclusion Criteria

- able to understand and give written informed consent
- 30 - 70 years of age
- mild to moderate Crow's Feet wrinkles (IGA 2-3) at rest
- moderate to severe Crow's Feet (IGA 3-4) on contraction
- willingness to refrain from the use of facial fillers, retinoids, injectible botulinum products, laser treatments, or any product affecting skin remodeling or that might cause an active dermal response during the course of the study
- subjects should be in good general health as determined by the investigator and free of any disease that may interfere with study evaluations or the Investigational Product

### Exclusion Criteria

- botulinum toxin treatment in the prior 6 months
- history of peri-ocular surgery, brow lift or related procedures
- soft tissue augmentation or any procedures affecting the lateral canthal region in the prior 12 months
- dermabrasion or laser treatment in the periocular region in the last 6 months
- topical prescription-strength retinoids in the prior 3 months
- application of any topical prescription medication to the treatment area within 14 days prior to treatment
- subjects on clinically significant, concomitant drug therapy
- present or history of neuromuscular disease, eyelid ptosis, muscle weakness or paralysis
- systemic aminoglycoside use in the week prior to treatment application
- participation in another investigational drug trial or receiving any investigational treatment(s) within 30 days of Baseline
- alcohol or drug abuse within the past 3 years
- female subjects who are pregnant or are nursing a child
- psychiatric disease interfering with the subject's ability to give informed consent
- refusal or inability to comply with the requirements of the protocol for any reason

### Treatment and Assessment Methods

### Clinical Visits

Prior to participating in any aspect of the study, each subject was fully informed, both verbally and in writing, of the conduct and consequences of the study. Each subject signed the written Informed Consent Form prior to the conduct of the screening evaluation to determine whether the subject was potentially eligible for the study. The investigator recorded the right and left Investigator's Global Assessment score of Crow's Feet, both, "at rest" and "on contraction". Prior to scheduling an initial visit to the investigator's study site, potential participants were queried with regards to their use of topical medications or prior cosmetic procedures to the treatment area. Subjects who met Exclusion Criteria were not enrolled.

### The Investigator's Global Assessment Score

The subject was asked to make an expressionless face for the "at rest" assessment.

The subject was asked to produce a maximally exaggerated smile for the "on contraction" assessment.

**Table 23: IGA Score standard**

| **Score** | **Grade** | **Description** |
|---|---|---|
| 0 | Absent | No visible wrinkles |
| 1 | Minimal | Very fine wrinkles (that are barely visible) |
| 2 | Mild | Fine wrinkles (that are shallow) |
| 3 | Moderate | Moderate wrinkles (that are moderately deep) |
| 4 | Severe | Severe wrinkles (that are severely deep) |

### Treatment application

If the subject was eligible for treatment on this basis, the subject was then treated. Emulsion V contained Emulsifying Wax, Gelatin Phosphate Buffer Solution, Isopropyl Myristate, Labrafac Lipophile, Methylparaben, Mineral Oil Heavy Viscosity Range, Polysorbate 80, Propylparaben, Purified Water, Sodium Chloride Injection, and White Petrolatum. All ingredients are either NF or USP grade. The average diameter (e.g., particle size) of nanoparticles contained in the Emusion V empty nanoparticle composition was approximately 77.1 nm.

For treatment, the subject was instructed to close his/her eyes which were then covered with an absorbent paper or cloth. The clinical investigator then applied the study medication using a latex-gloved finger to the skin of the periorbital region in the distribution of the muscles responsible for the Crow's Feet wrinkles. The preparation was administered in small increments to avoid run-off. The liquid was rubbed-in until vanished.

The subject was scheduled for follow-up office visits 1, 2, 4, 8, and 12 weeks after the treatment. At the follow-up office visits, the subject was questioned as to their compliance with the instructions regarding medications and procedures not to use after treatment that might interfere with the wrinkle assessment. If the subject was non-compliant, the subject was disqualified from the study. If the subject was compliant, the subject was re-assessed using the Investigators Global Assessment score.

### Treatment Results and Conclusion

The study was conducted at multiple study sites and conducted in compliance with Good Clinical Practice standards. 31 subjects were treated with Emulsion V. After treatment, each subject was re-assessed by the Investigators Global Assessment score at Week 1, 2, 4, 8, and Week 12.

On average, subjects treated with Emulsion V had a reduction in their wrinkle score as shown in the table below.

**Table 24: Percentage Wrinkle Reduction**

| | Wk 01 | Wk 02 | Wk 04 | Wk 08 | Wk 12 |
|---|---|---|---|---|---|
| At Rest | -10% | -15% | -10% | -14% | -15% |
| On Contraction | -12% | -19% | -18% | -25% | -24% |

As can be seen, patients treated with Emulsion V experienced an improvement of up to 15% when assessed "at rest". The improvement was evident as early as Week 2. In addition, participants showed an even greater improvement of up to 25% in their wrinkle assessment "on contraction".

Given these data, it is concluded that Emulsion V is (i) biologically active in reducing Lateral Canthal Lines, (ii) is an anti-wrinkle formulation, and (iii) may be used effectively in treating Crow's Feet.

### Equivalents

Those skilled in the art will recognize, or be able to ascertain using no more than routine experimentation, many equivalents to the specific embodiments of the invention described herein. The scope of the present invention is not intended to be limited to the above Description, but rather is as set forth in the following claims:

Aspects and features of the present disclosure are set out in the following numbered clauses which contain the subject matter of the claims of the parent application as filed:
1. A nanoparticle composition comprising a population of particles, wherein the majority of particles have diameters between approximately 10 and approximately 300 nanometers,
   wherein the nanoparticle composition comprises at least one aqueous dispersion medium, at least one oil, and at least one surfactant,
   wherein the oil is a medium chain triglyceride,
   wherein the surfactant is a polysorbate, and
   wherein the ratio of oil to surfactant is between about 0.5:1 to about 1:1.
2. The nanoparticle composition of clause 1, wherein the medium chain triglyceride is an acid containing 6-12 carbons atoms.
3. The nanoparticle composition of clause 1, wherein the medium chain triglyceride is 1349 oil.
4. The nanoparticle composition of clause 1, wherein the polysorbate is polysorbate 80.
5. The nanoparticle composition of clause 1, wherein the surfactant is super-refined polysorbate 80.
6. The nanoparticle composition of clause 1, further comprising a known therapeutic agent or independently active biologically active agent.
7. The nanoparticle composition of clause 6, wherein the known therapeutic agent or independently active biologically active agent is a protein.
8. The nanoparticle composition of clause 6, wherein the known therapeutic agent or independently active biologically active agent is an antibody.
9. The nanoparticle composition of clause 6, wherein the known therapeutic agent or independently active biologically active agent is a protein complex.
10. The nanoparticle composition of clause 6, wherein the known therapeutic agent or independently active biologically active agent is botulinum toxin.
11. The nanoparticle composition of clause 10, wherein the botulinum toxin is encapsulated within the particles.
12. The nanoparticle composition of clause 10, wherein the botulinum toxin is adsorbed on the surface of the particles.
13. The nanoparticle composition of clause 10, wherein the botulinum toxin is associated with the particle interface.
14. The nanoparticle composition of clause 10, wherein the botulinum toxin is selected from the group comprising type A, type Ab, type Af, type B, type Bf, type C1, type C2, type D, type E, type F, and type G.
15. The nanoparticle composition of clause 10, wherein the botulinum toxin is type A botulinum toxin.
16. The nanoparticle composition of clause 1, wherein the particles can penetrate skin without altering or changing the skin.
17. The nanoparticle composition of clause 1, wherein the particles can penetrate skin without the use of skin permeation enhancers or abrasives.
18. The nanoparticle composition of clause 1, wherein the particles can penetrate the top layer of skin without the use of skin permeation enhancers or abrasives.
19. The nanoparticle composition of clause 18, wherein the top layer of the skin is the surface of the stratum corneum.
20. The nanoparticle composition of clause 18, wherein the top layer of the skin includes dermal pores.
21. The nanoparticle composition of clause 18, wherein the top layer of the skin includes dermal glands.
22. The nanoparticle composition of any one of clauses 1-21, wherein the nanoparticle composition does not contain parabens.
23. A lotion comprising methylparaben, mineral oil, isopropyl myristate, white petrolatum, emulsifying wax, and propylparaben.
24. The lotion of clause 23, further comprising Polysorbate 80, 1349 oil, or combination thereof.
25. The lotion of clause 23, wherein the lotion comprises two phases admixed with one another, wherein the first phase comprises methylparaben.
26. The lotion of clause 23, wherein the lotion comprises two phases admixed with one another, wherein the second phase comprises mineral oil, isopropyl myristate, white petrolatum, emulsifying wax, and propylparaben.
27. The lotion of clause 23, wherein the lotion comprises two phases admixed with one another, wherein the first phase comprises methylparaben and the second phase comprises mineral oil, isopropyl myristate, white petrolatum, emulsifying wax, and propylparaben.
28. A lotion comprising mineral oil, isopropyl myristate, white petrolatum, and emulsifying wax.
29. The lotion of clause 28, further comprising Polysorbate 80, 1349 oil, or combination thereof.
30. The lotion of clause 28, wherein the lotion comprises two phases admixed with one another, wherein the second phase comprises mineral oil, isopropyl myristate, white petrolatum, and emulsifying wax.
31. A pharmaceutical composition comprising the lotion of any one of clauses 23-30 and a known therapeutic agent or independently active biologically active agent.
32. The pharmaceutical composition of clause 31, wherein the known therapeutic agent or independently active biologically active agent is a botulinum toxin, an antibody, or combination thereof.
33. A pharmaceutical composition comprising the nanoparticle composition of any one of clauses 1-21 and a lotion.
34. A pharmaceutical composition comprising the nanoparticle composition of any one of clauses 1-21 and the lotion of any one of clauses 23-30.
35. The pharmaceutical composition of either of clauses 31-34, wherein the pharmaceutical composition is formulated as a deodorant.
36. The pharmaceutical composition of either of clauses 31-34, wherein the pharmaceutical composition is formulated as an antiperspirant.
37. A method comprising steps of:
   providing a subject, and
   administering the nanoparticle composition of any one of clauses 1-21, the
      lotion of any one of clauses 23-30, or the pharmaceutical composition of any one of clauses 31-36 to the skin of the subject.
38. The method of clause 37, wherein the nanoparticle composition, lotion, or pharmaceutical composition is administered for treatment of a condition or disorder associated with sebaceous glands.
39. The method of clause 37, wherein the nanoparticle composition, lotion, or pharmaceutical composition is administered for treatment of a condition or disorder associated with sweat glands.
40. The method of clause 33, wherein the nanoparticle composition, lotion, or pharmaceutical composition is administered for treatment of a condition or disorder associated with hair follicles.
41. The method of clause 37, wherein the nanoparticle composition, lotion, or pharmaceutical composition is administered for treatment of the outer layer of the skin.
42. The method of clause 37, wherein the nanoparticle composition, lotion, or pharmaceutical composition is administered for treatment of a condition or disorder selected from the group consisting of acne, hyperhidrosis, unwanted sweating, bromhidrosis, body odor, chromhidrosis, excess sebum-producing disorders, seborrhea, seborrheic dermatitis, rosacea, hair loss, psoriasis, dermal infections, viral infection, bacterial infection, fungal infection, actinic keratosis, eczematous dermatitis, atopic dermatitis, burns, Raynaud's phenomenon, lupus erthythematosus, hyperpigmentation disorders, melasma, hypopigmentation disorders, vitiligo, skin cancer, squamous cell skin carcinoma, basal cell skin carcinoma, arthritis, osteoarthritis, bruxism, cervical neck pain, dry eyes, gastrointestinal disorders, achalasia, esophageal spasm, gastroparesis, spasm of the sphincter of oddi, anal fissure, anismus, lateral epicondylitis, back pain, lower back pain, upper back pain, masseter muscle hypertrophy, facial nerve disorders, facial wrinkles, wrinkles involving the forehead, glabellar, rhytids and/or periorbital regions, unsightly facial expressions, neck lines, hyperfunctional facial lines, hyperkinetic facial lines, platysma bands, neuromuscular disorders and conditions involving muscular spasm or contracture, facial palsy such as hemi facial spasm, cerebral palsy, spasticisty due to stroke, blepharospasm, facial contracture, dystonia, cervical dystonia, laryngeal dystonia, oromandibular dystonia, writer's cramp, neuralgias, trigeminal neuralgia, neuropathic pain, Parkinson's disease, plantar fasciitis pain, prostate hyperplasia, headache, migraine, essential headache, cervicogenic headache, tension headache, prostatic disorders, prostatic pain, prostatic hypertrophy, restless leg syndrome, rhinitis, allergic rhinitis, sialorrhea, skin pruritis, strabismus, temporomandibular joint ("TMJ") syndrome, tics, Tourette's syndrome, hemifacial spasm, tremor, essential tremor, urinary bladder dysfunction, detrusor sphincter dysnergia, painful bladder, bladder spasticity, overactive bladder, vaginismus, spasticity such as that resulting from multiple sclerosis, retroorbital muscle, various ophthalmologic conditions, and/or combinations thereof.
43. The method of clause 37, wherein the nanoparticle composition, lotion, or pharmaceutical composition is administered for treatment of hyperhidrosis.
44. The method of clause 37, wherein the nanoparticle composition, lotion, or pharmaceutical composition is administered for treatment of unwanted sweating.
45. The method of clause 37, wherein the nanoparticle composition, lotion, or pharmaceutical composition is administered for treatment of bromhidrosis.
46. The method of clause 37, wherein the nanoparticle composition, lotion, or pharmaceutical composition is administered for treatment of body odor.
47. The method of clause 37, wherein the nanoparticle composition, lotion, or pharmaceutical composition is administered for treatment of chromhidrosis.
48. The method of clause 37, wherein the nanoparticle composition, lotion, or pharmaceutical composition is administered for treatment of acne.
49. The method of clause 37, wherein the nanoparticle composition, lotion, or pharmaceutical composition is administered for treatment of wrinkles.
50. The method of clause 37, wherein the nanoparticle composition, lotion, or pharmaceutical composition is administered for treatment of headache.

## Claims

1. A topical formulation comprising a nanoparticle composition comprising a population of particles, wherein the majority of particles have diameters between approximately 10 and approximately 300 nanometers,
wherein the nanoparticle composition comprises at least one aqueous dispersion medium, at least one oil, at least one surfactant, and a botulinum toxin;
wherein the oil is a medium chain triglyceride,
wherein the surfactant is a polysorbate,
wherein the ratio of oil to surfactant is between about 0.5:1 to about 1:1; and
wherein the composition further comprises at least one excipient;
for use in preventing or treating a condition or disorder associated with sweat glands and/or sebaceous glands.

2. The topical formulation for use as claimed in claim 1,
wherein the oil is Caprylic/Capric triglyceride oil;
wherein the surfactant is Super Refined Polysorbate 80;
wherein the least one excipient is selected from the group consisting of ethylenediaminetetraacetic acid (EDTA), methylparaben, and propylparaben, or combinations thereof; and
wherein the condition or disorder associated with sweat glands and/or sebaceous glands is bromhidrosis, body odor, chromhidrosis, acne, excess sebum-producing disorders, seborrhea, and seborrheic dermatitis.

3. The topical formulation for use as claimed in claim 1 or claim 2, wherein the composition further comprises cetostearyl alcohol.

4. The topical formulation for use as claimed in any one of claims 1-3, wherein the composition further comprises 0.026% gelatin.

5. The topical formulation for use as claimed in any one of claims 1-4, wherein the botulinum toxin is encapsulated within the particles.

6. The topical formulation for use as claimed in any one of claims 1-4, wherein the botulinum toxin is adsorbed on the surface of the particles.

7. The topical formulation for use as claimed in any one of claims 1-4, wherein the botulinum toxin is associated with the particle interface.

8. The topical formulation for use as claimed in any one of claims 1-7, wherein the botulinum toxin is selected from the group comprising type A, type Ab, type Af, type B, type Bf, type C1, type C2, type D, type E, type F, and type G.

9. The topical formulation for use as claimed in claim 8, optionally wherein the botulinum toxin is type A botulinum toxin.

10. The topical formulation for use as claimed in any one of claims 1-9, wherein the particles can penetrate the top layer of skin without altering or changing the skin, and without the use of skin permeation enhancers or abrasives.

11. The topical formulation for use as claimed in claim 10, wherein the top layer of the skin is the surface of the stratum corneum; and wherein the top layer of the skin includes dermal pores or dermal glands.

12. The topical formulation for use as claimed in any one of claims 1-11, wherein the composition is formulated as a deodorant, a sunscreen, a shampoo, a conditioner, an antiperspirant, or any combination thereof.

13. The topical formulation for use as claimed in any one of claims 1-12, wherein the condition or disorder associated with sweat glands and/or sebaceous glands is bromhidrosis.

14. The topical formulation for use as claimed in any one of claims 1-12, wherein the condition or disorder associated with sweat glands and/or sebaceous glands is body odor.

15. The topical formulation for use as claimed in any one of claims 1-14 wherein the botulinum toxin remains stable for an extended period of time when stored at 2°C to 8°C.

16. The topical formulation for use as claimed in any one of claims 1-15, wherein the nanoparticles have a zeta potential ranging between -25 mV and +25 mV.

17. The topical formulation for use as claimed in any one of claims 1-16 wherein the composition is formulated into a cream, a gel, a liniment, a lotion, an ointment, drops, an oil, a foam, a spray, a patch, a liquid, or thickening lotion.

18. Use of a nanoparticle composition comprising a population of particles, wherein the majority of particles have diameters between approximately 10 and approximately 300 nanometers,
wherein the nanoparticle composition comprises at least one aqueous dispersion medium, at least one oil, at least one surfactant, and a botulinum toxin;
wherein the oil is a medium chain triglyceride,
wherein the surfactant is a polysorbate,
wherein the ratio of oil to surfactant is between about 0.5:1 to about 1:1; and
wherein the composition further comprises at least one excipient;
in the manufacture of a medicament for use in preventing or treating a condition or disorder associated with sweat glands and/or sebaceous glands.


SEQUENCE LISTING

<110>  Anterios, Inc.
       Edelson, Jonathan
       Kotyla, Timothy
       Theobald, Klaus

<120>  NANOPARTICLE COMPOSITIONS

<130>  2007339-0131

<150>  US 61/435,780
<151>  2011-01-24

<160>  62

<170>  PatentIn version 3.5

<210>  1
<211>  20
<212>  RNA
<213>  Artificial Sequence

<220>
<223>  synthetic siRNA oligonucleotide

<400>  1
aaacuuguuu uugagggucu                                                  20


<210>  2
<211>  19
<212>  RNA
<213>  Artificial Sequence

<220>
<223>  synthetic siRNA oligonucleotide

<400>  2
uuuuugaggg ucuugaucu                                                   19


<210>  3
<211>  20
<212>  RNA
<213>  Artificial Sequence

<220>
<223>  synthetic siRNA oligonucleotide

<400>  3
uuuugagggu cuugaucugu                                                  20


<210>  4
<211>  20
<212>  RNA
<213>  Artificial Sequence

<220>
<223>  synthetic siRNA oligonucleotide

<400>  4

uuugaggguc uugaucuguu                                                    20

<210>  5
<211>  20
<212>  RNA
<213>  Artificial Sequence

<220>
<223>  synthetic siRNA oligonucleotide

<400>  5
aaggaggcaa acuuguuuuu                                                    20

<210>  6
<211>  20
<212>  RNA
<213>  Artificial Sequence

<220>
<223>  synthetic siRNA oligonucleotide

<400>  6
aacuuguuga gggucuugau                                                    20

<210>  7
<211>  20
<212>  RNA
<213>  Artificial Sequence

<220>
<223>  synthetic siRNA oligonucleotide

<400>  7
aaacuuguug agggucuugu                                                    20

<210>  8
<211>  20
<212>  RNA
<213>  Artificial Sequence

<220>
<223>  synthetic siRNA oligonucleotide

<400>  8
caaacuuguu gagggucuuu                                                    20

<210>  9
<211>  20
<212>  DNA
<213>  Artificial Sequence

<220>
<223>  synthetic oligonucleotide

<400>  9
tccatgacgt tcctgacgtt                                                    20

<210>  10
<211>  20
<212>  DNA
<213>  Artificial Sequence

<220>
<223>  synthetic oligonucleotide

<400>  10
tccatgacgt tcctgacgtt                                                    20


<210>  11
<211>  19
<212>  DNA
<213>  Artificial Sequence

<220>
<223>  synthetic oligonucleotide

<400>  11
tccatgacgt tcctgacgt                                                     19


<210>  12
<211>  18
<212>  DNA
<213>  Artificial Sequence

<220>
<223>  synthetic oligonucleotide

<400>  12
tccatgacgt tcctgacg                                                      18


<210>  13
<211>  16
<212>  DNA
<213>  Artificial Sequence

<220>
<223>  synthetic oligonucleotide

<400>  13
tcctcgacgt ccctga                                                        16


<210>  14
<211>  12
<212>  DNA
<213>  Artificial Sequence

<220>
<223>  synthetic oligonucleotide

<400>  14
catgacgttc ct                                                            12


<210>  15
<211>  6
<212>  DNA

<213>  Artificial Sequence

<220>
<223>  synthetic oligonucleotide

<400>  15
gacgtt                                                                  6


<210>  16
<211>  20
<212>  DNA
<213>  Artificial Sequence

<220>
<223>  synthetic oligonucleotide

<400>  16
aacgtcagga acgtcatgga                                                  20


<210>  17
<211>  19
<212>  DNA
<213>  Artificial Sequence

<220>
<223>  synthetic oligonucleotide

<400>  17
cacccaagac agcagaaag                                                   19


<210>  18
<211>  19
<212>  DNA
<213>  Artificial Sequence

<220>
<223>  synthetic oligonucleotide

<400>  18
ctcccaagac agcagaaag                                                   19


<210>  19
<211>  19
<212>  DNA
<213>  Artificial Sequence

<220>
<223>  synthetic oligonucleotide

<400>  19
ctgccaagac agcagaaag                                                   19


<210>  20
<211>  19
<212>  DNA
<213>  Artificial Sequence

<220>

<223>  synthetic oligonucleotide

<400>  20
cacccaactc tccagaaag                                                    19


<210>  21
<211>  19
<212>  DNA
<213>  Artificial Sequence

<220>
<223>  synthetic oligonucleotide

<400>  21
cacccaagac agcagaatg                                                    19


<210>  22
<211>  19
<212>  DNA
<213>  Artificial Sequence

<220>
<223>  synthetic oligonucleotide

<400>  22
cacccaagac agcagattg                                                    19


<210>  23
<211>  6
<212>  DNA
<213>  Artificial Sequence

<220>
<223>  synthetic oligonucleotide

<400>  23
ttaggg                                                                   6


<210>  24
<211>  11
<212>  DNA
<213>  Artificial Sequence

<220>
<223>  synthetic oligonucleotide

<400>  24
gttagggtta g                                                            11


<210>  25
<211>  16
<212>  DNA
<213>  Artificial Sequence

<220>
<223>  synthetic oligonucleotide

<400>  25

ggttaggtgt aggttt                                                    16


<210>  26
<211>  9
<212>  DNA
<213>  Artificial Sequence

<220>
<223>  synthetic oligonucleotide

<400>  26
gttagggtt                                                             9


<210>  27
<211>  9
<212>  DNA
<213>  Artificial Sequence

<220>
<223>  synthetic oligonucleotide

<400>  27
ttagggtta                                                             9


<210>  28
<211>  15
<212>  DNA
<213>  Artificial Sequence

<220>
<223>  synthetic oligonucleotide

<400>  28
gttaggttta aggtt                                                     15


<210>  29
<211>  15
<212>  DNA
<213>  Artificial Sequence

<220>
<223>  synthetic oligonucleotide

<400>  29
ggtaggtgta gggtg                                                     15


<210>  30
<211>  15
<212>  DNA
<213>  Artificial Sequence

<220>
<223>  synthetic oligonucleotide

<400>  30
ggtcggtgtc gggtg                                                     15

<210>  31
<211>  15
<212>  DNA
<213>  Artificial Sequence

<220>
<223>  synthetic oligonucleotide

<400>  31
ggcaggcgca gggcg                                                        15


<210>  32
<211>  15
<212>  DNA
<213>  Artificial Sequence

<220>
<223>  synthetic oligonucleotide

<400>  32
gttagggtta gggtt                                                        15


<210>  33
<211>  16
<212>  DNA
<213>  Artificial Sequence

<220>
<223>  synthetic oligonucleotide

<400>  33
gataagggat tgggat                                                       16


<210>  34
<211>  9
<212>  DNA
<213>  Artificial Sequence

<220>
<223>  synthetic oligonucleotide

<400>  34
gagtatgag                                                                9


<210>  35
<211>  9
<212>  DNA
<213>  Artificial Sequence

<220>
<223>  synthetic oligonucleotide

<400>  35
gggttaggg                                                                9


<210>  36
<211>  11
<212>  DNA

<213>  Artificial Sequence

<220>
<223>  synthetic oligonucleotide

<400>  36
gttagggtta g                                                            11


<210>  37
<211>  15
<212>  DNA
<213>  Artificial Sequence

<220>
<223>  synthetic oligonucleotide

<400>  37
ggtaggtgta ggatt                                                        15


<210>  38
<211>  16
<212>  DNA
<213>  Artificial Sequence

<220>
<223>  synthetic oligonucleotide

<400>  38
ggtaggtgta ggattt                                                       16


<210>  39
<211>  16
<212>  DNA
<213>  Artificial Sequence

<220>
<223>  synthetic oligonucleotide

<400>  39
ggttaggtgt aggttt                                                       16


<210>  40
<211>  16
<212>  DNA
<213>  Artificial Sequence

<220>
<223>  synthetic oligonucleotide

<400>  40
ggttaggtgg aggttt                                                       16


<210>  41
<211>  16
<212>  DNA
<213>  Artificial Sequence

<220>

<223>  synthetic oligonucleotide

<400>  41
ggttaggttt aggttt                                                       16


<210>  42
<211>  16
<212>  DNA
<213>  Artificial Sequence

<220>
<223>  synthetic oligonucleotide

<400>  42
ggttaggtta aggtta                                                       16


<210>  43
<211>  15
<212>  DNA
<213>  Artificial Sequence

<220>
<223>  synthetic oligonucleotide

<400>  43
ggtaggtgta gggtg                                                        15


<210>  44
<211>  16
<212>  DNA
<213>  Artificial Sequence

<220>
<223>  synthetic oligonucleotide

<400>  44
gttagggtta gggtta                                                       16


<210>  45
<211>  16
<212>  DNA
<213>  Artificial Sequence

<220>
<223>  synthetic oligonucleotide

<400>  45
ggttggttgg ttggtt                                                       16


<210>  46
<211>  20
<212>  DNA
<213>  Artificial Sequence

<220>
<223>  synthetic oligonucleotide

<400>  46

ccttggttgg ttggttggtt                                                    20


<210>  47
<211>  20
<212>  DNA
<213>  Artificial Sequence

<220>
<223>  synthetic oligonucleotide

<400>  47
ggttggttgg ttggttggtt                                                    20


<210>  48
<211>  24
<212>  DNA
<213>  Artificial Sequence

<220>
<223>  synthetic oligonucleotide

<400>  48
tcgtcgtttt gtcgttttgt cgtt                                               24


<210>  49
<211>  5
<212>  PRT
<213>  Artificial Sequence

<220>
<223>  synthetic peptide

<400>  49

Lys Thr Thr Lys Ser
1               5


<210>  50
<211>  10
<212>  PRT
<213>  Artificial Sequence

<220>
<223>  synthetic peptide

<400>  50

Glu Tyr Lys Thr Thr Lys Ser Ser Arg Leu
1               5                   10


<210>  51
<211>  8
<212>  PRT
<213>  Artificial Sequence

<220>
<223>  synthetic peptide

<400>  51

Val Ile Glu Tyr Lys Thr Thr Lys
1               5


<210>  52
<211>  4
<212>  PRT
<213>  Artificial Sequence

<220>
<223>  synthetic peptide

<400>  52

Lys Thr Thr Lys
1


<210>  53
<211>  12
<212>  PRT
<213>  Artificial Sequence

<220>
<223>  synthetic peptide

<400>  53

Gly Lys Thr Val Ile Glu Tyr Lys Thr Thr Lys Ser
1               5                   10


<210>  54
<211>  15
<212>  PRT
<213>  Artificial Sequence

<220>
<223>  synthetic peptide

<400>  54

Gly Lys Thr Val Ile Glu Tyr Lys Thr Thr Lys Ser Ser Arg Leu
1               5                   10                  15


<210>  55
<211>  20
<212>  PRT
<213>  Artificial Sequence

<220>
<223>  synthetic peptide

<400>  55

Trp Gly Lys Thr Val Ile Glu Tyr Lys Thr Thr Lys Ser Ser Arg Leu
1               5                   10                  15


Pro Ile Ile Asp

            20

<210>  56
<211>  20
<212>  PRT
<213>  Artificial Sequence

<220>
<223>  synthetic peptide

<400>  56

Cys Thr Ser His Thr Gly Ala Trp Gly Lys Thr Val Ile Glu Tyr Lys
1               5                   10                  15

Thr Thr Lys Ser
            20

<210>  57
<211>  4
<212>  PRT
<213>  Artificial Sequence

<220>
<223>  synthetic peptide

<400>  57

Thr Thr Lys Ser
1

<210>  58
<211>  6
<212>  PRT
<213>  Artificial Sequence

<220>
<223>  synthetic peptide

<400>  58

Glu Glu Met Gln Arg Arg
1               5

<210>  59
<211>  6
<212>  PRT
<213>  Artificial Sequence

<220>
<223>  synthetic peptide

<400>  59

Val Gly Val Ala Pro Gly
1               5

<210>  60
<211>  6
<212>  PRT
<213>  Artificial Sequence

<220>
<223>  synthetic peptide

<400>  60

Tyr Tyr Arg Ala Asp Ala
1               5


<210>  61
<211>  3
<212>  PRT
<213>  Artificial Sequence

<220>
<223>  synthetic peptide

<400>  61

Gly His Lys
1


<210>  62
<211>  14
<212>  PRT
<213>  Artificial Sequence

<220>
<223>  synthetic peptide

<400>  62

Thr Ser Leu Asp Ala Ser Ile Ile Trp Ala Met Met Gln Asn
1               5                   10